# EUROPEAN PATENT APPLICATION

(11) **EP 4 601 446 A1**
(43) Date of publication of application: **13.08.2025**
(21) Application number: 24156436.8
(22) Date of filing: 07.02.2024
(51) Int. Cl.: H10K 85/30, C07C 49/92, C07D 213/50, C07D 221/04, H10K 30/86

(54) **ORGANIC PHOTODETECTOR COMPRISING A METAL COMPLEX OF FORMULA (I), AN ORGANIC ELECTRONIC DEVICE COMPRISING THE ORGANIC PHOTOTECTOR AS WELL AS A DISPLAY DEVICE COMPRISING THE ORGANIC ELECTRONIC DEVICE**

(71) Applicant: Novaled GmbH, 01099 Dresden (DE)
(72) Inventor: RUNGE, Steffen, 01099 Dresden (DE); UVAROV, Vladimir, 01099 Dresden (DE); PINTER, Piermaria, 01099 Dresden (DE); CHENG, Huan, 01099 Dresden (DE); WERNER, Ansgar, 01099 Dresden (DE)
(74) Representative: Michalski Hüttermann & Partner Patentanwälte mbB

(57) **Abstract**

The present invention relates to an organic photodetector comprising a metal complex of formula (I), an organic electronic device comprising the organic phototector as well as an display device comprising the organic electronic device.

## Description

### Technical Field

The present invention relates to an organic photodetector comprising a metal complex of formula (I), an organic electronic device comprising the organic phototector as well as a display device comprising the organic electronic device

### Background Art

Organic photodetectors have been developed inter alia for use in organic electronic devices such as displays. However, there is a constant need for improved photodetectors and materials for such photodetectors, especially in view of improving/lowering the dark current and improving the signal-to-noise ratio.

### DISCLOSURE

An aspect of the present invention provides an organic photodetector comprising an anode layer, a cathode layer, a hole transport region, a photoconversion unit, a semiconductor layer,
wherein the hole transport region, the photoconversion unit, and the semiconductor layer are arranged between the anode layer and the cathode layer;
wherein the hole transport region comprises one or more layer;
wherein the hole transport region is arranged between the anode layer and the photoconversion unit;
wherein the semiconductor layer is arranged between the hole transport region and photoconversion unit;
wherein the semiconductor layer is arranged adjacent or in direct contact to the photoconversion unit;
wherein the photoconversion unit comprises one or more layers;
wherein the photoconversion unit comprises an electron donor compound and electron acceptor compound;
wherein the semiconductor layer or the photoconversion unit comprises a metal complex; metal complex of formula (I):

   M^{n⊕}(L^{⊝})ₙ(AL)ₘ (I),

   wherein:
   - M: is a metal ion,
   - n: is the valency of M and selected from 1 to 4;
   - L: is a ligand independently selected from formula (II) 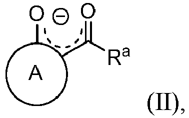
A is selected from a substituted or unsubstituted C₃ to C₄₀ carbocyclic ring or ring system, substituted or unsubstituted C₂ to C₄₀ heterocyclic ring or ring system, substituted or unsubstituted C₆ to C₄₀ aryl ring or ring system, or substituted or unsubstituted C₂ to C₄₀ heteroaryl ring or ring system, wherein the carbocyclic ring or the heterocyclic ring may contain one or more double bonds, and wherein the ring system may comprise two or three rings, preferably two rings;
R^{a} is selected from CN, substituted or unsubstituted C₁ to C₂₀ alkyl, CH₃, CH₂D, CHD₂, CD₃, a partially fluorinated or perfluorinated C₁ to C₂₀ alkyl, CFH₂, CFDH, CFD₂, CF₂H, CF₂D, CF₃, substituted or unsubstituted C₁ to C₂₀ alkenyl, substituted or unsubstituted C₁ to C₂₀ alkinyl, substituted or unsubstituted C₃ to C₄₀ cycloalkyl, substituted or unsubstituted C₂ to C₄₀ heterocycloalkyl, substituted or unsubstituted C₃ to C₄₀ carbocyclyl ring, substituted or unsubstituted C₂ to C₄₀ heterocyclyl, substituted or unsubstituted C₂ to C₄₀ heteroaryl, or substituted or unsubstituted C₆ to C₄₀ aryl;
   - AL: is an ancillary ligand which coordinates to the metal ion M;
   - m: is an integer selected from 0 to 2.

The negative charge in the compounds of Formula (I) may be delocalised partially or fully over the β-dicarbonyl group and optionally also over the aryl group(s).

It should be noted that throughout the application and the claims any R^{k} etc. always refer to the same moieties, unless otherwise noted.

It should be noted that throughout the application and the claims the term "substituents", when referring to Formula (I) and/or ligand L always are meant to be selected from H, D, halogen, Cl, F, CN, NO₂, C₁ to C₁₂ alkyl, C₁ to C₁₂ alkoxy, partially or perfluorinated C₁ to C₁₂ alkyl, CF₃, CF₂H, partially or perfluorinated C₁ to C₁₂ alkoxy, or a combination thereof.

Throughout the applications and the claims the term "ring system" shall especially mean that adjacent rings share two common atoms.

In the present specification, when a definition is not otherwise provided, "partially fluorinated" refers to an alkyl group or an alkoxy group in which only part of the hydrogen atoms are replaced by fluorine atoms.

In the present specification, when a definition is not otherwise provided, "perfluorinated" refers to an alkyl group or an alkoxy group in which all hydrogen atoms are replaced by fluorine atoms.

In the present specification, when a definition is not otherwise provided, "substituted" refers to one substituted with a deuterium, C₁ to C₁₂ alkyl and C₁ to C₁₂ alkoxy.

However, in the present specification "aryl substituted" refers to a substitution with one or more aryl groups, which themselves may be substituted with one or more aryl and/or heteroaryl groups.

Correspondingly, in the present specification "heteroaryl substituted" refers to a substitution with one or more heteroaryl groups, which themselves may be substituted with one or more aryl and/or heteroaryl groups.

In the present specification, when a definition is not otherwise provided, an "alkyl group" refers to a saturated aliphatic hydrocarbyl group. The alkyl group may be a C₁ to C₁₂ alkyl group. More specifically, the alkyl group may be a C₁ to C₁₀ alkyl group or a C₁ to C₆ alkyl group. For example, a C₁ to C₄ alkyl group includes 1 to 4 carbons in alkyl chain, and may be selected from methyl, ethyl, propyl, iso-propyl, n-butyl, iso-butyl, sec-butyl, and tert-butyl.

Specific examples of the alkyl group may be a methyl group, an ethyl group, a propyl group, an iso-propyl group, a butyl group, an iso-butyl group, a tert-butyl group, a pentyl group, a hexyl group.

In the context of the present invention, "ⁱCₙH₍₂ₙ₊₁₎" denotes an iso-alkyl group and "ⁱCₙF₍₂ₙ₊₁₎" denotes a perfluorinated iso-alkyl group.

The term "cycloalkyl" refers to saturated hydrocarbyl groups derived from a cycloalkane by formal abstraction of one hydrogen atom from a Atom comprised in the corresponding cycloalkane. Examples of the cycloalkyl group may be a cyclopropyl group, a cyclobutyl group, a cyclopentyl group, a cyclohexyl group, a methylcyclohexyl group, an adamantly group and the like.

The term "hetero" is understood the way that at least one carbon atom, in a structure which may be formed by covalently bound carbon atoms, is replaced by another polyvalent atom. Preferably, the heteroatoms are selected from B, Si, N, P, O, S; more preferably from N, P, O, S.

In the present specification, "aryl group" refers to a hydrocarbyl group which can be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenyl, and polycyclic groups comprising fused rings, like naphthyl or fluorenyl.

Analogously, under heteroaryl, it is especially where suitable understood a group derived by formal abstraction of one ring hydrogen from a heterocyclic aromatic ring in a compound comprising at least one such ring.

Under heterocycloalkyl, it is especially where suitable understood a group derived by formal abstraction of one ring hydrogen from a saturated cycloalkyl ring in a compound comprising at least one such ring.

The term "fused aryl rings" or "condensed aryl rings" is understood the way that two aryl rings are considered fused or condensed when they share at least two common sp²-hybridized carbon atoms

In the present specification, the single bond refers to a direct bond.

In the context of the present invention, "different" means that the compounds do not have an identical chemical structure.

The term "free of", "does not contain", "does not comprise" does not exclude impurities which may be present in the compounds prior to deposition. Impurities have no technical effect with respect to the object achieved by the present invention.

The term "contacting sandwiched" refers to an arrangement of three layers whereby the layer in the middle is in direct contact with the two adjacent layers.

The term "adjacent" in the context of layers especially means that one or two layers may be present between the adjacent layers.

The term "shared" and/or "common" in the context of layers especially means that the layer that is "shared" or "common" forms part of the device(s) that share the layer.

The term "contacting sandwiched" refers to an arrangement of three layers whereby the layer in the middle is in direct contact with the two adjacent layers.

The terms "light-absorbing layer" and "light absorption layer" are used synonymously.

The terms "light-emitting layer", "light emission layer" and "emission layer" are used synonymously.

The terms "OLED", "organic light-emitting diode" and "organic light-emitting device" are used synonymously.

The terms anode, anode layer and anode electrode are used synonymously.

The terms cathode, cathode layer and cathode electrode are used synonymously.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electrons formed in the cathode may be easily injected into the emission layer and transported in the emission layer due to conductive characteristics according to a lowest unoccupied molecular orbital (LUMO) level.

The term "LUMO level" is understood to mean the lowest unoccupied molecular orbital and is determined in eV (electron volt). The LUMO may also be named "LUMO" or "LUMO energy level".

The term "LUMO level further away from vacuum level" is understood to mean that the absolute value of the LUMO level is higher than the absolute value of the LUMO level of the reference compound.

The term "HOMO level" is understood to mean the highest occupied molecular orbital and is determined in eV (electron volt).

The term "HOMO level further away from vacuum level" is understood to mean that the absolute value of the HOMO level is higher than the absolute value of the HOMO level of the reference compound. For example, the term "further away from vacuum level than the HOMO level of N2,N2,N2',N2',N7,N7,N7',N7'-octakis(4-methoxyphenyl)-9,9'-spirobi[fluorene] - 2,2',7,7'-tetraamine is understood to mean that the absolute value of the HOMO level of the matrix compound of the hole injection layer is higher than the HOMO level of N2,N2,N2',N2', N7,N7, N7',N7'-octakis(4-methoxyphenyl)-9,9'-spirobi[fluorene]-2,2',7,7'-tetraamine.

The term "absolute value" is understood to mean the value without the "- "symbol. According to one embodiment of the present invention, the HOMO level of the matrix compound of the hole injection layer may be calculated by quantum mechanical methods.

### Advantageous Effects

Surprisingly, it was found that many organic photodetectors according to the present invention have a lower dark current and/or an improved signal-to-noise ratio.

According to one embodiment, the molecular mass of L is selected in the range of ≤ 600 Da and ≥ 176 , preferably ≤ 550 Da and ≥ 280 Da and most preferred ≤ 550 Da and ≥ 240 Da.

According to one embodiment of the invention, the metal complex of formula (I) comprises at least two fluorine atoms and 10 fluorine atoms or less, preferably 8 fluorine atoms or less.

According to an embodiment, wherein the ligand L of formula (II) comprises at least one fluorine atom.

According to an embodiment, wherein the ligand L of formula (II) comprises at least two fluorine atoms.

According to an embodiment, wherein the ligand L of formula (II) comprises one to ten fluorine atoms; preferably two to ten fluorine atoms; more preferably two to eight fluorine atoms.

According to one embodiment of the present invention, the ligand of formula (II) is free of alkoxy groups.

According to an embodiment, if n>1 then each L is selected the same.

According to an embodiment, Ligand L is selected from formula (IIa): 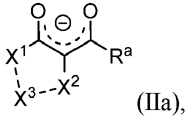 wherein the dashed lines in formula (IIa) denote a single or double bond;
X¹ is selected from a direct bond, N, O, NR¹, CR¹;
X² is selected from N, O, CR², CO, SO₂;
X³ is selected from N, O, CR³, NR³ or substituted or unsubstituted C₁ to C₃ alkylenediyl group;
R¹ is selected from substituted or unsubstituted C₁ to C₁₂ alkyl, substituted or unsubstituted C₆ to C₁₉ aryl or C₂ to C₂₀ heteroaryl group;
R² is selected from H, D, CN, substituted or unsubstituted C₁ to C₁₂ alkyl, substituted or unsubstituted C₃ to C₅ alkenyl, substituted or unsubstituted C₆ to C₁₉ aryl or C₂ to C₂₀ heteroaryl group;
R³ is selected from H, D, substituted or unsubstituted C₁ to C₁₂ alkyl, substituted or unsubstituted C₃ to C₅ alkenyl, substituted or unsubstituted C₆ to C₁₉ aryl or C₂ to C₂₀ heteroaryl group;
wherein two of X¹, X³, R¹ and R³ may form a 5 to 7 membered ring;
R^{a} is selected from CN, substituted or unsubstituted C₁ to C₂₀ alkyl, CH₃, CH₂D, CHD₂, CD₃, a partially fluorinated or perfluorinated C₁ to C₂₀ alkyl, CFH₂, CFDH, CFD₂, CF₂H, CF₂D, CF₃, substituted or unsubstituted C₁ to C₂₀ alkenyl, substituted or unsubstituted C₁ to C₂₀ alkinyl, substituted or unsubstituted C₃ to C₄₀ cycloalkyl, substituted or unsubstituted C₂ to C₄₀ heterocycloalkyl, substituted or unsubstituted C₃ to C₄₀ carbocyclyl ring, substituted or unsubstituted C₂ to C₄₀ heterocyclyl, substituted or unsubstituted C₂ to C₄₀ heteroaryl, or substituted or unsubstituted C₆ to C₄₀ aryl.

The term "alkylenediyl" group especially means and/or comprises moities with a "carbon backbone" selected from C=, C=C and C=C-C=.

According to one embodiment, Ligand L is selected from formulae (IIb) to (IIe): 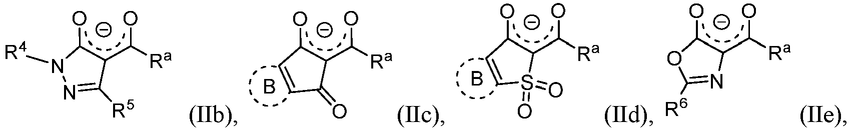 Wherein
R⁴ is selected from unsubstituted and substituted C₁ to C₁₂ alkyl, substituted or unsubstituted C₆ to C₁₉ aryl, substituted or unsubstituted C₂ to C₂₀ heteroaryl, substituted or unsubstituted 6-membered heteroaryl, preferably R² is selected from substituted or unsubstituted C₆ to C₁₉ aryl, substituted or unsubstituted C₂ to C₂₀ heteroaryl, substituted or unsubstituted 6-membered heteroaryl;
R⁵ is selected from substituted or unsubstituted C₁ to C₁₂ alkyl, substituted or unsubstituted C₆ to C₁₉ aryl, substituted or unsubstituted C₂ to C₂₀ heteroaryl, substituted or unsubstituted 6-membered heteroaryl, or CN;
R⁶ is selected from unsubstituted and substituted C₁ to C₁₂ alkyl, substituted or unsubstituted C₆ to C₁₉ aryl, substituted or unsubstituted C₂ to C₂₀ heteroaryl, substituted or unsubstituted 6-membered heteroaryl;
B is selected from substituted or unsubstituted C₆ to C₁₉ aryl, substituted or unsubstituted C₂ to C₂₀ heteroaryl ring or a ring system, wherein the ring system may comprise one or two rings, preferably one ring;
R^{a} is selected from CN, substituted or unsubstituted C₁ to C₂₀ alkyl, CH₃, CH₂D, CHD₂, CD₃, a partially fluorinated or perfluorinated C₁ to C₂₀ alkyl, CFH₂, CFDH, CFD₂, CF₂H, CF₂D, CF₃, substituted or unsubstituted C₁ to C₂₀ alkenyl, substituted or unsubstituted C₁ to C₂₀ alkinyl, substituted or unsubstituted C₃ to C₄₀ cycloalkyl, substituted or unsubstituted C₂ to C₄₀ heterocycloalkyl, substituted or unsubstituted C₃ to C₄₀ carbocyclyl ring, substituted or unsubstituted C₂ to C₄₀ heterocyclyl, substituted or unsubstituted C₂ to C₄₀ heteroaryl, or substituted or unsubstituted C₆ to C₄₀ aryl.

According to one embodiment of the invention, ligand L, ligand of formula (II), ligand of formula (IIa) and/or the ligands of formulae (IIb) to (IIe) comprise two or more and ten or less fluorine atoms, preferably 8 fluorine atoms or less.

According to one embodiment of the invention, ligand L, ligand of formula (II), ligand of formula (IIa) and/or the ligands of formulae (IIb) to (IIe) comprise at least one fluorine atom.

According to one embodiment of the invention, ligand L, ligand of formula (II), ligand of formula (IIa) and/or the ligands of formulae (IIb) to (IIe) comprise comprises at least two fluorine atoms.

According to one embodiment of the invention, ligand L, ligand of formula (II), ligand of formula (IIa) and/or the ligands of formulae (IIb) to (IIe) comprise one to ten fluorine atoms; preferably two to ten fluorine atoms; more preferably two to eight fluorine atoms.

According to one embodiment, L is selected from formulae (IIf) or (IIg): 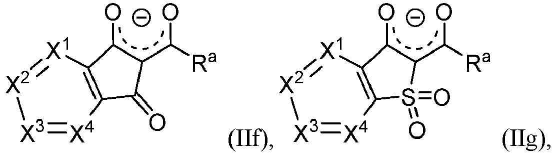 wherein
- X¹: is selected from CR²¹ or N;
- X²: is selected from CR²² or N;
- X³: is selected from CR²³ or N;
- X⁴: is selected from CR²⁴ or N;
wherein 0, 1 or 2 of the group consisting of X¹, X², X³, X⁴ are selected from N;
R²¹ to R²⁴ are independently selected from H, D, halogen, Cl, F, CN, NO₂, C₁ to C₁₂ alkyl, C₁ to C₁₂ alkoxy, partially or perfluorinated C₁ to C₁₂ alkyl, CF₃, CF₂R, CFR₂, CF₂H, CFH₂, partially or perfluorinated C₁ to C₁₂ alkoxy, or a combination thereof, wherein R is H or D, preferably H; and whereby any R^{k} to R^{k+1} may form a ring, wherein k is an integer from 1 to 3;
R^{a} is selected from CN, substituted or unsubstituted C₁ to C₂₀ alkyl, CH₃, CH₂D, CHD₂, CD₃, a partially fluorinated or perfluorinated C₁ to C₂₀ alkyl, CFH₂, CFDH, CFD₂, CF₂H, CF₂D, CF₃, substituted or unsubstituted C₁ to C₂₀ alkenyl, substituted or unsubstituted C₁ to C₂₀ alkinyl, substituted or unsubstituted C₃ to C₄₀ cycloalkyl, substituted or unsubstituted C₂ to C₄₀ heterocycloalkyl, substituted or unsubstituted C₃ to C₄₀ carbocyclyl ring, substituted or unsubstituted C₂ to C₄₀ heterocyclyl, substituted or unsubstituted C₂ to C₄₀ heteroaryl, or substituted or unsubstituted C₆ to C₄₀ aryl.

Preferably, R²¹ to R²⁴ are independently selected from H, D, halogen, Cl, F, CN, partially or perfluorinated C₁ to C₁₂ alkyl, CF₃, CF₂R, CFR₂, CF₂H, CFH₂, or a combination thereof, wherein R is H or D, preferably H; more preferred R²¹ to R²⁴ are independently selected from H, D, F, CF₃, CF₂R, CFR₂, CF₂H, CFH₂, or a combination thereof, wherein R is H or D, preferably H.

According to one embodiment of the invention, the ligands of formulae (IIf) and (IIg) comprise between two and 10 fluorine atoms, preferably 8 fluorine atoms or less.

According to one embodiment of the invention, the ligands of formulae (IIf) and (IIg) comprise comprises at least one fluorine atom.

According to one embodiment of the invention, the ligands of formulae (IIf) and (IIg) comprise at least two fluorine atoms.

According to one embodiment of the invention, the ligands of formulae (IIf) and (IIg) comprise one to ten fluorine atoms; preferably two to ten fluorine atoms; more preferably two to eight fluorine atoms.

According to one embodiment of the invention, wherein ligand L, ligand of formula (II), ligand of formula (IIa), the ligands of formulae (IIb) to (IIe) and/or the ligands of formulae (IIf) and (IIg) comprise between two and 10 fluorine atoms.

According to one embodiment of the invention, wherein ligand L, ligand of formula (II), ligand of formula (IIa), the ligands of formulae (IIb) to (IIe) and/or the ligands of formulae (IIf) and (IIg) comprise at least one fluorine atom.

According to one embodiment of the invention, wherein ligand L, ligand of formula (II), ligand of formula (IIa), the ligands of formulae (IIb) to (IIe) and/or the ligands of formulae (IIf) and (IIg) comprise at least two fluorine atoms.

According to one embodiment of the invention, wherein ligand L, ligand of formula (II), ligand of formula (IIa), the ligands of formulae (IIb) to (IIe) and/or the ligands of formulae (IIf) and (IIg) comprise one to ten fluorine atoms; preferably two to ten fluorine atoms; more preferably two to eight fluorine atoms.

According to one embodiment, Ligand L is selected from formulae (IIb), (IIe), (IIf) and/or (IIg).

According to one embodiment, A is selected from substituted or unsubstituted C₅ to C₁₉ carbocyclic ring or a ring system, substituted or unsubstituted C₂ to C₂₀ heterocyclic ring or a ring system, substituted or unsubstituted C₆ to C₁₉ aryl, or substituted or unsubstituted C₂ to C₂₀ heteroaryl, wherein the carbocyclic ring or the heterocyclic ring may contain one or more double bonds.

According to one embodiment, one or more substituents of A are independently selected from D, electron-withdrawing group, =O, =S, CN, halogen, Cl, F, substituted or unsubstituted C₁ to C₂₀ alkyl, CH₃, CH₂D, CHD₂, CD₃, a partially fluorinated or perfluorinated C₁ to C₂₀ alkyl, CFH₂, CFDH, CFD₂, CF₂H, CF₂D, CF₃, substituted or unsubstituted C₁ to C₂₀ alkenyl, substituted or unsubstituted C₁ to C₂₀ alkinyl, substituted or unsubstituted C₃ to C₄₀ cycloalkyl, substituted or unsubstituted C₂ to C₄₀ heterocycloalkyl, substituted or unsubstituted C₃ to C₄₀ carbocyclyl ring, or substituted or unsubstituted C₂ to C₄₀ heterocyclyl, substituted or unsubstituted C₂ to C₄₀ heteroaryl ring or ring system, or substituted or unsubstituted C₆ to C₄₀ aryl ring or ring system.

According to one embodiment, one or more substituents of A are independently selected from D, electron-withdrawing group, =O, =S, CN, halogen, Cl, F, substituted or unsubstituted C₁ to C₁₂ alkyl, CH₃, CH₂D, CHD₂, CD₃, a partially fluorinated or perfluorinated C₁ to C₁₂ alkyl, CFH₂, CFDH, CFD₂, CF₂H, CF₂D, CF₃, substituted or unsubstituted C₁ to C₁₂ alkenyl, substituted or unsubstituted C₁ to C₁₂ alkinyl, substituted or unsubstituted C₅ to C₁₉ cycloalkyl, substituted or unsubstituted C₂ to C₂₀ heterocycloalkyl, substituted or unsubstituted C₅ to C₁₉ carbocyclyl ring, or substituted or unsubstituted C₂ to C₂₀ heterocyclyl, substituted or unsubstituted C₂ to C₂₀ heteroaryl, or substituted or unsubstituted C₆ to C₁₉ aryl.

According to one embodiment, one or more substituent of the one or more substituent of A are independently selected from D, electron-withdrawing group, halogen, Cl, F, CN, CH₃, CH₂D, CHD₂, CD₃, partially or perfluorinated C₁ to C₈ alkyl, CFH₂, CFDH, CFD₂, CF₂H, CF₂D, CF₃, partially or perfluorinated C₁ to C₈ alkoxy, OCH₃, OCH₂D, OCHD₂, OCD₃, OCFH₂, OCFDH, OCFD₂, OCF₂H, OCF₂D, OCF₃.

According to one embodiment, one or more substituents of A are independently selected from D, electron-withdrawing group, =O, =S, CN, halogen, Cl, F, substituted or unsubstituted C₁ to C₂₀ alkyl, CH₃, CH₂D, CHD₂, CD₃, a partially fluorinated or perfluorinated C₁ to C₂₀ alkyl, CFH₂, CFDH, CFD₂, CF₂H, CF₂D, CF₃, substituted or unsubstituted C₁ to C₂₀ alkenyl, substituted or unsubstituted C₁ to C₂₀ alkinyl, substituted or unsubstituted C₃ to C₄₀ cycloalkyl, substituted or unsubstituted C₂ to C₄₀ heterocycloalkyl, substituted or unsubstituted C₃ to C₄₀ carbocyclyl ring, or substituted or unsubstituted C₂ to C₄₀ heterocyclyl, substituted or unsubstituted C₂ to C₄₀ heteroaryl ring or ring system, or substituted or unsubstituted C₆ to C₄₀ aryl ring or ring system and one or more substituents on R^{a} are independently selected from an D, electron-withdrawing group, halogen, Cl, F, CN, partially or perfluorinated C₁ to C₈ alkyl, CH₃, CH₂D, CHD₂, CD₃, CFH₂, CFDH, CFD₂, CF₂H, CF₂D, CF₃, partially or perfluorinated C₁ to C₈ alkoxy, OCH₃, OCH₂D, OCHD₂, OCD₃, OCFH₂, OCFDH, OCFD₂, OCF₂H, OCF₂D, OCF₃.

According to one embodiment, one or more substituents on R^{a} are independently selected from an D, electron-withdrawing group, halogen, Cl, F, CN, partially or perfluorinated C₁ to C₈ alkyl, CH₃, CH₂D, CHD₂, CD₃, CFH₂, CFDH, CFD₂, CF₂H, CF₂D, CF₃, partially or perfluorinated C₁ to C₈ alkoxy, OCH₃, OCH₂D, OCHD₂, OCD₃, OCFH₂, OCFDH, OCFD₂, OCF₂H, OCF₂D, OCF₃.

According to one embodiment, one or more substituents of A are independently selected from D, electron-withdrawing group, =O, =S, CN, halogen, Cl, F, substituted or unsubstituted C₁ to C₂₀ alkyl, CH₃, CH₂D, CHD₂, CD₃, a partially fluorinated or perfluorinated C₁ to C₂₀ alkyl, CFH₂, CFDH, CFD₂, CF₂H, CF₂D, CF₃, substituted or unsubstituted C₁ to C₂₀ alkenyl, substituted or unsubstituted C₁ to C₂₀ alkinyl, substituted or unsubstituted C₃ to C₄₀ cycloalkyl, substituted or unsubstituted C₂ to C₄₀ heterocycloalkyl, substituted or unsubstituted C₃ to C₄₀ carbocyclyl ring, or substituted or unsubstituted C₂ to C₄₀ heterocyclyl, substituted or unsubstituted C₂ to C₄₀ heteroaryl ring or ring system, or substituted or unsubstituted C₆ to C₄₀ aryl ring or ring system;
and one or more substituents on R^{a} are independently selected from an D, electron-withdrawing group, halogen, Cl, F, CN, partially or perfluorinated C₁ to C₈ alkyl, CH₃, CH₂D, CHD₂, CD₃, CFH₂, CFDH, CFD₂, CF₂H, CF₂D, CF₃, partially or perfluorinated C₁ to C₈ alkoxy, OCH₃, OCH₂D, OCHD₂, OCD₃, OCFH₂, OCFDH, OCFD₂, OCF₂H, OCF₂D, OCF₃.

According to an embodiment, one or more substituent of A are independently selected from D, electron-withdrawing group, =O, =S, CN, halogen, Cl, F, substituted or unsubstituted C₁ to C₂₀ alkyl, CH₃, CH₂D, CHD₂, CD₃, a partially fluorinated or perfluorinated C₁ to C₂₀ alkyl, CFH₂, CFDH, CFD₂, CF₂H, CF₂D, CF₃, substituted or unsubstituted C₁ to C₂₀ alkenyl, substituted or unsubstituted C₁ to C₂₀ alkinyl, substituted or unsubstituted C₃ to C₄₀ cycloalkyl, substituted or unsubstituted C₂ to C₄₀ heterocycloalkyl, substituted or unsubstituted C₃ to C₄₀ carbocyclyl ring, or substituted or unsubstituted C₂ to C₄₀ heterocyclyl, substituted or unsubstituted C₂ to C₄₀ heteroaryl ring or ring system, or substituted or unsubstituted C₆ to C₄₀ aryl ring or ring system; and one or more substituent on R^{a} are independently selected from an D, electron-withdrawing group, halogen, Cl, F, CN, partially or perfluorinated C₁ to C₈ alkyl, CH₃, CH₂D, CHD₂, CD₃, CFH₂, CFDH, CFD₂, CF₂H, CF₂D, CF₃, partially or perfluorinated C₁ to C₈ alkoxy, OCH₃, OCH₂D, OCHD₂, OCD₃, OCFH₂, OCFDH, OCFD₂, OCF₂H, OCF₂D, OCF₃.

According to an embodiment, the one or more substituent of the one or more substituent of A are independently selected from D, electron-withdrawing group, halogen, Cl, F, CN, CH₃, CH₂D, CHD₂, CD₃, partially or perfluorinated C₁ to C₈ alkyl, CFH₂, CFDH, CFD₂, CF₂H, CF₂D, CF₃, partially or perfluorinated C₁ to C₈ alkoxy, OCH₃, OCH₂D, OCHD₂, OCD₃, OCFH₂, OCFDH, OCFD₂, OCF₂H, OCF₂D, OCF₃; and one or more substituent on R^{a} are independently selected from an D, electron-withdrawing group, halogen, Cl, F, CN, partially or perfluorinated C₁ to C₈ alkyl, CH₃, CH₂D, CHD₂, CD₃, CFH₂, CFDH, CFD₂, CF₂H, CF₂D, CF₃, partially or perfluorinated C₁ to C₈ alkoxy, OCH₃, OCH₂D, OCHD₂, OCD₃, OCFH₂, OCFDH, OCFD₂, OCF₂H, OCF₂D, OCF₃.i

According to an embodiment, A is selected from substituted or unsubstituted C₅ to C₁₉ carbocyclic ring or ring system, substituted or unsubstituted C₂ to C₂₀ heterocyclic ring or ring system, substituted or unsubstituted C₆ to C₁₉ aryl, or substituted or unsubstituted C₂ to C₂₀ heteroaryl, wherein the carbocyclic ring or the heterocyclic ring may contain one or more double bonds, wherein one or more substituents of A are independently selected from D, electron-withdrawing group, =O, =S, CN, halogen, Cl, F, substituted or unsubstituted C₁ to C₂₀ alkyl, CH₃, CH₂D, CHD₂, CD₃, a partially fluorinated or perfluorinated C₁ to C₂₀ alkyl, CFH₂, CFDH, CFD₂, CF₂H, CF₂D, CF₃, substituted or unsubstituted C₁ to C₂₀ alkenyl, substituted or unsubstituted C₁ to C₂₀ alkinyl, substituted or unsubstituted C₃ to C₄₀ cycloalkyl, substituted or unsubstituted C₂ to C₄₀ heterocycloalkyl, substituted or unsubstituted C₃ to C₄₀ carbocyclyl ring, or substituted or unsubstituted C₂ to C₄₀ heterocyclyl, substituted or unsubstituted C₂ to C₄₀ heteroaryl ring or ring system, or substituted or unsubstituted C₆ to C₄₀ aryl ring or ring system.

According to an embodiment, A is selected from substituted or unsubstituted C₅ to C₁₉ carbocyclic ring, substituted or unsubstituted C₂ to C₂₀ heterocyclic ring, substituted or unsubstituted C₆ to C₁₉ aryl, or substituted or unsubstituted C₂ to C₂₀ heteroaryl, wherein the carbocyclic ring or the heterocyclic ring may contain one or more double bonds,
wherein the one or more substituent of A are independently selected from D, electron-withdrawing group, =O, =S, CN, halogen, Cl, F, substituted or unsubstituted C₁ to C₂₀ alkyl, CH₃, CH₂D, CHD₂, CD₃, a partially fluorinated or perfluorinated C₁ to C₂₀ alkyl, CFH₂, CFDH, CFD₂, CF₂H, CF₂D, CF₃, substituted or unsubstituted C₁ to C₂₀ alkenyl, substituted or unsubstituted C₁ to C₂₀ alkinyl, substituted or unsubstituted C₃ to C₄₀ cycloalkyl, substituted or unsubstituted C₂ to C₄₀ heterocycloalkyl, substituted or unsubstituted C₃ to C₄₀ carbocyclyl ring, or substituted or unsubstituted C₂ to C₄₀ heterocyclyl, substituted or unsubstituted C₂ to C₄₀ heteroaryl ring or ring system, or substituted or unsubstituted C₆ to C₄₀ aryl ring or ring system; wherein the one or more substituents of the one or more substituents of A are independently selected from D, electron-withdrawing group, halogen, Cl, F, CN, CH₃, CH₂D, CHD₂, CD₃, partially or perfluorinated C₁ to C₈ alkyl, CFH₂, CFDH, CFD₂, CF₂H, CF₂D, CF₃, partially or perfluorinated C₁ to C₈ alkoxy, OCH₃, OCH₂D, OCHD₂, OCD₃, OCFH₂, OCFDH, OCFD₂, OCF₂H, OCF₂D, OCF₃.

According to an embodiment, A is selected from substituted or unsubstituted C₅ to C₁₉ carbocyclic ring, substituted or unsubstituted C₂ to C₂₀ heterocyclic ring, substituted or unsubstituted C₆ to C₁₉ aryl, or substituted or unsubstituted C₂ to C₂₀ heteroaryl, wherein the carbocyclic ring or the heterocyclic ring may contain one or more double bonds; wherein one or more substituents on R^{a} are independently selected from an D, electron-withdrawing group, halogen, Cl, F, CN, partially or perfluorinated C₁ to C₈ alkyl, CH₃, CH₂D, CHD₂, CD₃, CFH₂, CFDH, CFD₂, CF₂H, CF₂D, CF₃, partially or perfluorinated C₁ to C₈ alkoxy, OCH₃, OCH₂D, OCHD₂, OCD₃, OCFH₂, OCFDH, OCFD₂, OCF₂H, OCF₂D, OCF₃.

According to an embodiment, A is selected from substituted or unsubstituted C₅ to C₁₉ carbocyclic ring, substituted or unsubstituted C₂ to C₂₀ heterocyclic ring, substituted or unsubstituted C₆ to C₁₉ aryl, or substituted or unsubstituted C₂ to C₂₀ heteroaryl, wherein the carbocyclic ring or the heterocyclic ring may contain one or more double bonds, wherein the one or more substituent of A are independently selected from D, electron-withdrawing group, =O, =S, CN, halogen, Cl, F, substituted or unsubstituted C₁ to C₂₀ alkyl, CH₃, CH₂D, CHD₂, CD₃, a partially fluorinated or perfluorinated C₁ to C₂₀ alkyl, CFH₂, CFDH, CFD₂, CF₂H, CF₂D, CF₃, substituted or unsubstituted C₁ to C₂₀ alkenyl, substituted or unsubstituted C₁ to C₂₀ alkinyl, substituted or unsubstituted C₃ to C₄₀ cycloalkyl, substituted or unsubstituted C₂ to C₄₀ heterocycloalkyl, substituted or unsubstituted C₃ to C₄₀ carbocyclyl ring, or substituted or unsubstituted C₂ to C₄₀ heterocyclyl, substituted or unsubstituted C₂ to C₄₀ heteroaryl, or substituted or unsubstituted C₆ to C₄₀ aryl; and wherein one or more substituents on R^{a} are independently selected from an D, electron-withdrawing group, halogen, Cl, F, CN, partially or perfluorinated C₁ to C₈ alkyl, CH₃, CH₂D, CHD₂, CD₃, CFH₂, CFDH, CFD₂, CF₂H, CF₂D, CF₃, partially or perfluorinated C₁ to C₈ alkoxy, OCH₃, OCH₂D, OCHD₂, OCD₃, OCFH₂, OCFDH, OCFD₂, OCF₂H, OCF₂D, OCF₃.

According to an embodiment, A is selected from substituted or unsubstituted C₅ to C₁₉ carbocyclic ring or ring system, substituted or unsubstituted C₂ to C₂₀ heterocyclic ring or ring system, substituted or unsubstituted C₆ to C₁₉ aryl, or substituted or unsubstituted C₂ to C₂₀ heteroaryl, wherein the carbocyclic ring or the heterocyclic ring may contain one or more double bonds,

According to an embodiment, A is selected from a substituted or unsubstituted C₅ to C₁₉ carbocyclic ring or ring system, substituted or unsubstituted C₂ to C₂₀ heterocyclic ring or ring system, substituted or unsubstituted C₆ to C₁₉ aryl, or substituted or unsubstituted C₂ to C₂₀ heteroaryl, wherein the carbocyclic ring or the heterocyclic ring may contain one or more double bonds,
wherein the one or more substituent of A are independently selected from D, electron-withdrawing group, =O, =S, CN, halogen, Cl, F, substituted or unsubstituted C₁ to C₂₀ alkyl, CH₃, CH₂D, CHD₂, CD₃, a partially fluorinated or perfluorinated C₁ to C₂₀ alkyl, CFH₂, CFDH, CFD₂, CF₂H, CF₂D, CF₃, substituted or unsubstituted C₁ to C₂₀ alkenyl, substituted or unsubstituted C₁ to C₂₀ alkinyl, substituted or unsubstituted C₃ to C₄₀ cycloalkyl, substituted or unsubstituted C₂ to C₄₀ heterocycloalkyl, substituted or unsubstituted C₃ to C₄₀ carbocyclyl ring, or substituted or unsubstituted C₂ to C₄₀ heterocyclyl, substituted or unsubstituted C₂ to C₄₀ heteroaryl ring or ring system, or substituted or unsubstituted C₆ to C₄₀ aryl ring or ring system;
wherein the one or more substituents of the one or more substituent of A are independently selected from D, electron-withdrawing group, halogen, Cl, F, CN, CH₃, CH₂D, CHD₂, CD₃, partially or perfluorinated C₁ to C₈ alkyl, CFH₂, CFDH, CFD₂, CF₂H, CF₂D, CF₃, partially or perfluorinated C₁ to C₈ alkoxy, OCH₃, OCH₂D, OCHD₂, OCD₃, OCFH₂, OCFDH, OCFD₂, OCF₂H, OCF₂D, OCF₃;
and wherein one or more substituent on R^{a} are independently selected from an D, electron-withdrawing group, halogen, Cl, F, CN, partially or perfluorinated C₁ to C₈ alkyl, CH₃, CH₂D, CHD₂, CD₃, CFH₂, CFDH, CFD₂, CF₂H, CF₂D, CF₃, partially or perfluorinated C₁ to C₈ alkoxy, OCH₃, OCH₂D, OCHD₂, OCD₃, OCFH₂, OCFDH, OCFD₂, OCF₂H, OCF₂D, OCF₃.

According to one emodiment, Ligand L of formula (II) comprises at least one group selected from substituted or unsubstituted C₁ to C₂₀ alkyl, a partially fluorinated or perfluorinated C₁ to C₂₀ alkyl.

According to an embodiment, wherein R^{a} of formulae (II), (IIa), (IIb), (IIc), (IId),(IIe) is selected from CH₃, CF₃, C₂F₅, C₃F₇, C₄F₉, CFH₂, CF₂H, CF₂CF₂H, (CF₂)₂CF₂H, CH(CF₂H)₂, CF(CF₂H)₂, C(CF₂H)₃ and the following formulae **J1 to J114:** 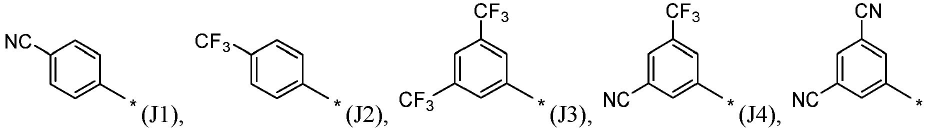 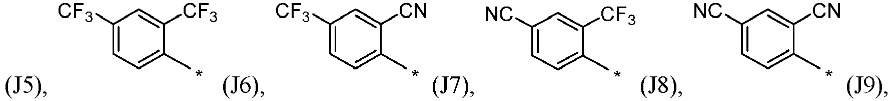 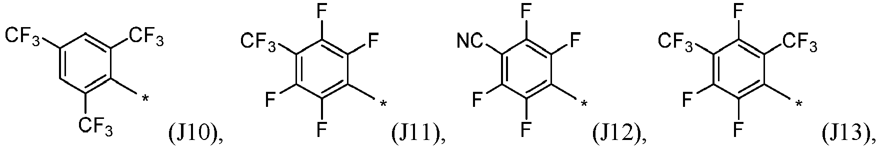 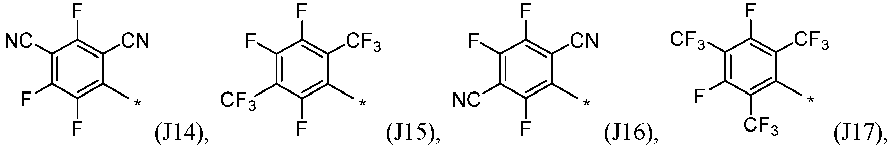 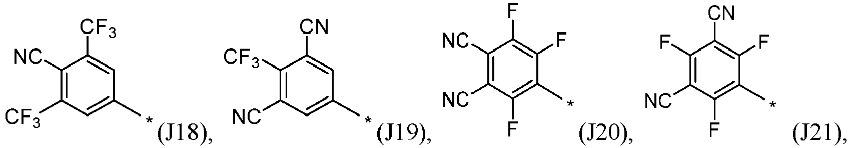 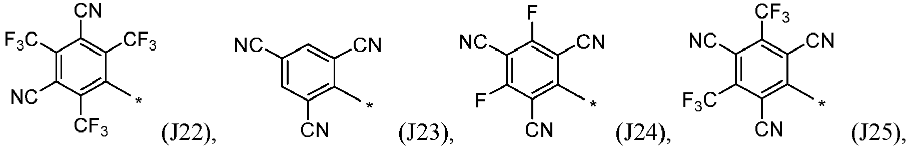 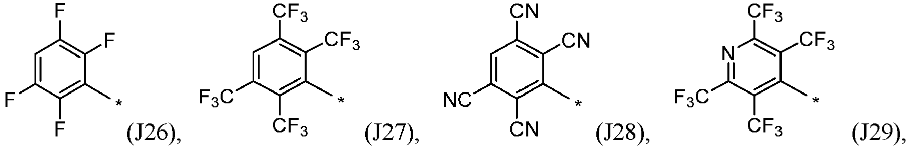 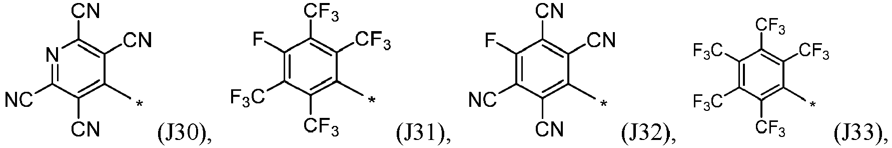 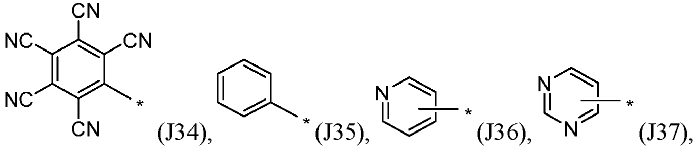 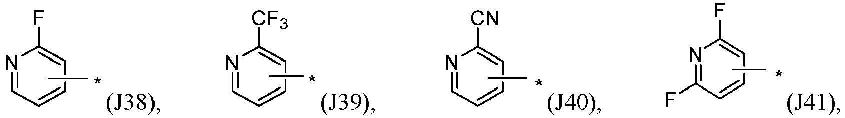 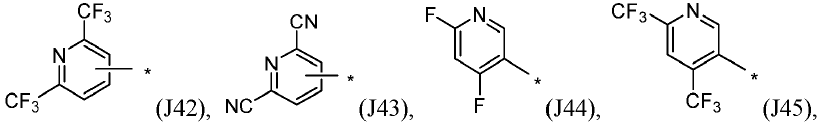 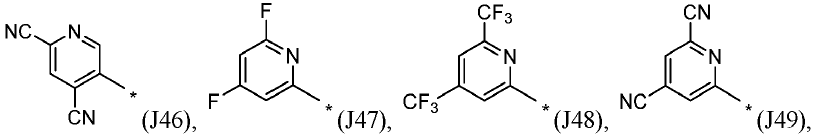 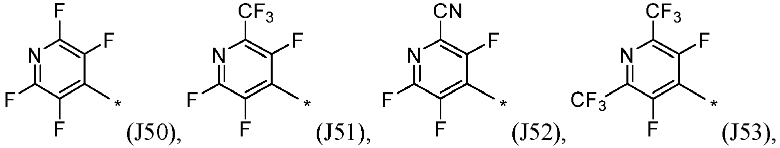 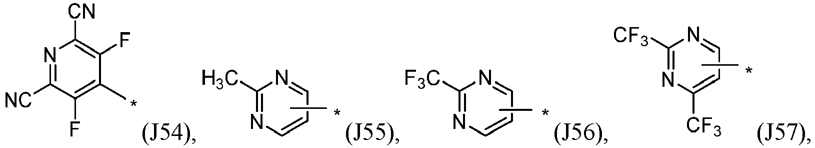 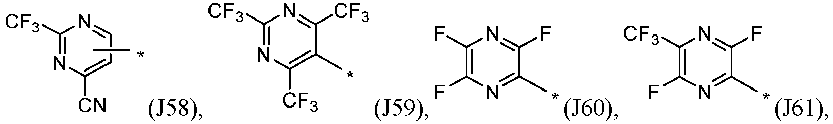 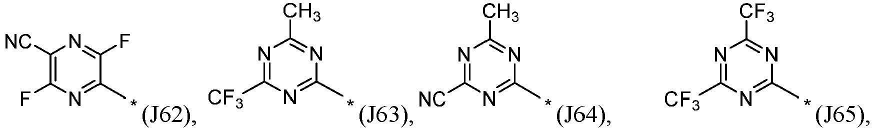 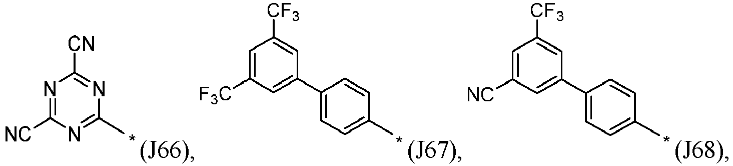 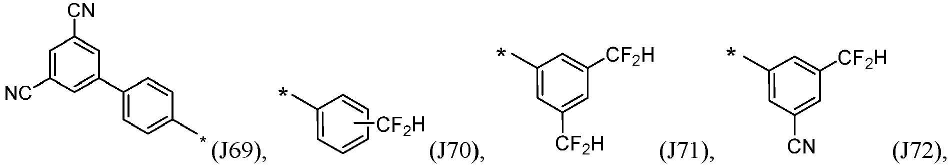 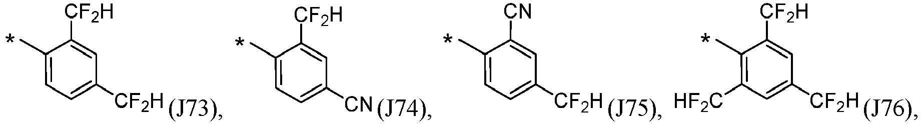 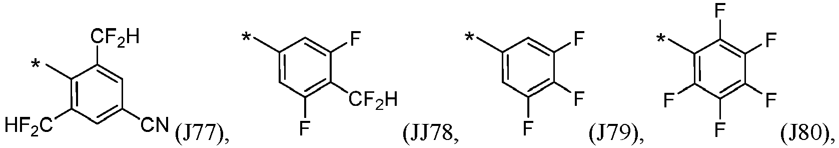 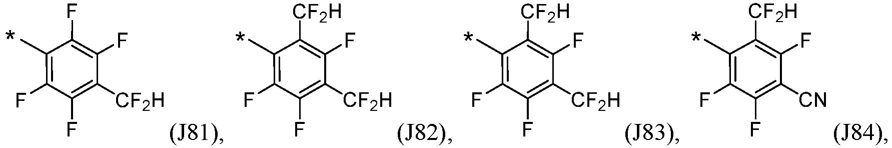 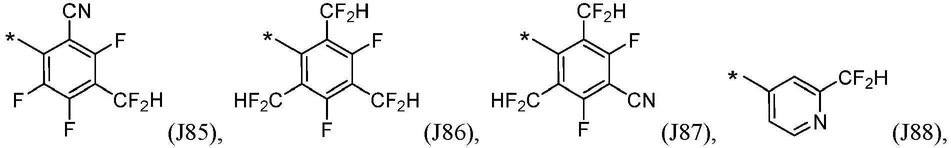 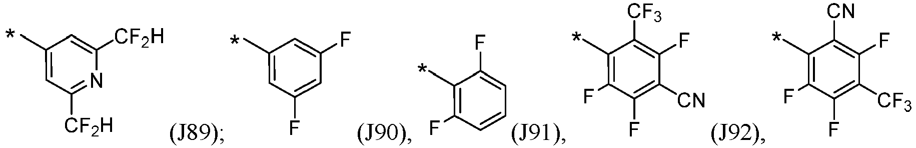 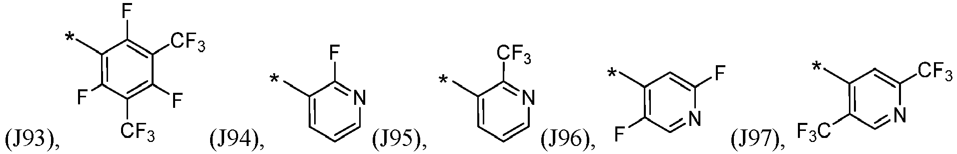 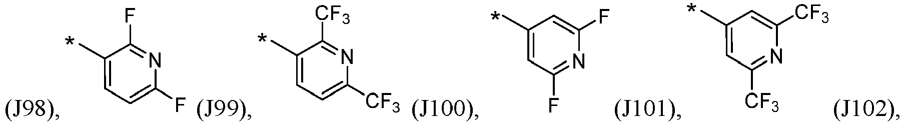 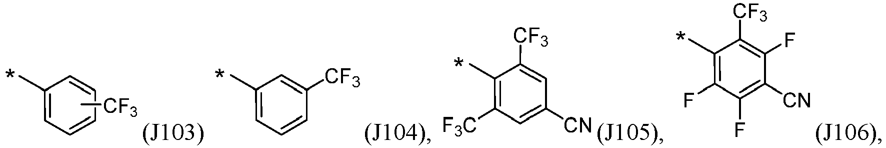 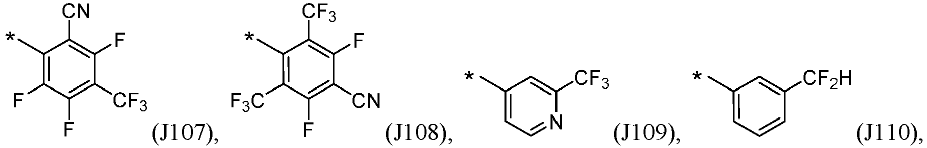 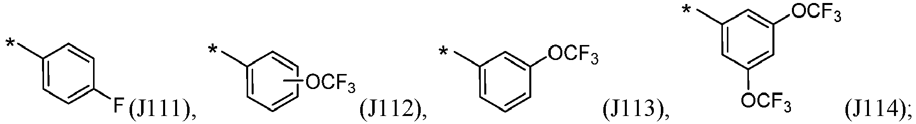 wherein the "*" denotes the binding position.
According to one embodiment, R^{a} of formulae (II), (IIa), (IIb), (IIc), (IId),(IIe) is selected from CH₃, CF₃, CF₂H, and from the following moieties 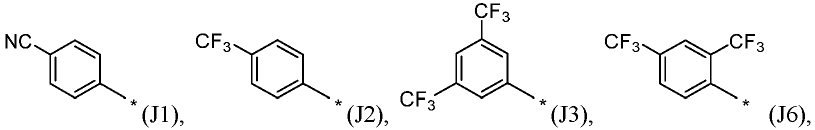 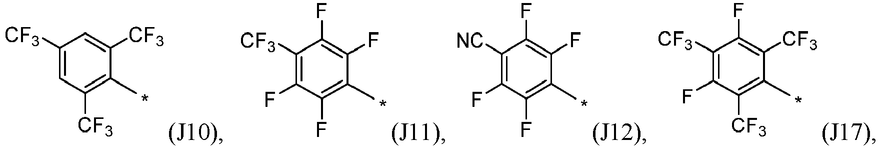 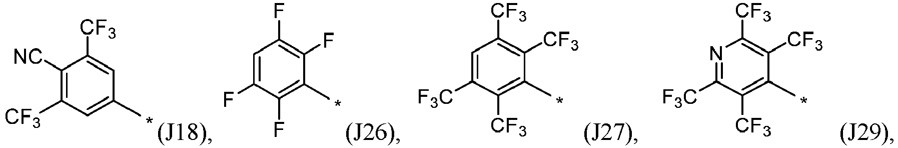 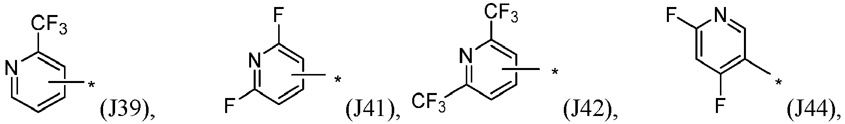 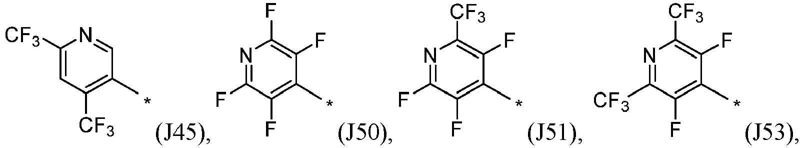 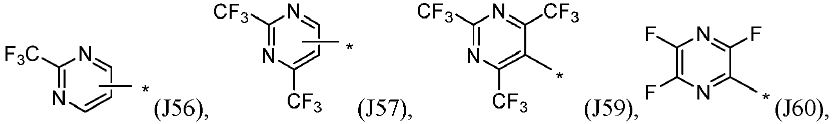 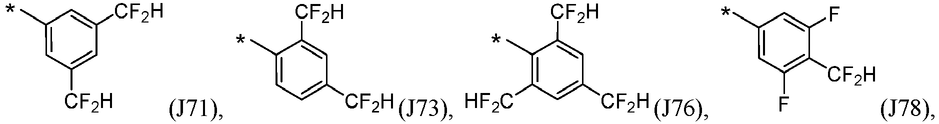 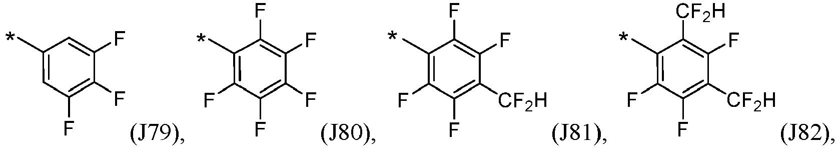 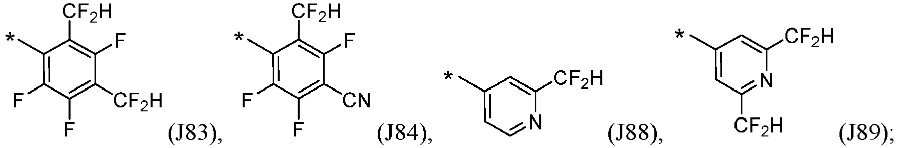 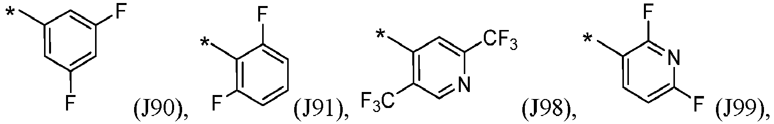 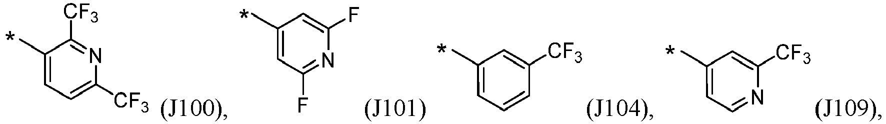 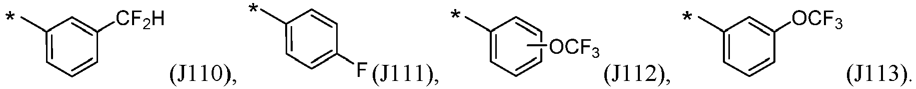 According to one embodiment, R^{a} of formulae (II), (IIa), (IIb), (IIc), (IId),(IIe) is selected from CF₃, CF₂H, and from the following moieties 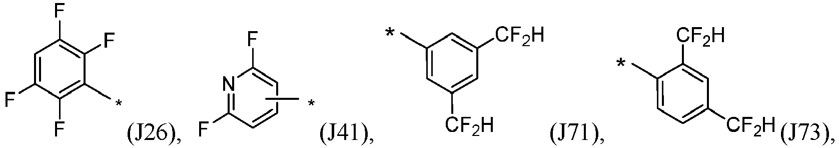 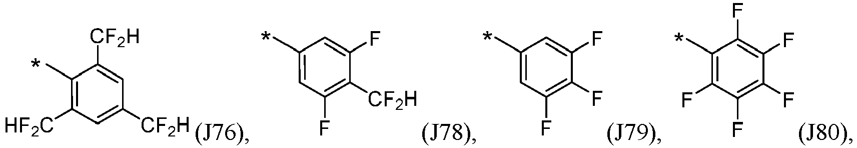 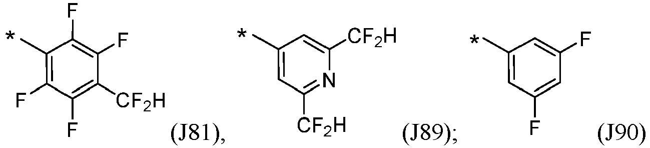 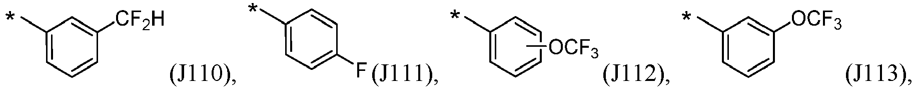 According to one embodiment, R^{a} of formulae (II), (IIa), (IIb), (IIc), (IId),(IIe) is selected from CF₂H, and from the following moieties

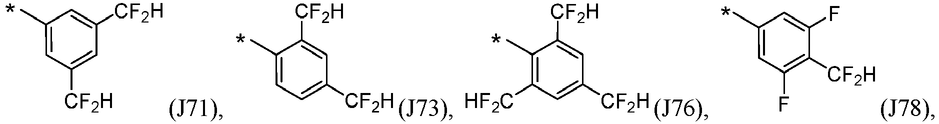

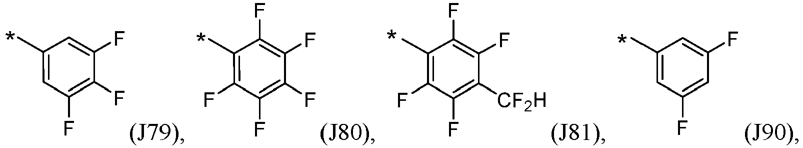

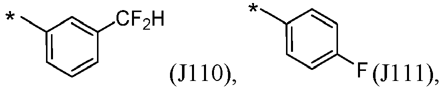

According to an embodiment of Formula (I) R⁴ of formula (IIb) is selected from **K1 to K114** 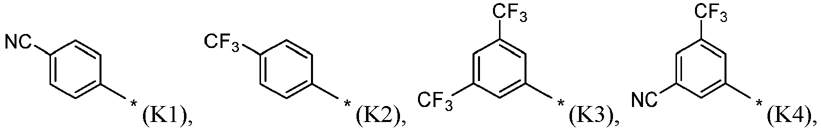 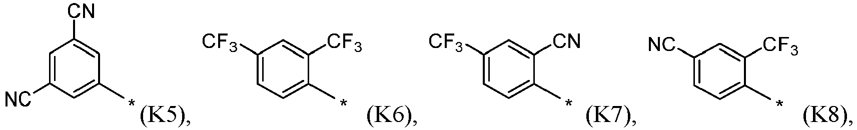 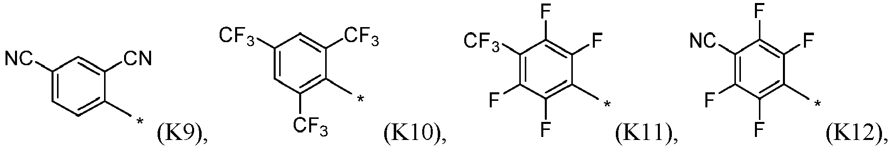 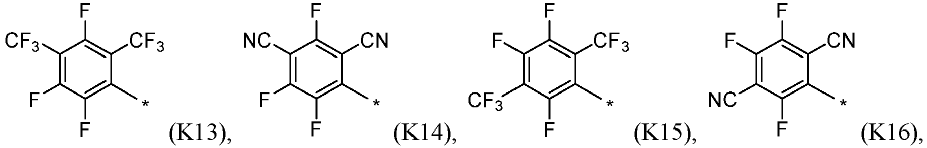 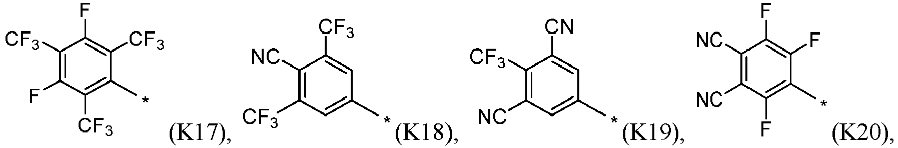 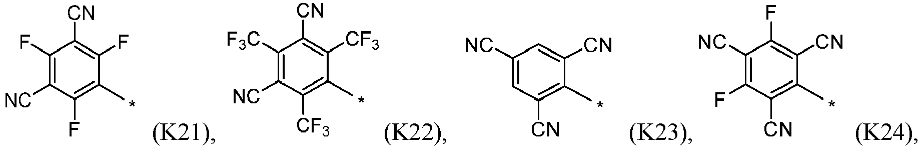 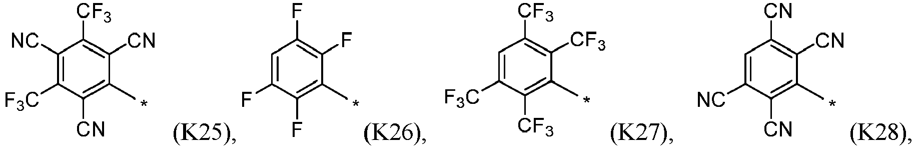 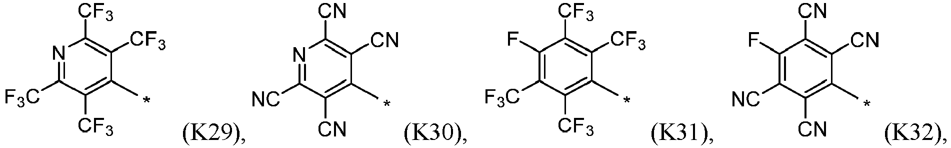 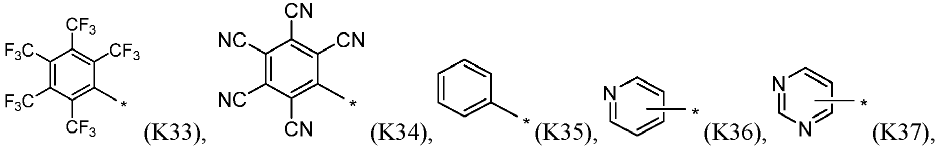 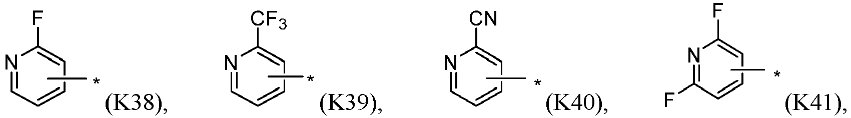 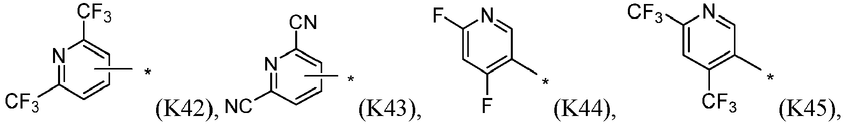 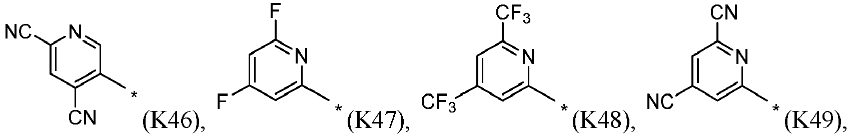 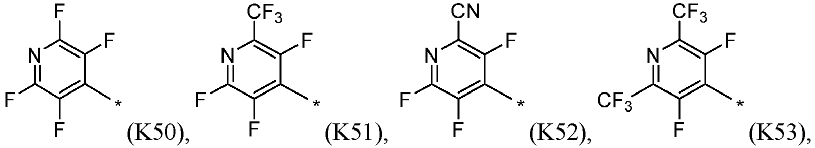 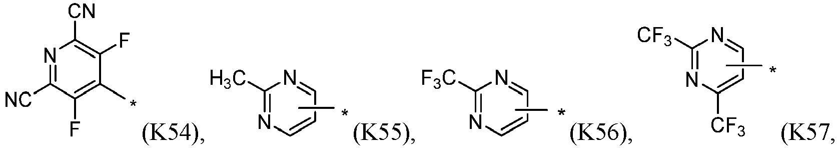 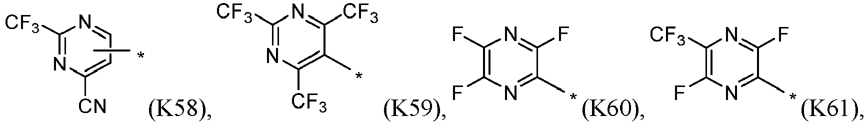 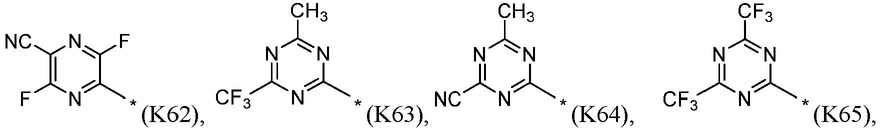 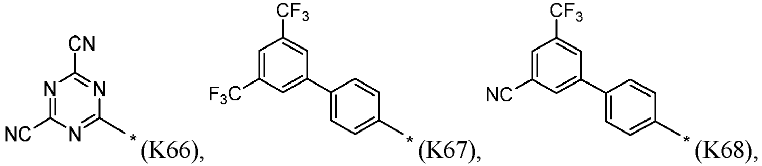 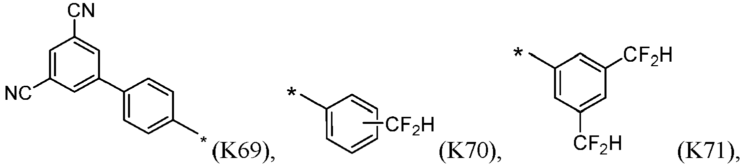 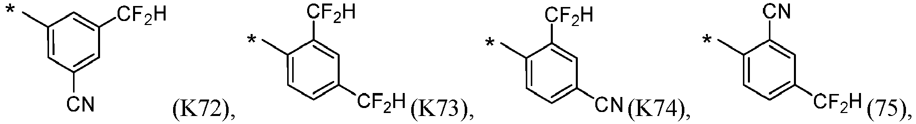 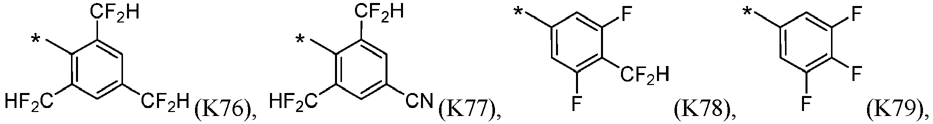 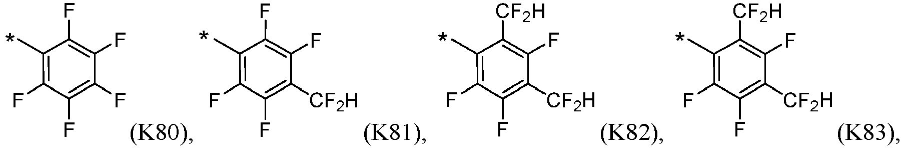 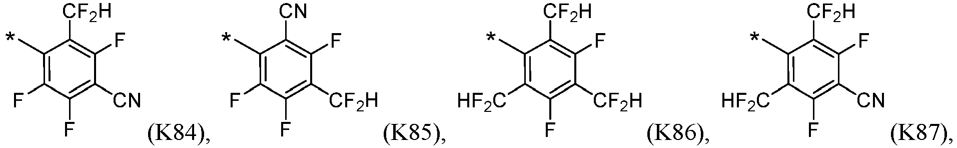 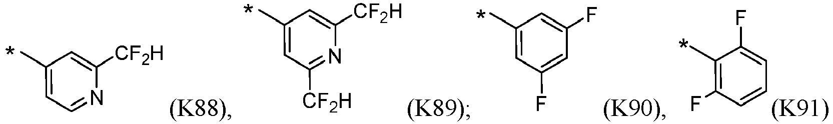 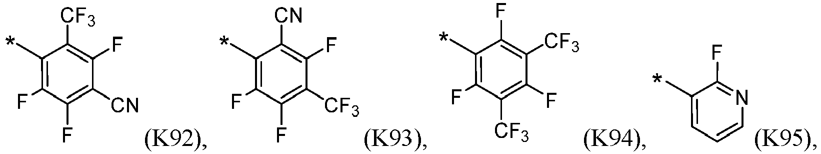 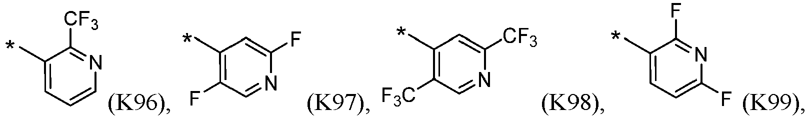 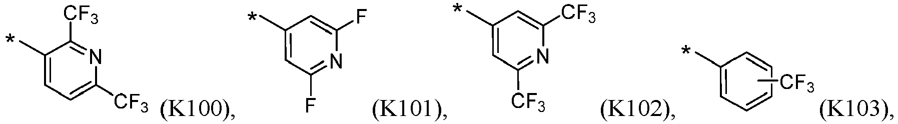 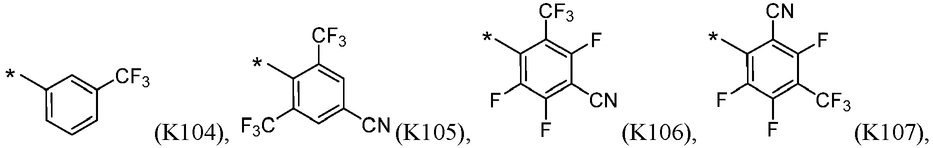 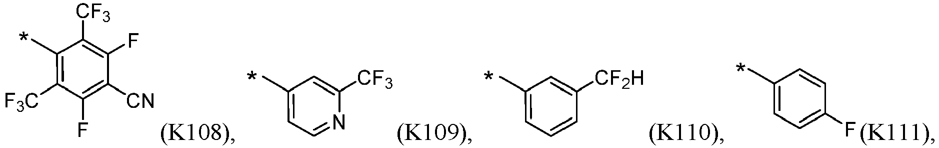 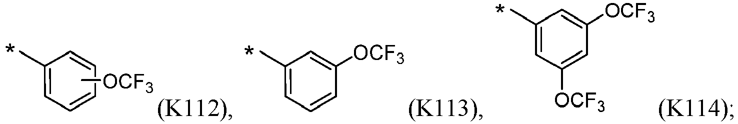 wherein the "*" denotes the binding position.

According to an embodiment of Formula (I) R⁴ of formula (IIb) is selected from the following moieties: 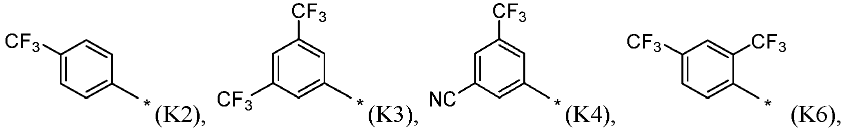 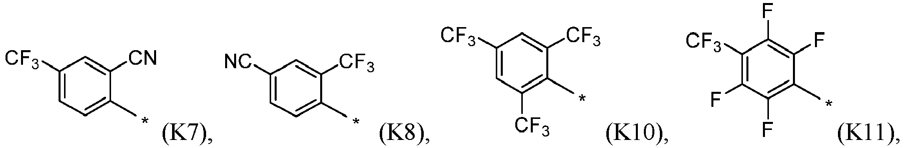 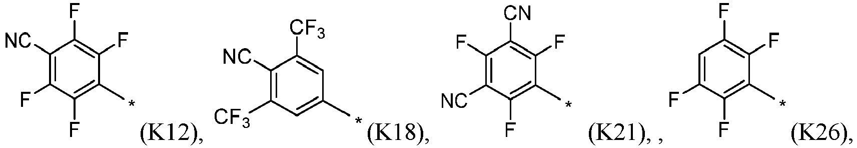 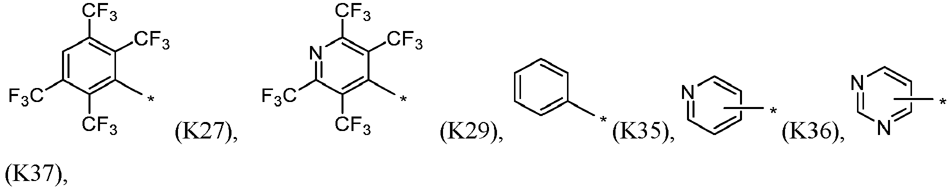 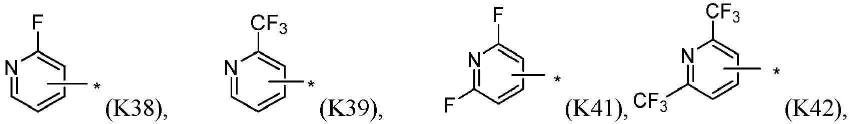 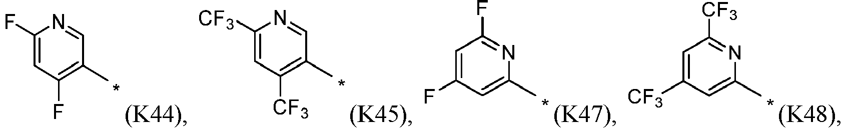 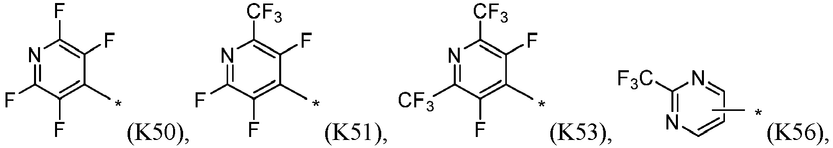 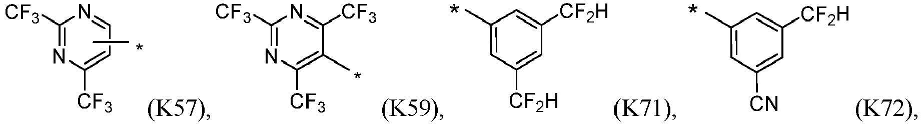 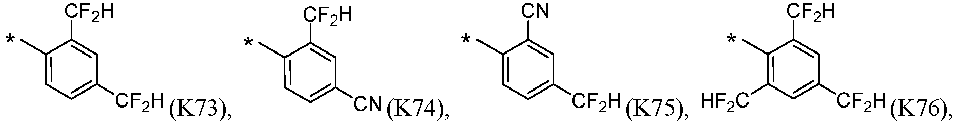 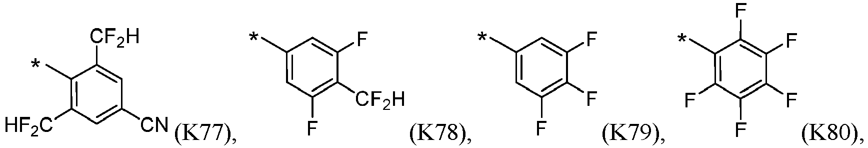 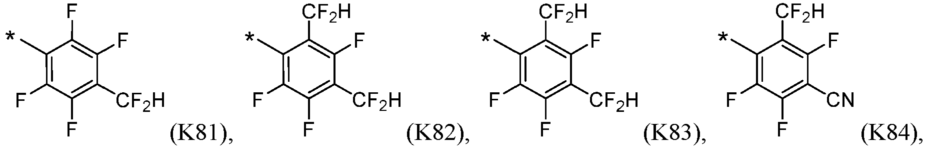 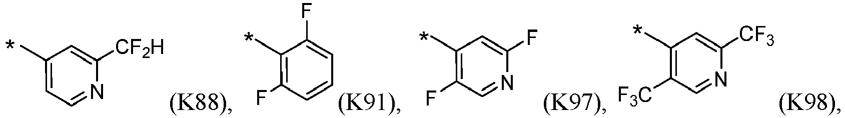 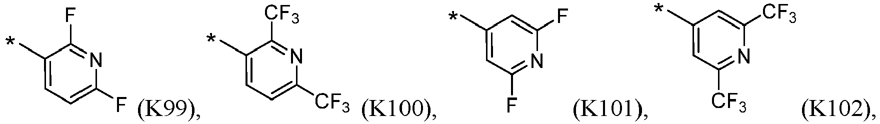 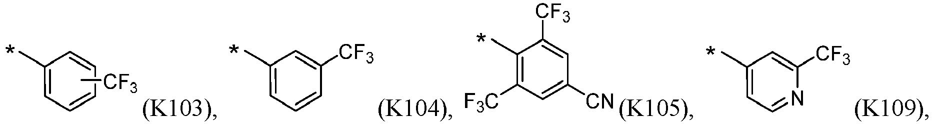 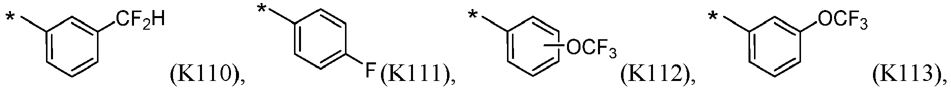 wherein the "*" denotes the binding position.

According to an embodiment of Formula (I) R⁴ of formula (IIb) is selected from the following moieties: 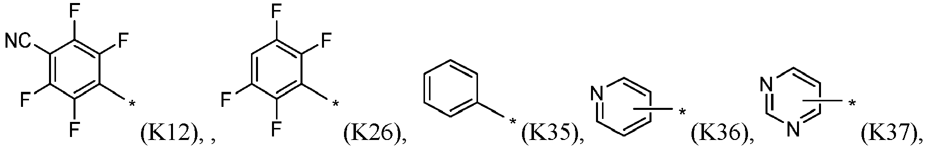 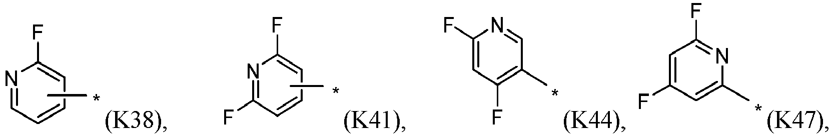 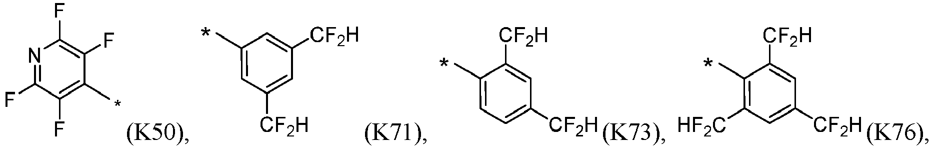 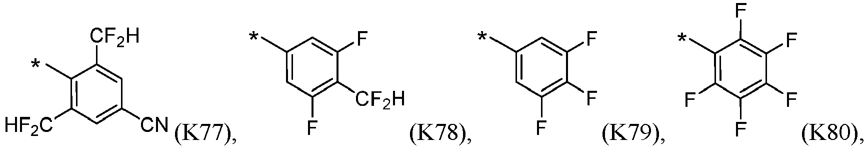 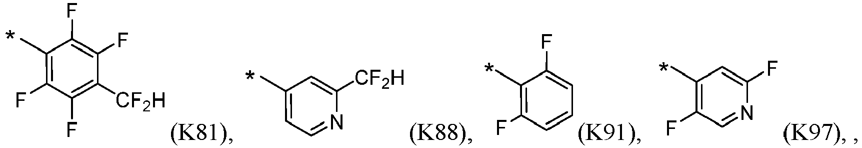 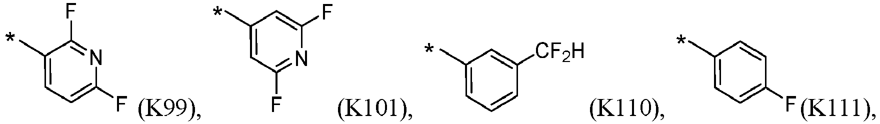 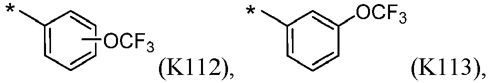 wherein the "*" denotes the binding position.

According to an embodiment of Formula (I) R⁴ of formula (IIb) is selected from the following moieties: 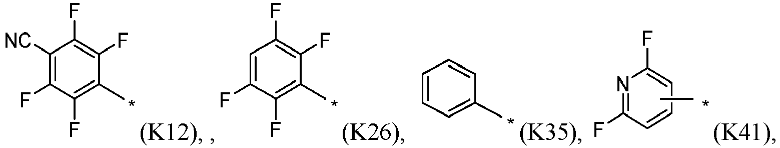 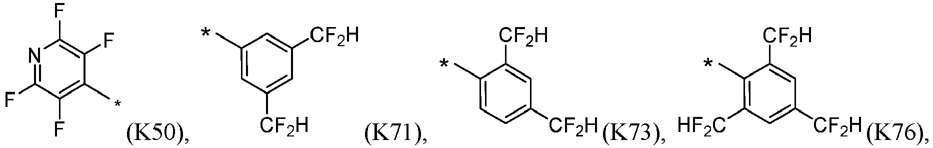 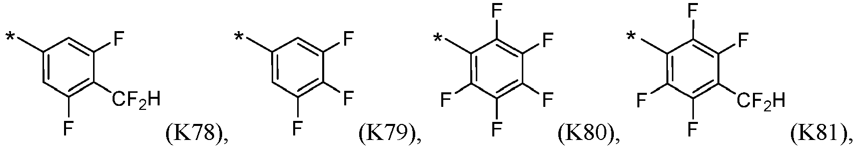 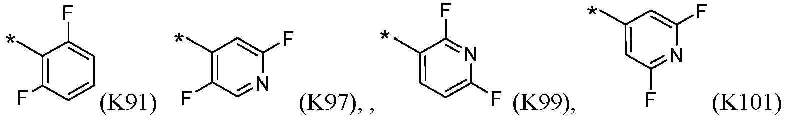 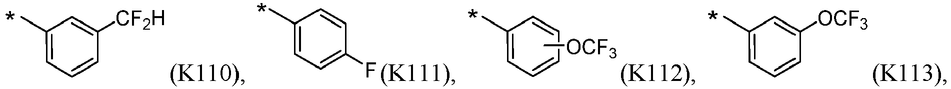 wherein the "*" denotes the binding position.

According to an embodiment of Formula (I), wherein R⁵ of formula (IIb) is selected from CH₃, CF₃, C₂F₅, C₃F₇, C₄F₉, CFH₂, CF₂H, CF₂CF₂H, (CF₂)₂CF₂H, CH(CF₂H)₂, CF(CF₂H)₂, C(CF₂H)₃, CN and the following formulae **L1 to L114:** 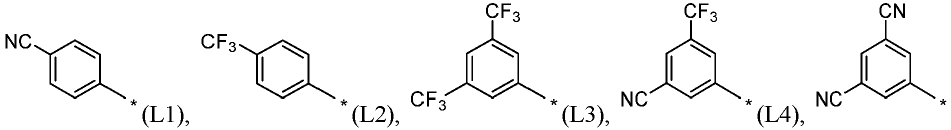 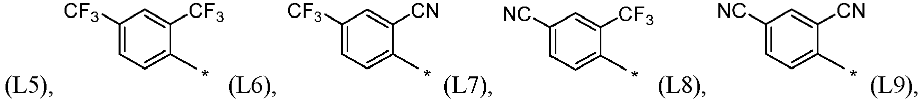 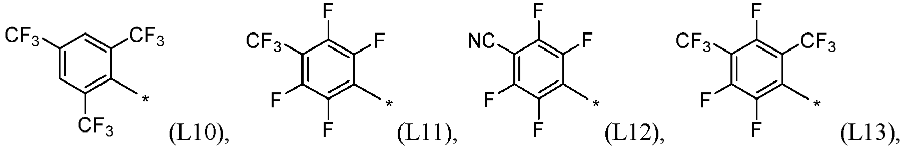 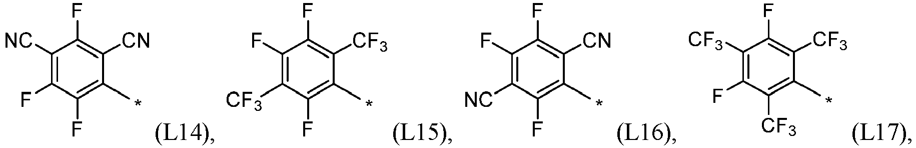 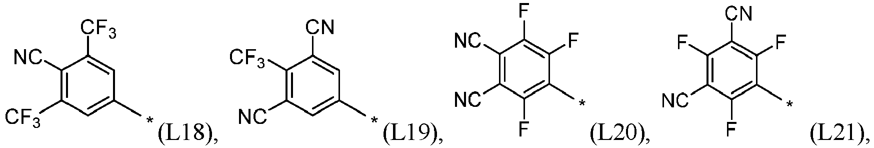 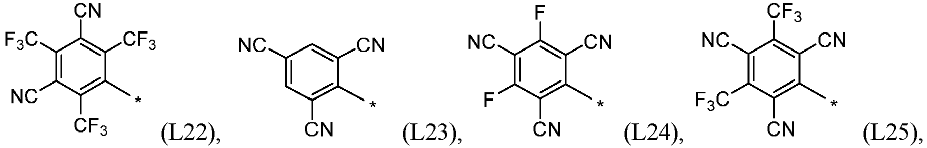 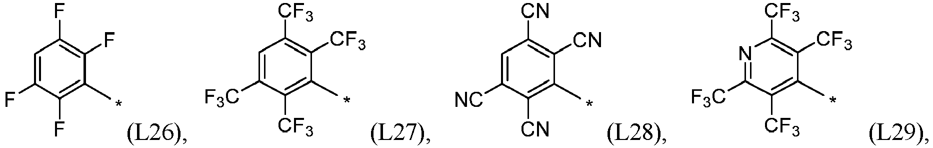 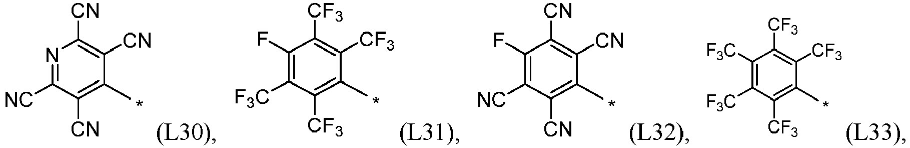 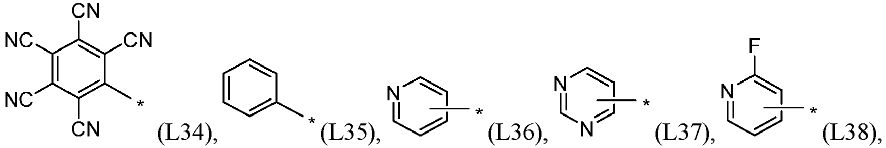 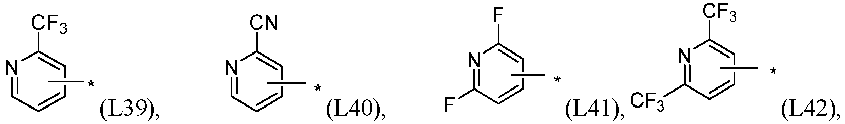 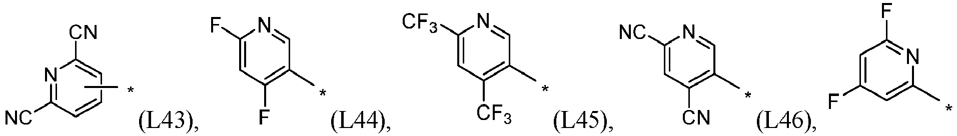 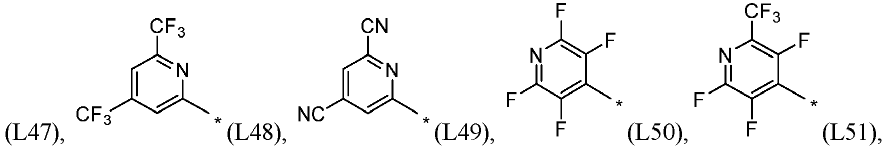 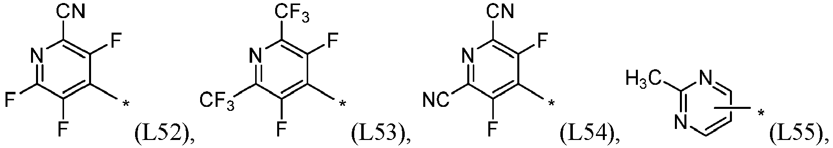 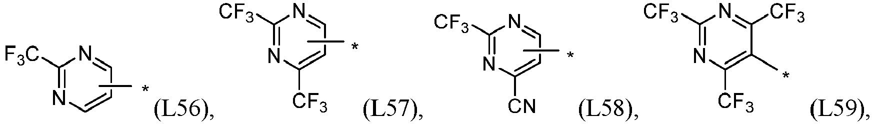 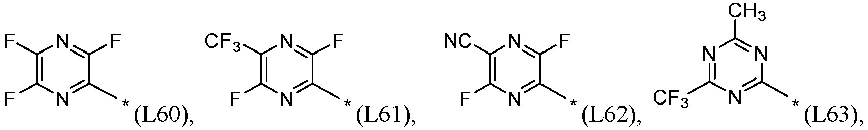 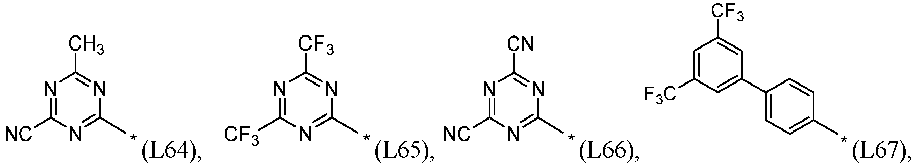 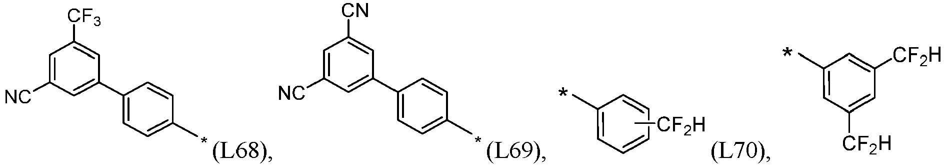 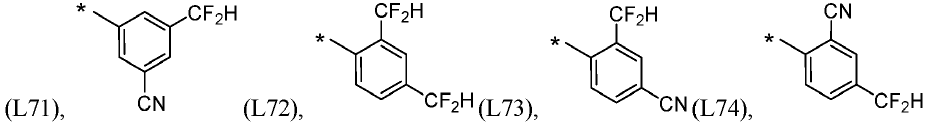 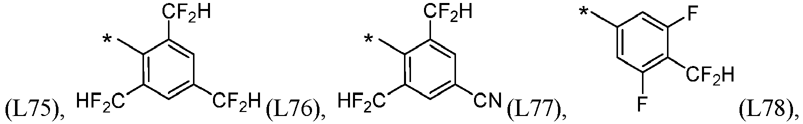 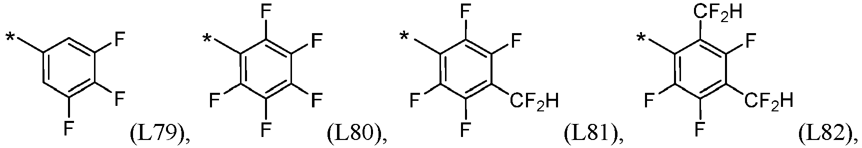 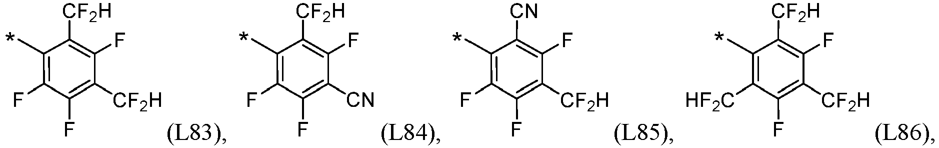 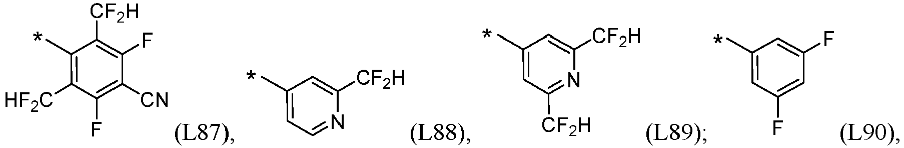 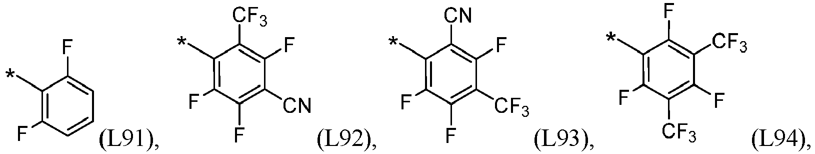 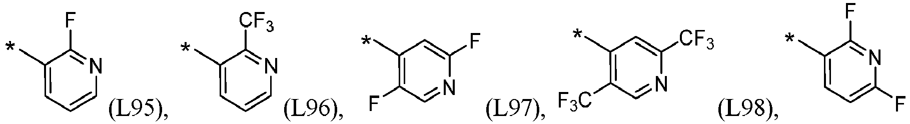 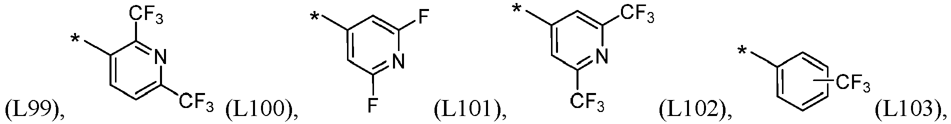 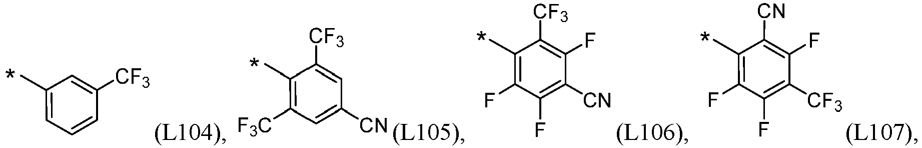 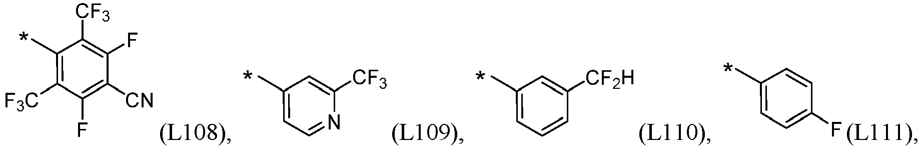 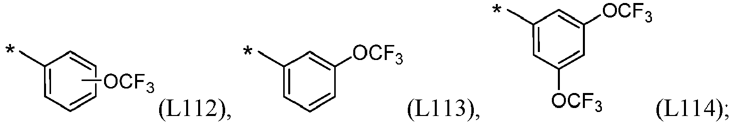 wherein the "*" denotes the binding position.

According to an embodiment of Formula (I), wherein R⁵ of formula (IIb) is selected from CH₃, CF₃, CF₂H, CN, and the following moieties: 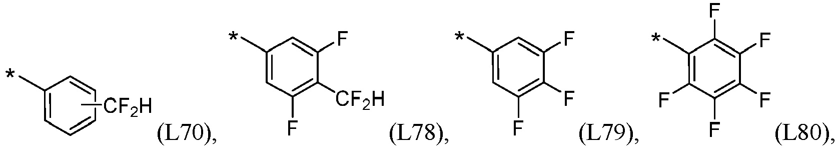 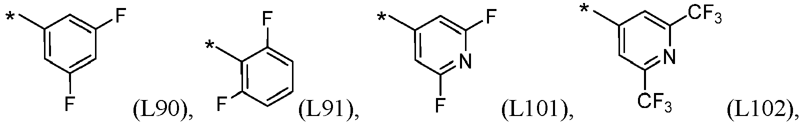 wherein the "*" denotes the binding position.

According to an embodiment of Formula (I), wherein R⁵ of formula (IIb) is selected from CH₃, CF₃, CF₂H,, CN and the following moieties: 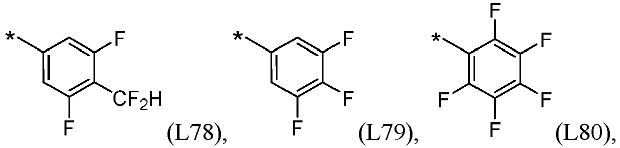 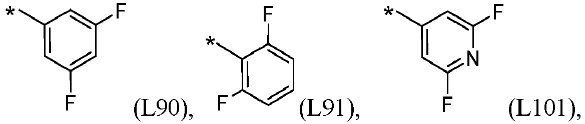 wherein the "*" denotes the binding position.
According to an embodiment of Formula (I), wherein R⁵ of formula (IIb) is selected from CH₃,, CN and CF₂H,

According to one embodiment of Formula (IIb), R⁴ of formula (IIb) is selected from the formulae **K1 to K114**, and R⁵ of formula (IIb) is selected from CH₃, CF₃, C₂F₅, C₃F₇, C₄F₉, CFH₂, CF₂H, CF₂CF₂H, (CF₂)₂CF₂H, CH(CF₂H)₂, CF(CF₂H)₂, C(CF₂H)₃, CN, and the formulae **L1 to L114.**

According to an embodiment of Formula (I) R⁶ of formula (IIe) is selected from **M1 to M114:** 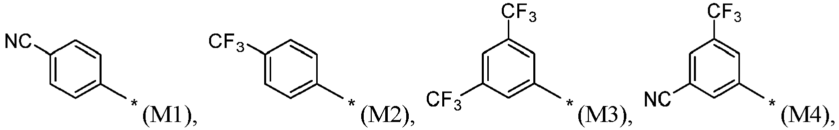 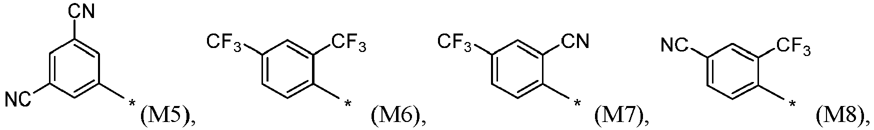 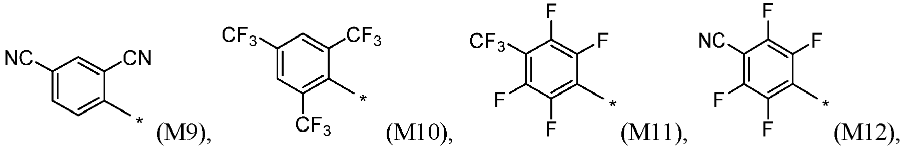 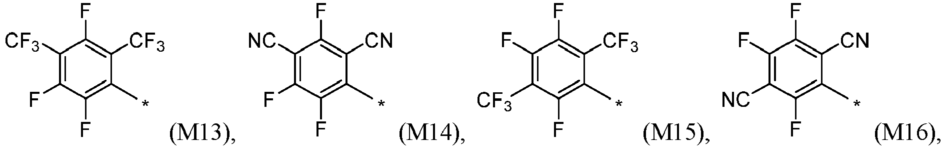 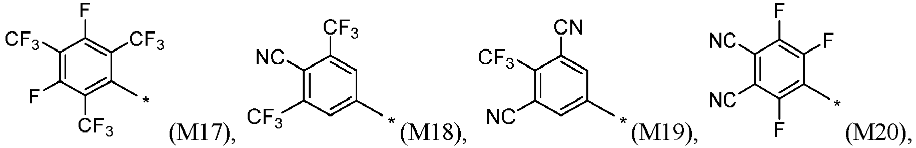 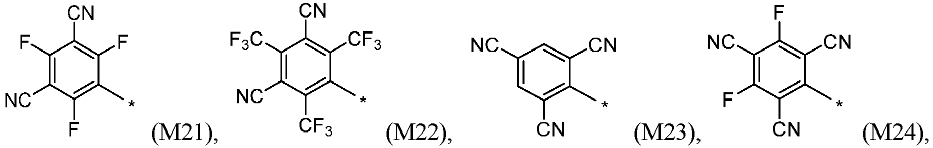 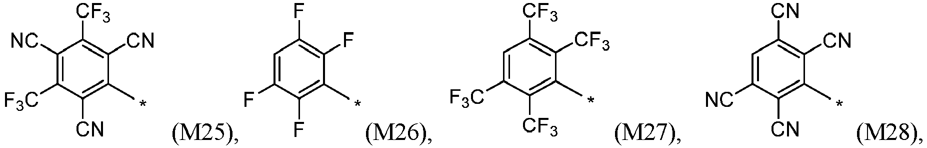 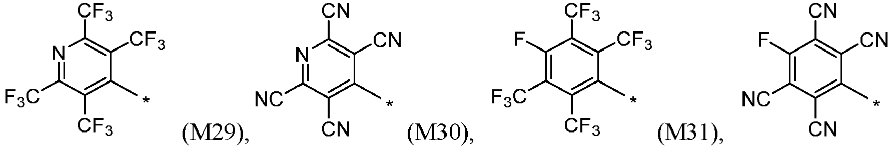 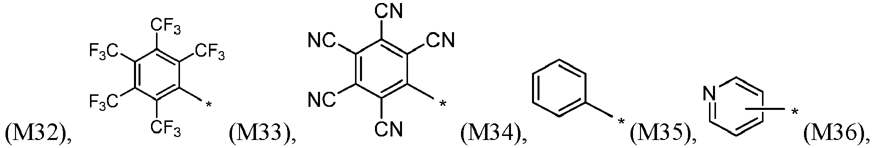 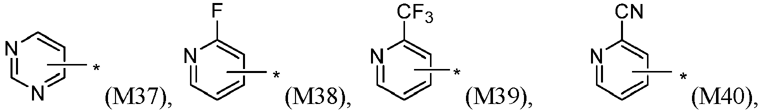 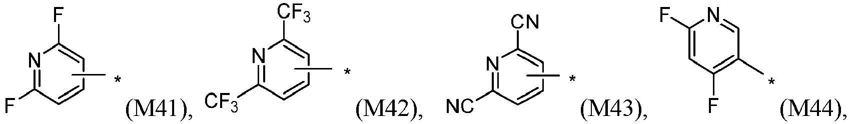 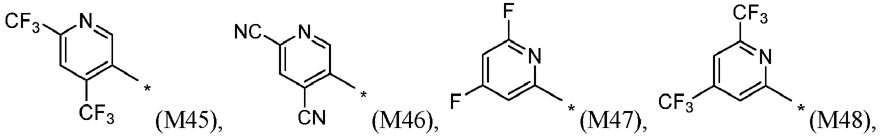 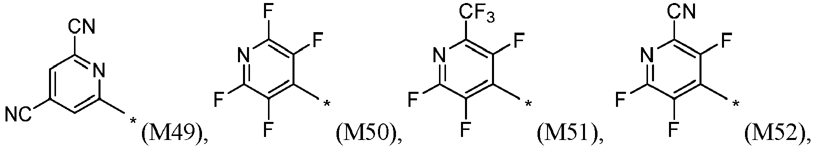 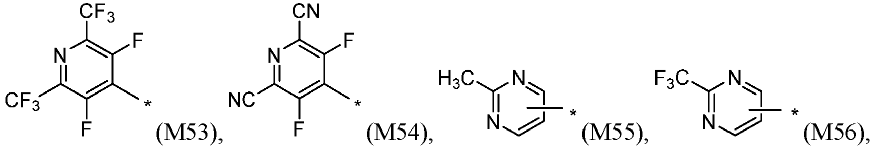 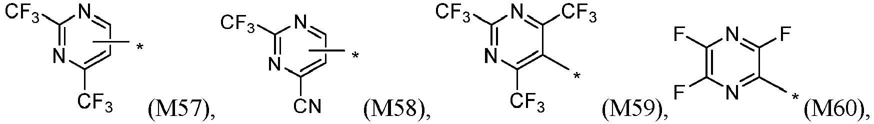 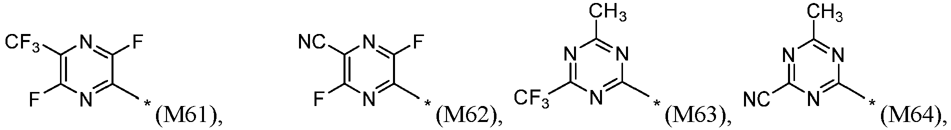 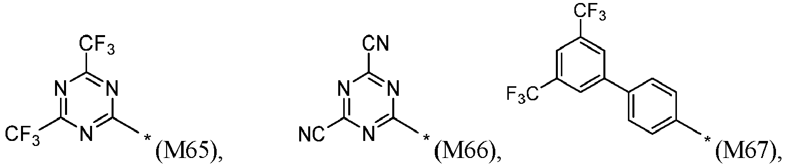 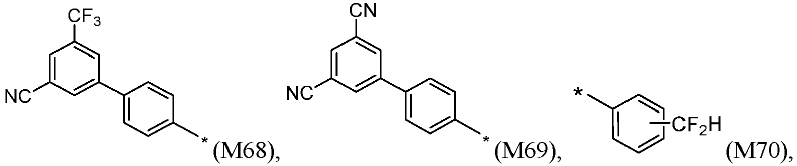 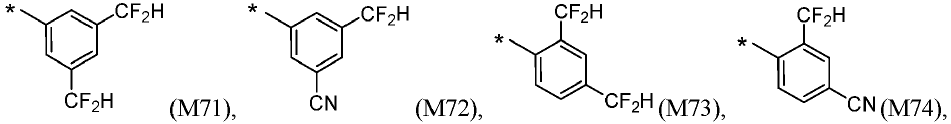 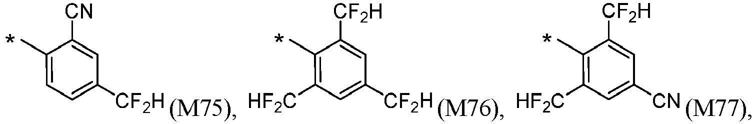 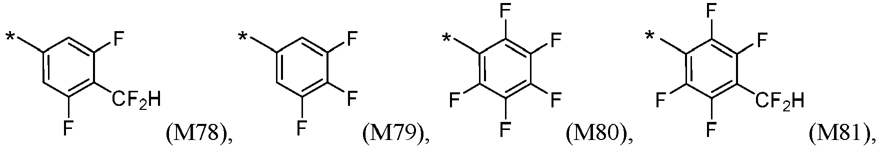 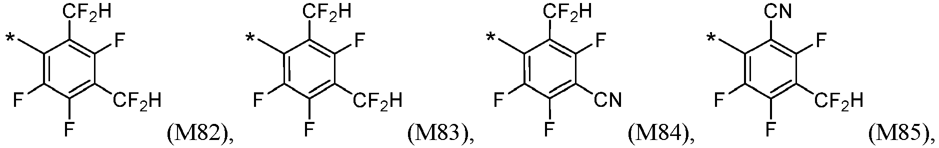 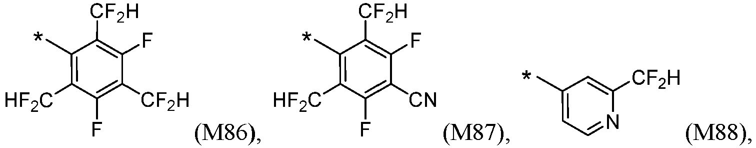 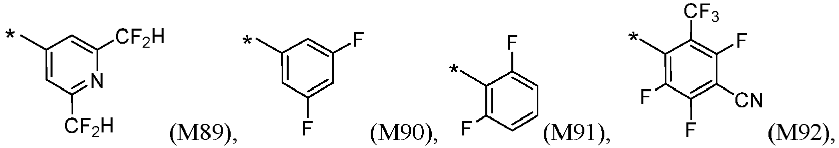 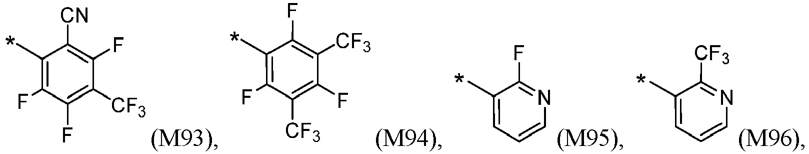 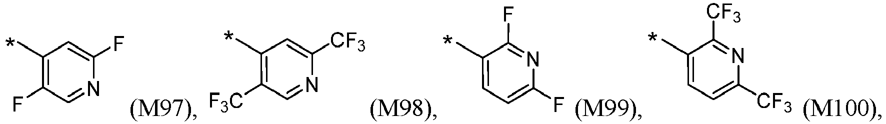 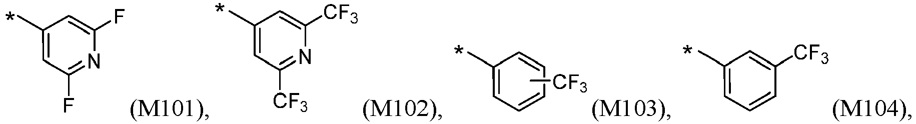 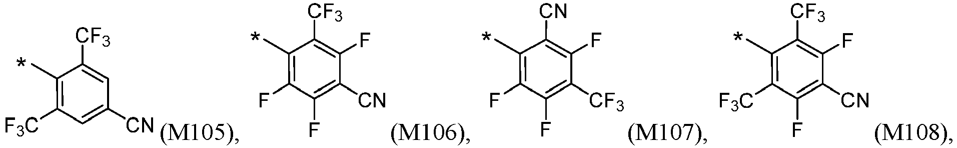 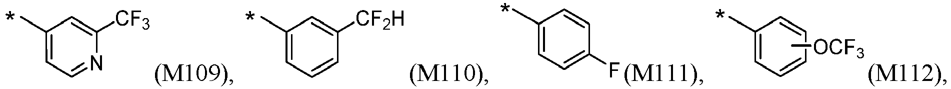 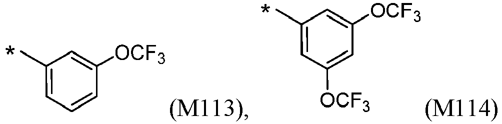 wherein the "*" denotes the binding position.

According to an embodiment of Formula (I) R⁶ of formula (IIe) is selected from the following moieties: 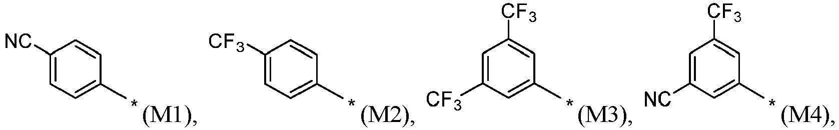 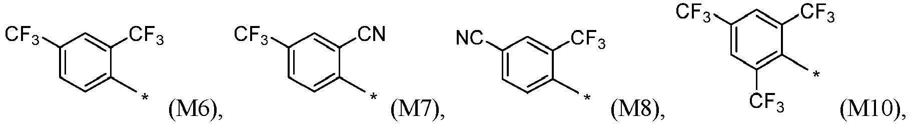 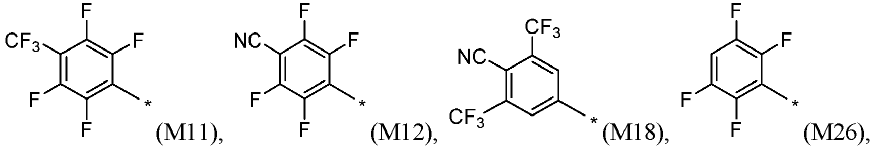 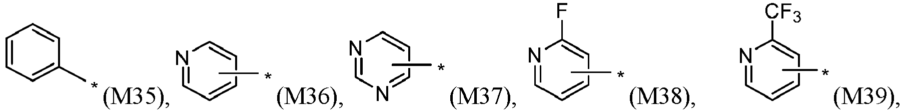 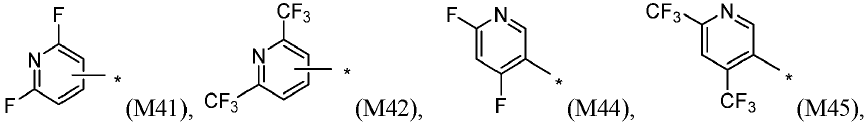 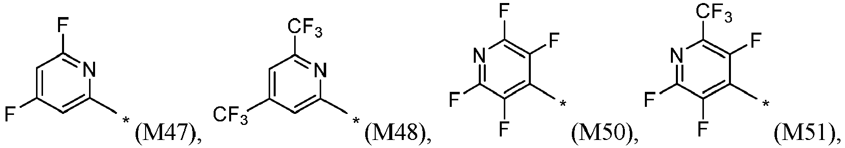 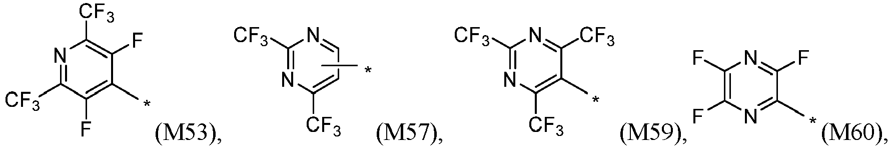 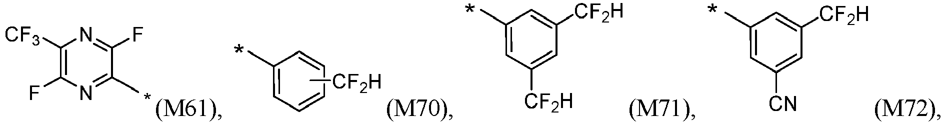 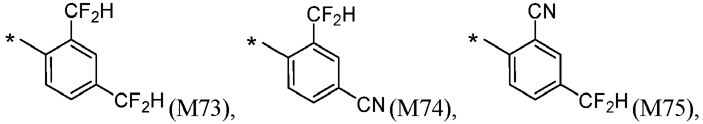 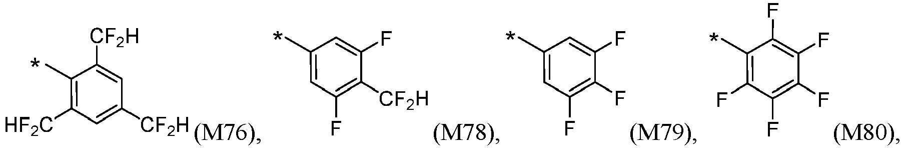 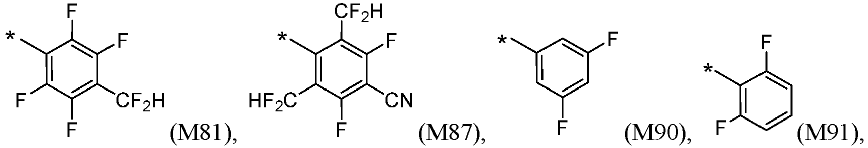 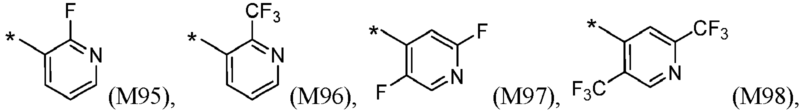 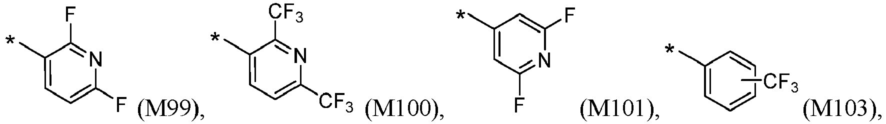 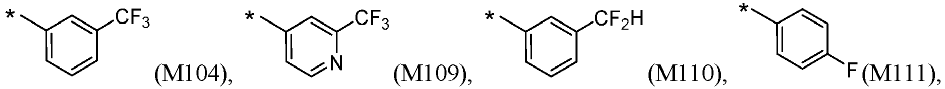 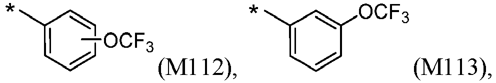 wherein the "*" denotes the binding position.

According to an embodiment of Formula (I) R⁶ of formula (IIe) is selected from the following moieties: 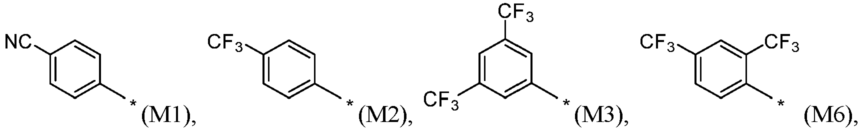 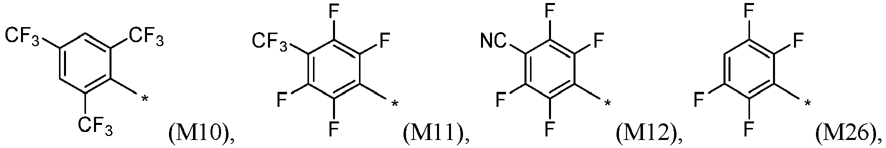 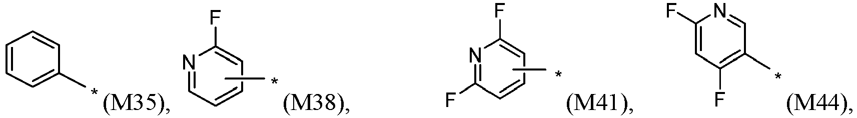 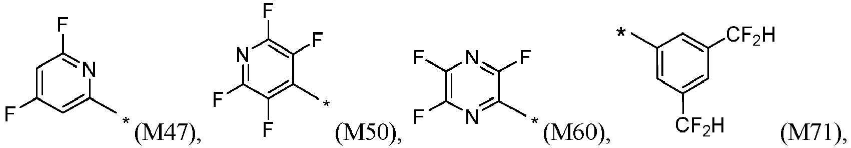 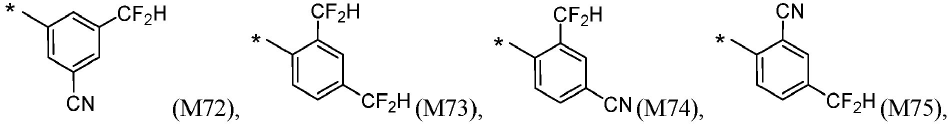 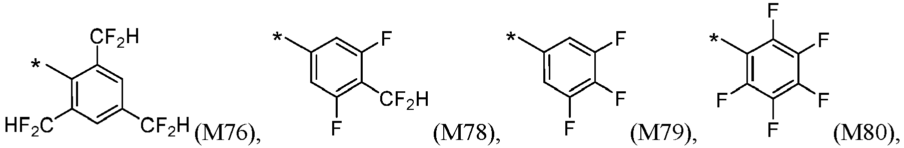 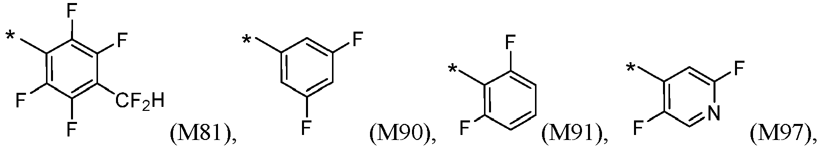 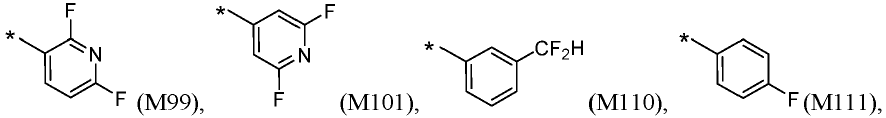 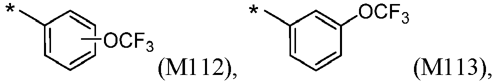 wherein the "*" denotes the binding position.

According to an embodiment of Formula (I) R⁶ of formula (IIe) is selected from the following moieties: 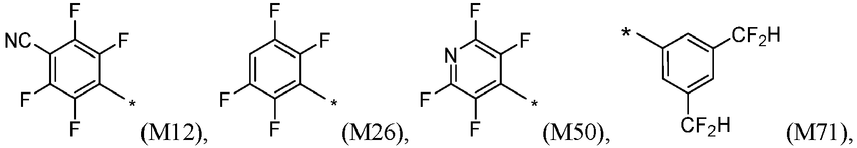 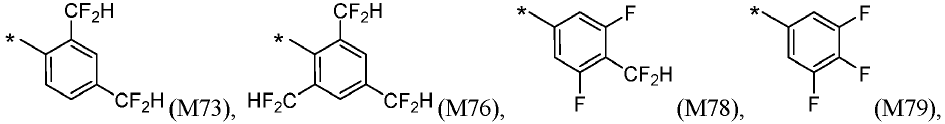 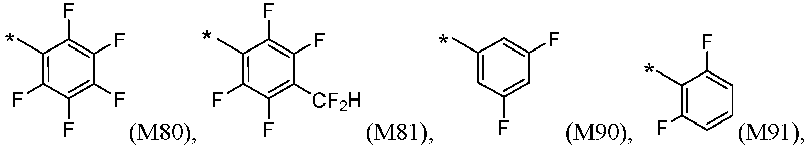 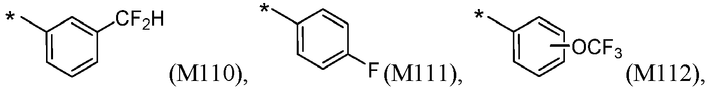 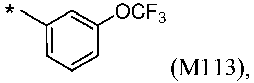 wherein the "*" denotes the binding position.

According to one embodiment, Ligand L is selected from formulae (IIb) to (IIe); wherein
R^{a} of formulae (IIb) to (IIe) is selected from CH₃, CF₃, C₂F₅, C₃F₇, C₄F₉, CFH₂, CF₂H, CF₂CF₂H, (CF₂)₂CF₂H, CH(CF₂H)₂, CF(CF₂H)₂, C(CF₂H)₃ and formulae **J1to J114;**
R⁴ of formula (IIb) is selected from the formulae **K1 to K114,** and R⁵ of formula (IIb) is selected from CH₃, CF₃, C₂F₅, C₃F₇, C₄F₉, CFH₂, CF₂H, CF₂CF₂H, (CF₂)₂CF₂H, CH(CF₂H)₂, CF(CF₂H)₂, C(CF₂H)₃, CN, and the formulae **L1 to L114**;
R⁶ of formula (IIe) is selected from formulae **M1 to M114.**

(IIg). According to one embodiment, Ligand L is selected from formulae (IIb), (IIe), (IIf) and/or

According to one embodiment, Ligand L is selected from formulae (IIb), (IIe), (IIf) and/or (IIg); wherein
R^{a} of formulae (IIb), (IIe), (IIf) and/or (IIg) is selected from CH₃, CF₃, C₂F₅, C₃F₇, C₄F₉, CFH₂, CF₂H, CF₂CF₂H, (CF₂)₂CF₂H, CH(CF₂H)₂, CF(CF₂H)₂, C(CF₂H)₃ and formulae **J1 to J114;**
R⁴ of formula (IIb) is selected from formulae **K1 to K112,** and R⁵ of formula (IIb) is selected from CH₃, CF₃, C₂F₅, C₃F₇, C₄F₉, CFH₂, CF₂H, CF₂CF₂H, (CF₂)₂CF₂H, CH(CF₂H)₂, CF(CF₂H)₂, C(CF₂H)₃, CN, phenyl and the formulae **L1 to L114**;
R⁶ of formula (IIe) is selected from formulae **M1 to M114;**
R²¹ to R²⁴ of formulae (IIf) and (IIg) are independently selected from H, D, halogen, Cl, F, CN, C₁ to C₁₂ alkyl, C₁ to C₁₂ alkoxy, partially or perfluorinated C₁ to C₁₂ alkyl, CF₃, CF₂R, CFR₂, CF₂H, CFH₂, or a combination thereof, wherein R is H or D, preferably H; more preferred R²¹ to R²⁴ are independently selected from H, D, F, CF₃, CF₂R, CFR₂, CF₂H, CFH₂, or a combination thereof, wherein R is H or D, preferably H.

According to one embodiment, Ligand L is selected from formulae (IIc), (IId), (IIe), (IIf) and/or (IIg).

According to one embodiment, Ligand L is selected from formulae (IIc), (IId), (IIe), (IIf) and/or (IIg), wherein
R^{a} of formulae (IIe), (IIf) and/or (IIg) is selected from CH₃, CF₃, C₂F₅, C₃F₇, C₄F₉, CFH₂, CF₂H, CF₂CF₂H, (CF₂)₂CF₂H, CH(CF₂H)₂, CF(CF₂H)₂, C(CF₂H)₃ and formulae J1 to J114;
R⁶ of formula (IIe) is selected from formulae **M1 to M114**;
R²¹ to R²⁴ of formulae (IIf) and (IIg) are independently selected from H, D, halogen, Cl, F, CN, C₁ to C₁₂ alkyl, C₁ to C₁₂ alkoxy, partially or perfluorinated C₁ to C₁₂ alkyl, CF₃, CF₂R, CFR₂, CF₂H, CFH₂, or a combination thereof, wherein R is H or D, preferably H; more preferred R²¹ to R²⁴ are independently selected from H, D, F, CF₃, CF₂R, CFR₂, CF₂H, CFH₂, or a combination thereof, wherein R is H or D, preferably H.

According to one embodiment, Ligand L is selected from formulae (IIe).

According to one embodiment, Ligand L is selected from formulae (IIe), wherein
R⁶ of formula (IIe) is selected from formulae **M1 to M114.**

According to one embodiment, Ligand L is selected from formulae (IIf) and/or (IIg); wherein
R^{a} of formulae (IIf) and/or (IIg) is selected from CH₃, CF₃, C₂F₅, C₃F₇, C₄F₉, CFH₂, CF₂H, CF₂CF₂H, (CF₂)₂CF₂H, CH(CF₂H)₂, CF(CF₂H)₂, C(CF₂H)₃ and formulae **J1 to J112**;
R²¹ to R²⁴ of formulae (IIf) and (IIg) are independently selected from H, D, halogen, Cl, F, CN, C₁ to C₁₂ alkyl, C₁ to C₁₂ alkoxy, partially or perfluorinated C₁ to C₁₂ alkyl, CF₃, CF₂R, CFR₂, CF₂H, CFH₂, or a combination thereof, wherein R is H or D, preferably H; more preferred R²¹ to R²⁴ are independently selected from H, D, F, CF₃, CF₂R, CFR₂, CF₂H, CFH₂, or a combination thereof, wherein R is H or D, preferably H.

According to an embodiment, Ligand L of formula (II) is selected from A1 to A217, B1 to B117, C1 to C31, D1 to D83, E1 to E173:

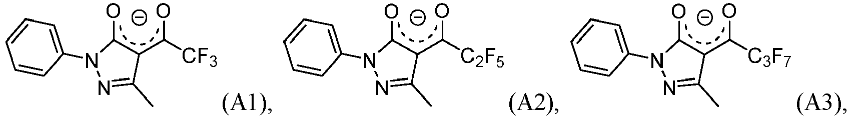

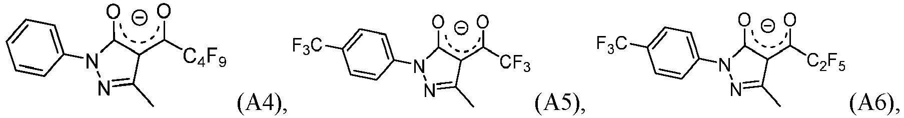

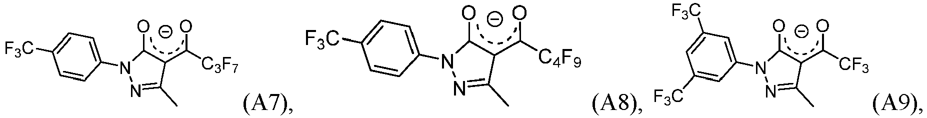

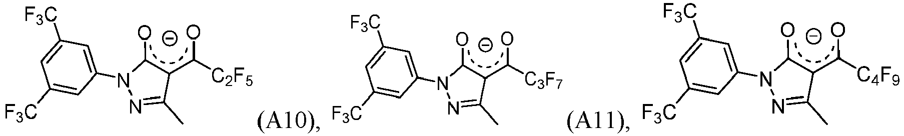

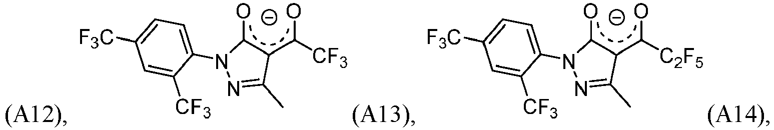

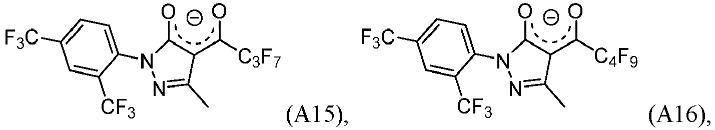

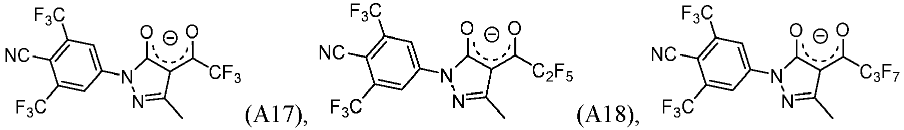

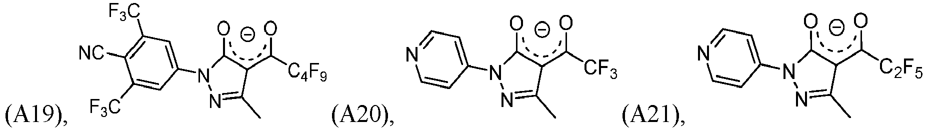

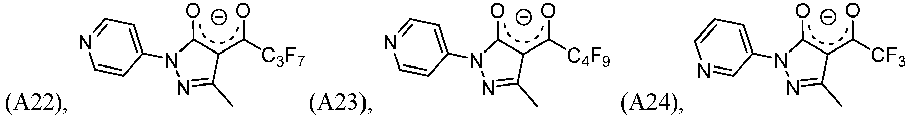

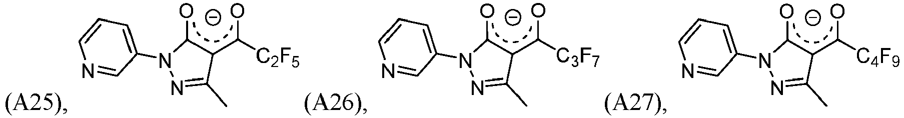

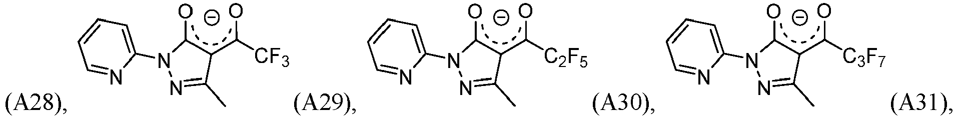

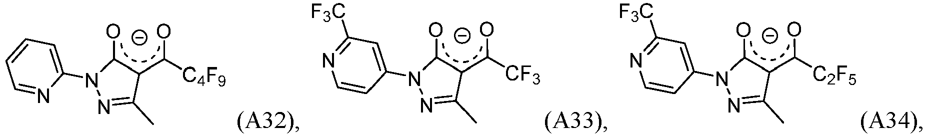

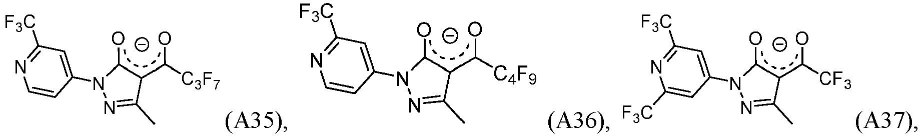

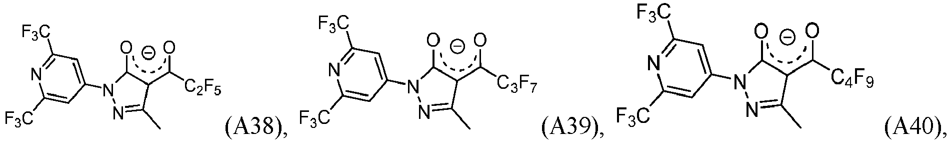

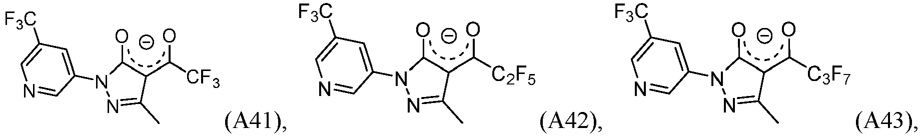

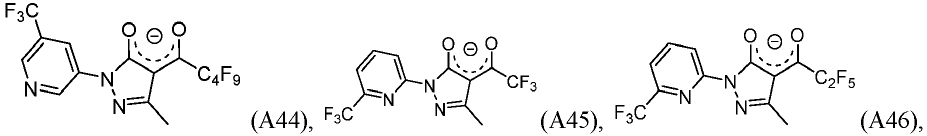

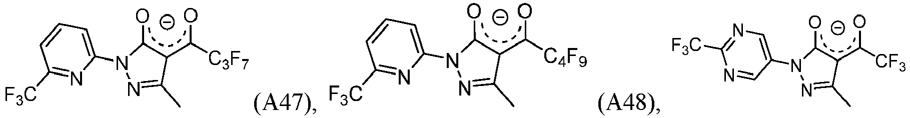

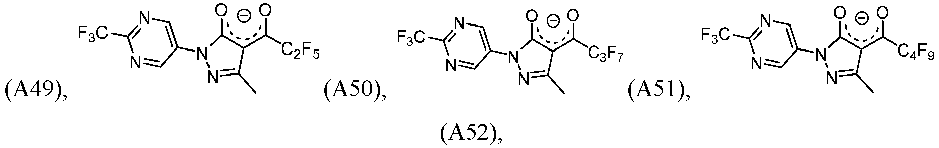

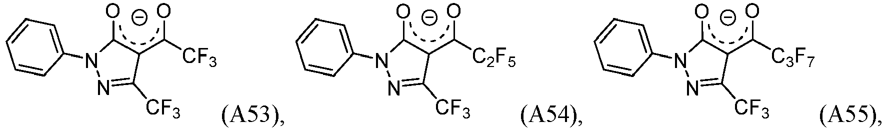

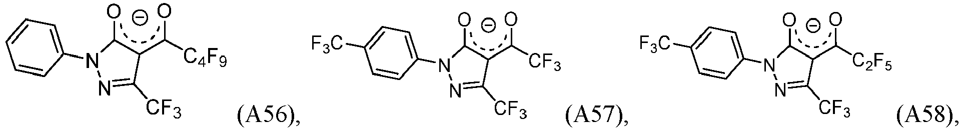

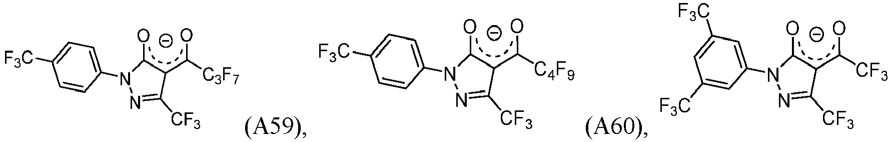

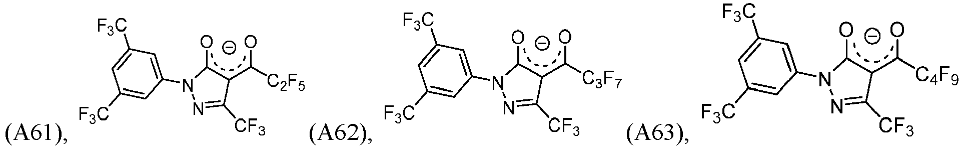

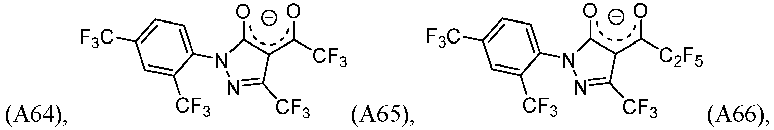

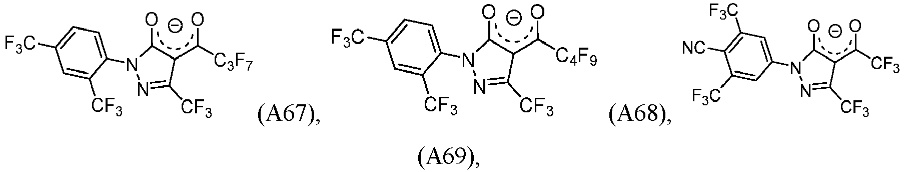

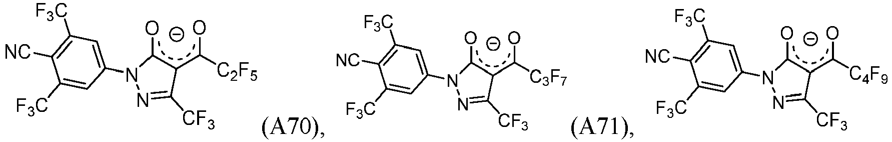

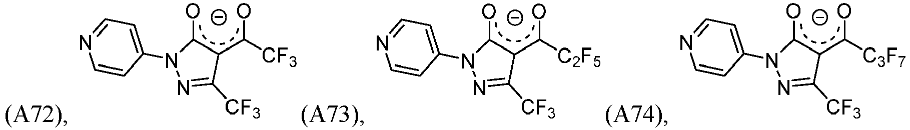

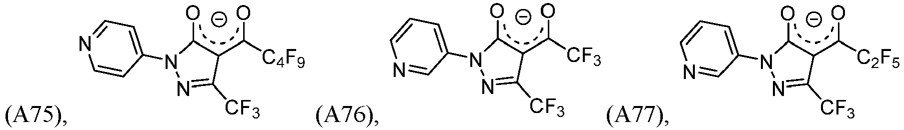

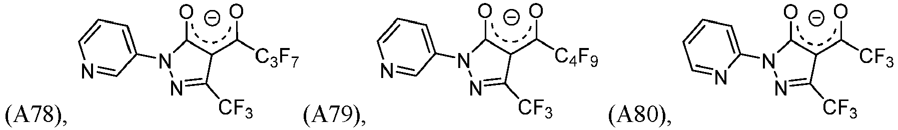

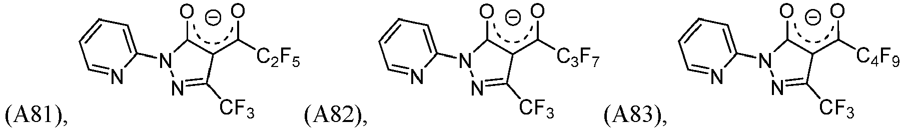

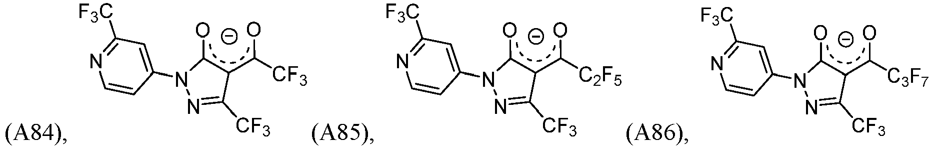

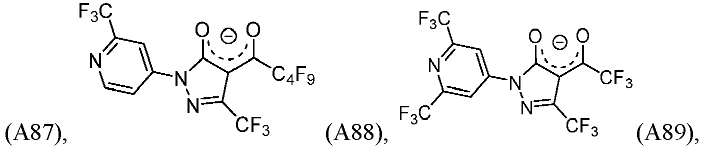

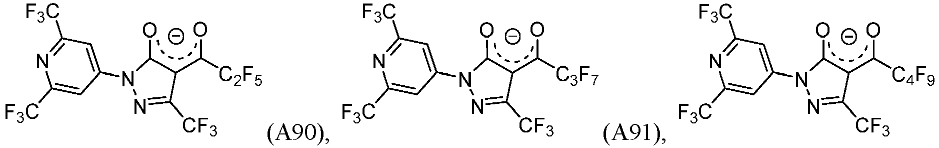

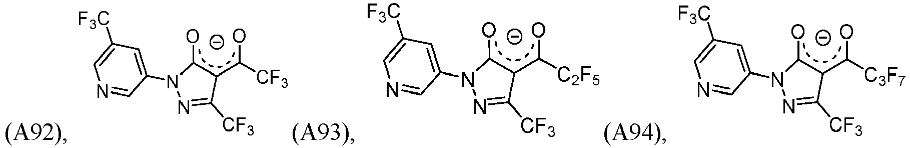

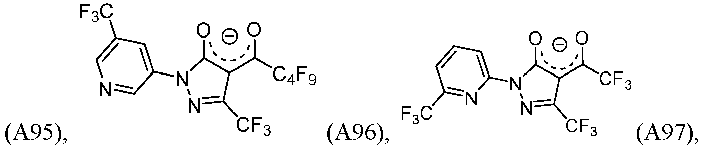

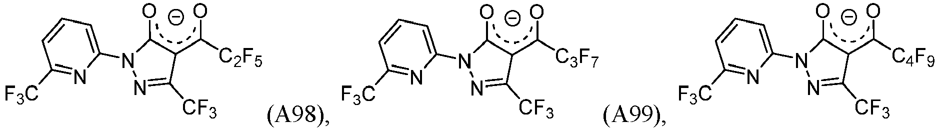

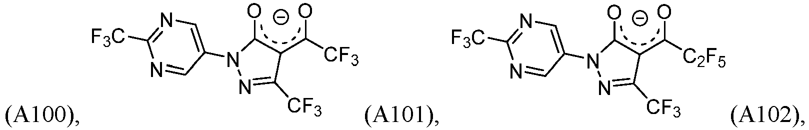

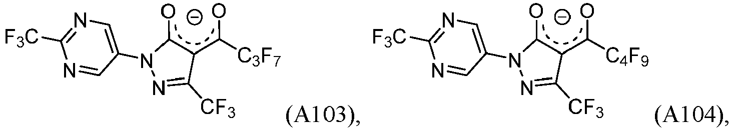

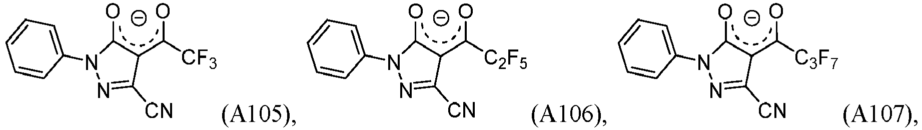

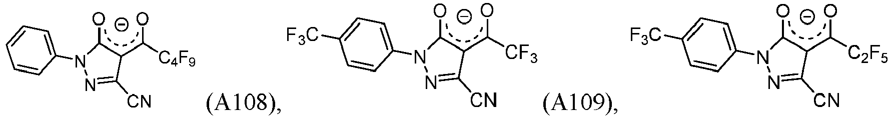

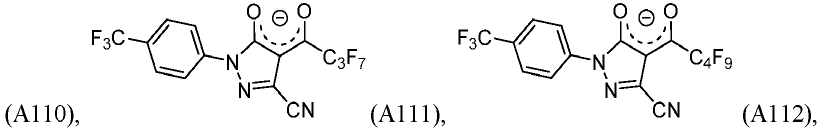

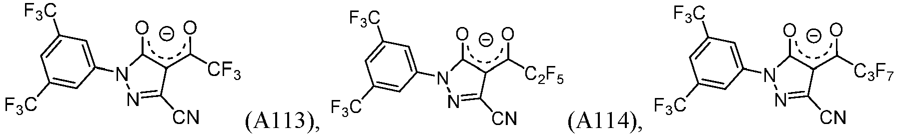

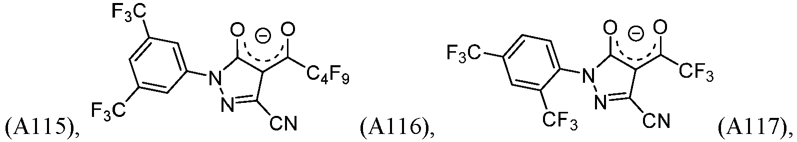

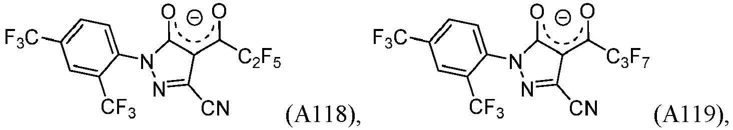

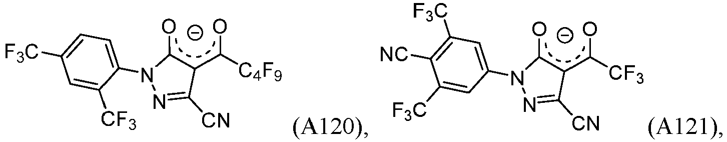

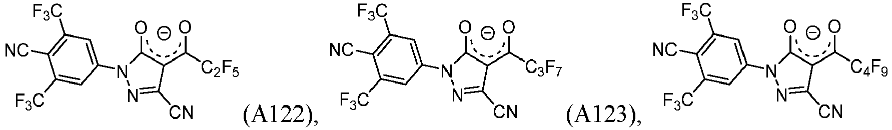

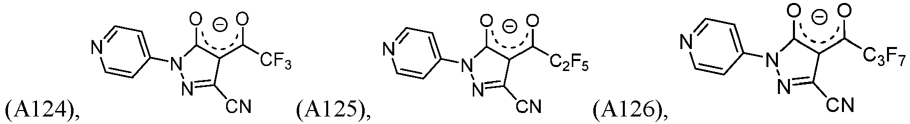

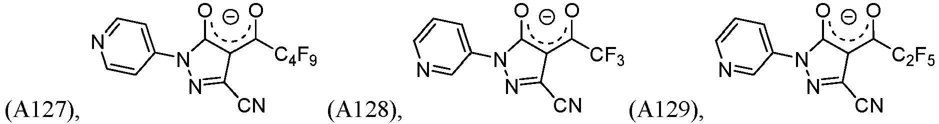

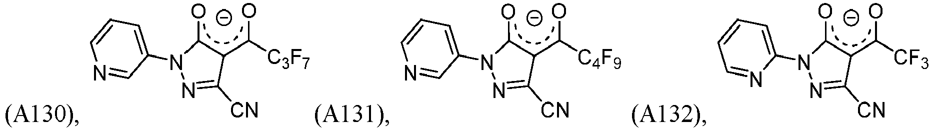

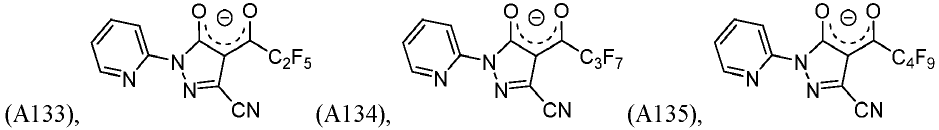

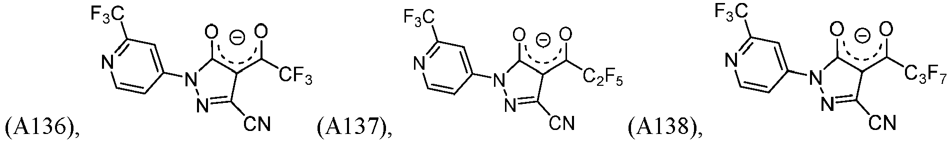

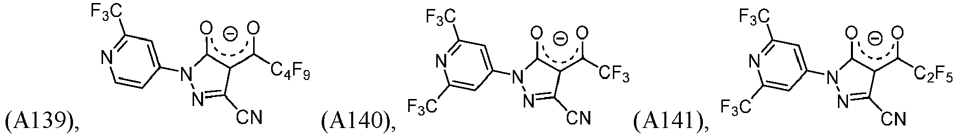

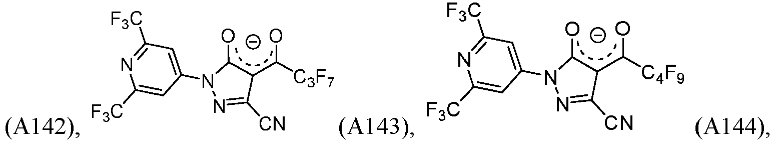

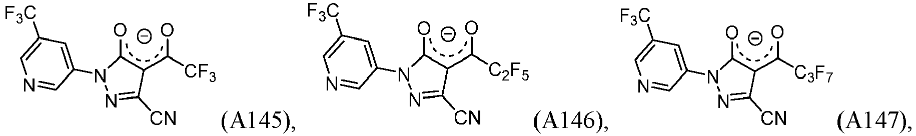

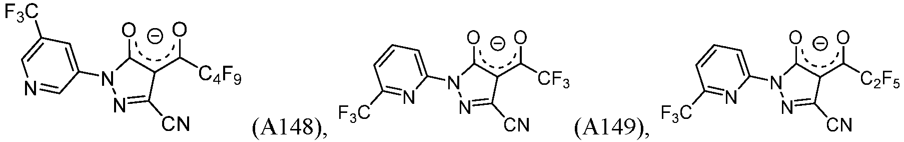

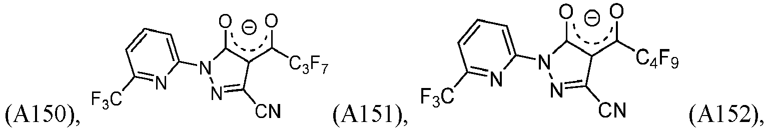

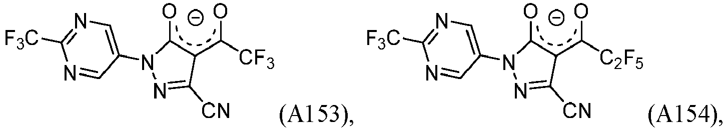

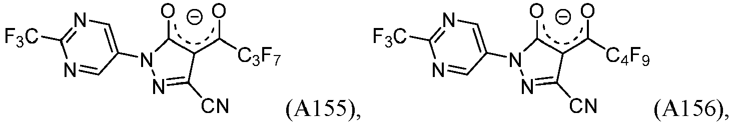

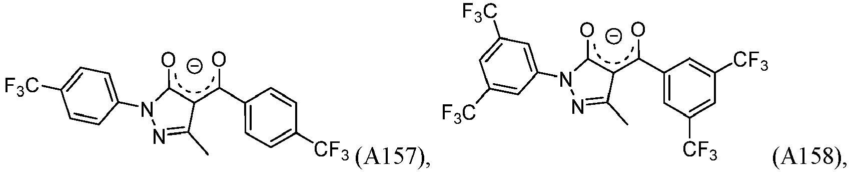

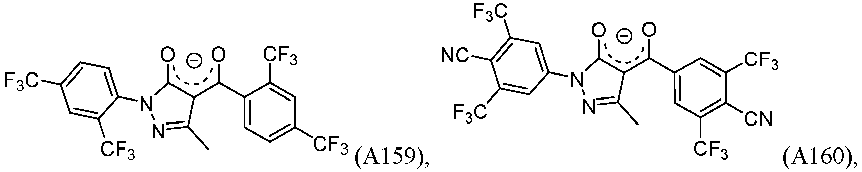

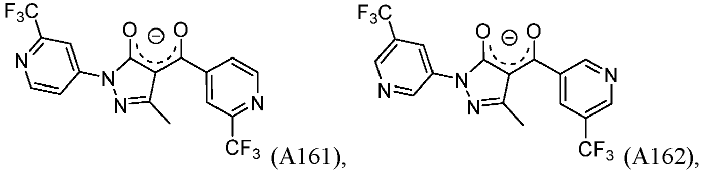

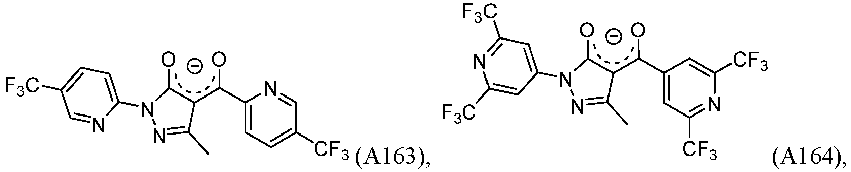

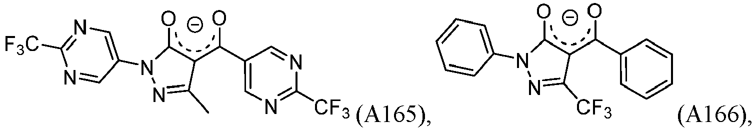

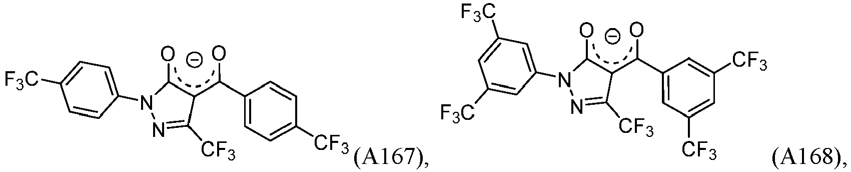

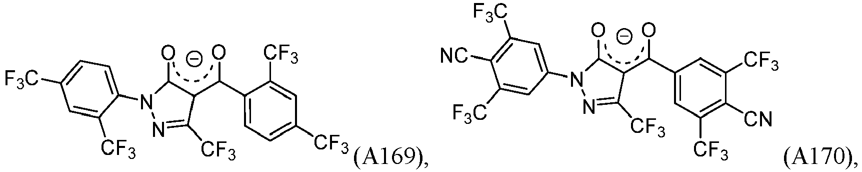

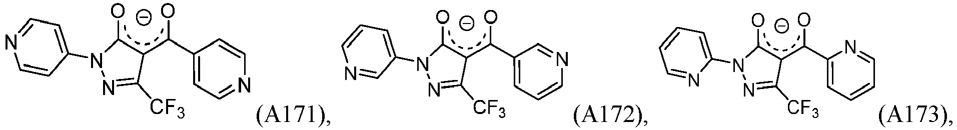

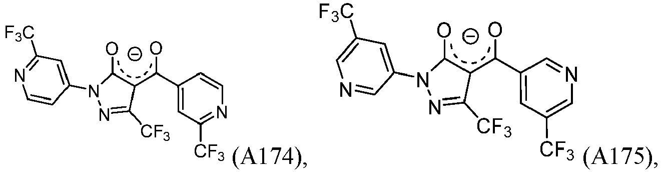

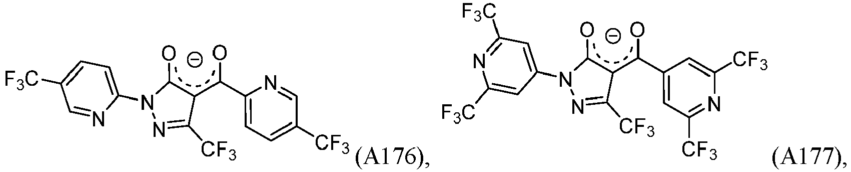

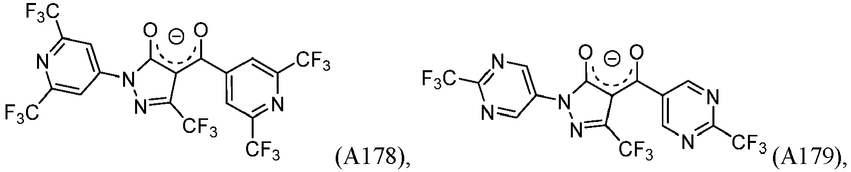

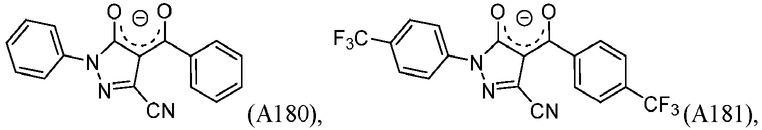

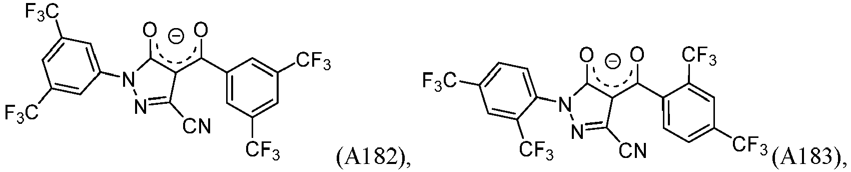

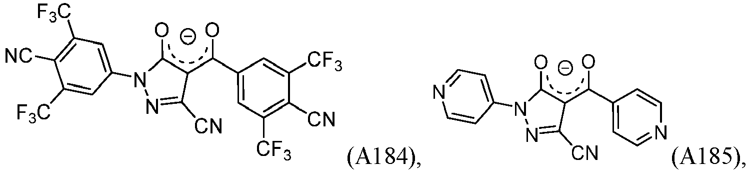

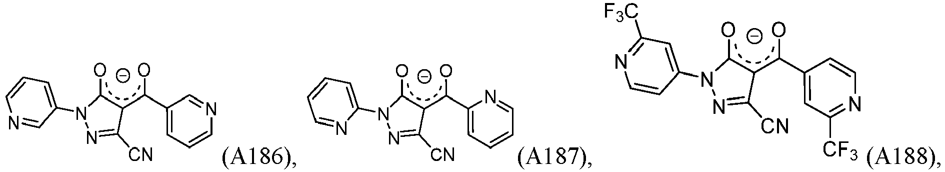

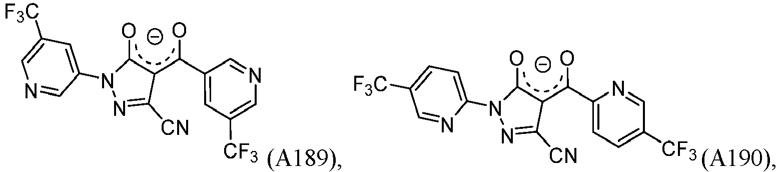

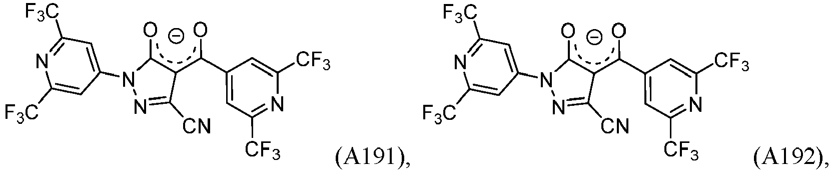

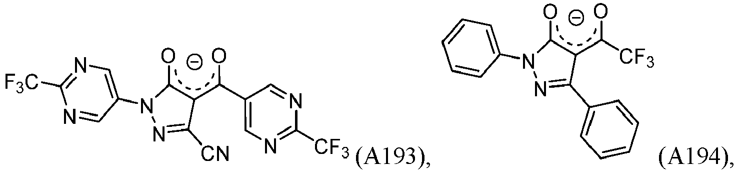

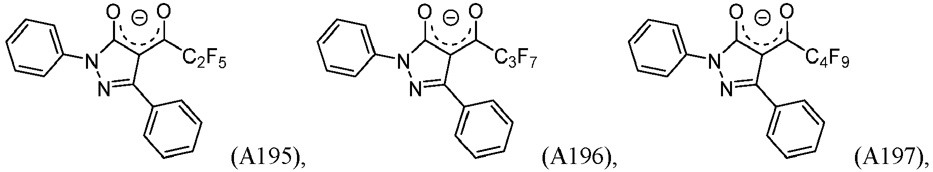

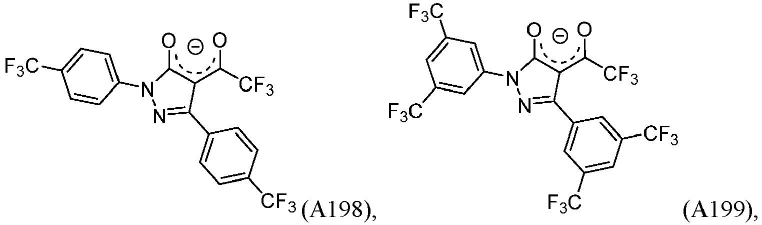

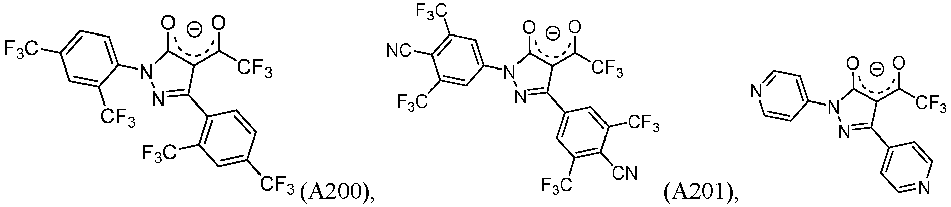

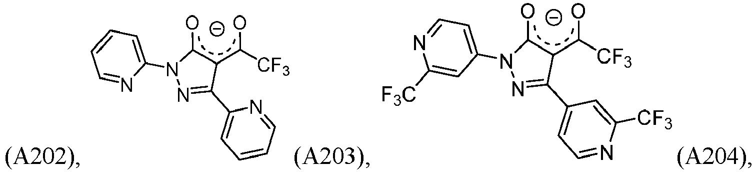

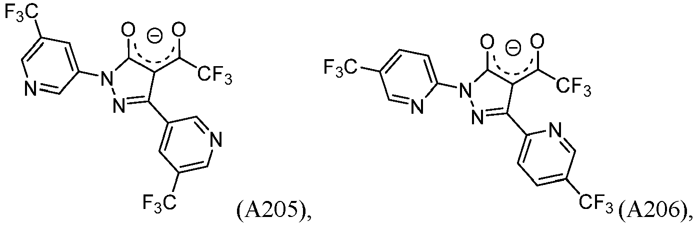

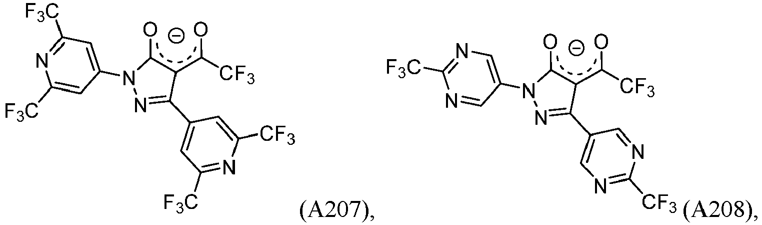

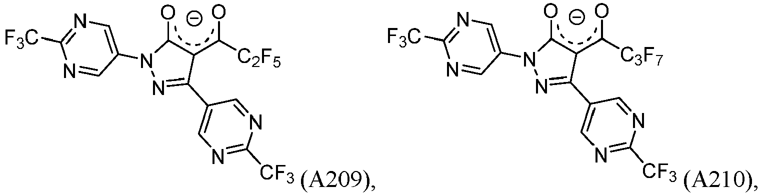

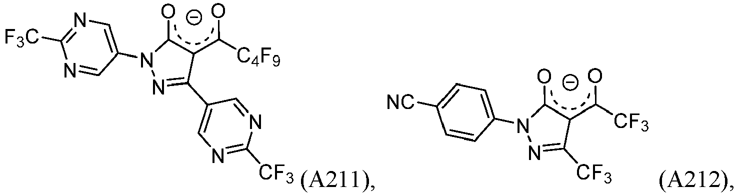

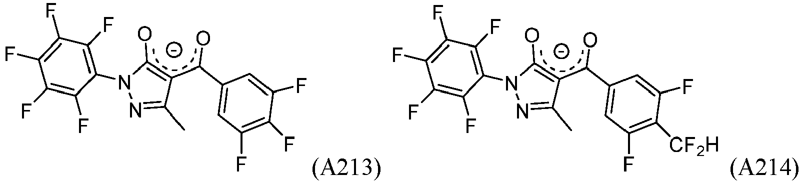

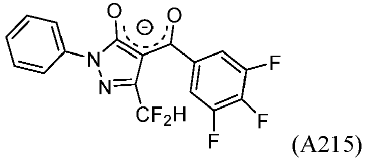

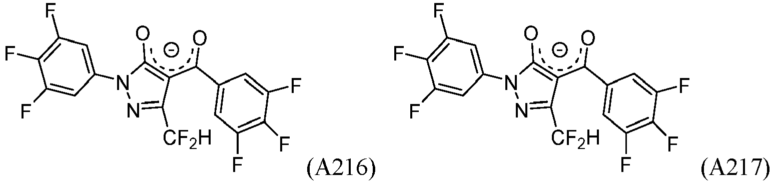

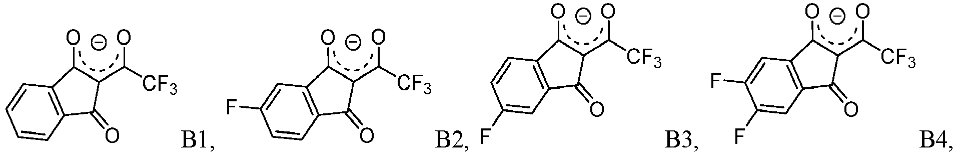

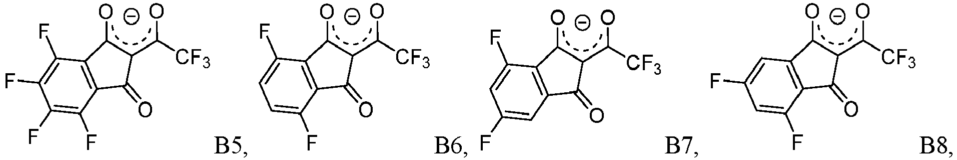

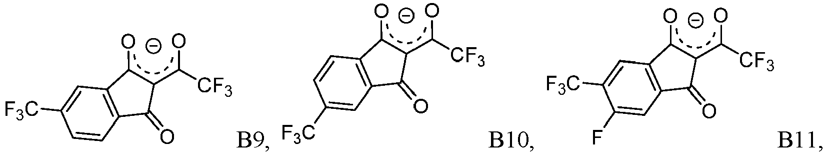

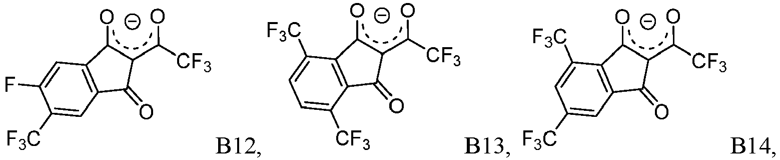

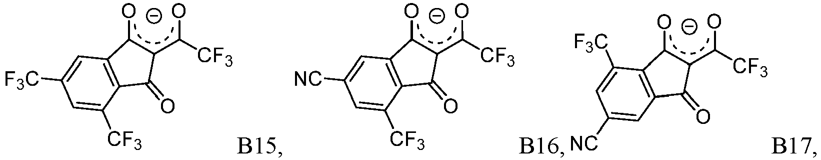

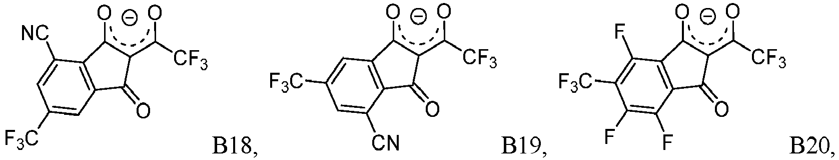

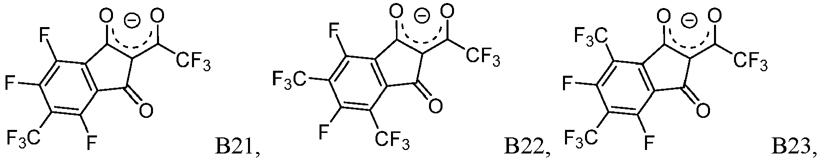

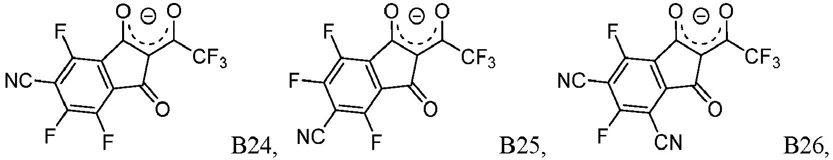

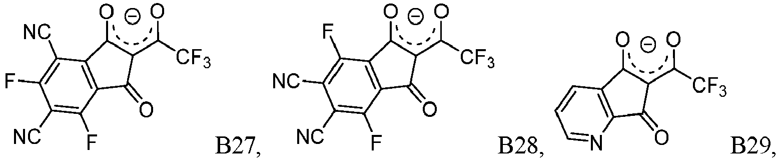

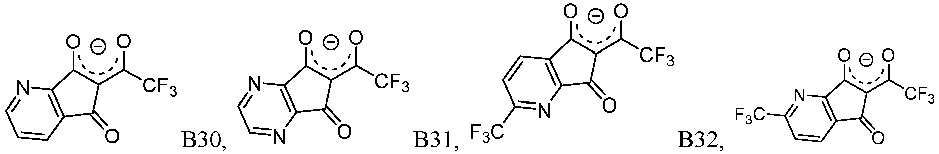

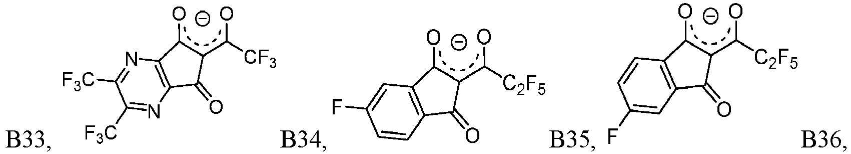

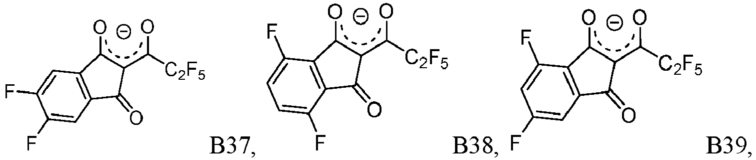

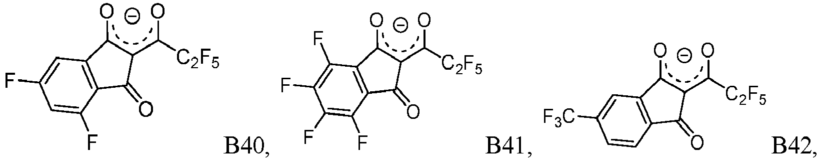

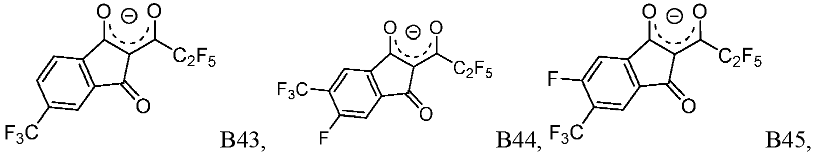

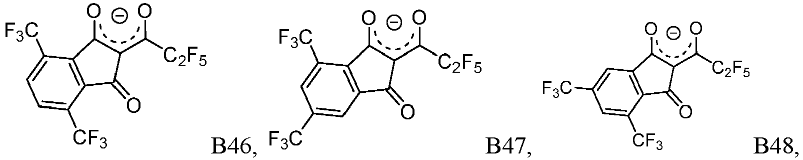

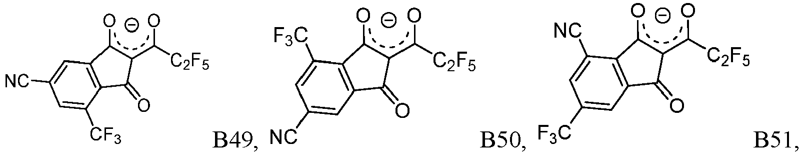

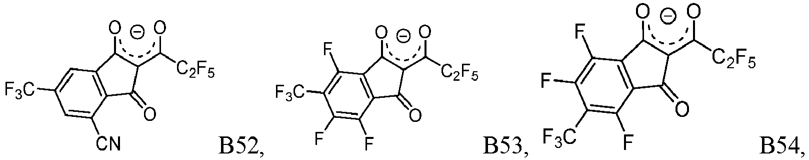

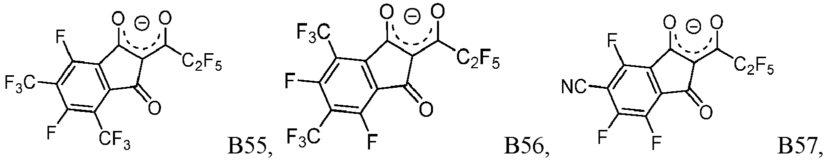

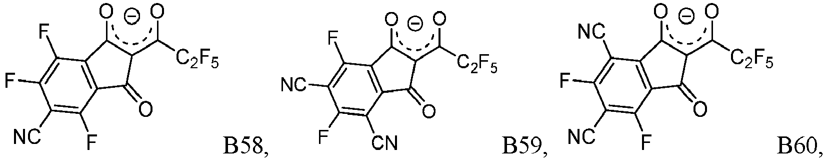

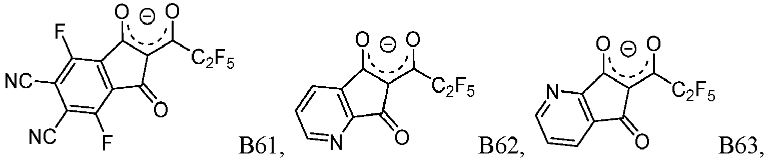

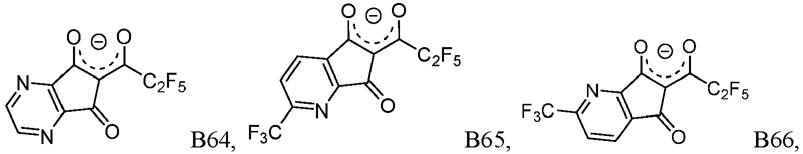

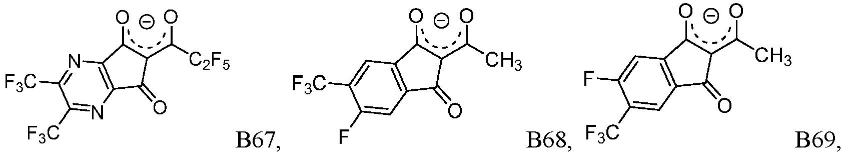

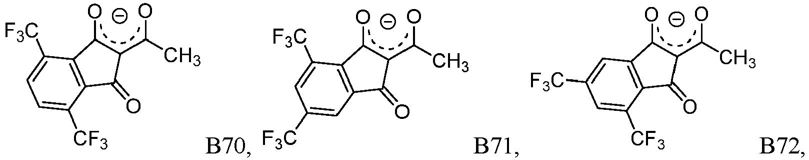

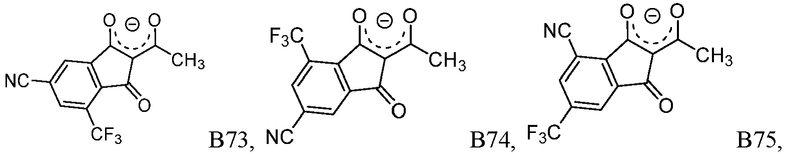

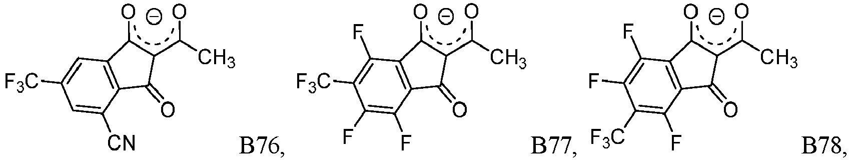

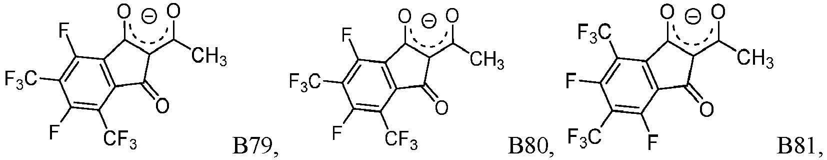

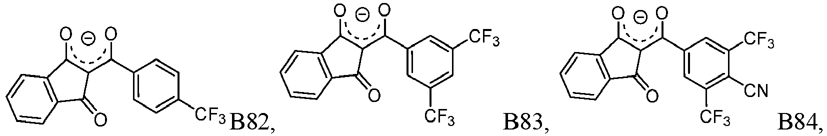

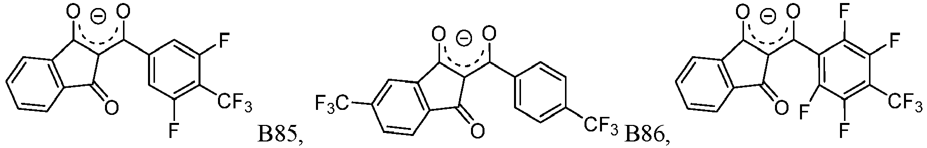

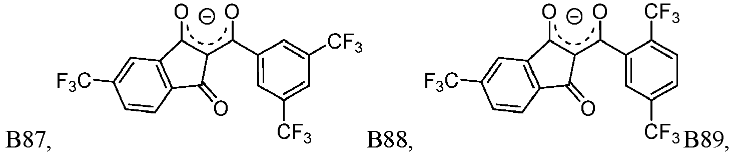

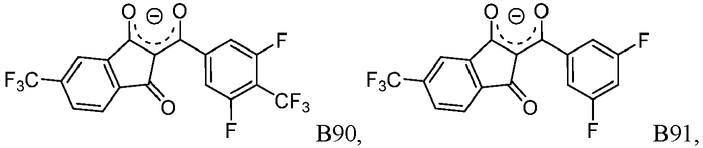

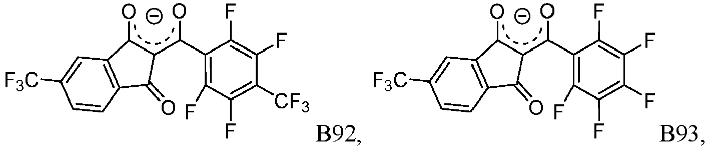

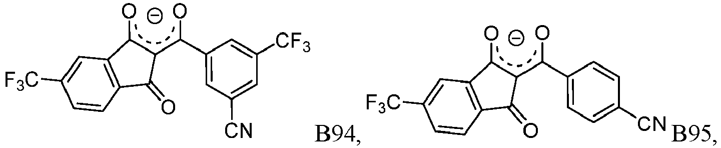

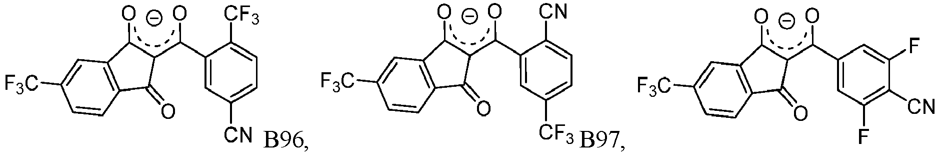

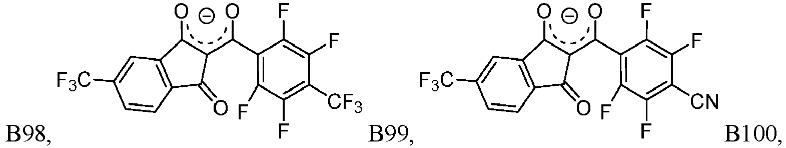

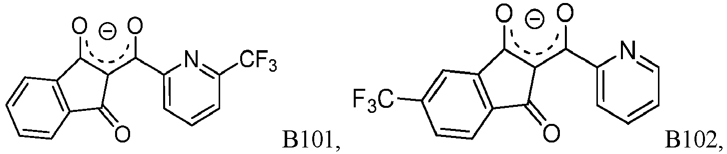

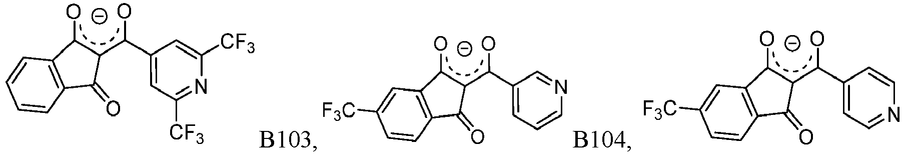

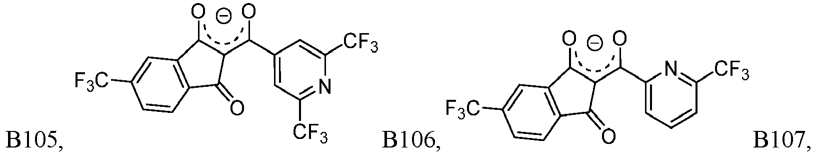

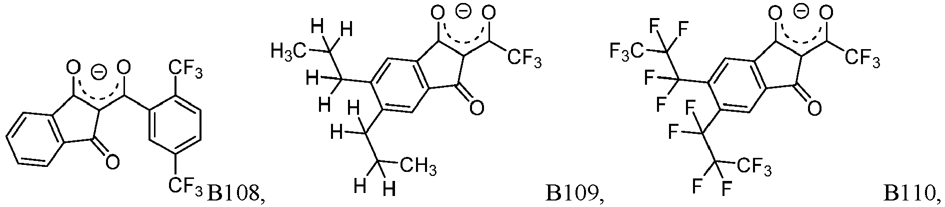

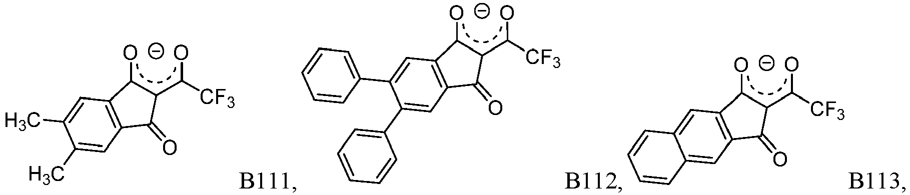

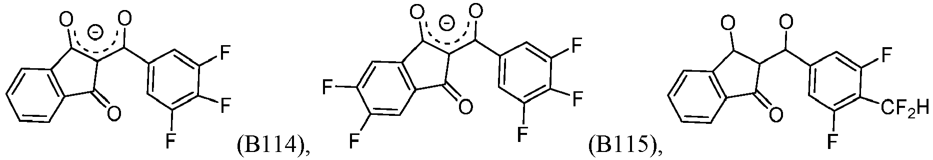

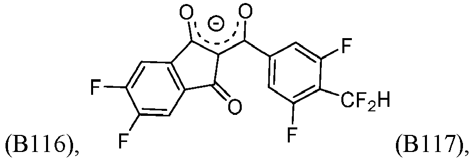

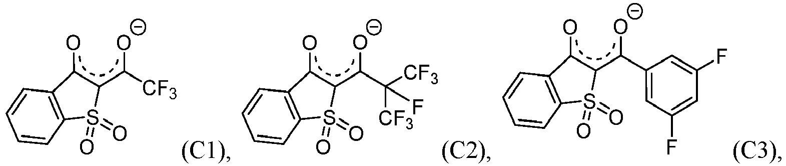

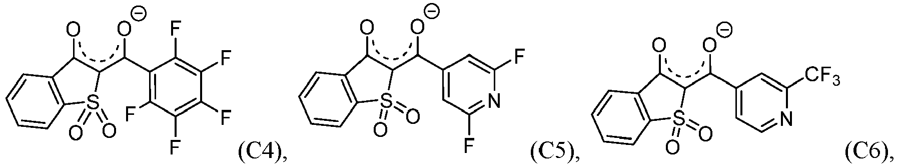

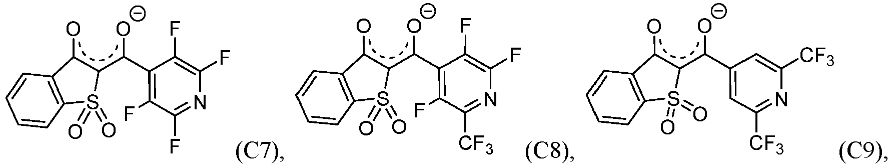

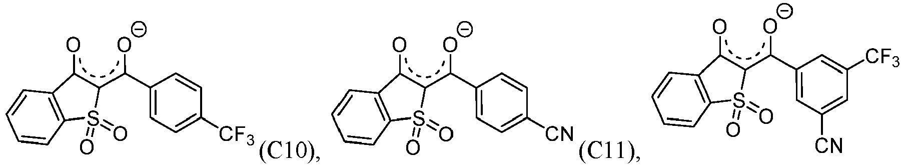

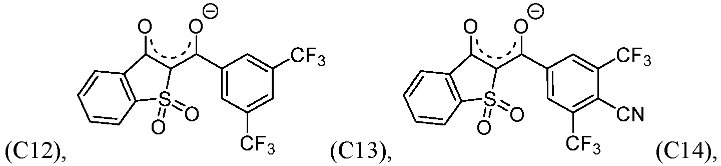

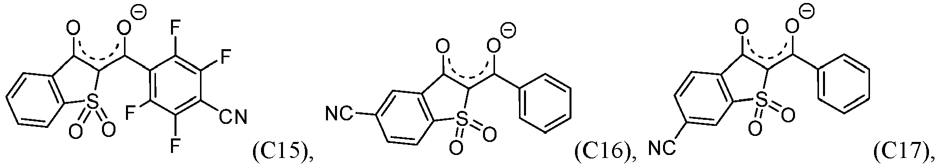

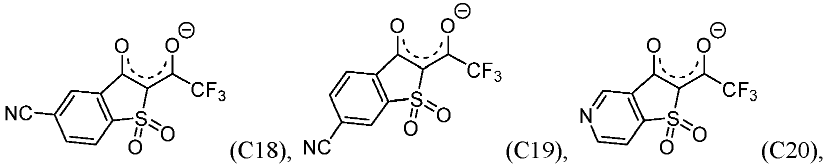

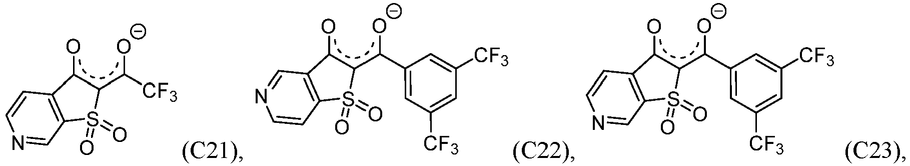

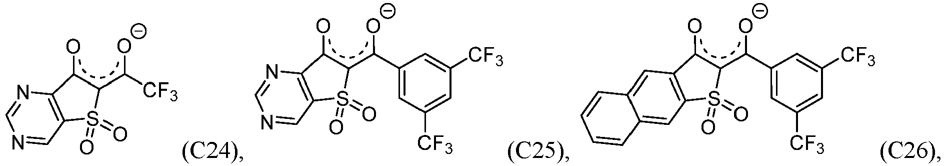

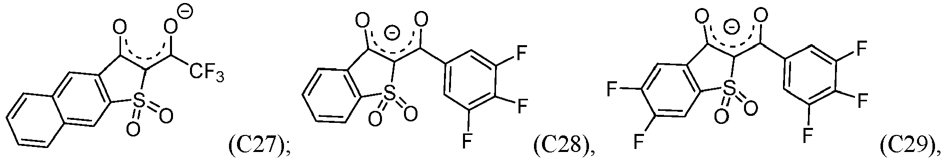

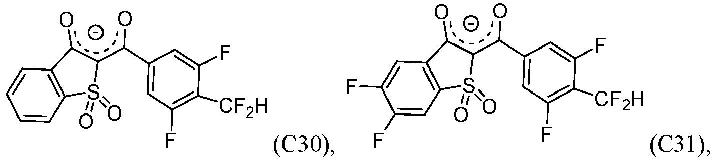

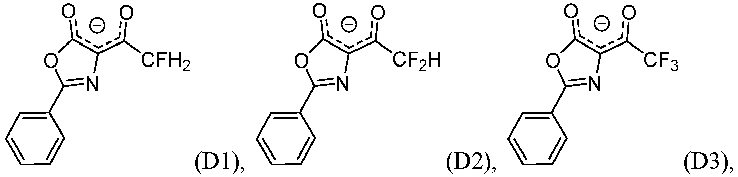

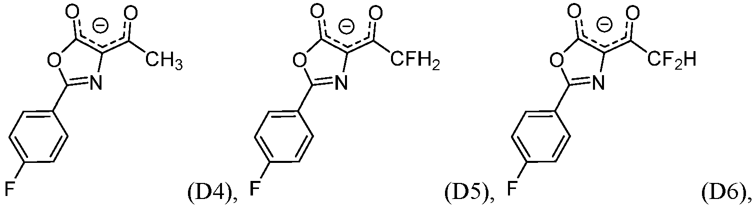

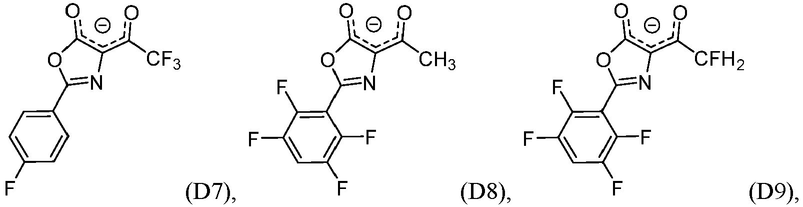

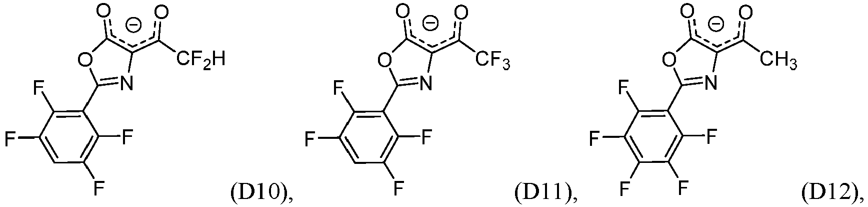

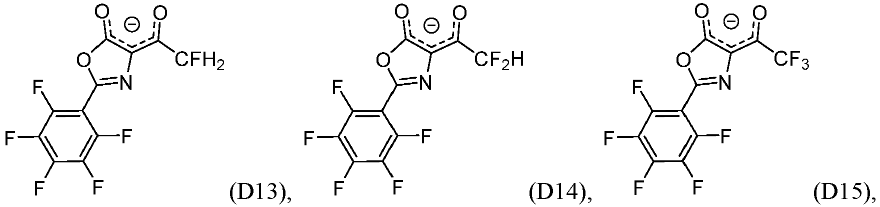

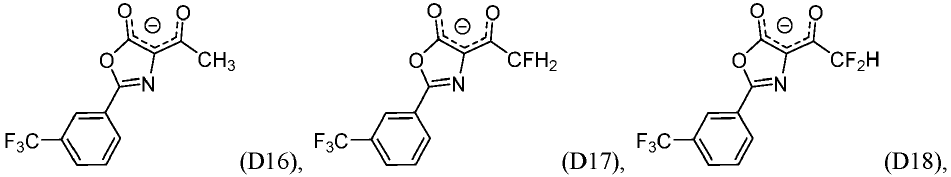

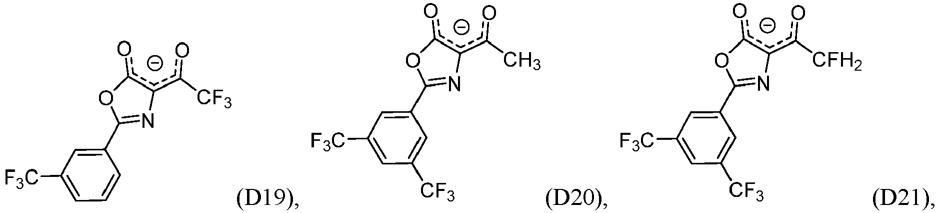

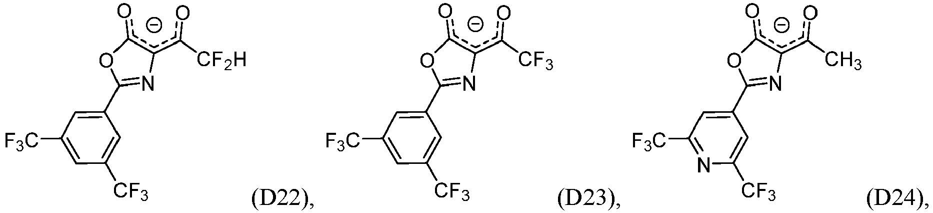

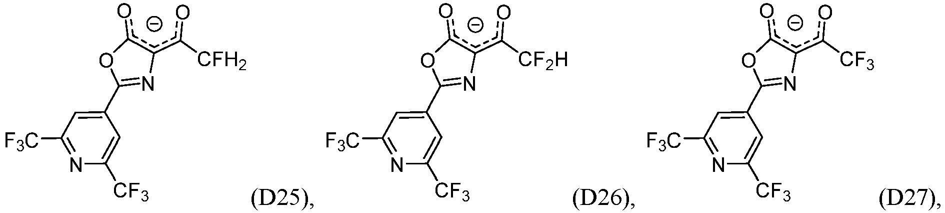

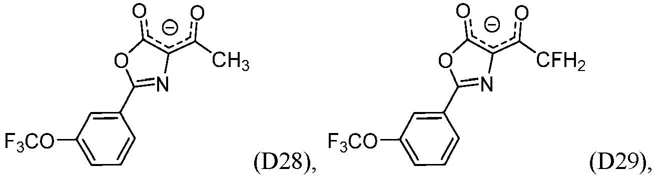

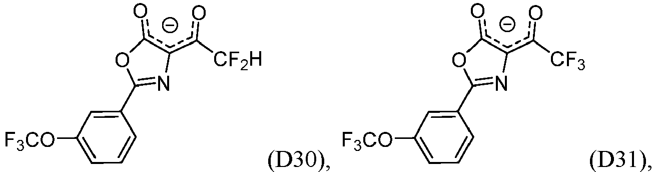

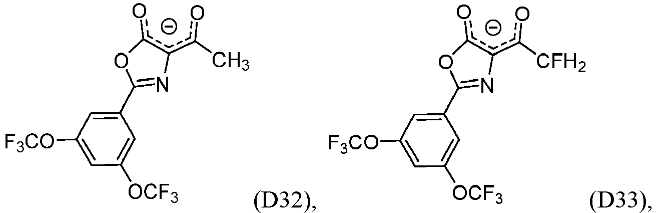

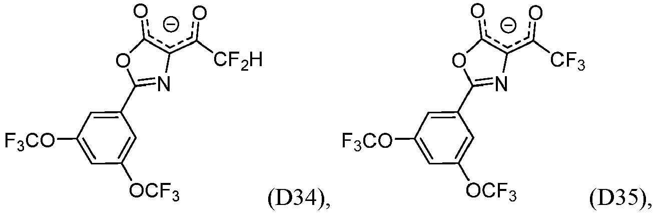

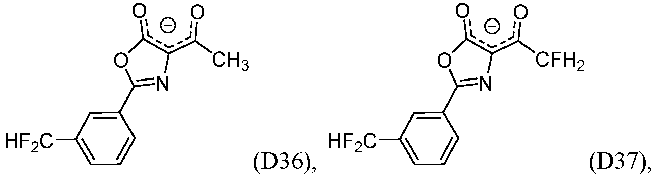

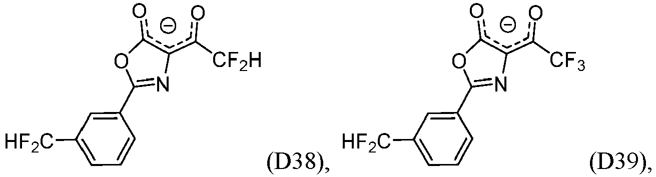

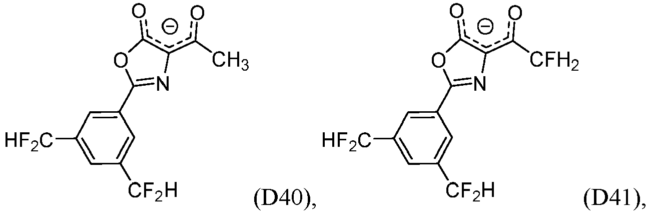

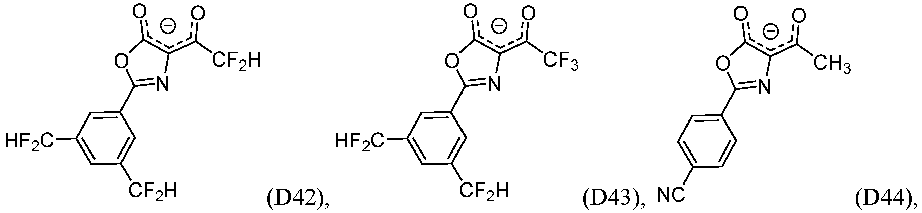

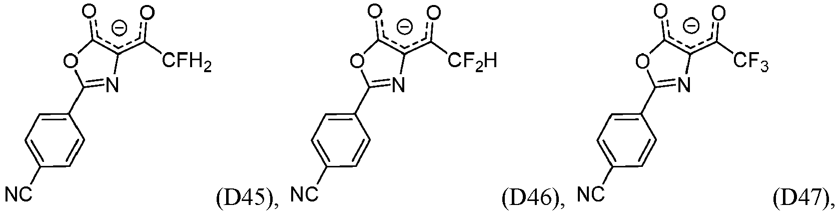

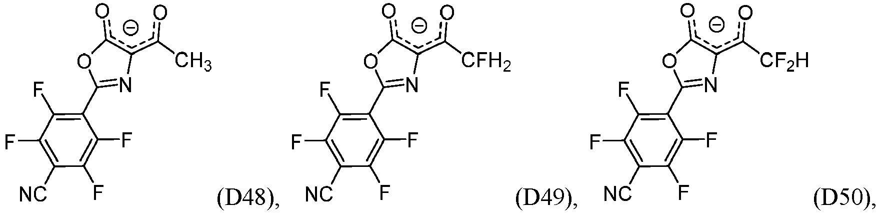

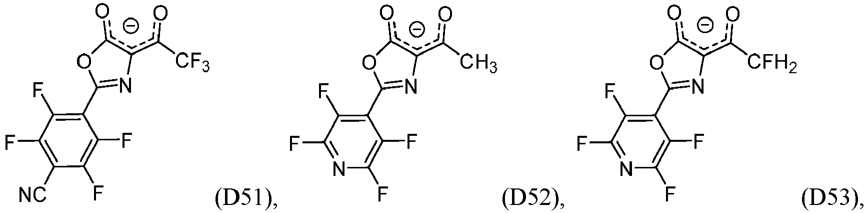

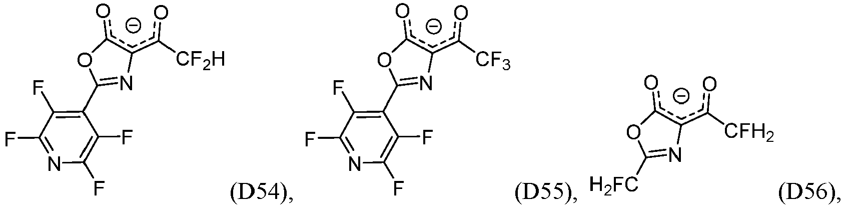

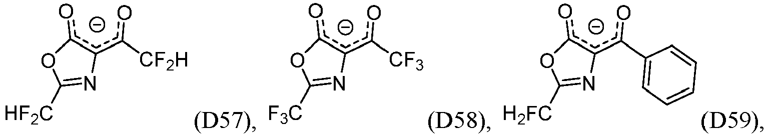

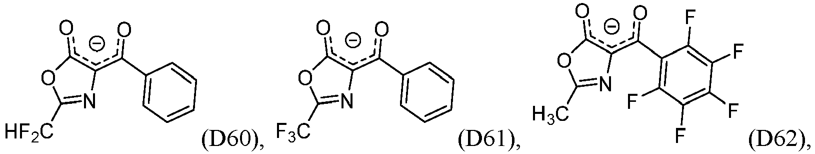

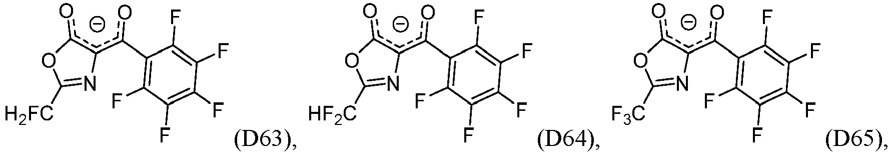

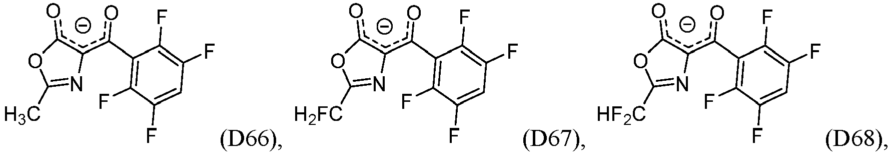

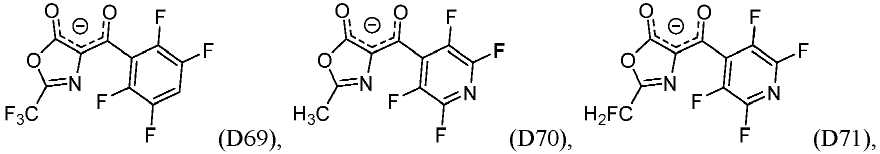

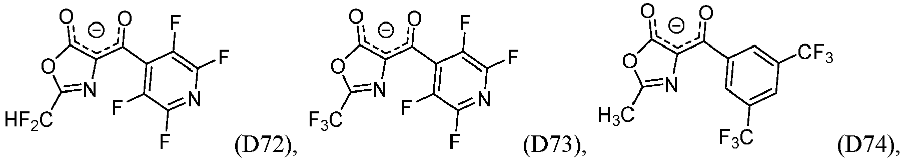

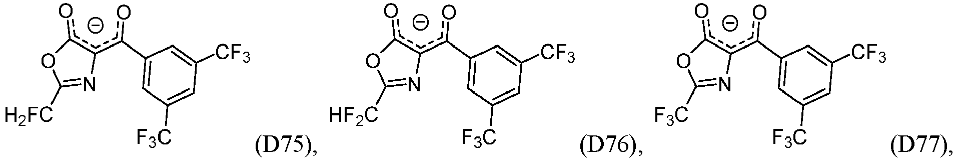

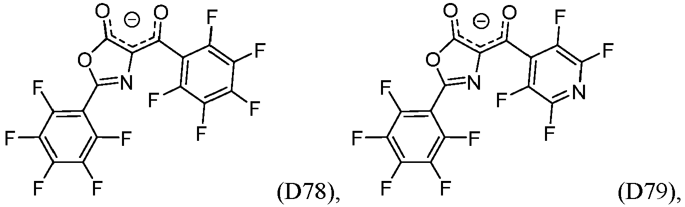

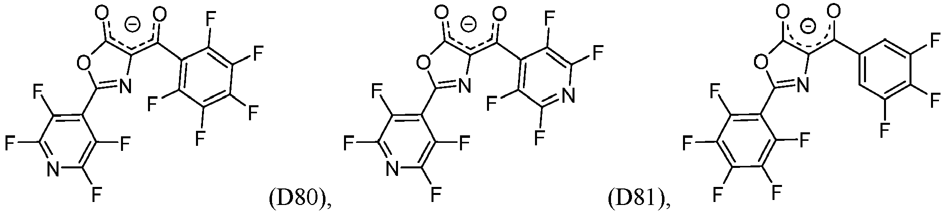

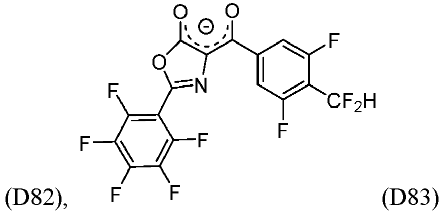

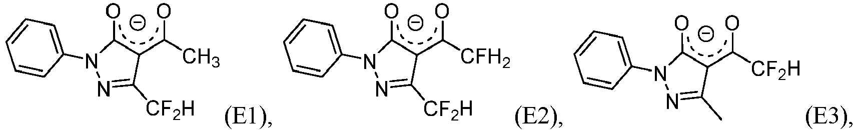

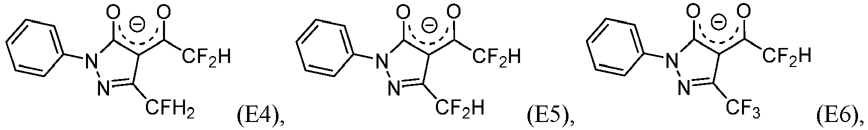

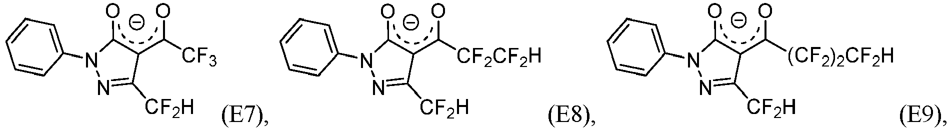

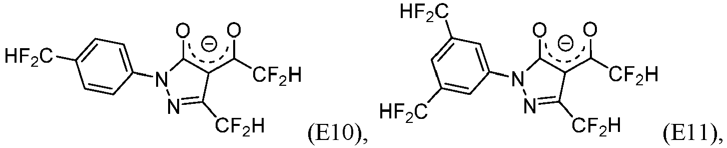

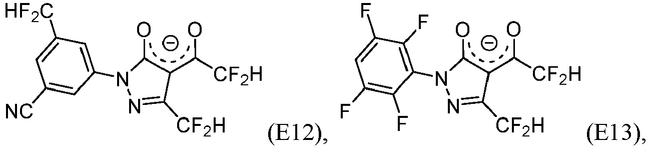

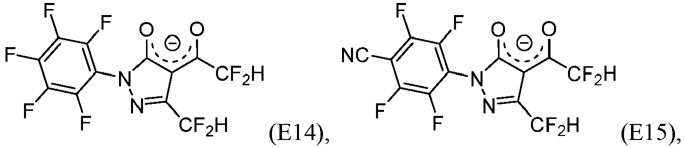

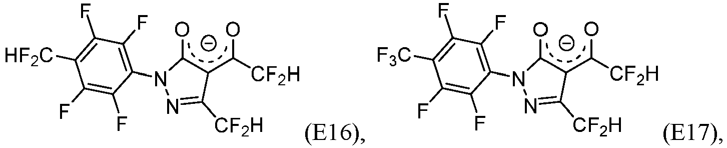

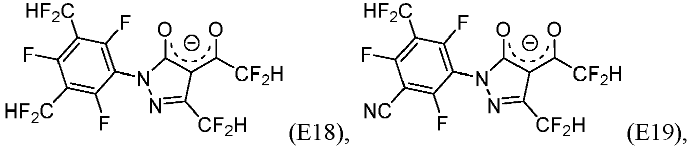

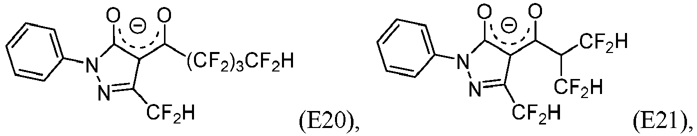

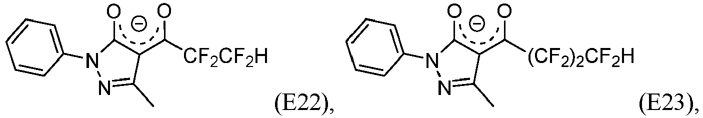

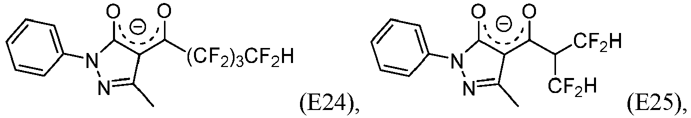

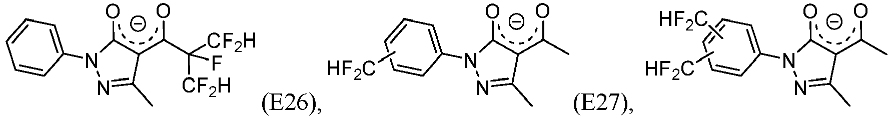

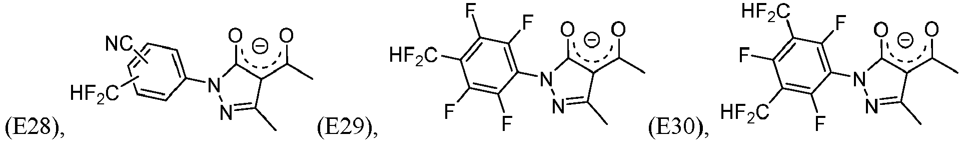

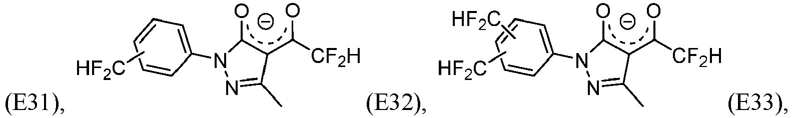

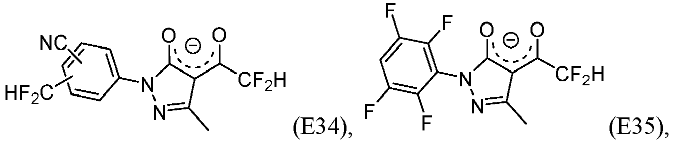

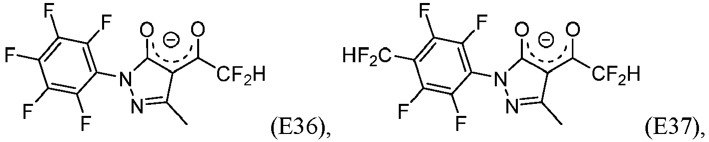

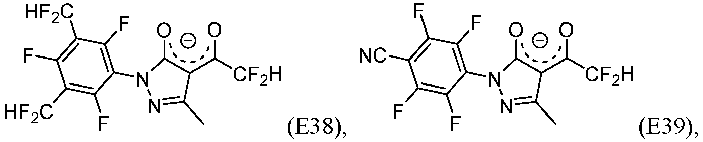

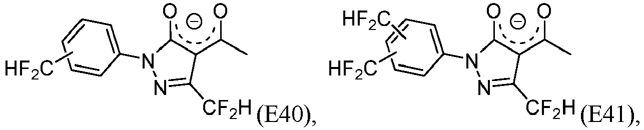

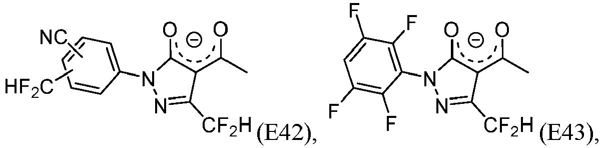

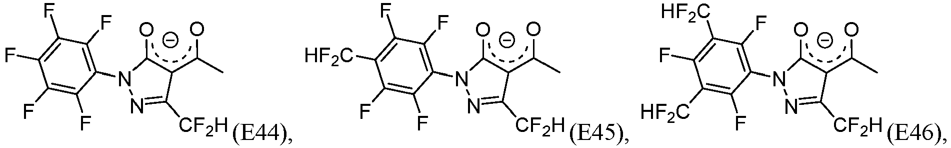

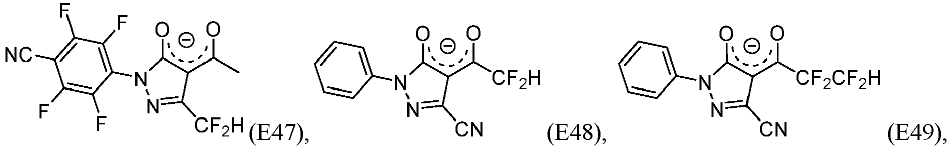

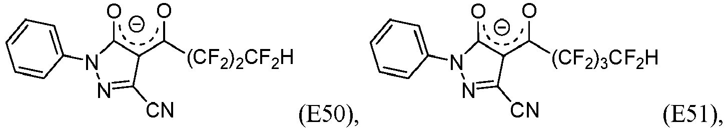

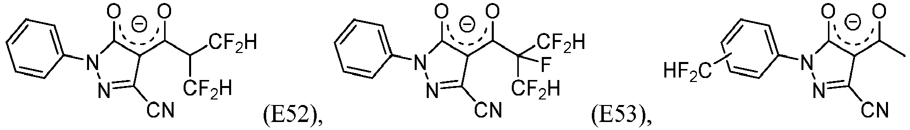

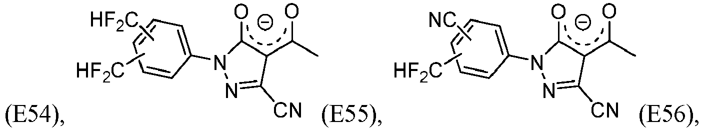

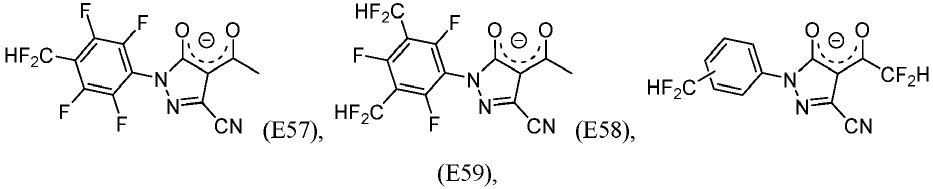

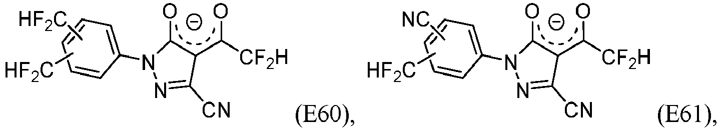

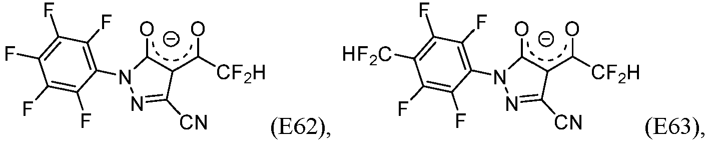

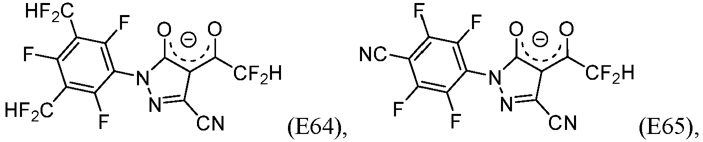

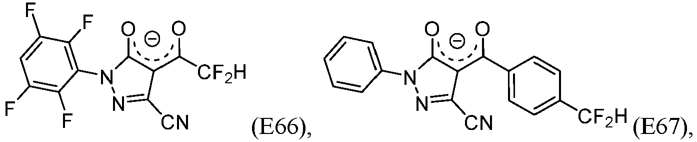

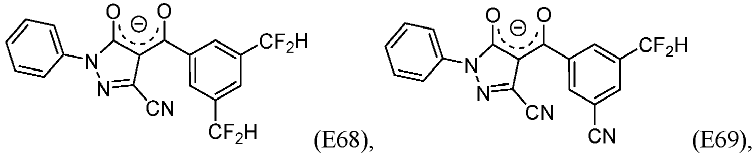

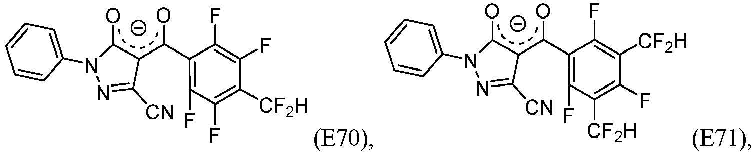

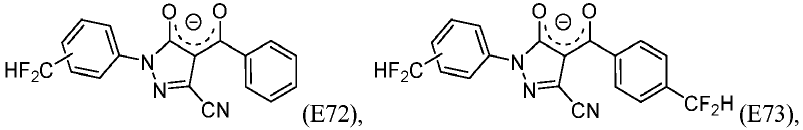

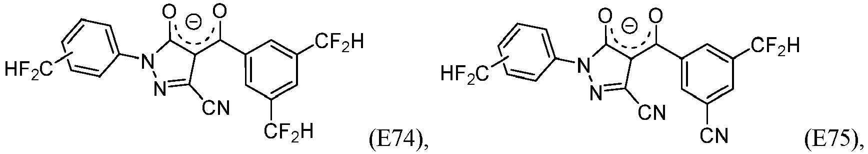

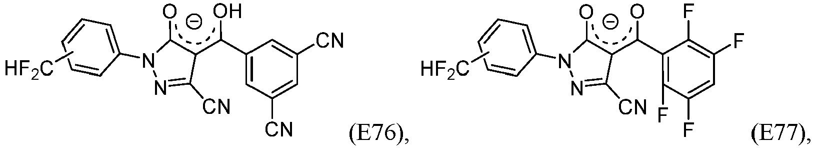

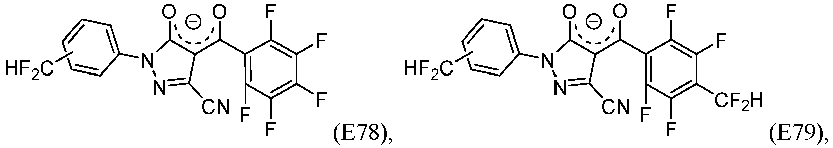

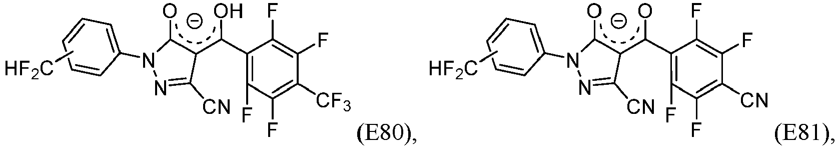

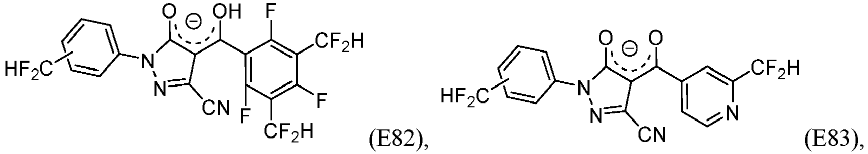

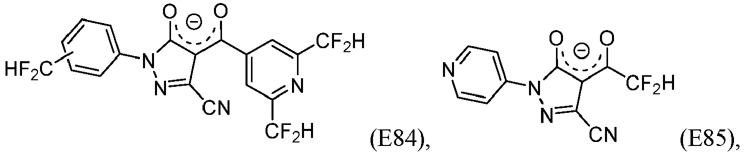

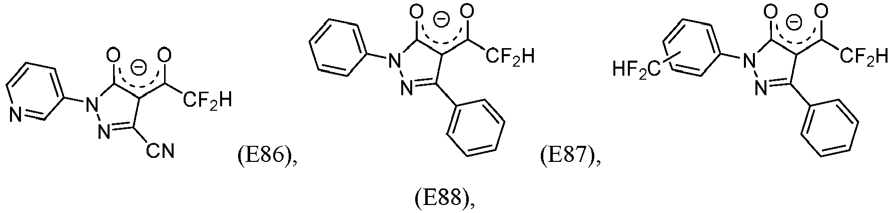

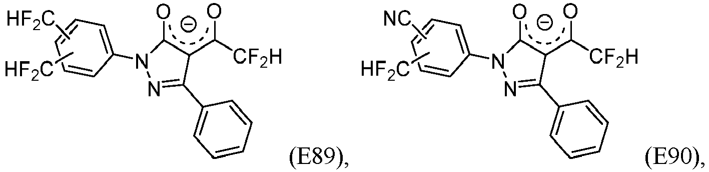

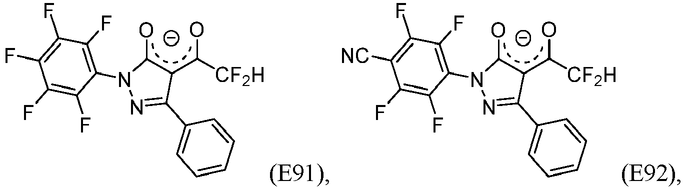

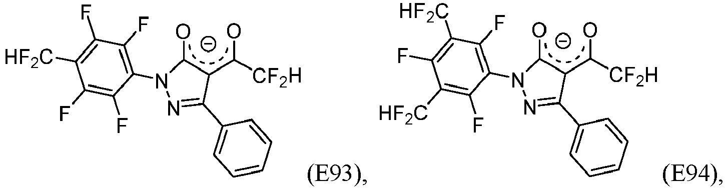

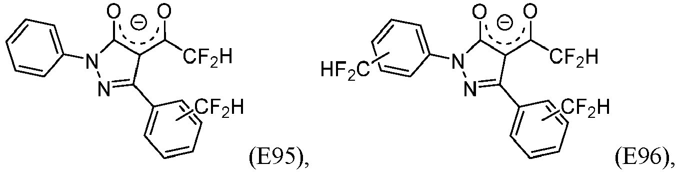

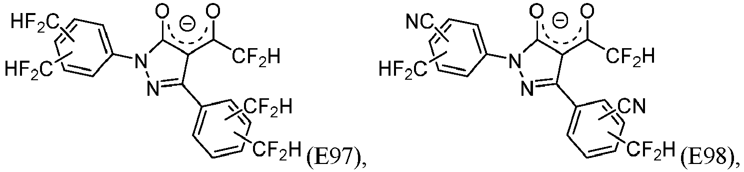

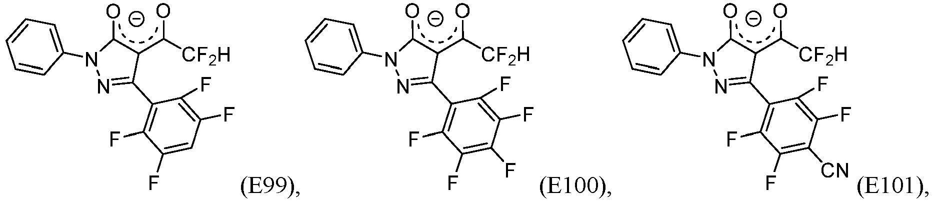

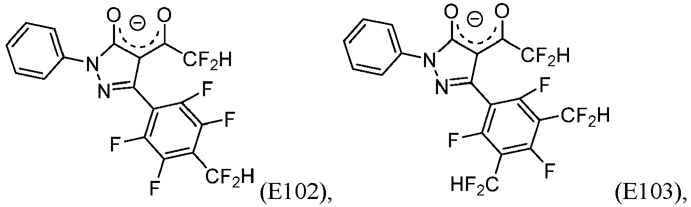

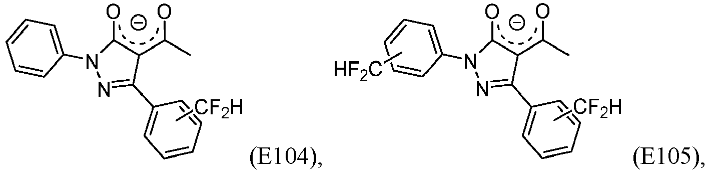

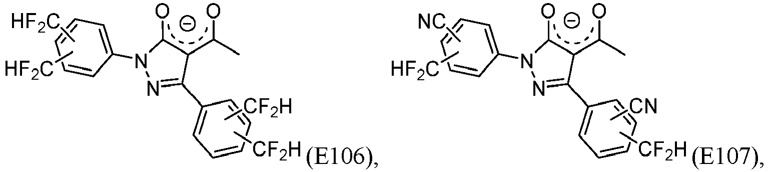

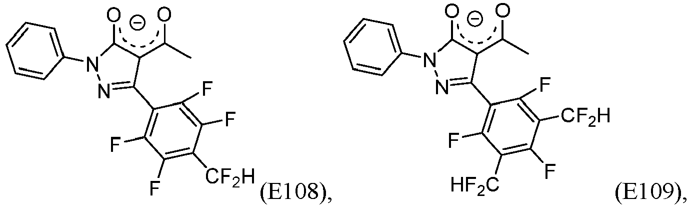

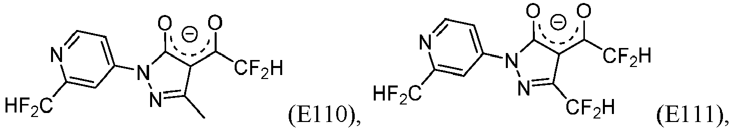

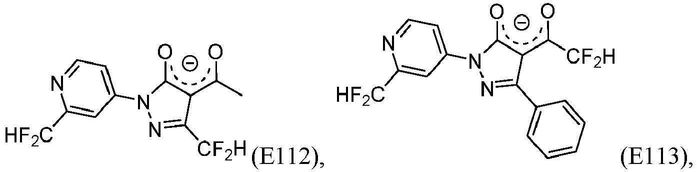

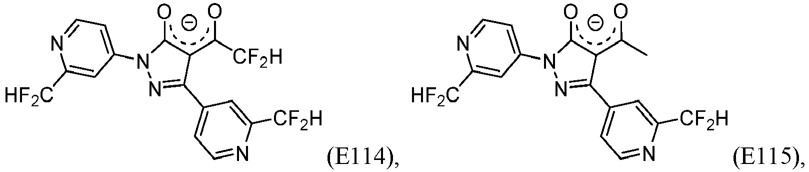

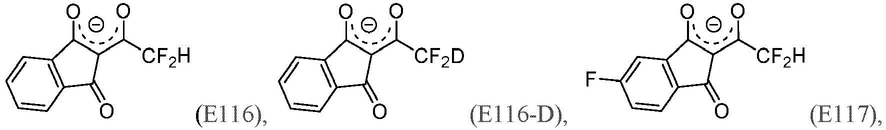

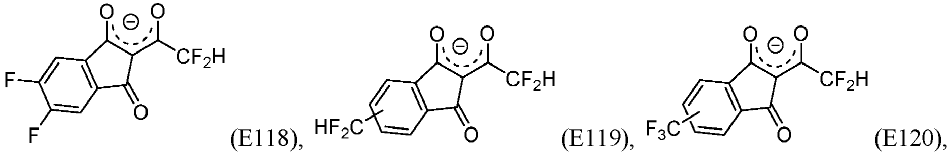

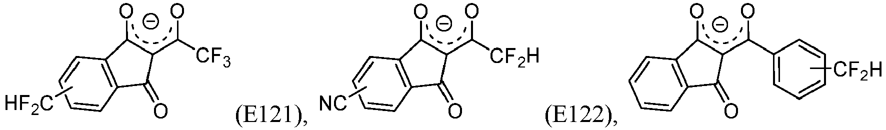

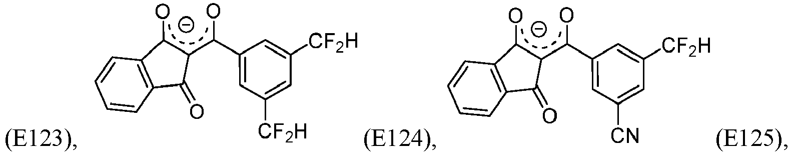

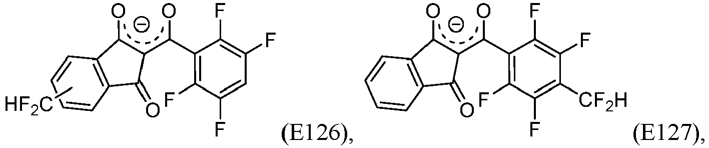

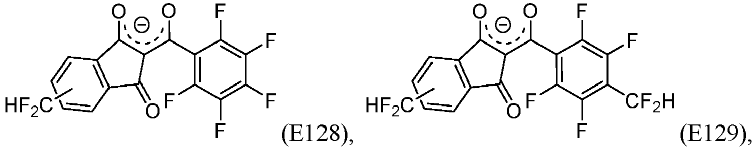

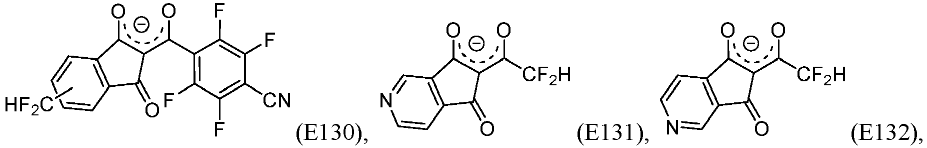

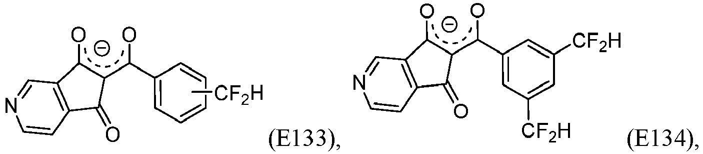

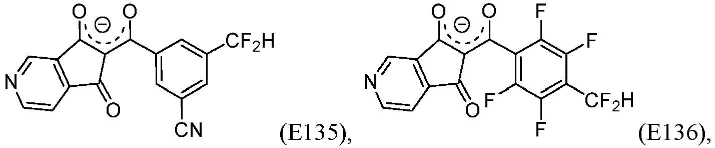

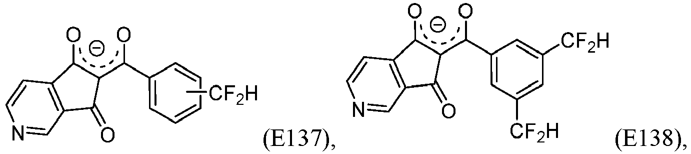

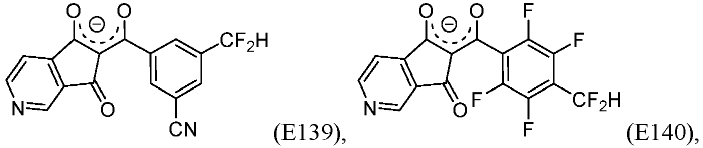

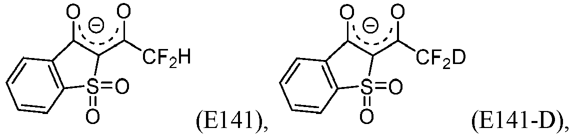

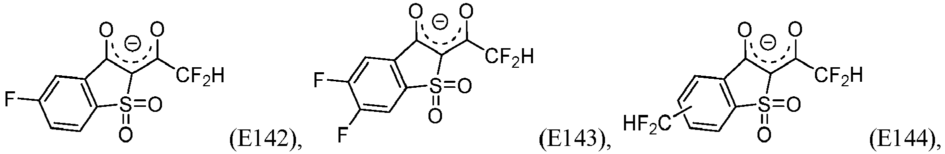

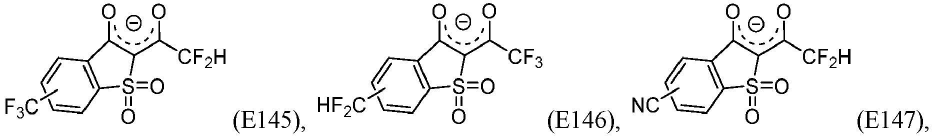

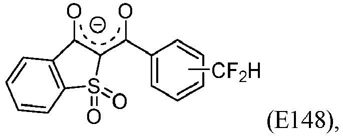

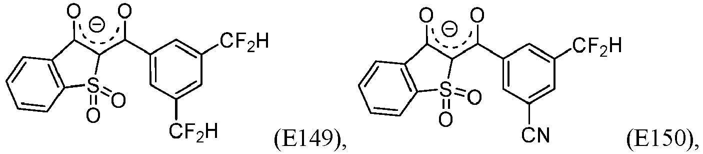

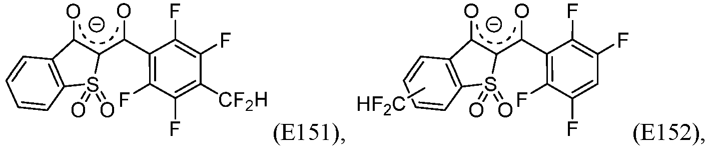

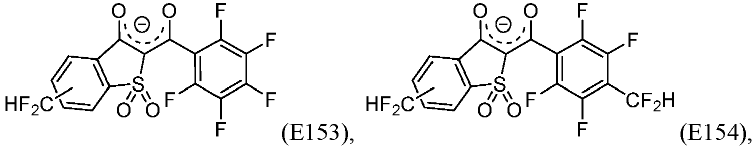

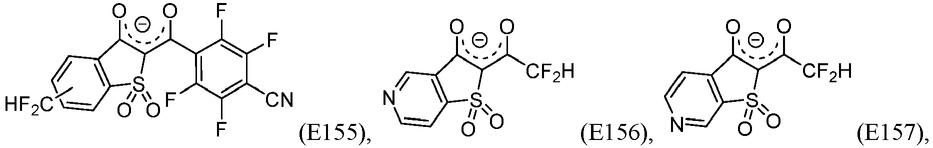

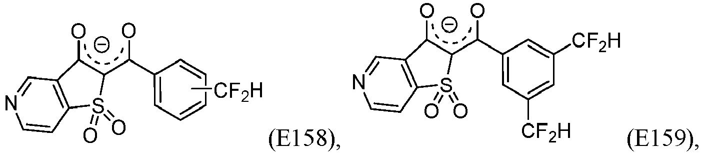

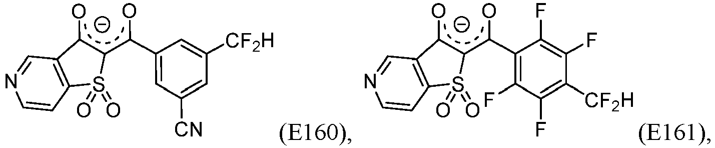

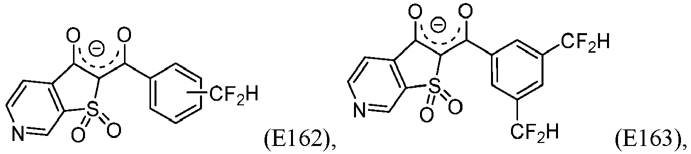

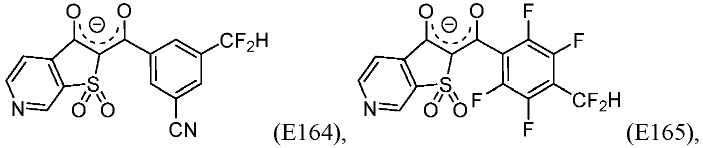

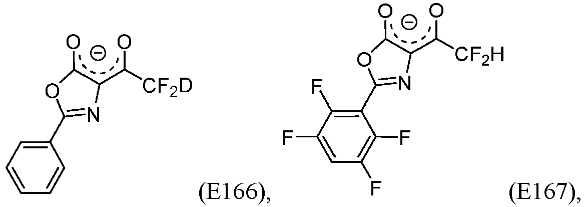

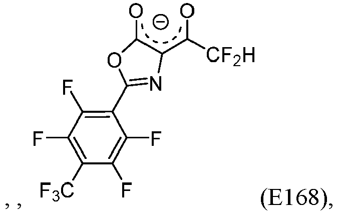

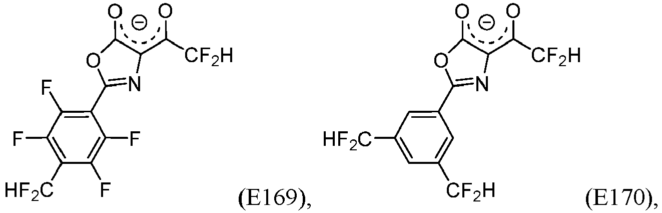

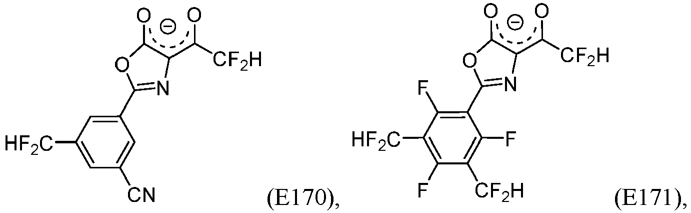

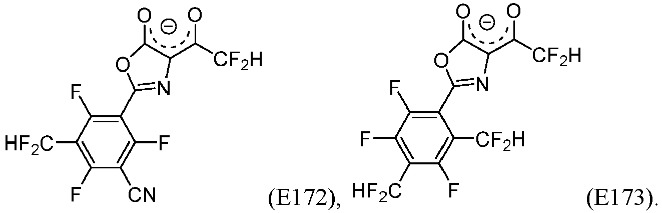

According to an embodiment, Ligand L of formula (II) is selected from the following compounds:

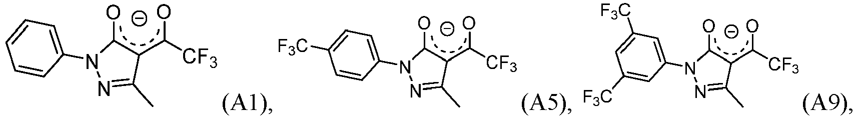

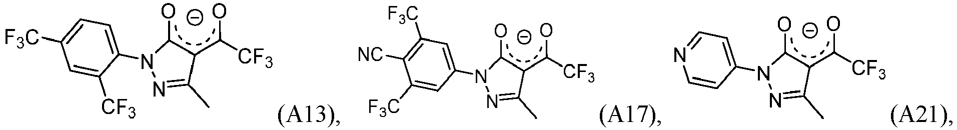

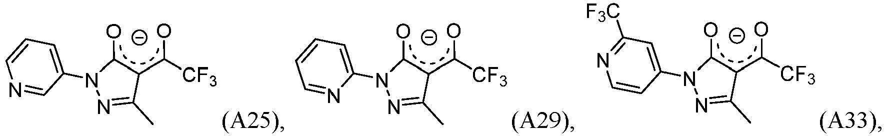

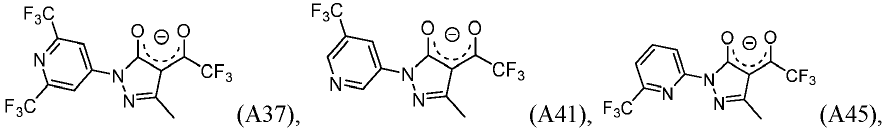

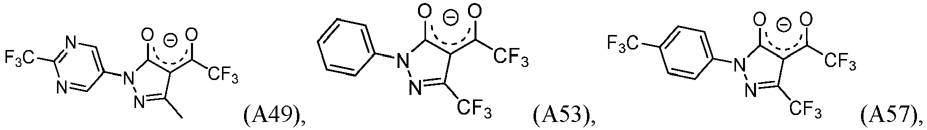

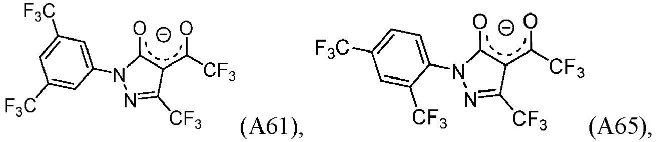

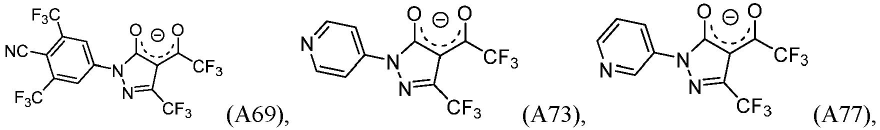

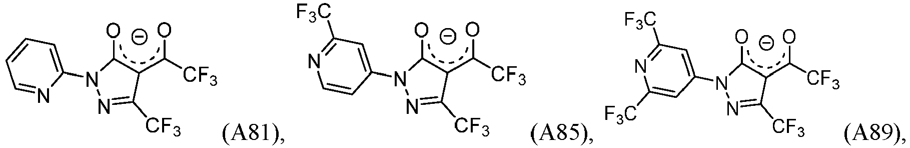

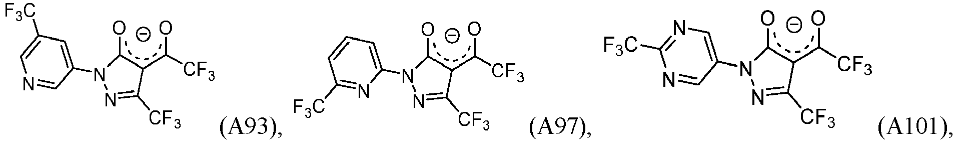

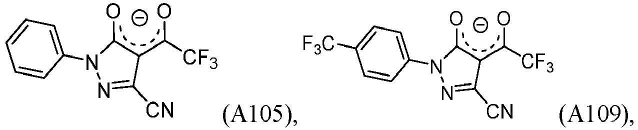

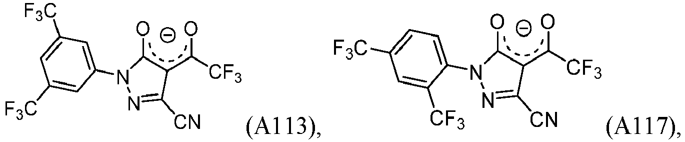

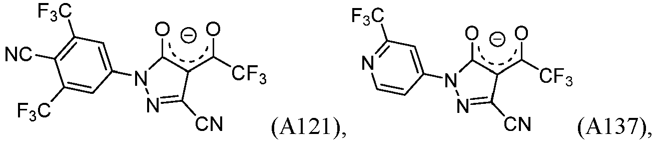

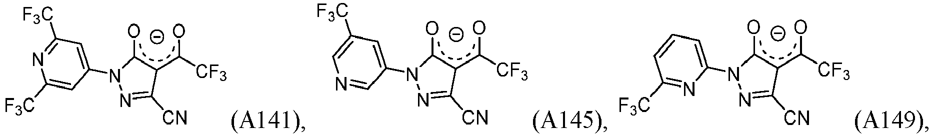

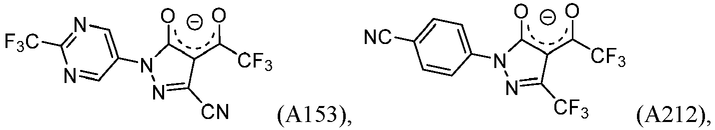

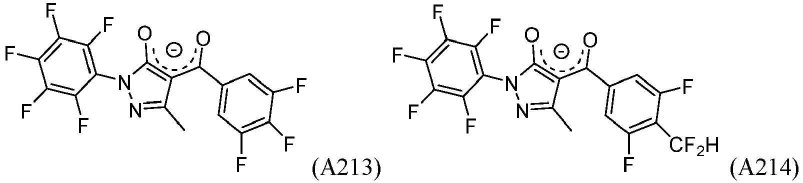

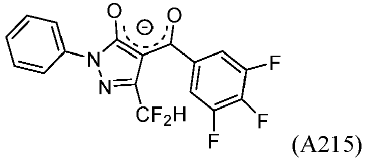

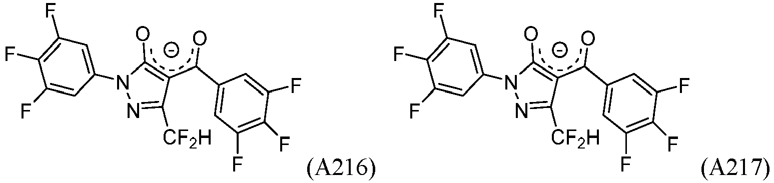

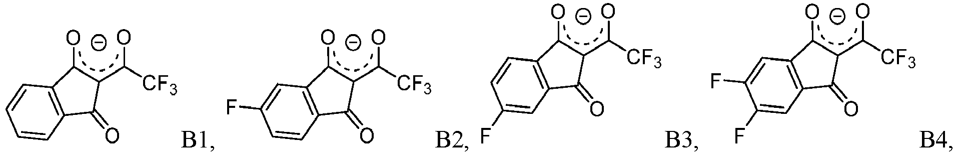

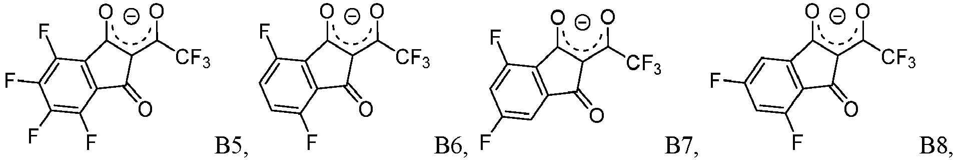

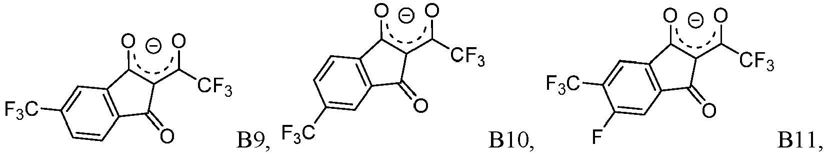

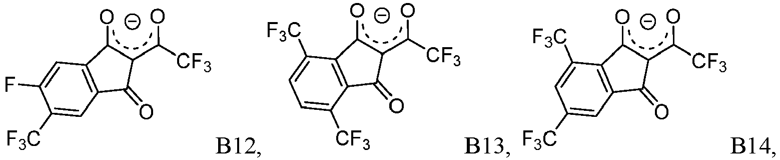

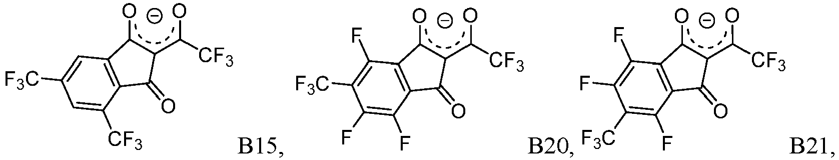

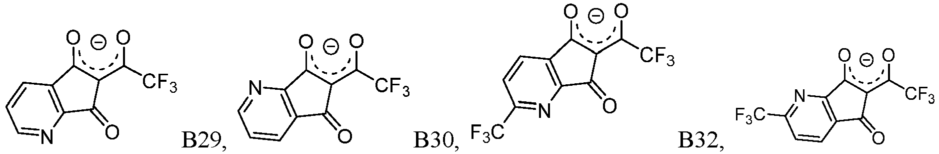

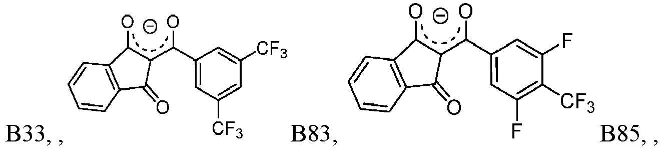

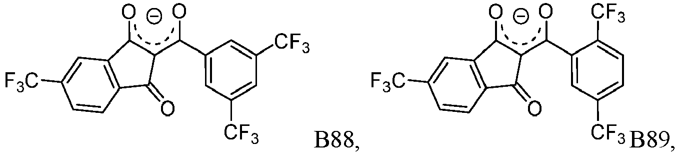

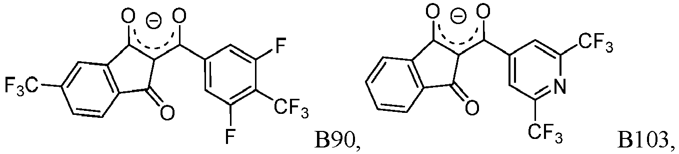

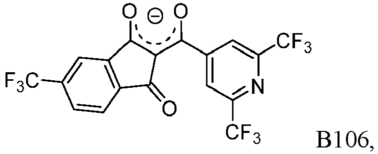

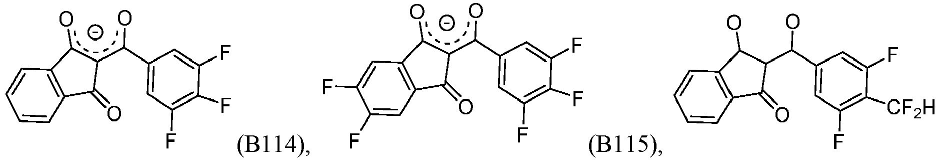

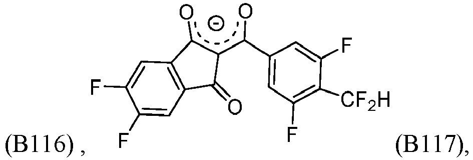

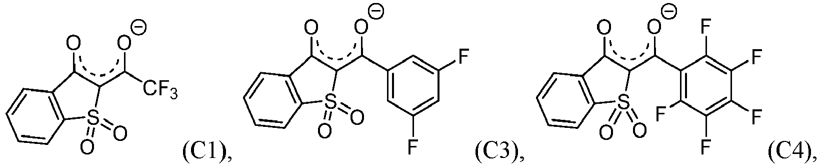

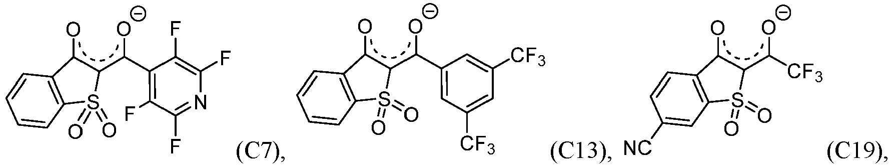

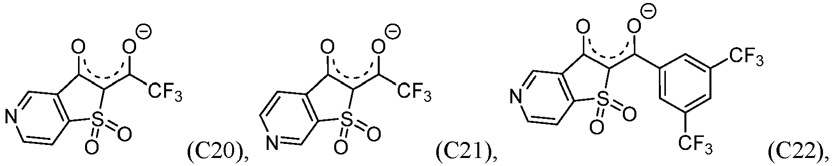

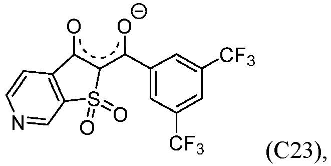

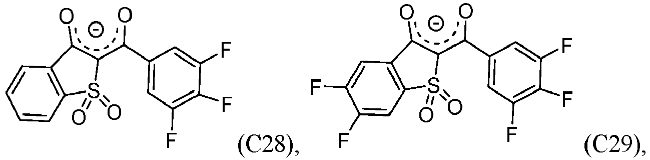

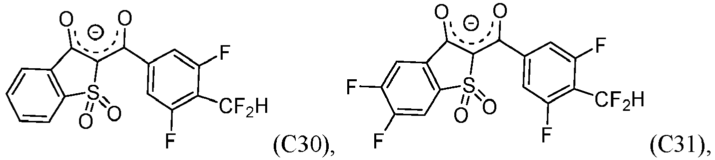

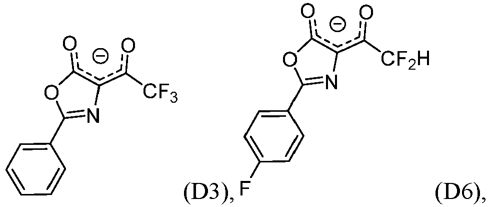

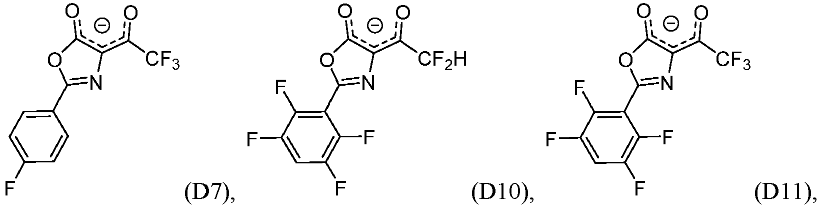

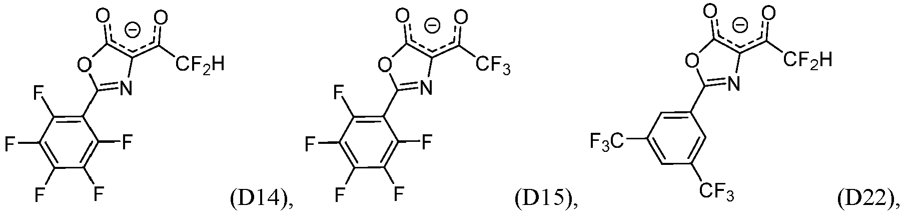

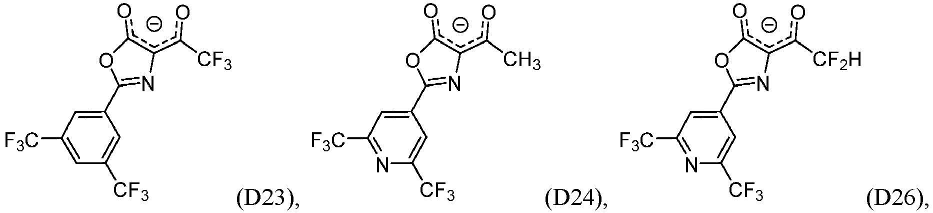

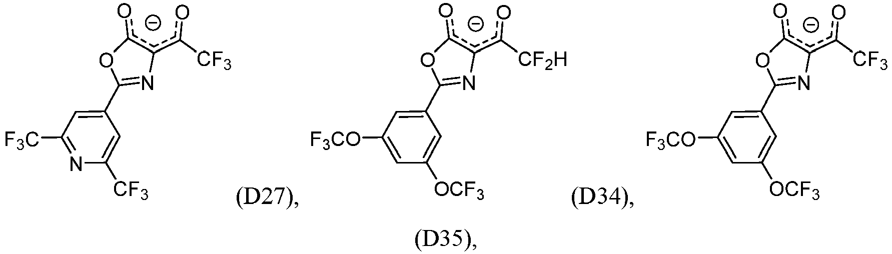

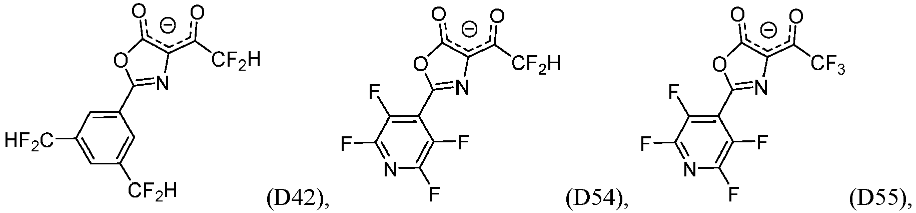

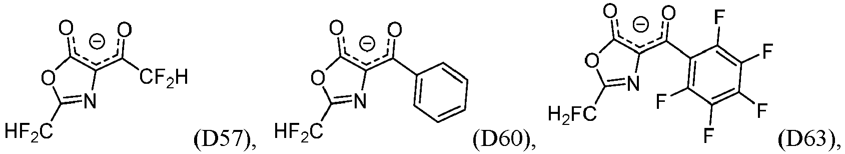

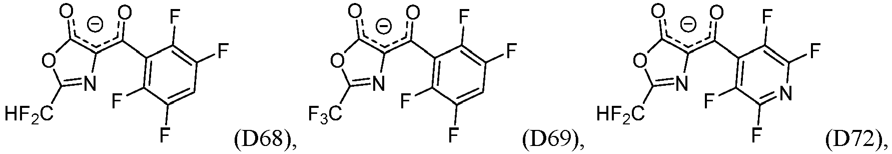

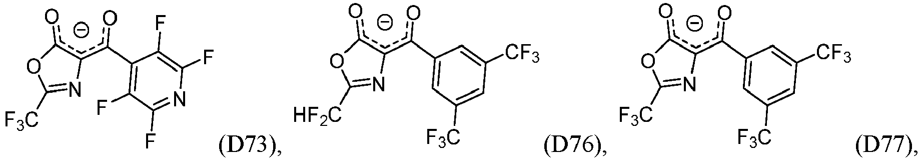

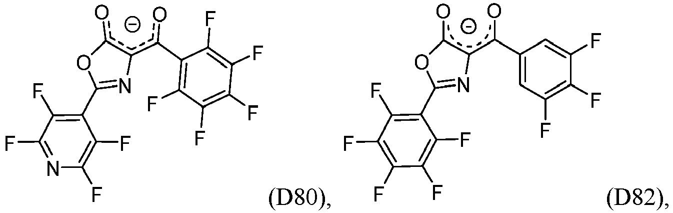

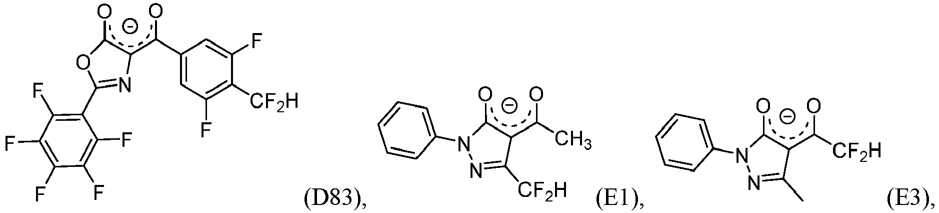

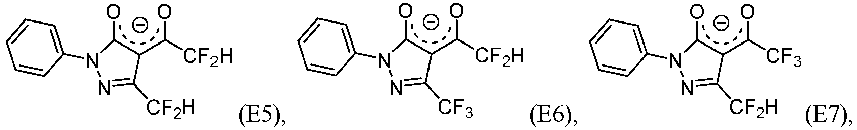

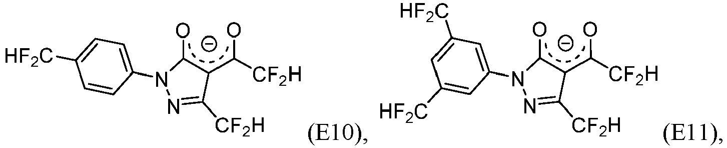

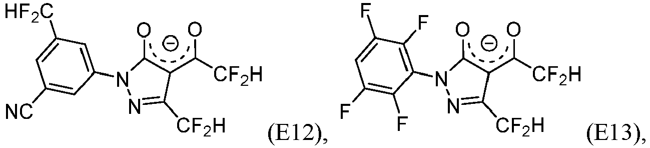

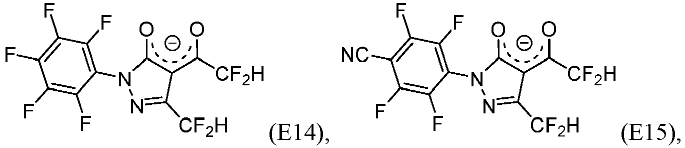

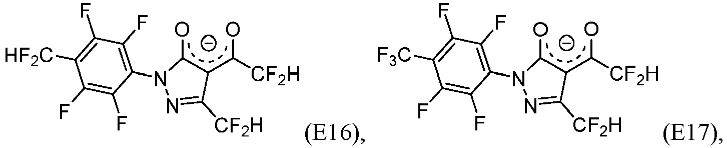

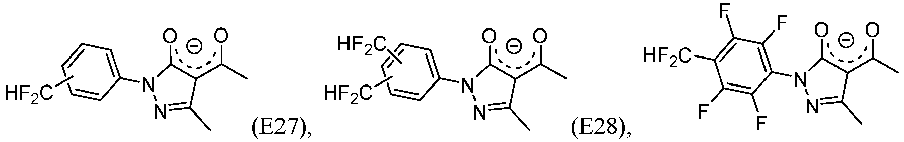

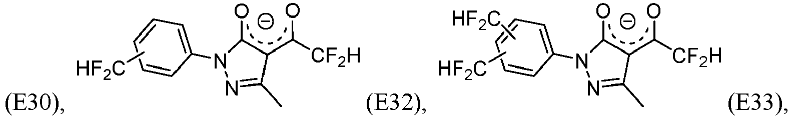

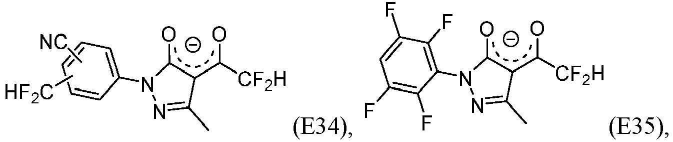

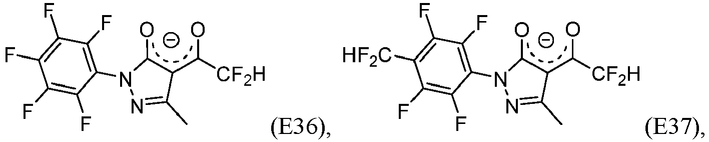

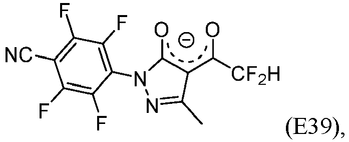

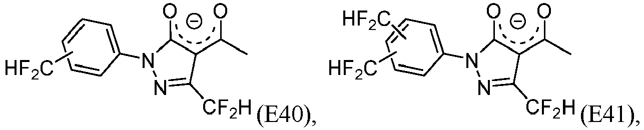

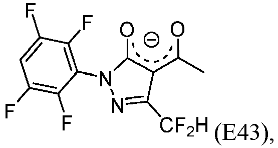

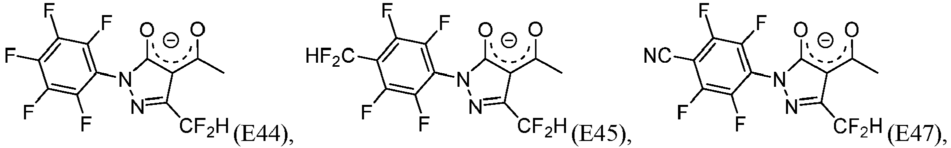

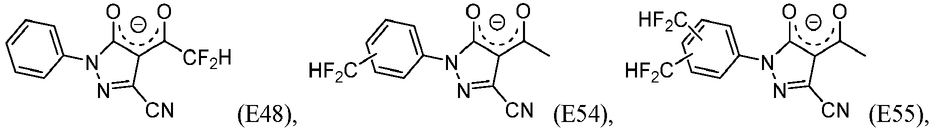

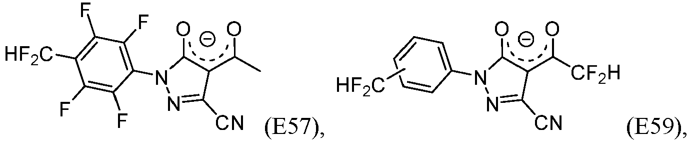

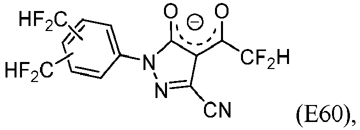

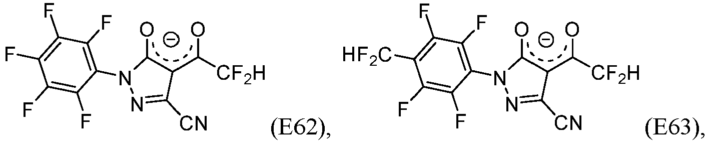

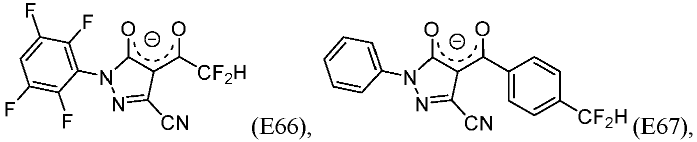

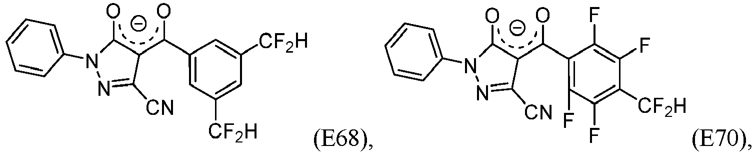

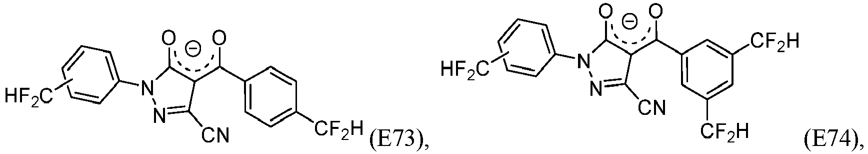

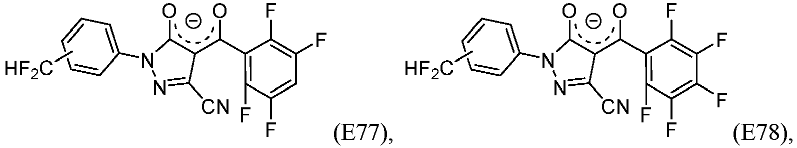

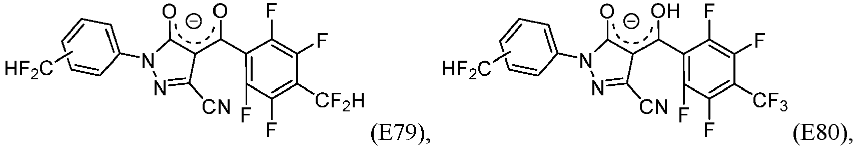

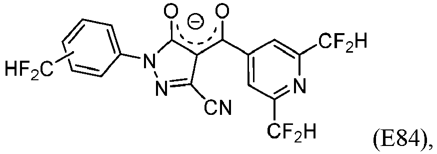

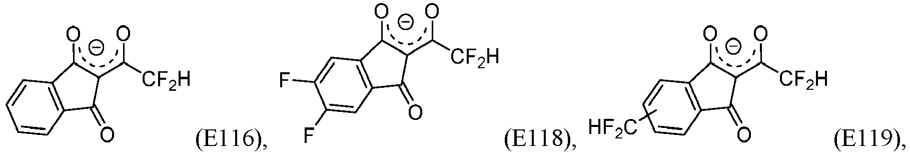

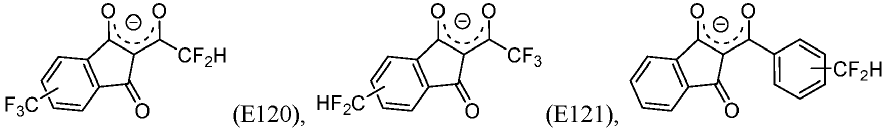

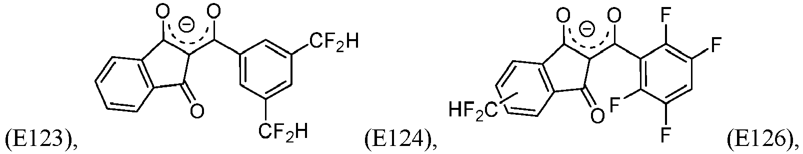

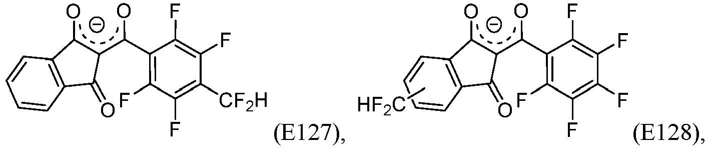

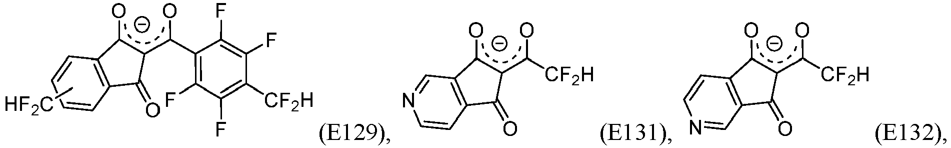

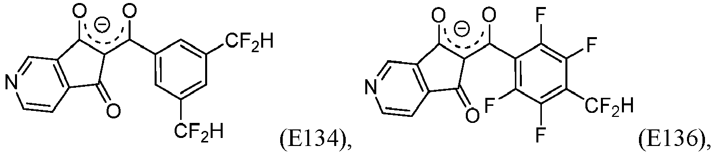

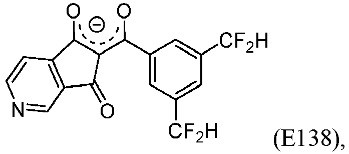

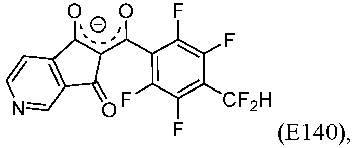

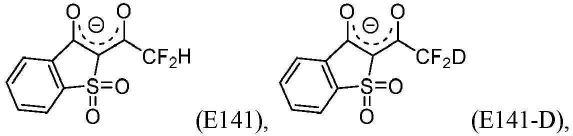

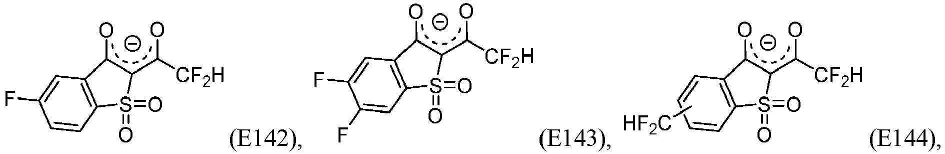

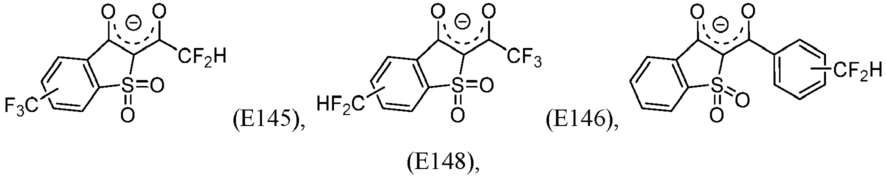

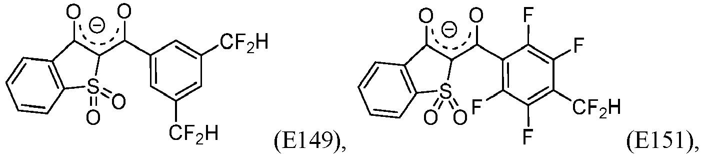

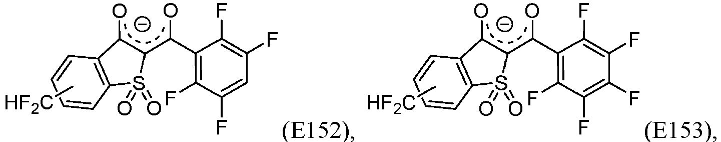

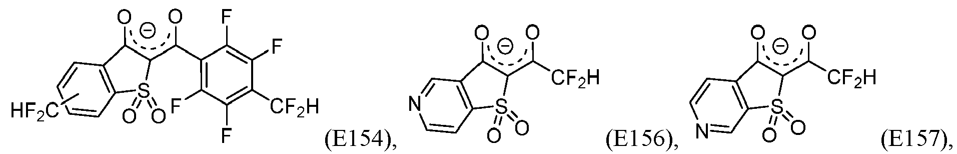

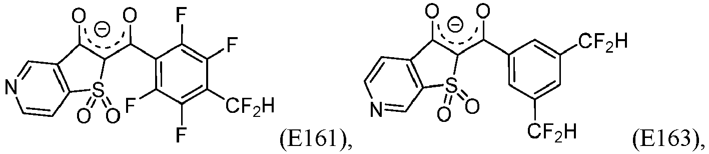

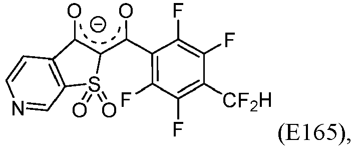

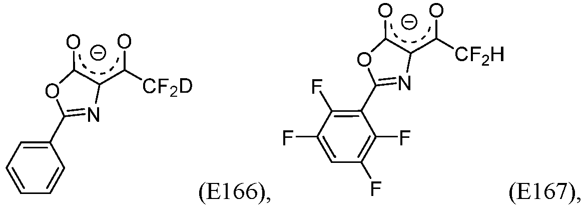

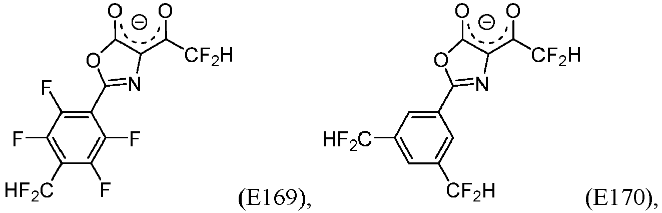

According to an embodiment, Ligand L of formula (II) is selected from the following compounds:

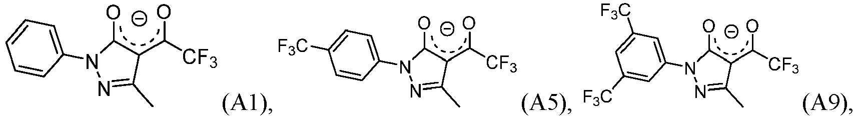

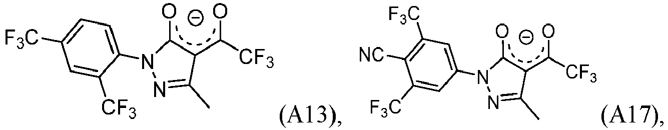

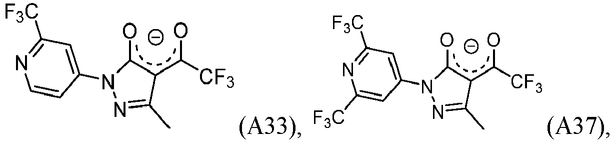

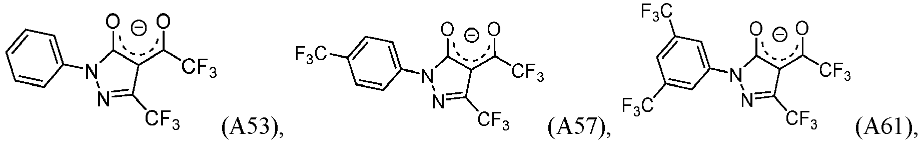

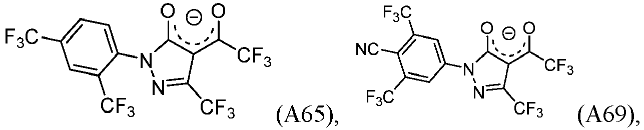

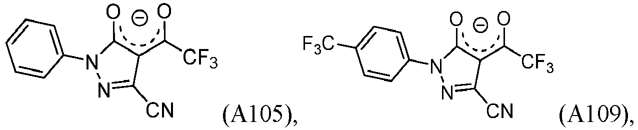

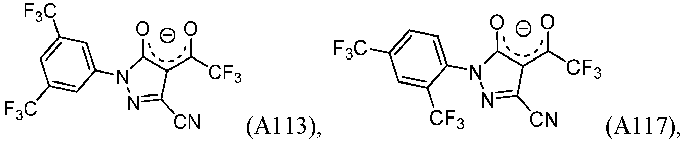

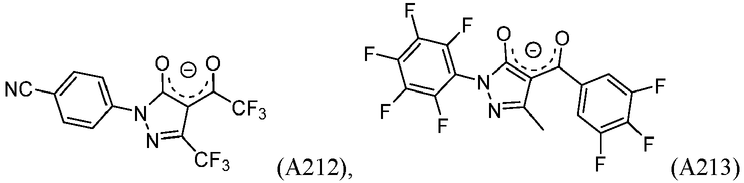

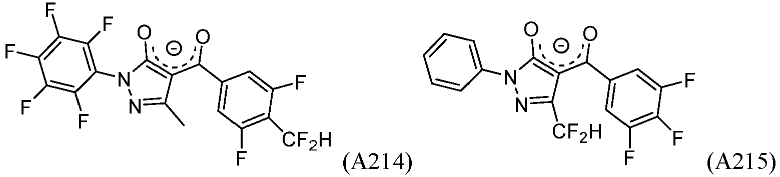

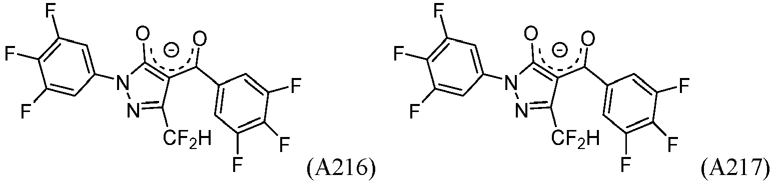

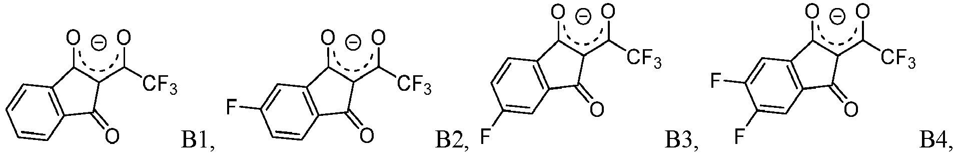

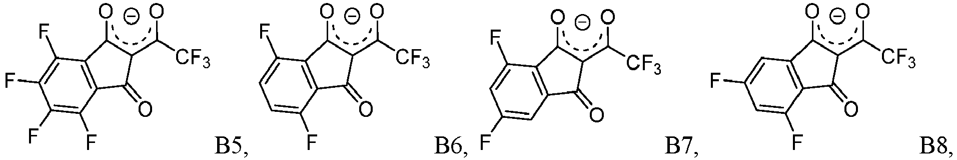

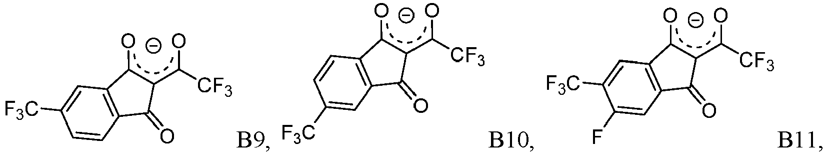

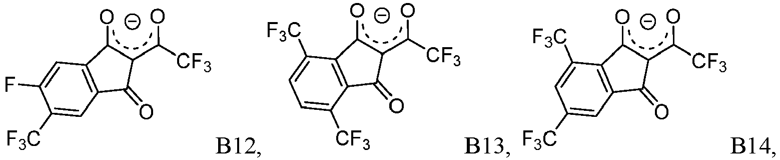

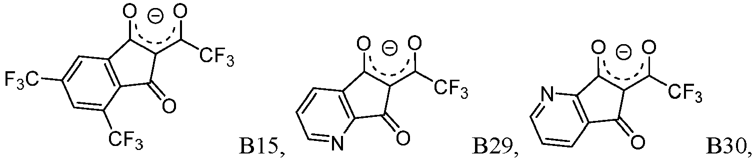

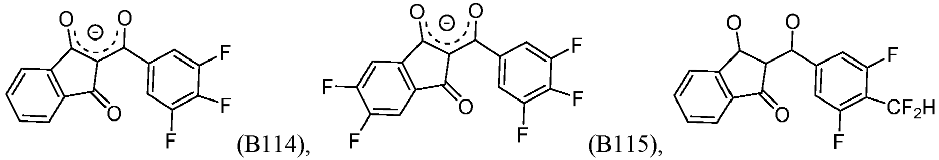

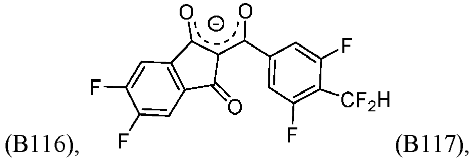

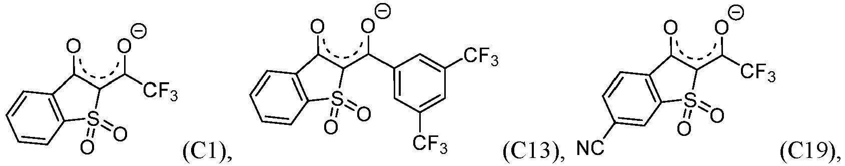

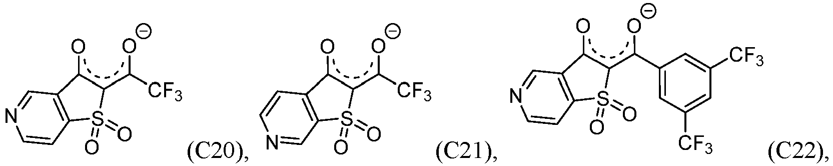

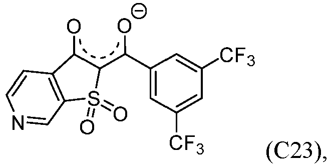

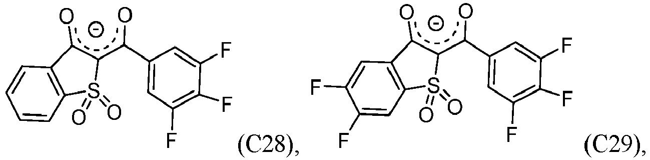

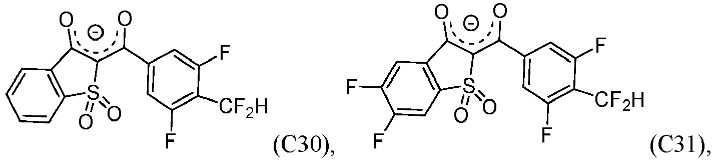

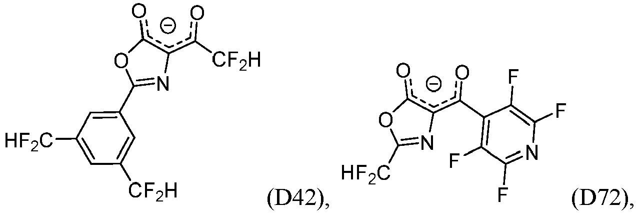

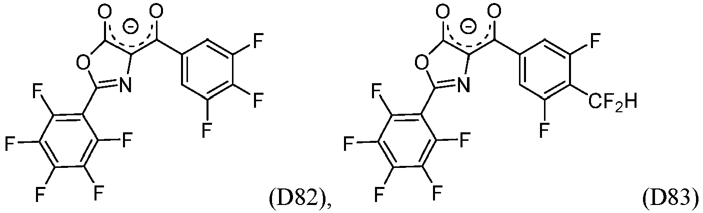

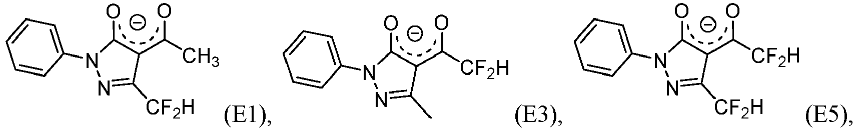

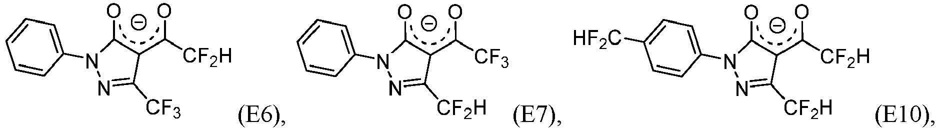

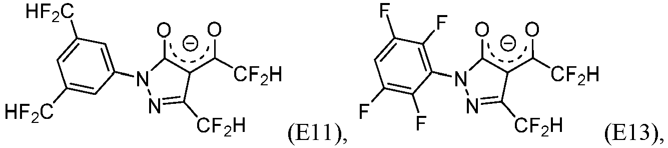

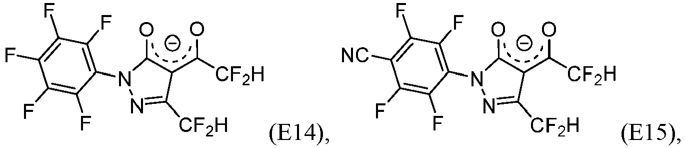

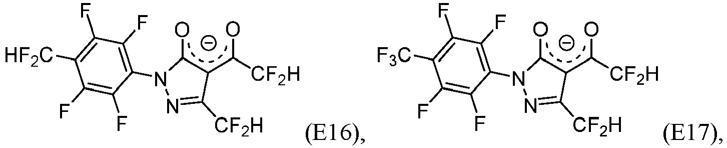

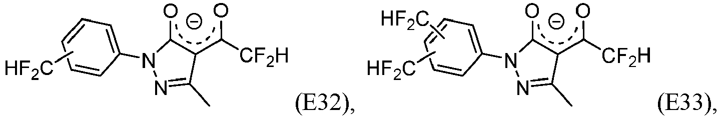

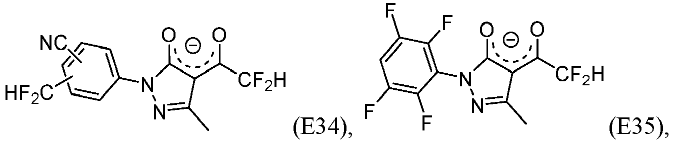

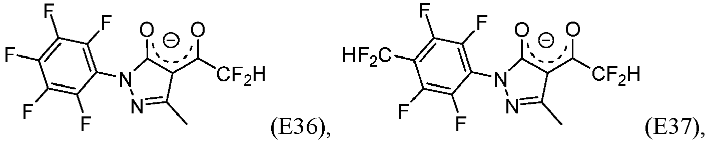

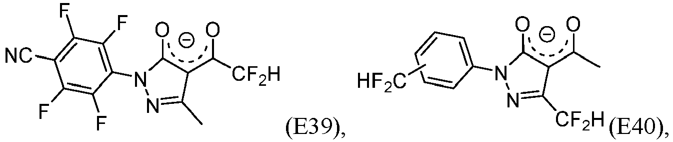

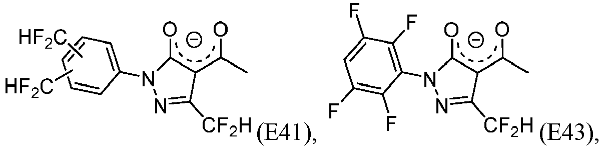

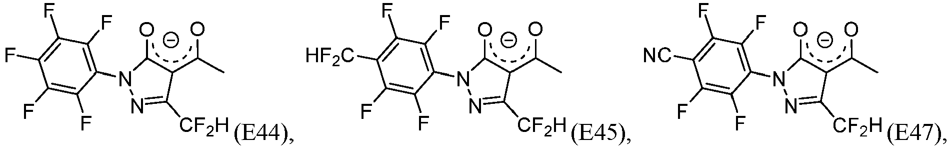

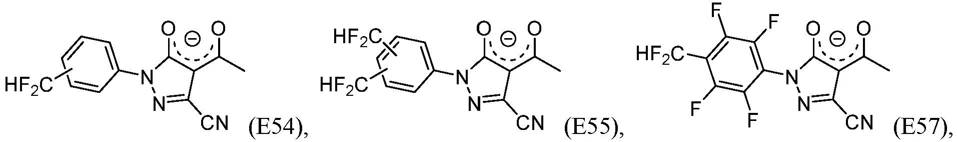

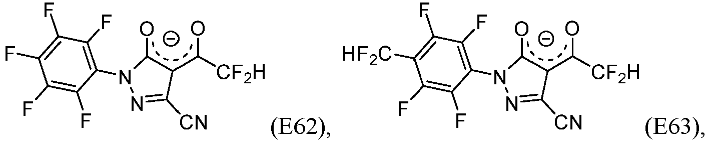

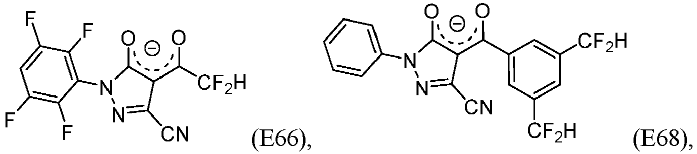

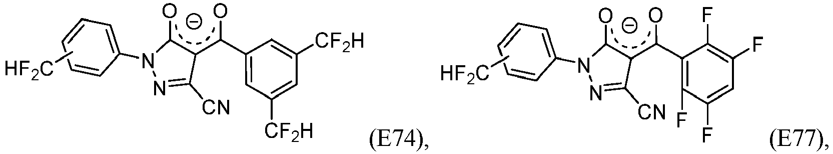

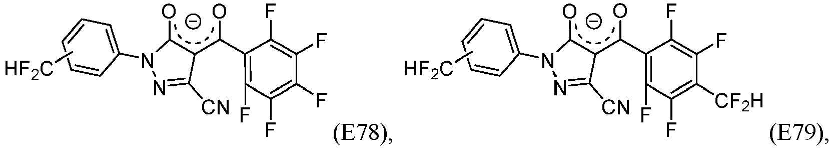

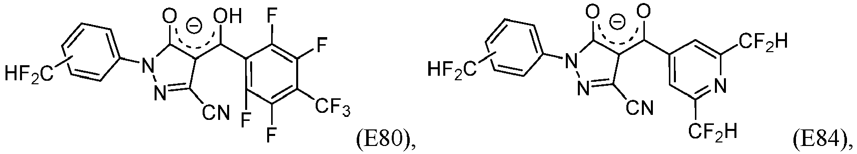

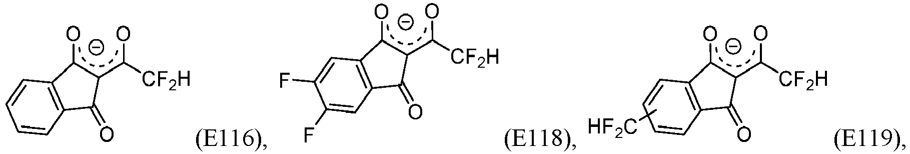

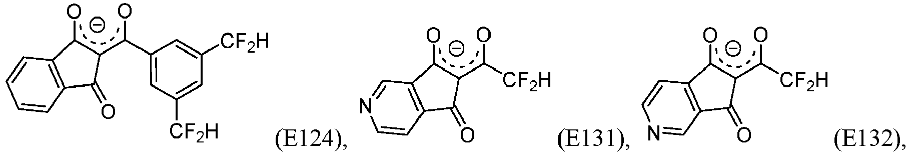

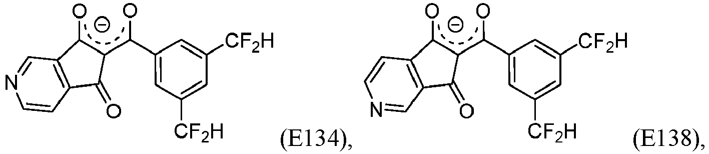

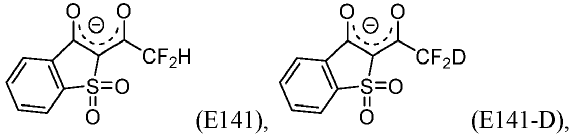

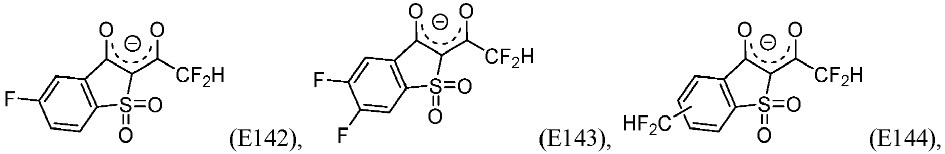

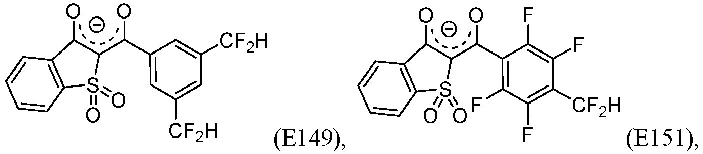

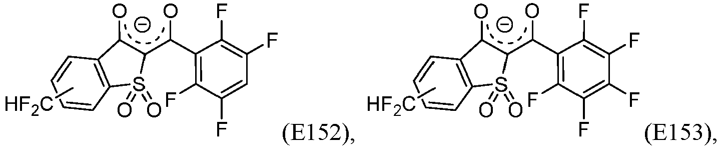

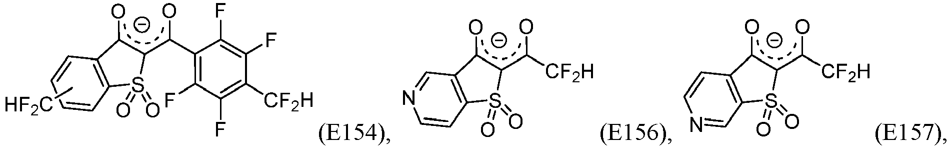

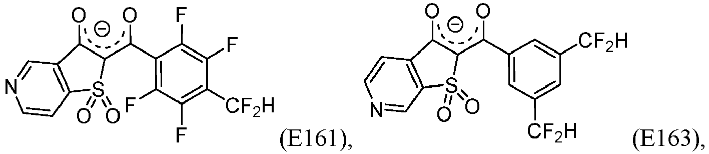

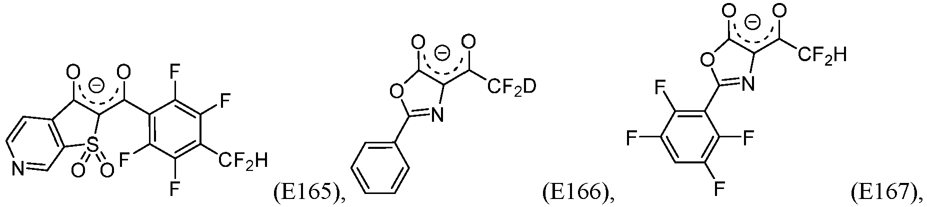

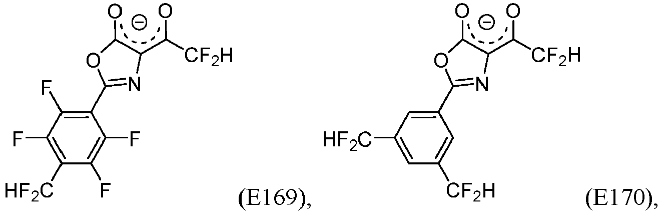

According to an embodiment, Ligand L of formula (II) is selected from the following compounds:

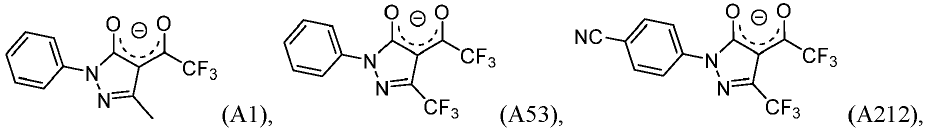

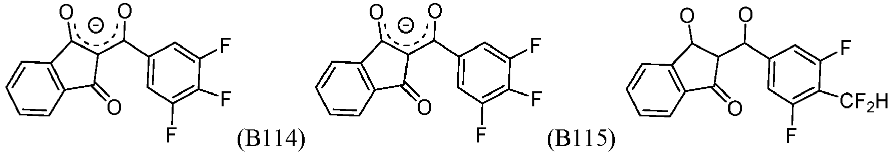

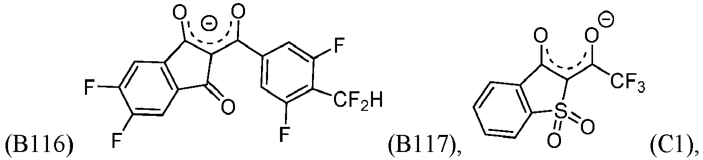

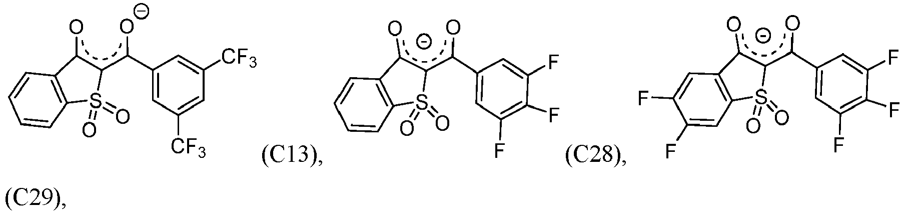

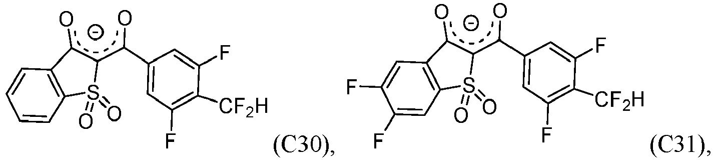

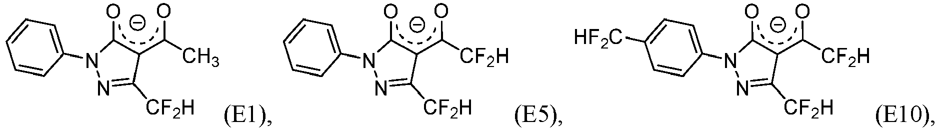

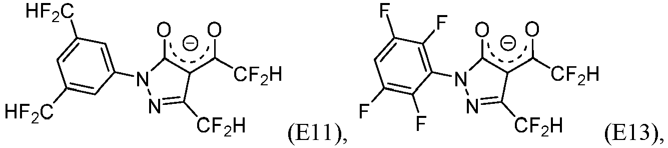

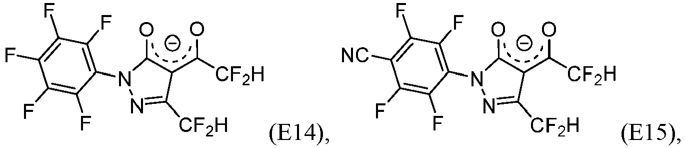

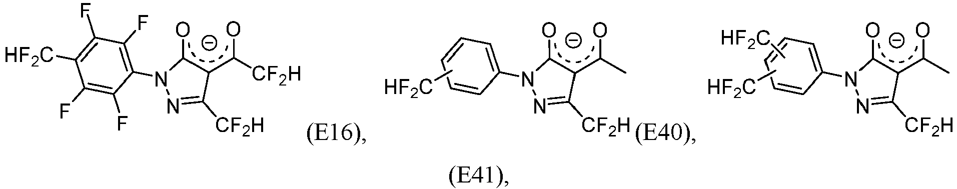

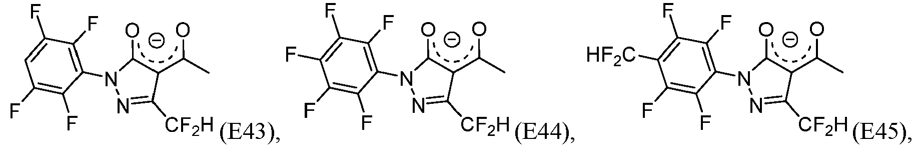

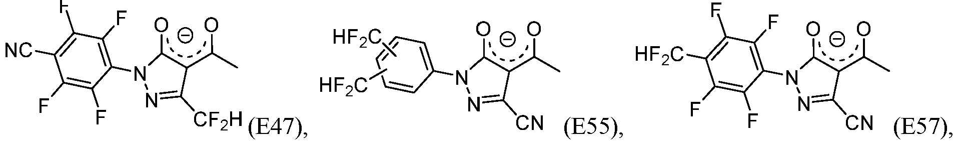

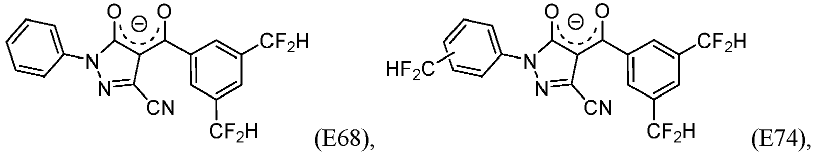

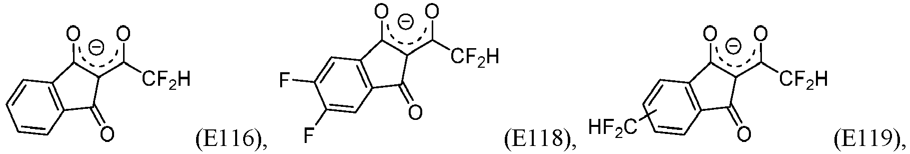

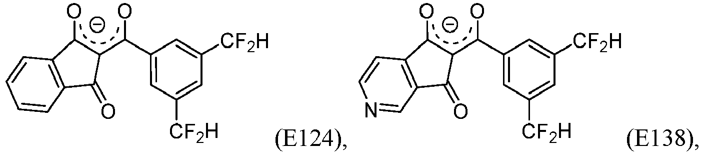

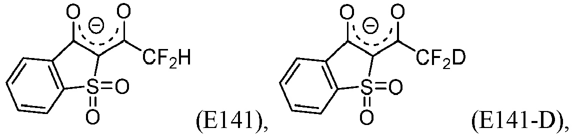

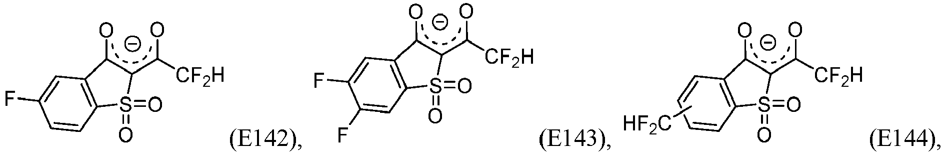

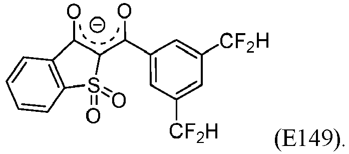

### Ancillary Ligand "AL"

According to one embodiment of the application, AL (the ancillary ligand) is selected from the group comprising H₂O, C₂ to C₄₀ mono- or multi-dentate ethers and C₂ to C₄₀ thioethers, C₂ to C₄₀ amines, C₂ to C₄₀ phosphine, C₂ to C₂₀ alkyl nitrile or C₂ to C₄₀ aryl nitrile, or a compound according to Formula (AL-I); 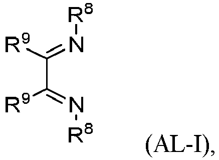 wherein
- R⁸ and R⁹: are independently selected from C₁ to C₂₀ alkyl, C₁ to C₂₀ heteroalkyl, C₆ to C₂₀ aryl, heteroaryl with 5 to 20 ring-forming atoms, halogenated or perhalogenated C₁ to C₂₀ alkyl, halogenated or perhalogenated C₁ to C₂₀ heteroalkyl, halogenated or perhalogenated C₆ to C₂₀ aryl, halogenated or perhalogenated heteroaryl with 5 to 20 ring-forming atoms, or at least one R⁶ and R⁷ are bridged and form a 5 to 20 member ring, or the two R⁶ and/or the two R⁷ are bridged and form a 5 to 40 member ring or form a 5 to 40 member ring comprising an unsubstituted or C₁ to C₁₂ substituted phenanthroline.

### The term "m"

The term "m" is an integer selected from 0 to 2, which corresponds to the oxidation number of M. According to one embodiment "m" is an integer selected from 0 or 1. According to another embodiment "m" is an integer selected from 1. According to another embodiment "m" is an integer selected from 2. Preferably "m" is an integer and may be selected from 0.

According to another embodiment of metal complex of formula (I), wherein n = 2 or 3; and/or m is an integer selected from 0 or 1, preferably 0.

### M of the metal complex of formula (I)

The term "M" represents a metal ion.

According to one embodiment, M of the metal complex of formula (I) may be selected from a metal ion wherein the corresponding metal has an electronegativity value according to Allen of ≥ 0.65 and ≤ 1.9.

The term "electronegativity value according to Allen" especially refers to Allen, Leland C. (1989). "Electronegativity is the average one-electron energy of the valence-shell electrons in ground-state free atoms", Journal of the American Chemical Society 111 (25): 9003-9014.

According to one embodiment, M may be selected from a transition metal or group III or V metal.

According to one embodiment, the atomic mass of M is selected in the range of ≥ 54 Da and ≤ 200 Da, preferably in the range of ≥ 55 Da and ≤ 138 Da.

According to an embodiment, M is not selected from Ce(IV); preferably not from Ce(IV), or Al(III).

According to an embodiment, M is a metal ion selected from Li(I), K(I), Rb(I), Cs(I), Ag(I), Cu(II), Zn(II), Pd(II), Ir(III), Al(III), Ga(III), Mn(II), Mn(III), Ru(III), In(III), Fe(II) or Fe(III) and Ce(IV); even more preferred M is a metal ion selected from Cu(II), Mn (II), Zn(II), In(III), Fe(III) and/or Ce(IV); especially preferred M is Cu(II), Zn(II) and/or Fe(III); wherein the number in brackets denotes the oxidation state.

Thereby, the LUMO energy level and/or thermal properties of the metal complex of formula (I) may be in the range suitable for use in organic electronic devices.

According to one embodiment, M is a metal ion selected from Li(I), K(I), Rb(I), Cs(I), Ag(I), Cu(II), Zn(II), Pd(II), Ir(III), Al(III), Ga(III), Mn(II), Mn(III), Ru(III), In(III), Fe(II) or Fe(III) and Ce(IV); even more preferred M is a metal ion selected from Cu(II), Mn (II), Zn(II), Fe(III) and/or Ce(IV); especially preferred M is Cu(II), Zn(II) and/or Fe(III); and L is selected from the formulae (II) and (IIa) to (IIg).

According to one embodiment, M is a metal ion selected from Li(I), K(I), Rb(I), Cs(I), Ag(I), Cu(II), Zn(II), Pd(II), Ir(III), Al(III), Ga(III), Mn(II), Mn(III), Ru(III), In(III), Fe(II) or Fe(III) and Ce(IV); even more preferred M is a metal ion selected from Cu(II), Mn (II), Zn(II), Fe(III) and/or Ce(IV); especially preferred M is Cu(II), Zn(II) and/or Fe(III); and L is selected from the formulae (IIb) to (IIg).

### Metal complex of formula (I)

According to an embodiment, wherein the metal complex of formula (I) is selected from one of the following compounds:

| **Name:** | **Metal M** | **Valency n** | **Ligand L** |
|---|---|---|---|
| 5MC1 | Ag(I) | 1 | A1 |
| 5MC2 | Ir(III) | 3 | A1 |
| 5MC3 | In(III) | 3 | A1 |
| 5MC4 | Al(III) | 3 | A1 |
| 5MC5 | Mn(III) | 3 | A1 |
| 5MC6 | Cu(II) | 2 | A1 |
| 5MC7 | Fe(III) | 3 | A1 |
| 5MC8 | Ru(III) | 3 | A1 |
| 5MC9 | Ce(IV) | 4 | A1 |
| 5MC10 | Cu(II) | 2 | A161 |
| 5MC11 | Cu(II) | 2 | A158 |
| 5MC12 | Cu(II) | 2 | A164 |
| 5MC13 | Cu(II) | 2 | A160 |
| 5MC14 | Cu(II) | 2 | A166 |
| 5MC15 | Cu(II) | 2 | A171 |
| 5MC16 | Cu(II) | 2 | A174 |
| 5MC17 | Cu(II) | 2 | A168 |
| 5MC18 | Cu(II) | 2 | A177 |
| 5MC19 | Cu(II) | 2 | A170 |
| 5MC20 | Cu(II) | 2 | A180 |
| 5MC21 | Cu(II) | 2 | A185 |
| 5MC22 | Cu(II) | 2 | A188 |
| 5MC23 | Cu(II) | 2 | A182 |
| 5MC24 | Cu(II) | 2 | A191 |
| 5MC25 | Cu(II) | 2 | A184 |
| 5MC26 | Cu(II) | 2 | A194 |
| 5MC27 | Cu(II) | 2 | A202 |
| 5MC28 | Cu(II) | 2 | A204 |
| 5MC29 | Cu(II) | 2 | A199 |
| 5MC30 | Cu(II) | 2 | A207 |
| 5MC31 | Cu(II) | 2 | A201 |
| 5MC32 | Cu(II) | 2 | A169 |
| 5MC33 | Fe(III) | 3 | A161 |
| 5MC34 | Fe(III) | 3 | A158 |
| 5MC35 | Fe(III) | 3 | A164 |
| 5MC36 | Fe(III) | 3 | A160 |
| 5MC37 | Fe(III) | 3 | A166 |
| 5MC38 | Fe(III) | 3 | A171 |
| 5MC39 | Fe(III) | 3 | A174 |
| 5MC40 | Fe(III) | 3 | A168 |
| 5MC41 | Fe(III) | 3 | A178 |
| 5MC42 | Fe(III) | 3 | A170 |
| 5MC43 | Fe(III) | 3 | A180 |
| 5MC44 | Fe(III) | 3 | A185 |
| 5MC45 | Fe(III) | 3 | A188 |
| 5MC46 | Fe(III) | 3 | A182 |
| 5MC47 | Fe(III) | 3 | A191 |
| 5MC48 | Fe(III) | 3 | A184 |
| 5MC49 | Fe(III) | 3 | A194 |
| 5MC50 | Fe(III) | 3 | A202 |
| 5MC51 | Fe(III) | 3 | A204 |
| 5MC52 | Fe(III) | 3 | A199 |
| 5MC53 | Fe(III) | 3 | A207 |
| 5MC54 | Fe(III) | 3 | A201 |
| 5MC55 | Fe(III) | 3 | A169 |
| 5MC56 | Cu(II) | 2 | A9 |
| 5MC57 | Cu(II) | 2 | A53 |
| 5MC58 | Cu(II) | 2 | A85 |
| 5MC59 | Cu(II) | 2 | A212 |
| 5MC60 | Fe(III) | 3 | A212 |
| 5MC61 | Cu | 2 | A213 |
| 5MC62 | Cu | 2 | A214 |
| 5MC63 | Cu | 2 | A215 |
| 5MC64 | Cu | 2 | A216 |
| 5MC65 | Cu | 2 | A217 |
| 5MC66 | Mn(II) | 2 | A213 |
| 5MC67 | Mn(II) | 2 | A216 |
| 5MC68 | Zn | 2 | A213 |
| 5MC69 | Zn | 2 | A216 |
| MC-1 | Cu | 2 | E1 |
| MC-2 | Fe | 3 | E1 |
| MC-3 | Mn | 2 | E1 |
| MC-4 | Zn | 2 | E1 |
| MC-66 | Mn | 3 | E1 |
| MC-75 | Ru | 3 | E1 |
| MC-84 | In | 3 | E1 |
| MC-93 | Al | 3 | E1 |
| MC-102 | Ce | 4 | E1 |
| MC-5 | Cu | 2 | E3 |
| MC-6 | Fe | 3 | E3 |
| MC-7 | Mn | 2 | E3 |
| MC-8 | Zn | 2 | E3 |
| MC-67 | Mn | 3 | E3 |
| MC-76 | Ru | 3 | E3 |
| MC-8 5 | In | 3 | E3 |
| MC-94 | Al | 3 | E3 |
| MC-103 | Ce | 4 | E3 |
| MC-9 | Cu | 2 | E5 |
| MC-10 | Fe | 3 | E5 |
| MC-11 | Mn | 2 | E5 |
| MC-12 | Zn | 2 | E5 |
| MC-68 | Mn | 3 | E5 |
| MC-77 | Ru | 3 | E5 |
| MC-86 | In | 3 | E5 |
| MC-95 | Al | 3 | E5 |
| MC-104 | Ce | 4 | E5 |
| MC-13 | Cu | 2 | E6 |
| MC-14 | Fe | 3 | E6 |
| MC-15 | Mn | 2 | E6 |
| MC-16 | Zn | 2 | E6 |
| MC-69 | Mn | 3 | E6 |
| MC-78 | Ru | 3 | E6 |
| MC-87 | In | 3 | E6 |
| MC-96 | Al | 3 | E6 |
| MC-105 | Ce | 4 | E6 |
| MC-17 | Cu | 2 | E7 |
| MC-18 | Fe | 3 | E7 |
| MC-19 | Mn | 2 | E7 |
| MC-20 | Zn | 2 | E7 |
| MC-71 | Mn | 3 | E7 |
| MC-80 | Ru | 3 | E7 |
| MC-89 | In | 3 | E7 |
| MC-98 | Al | 3 | E7 |
| MC-107 | Ce | 4 | E7 |
| MC-65 | Cu | 2 | E13 |
| MC-21 | Cu | 2 | E48 |
| MC-22 | Fe | 3 | E48 |
| MC-23 | Mn | 2 | E48 |
| MC-24 | Zn | 2 | E48 |
| MC-70 | Mn | 3 | E48 |
| MC-79 | Ru | 3 | E48 |
| MC-88 | In | 3 | E48 |
| MC-97 | Al | 3 | E48 |
| MC-106 | Ce | 4 | E48 |
| 6MC1 | Cu | 2 | B1 |
| 6MC2 | Cu | 2 | B9 |
| 6MC3 | Cu | 2 | B14 |
| 6MC4 | Cu | 2 | B71 |
| 6MC5 | Cu | 2 | B17 |
| 6MC6 | Cu | 2 | B30 |
| 6MC7 | Cu | 2 | B33 |
| 6MC8 | Cu | 2 | B83 |
| 6MC9 | Cu | 2 | B84 |
| 6MC10 | Cu | 2 | B103 |
| 6MC11 | Fe | 2 | B1 |
| 6MC12 | Fe | 2 | B9 |
| 6MC13 | Fe | 2 | B14 |
| 6MC14 | Fe | 2 | B71 |
| 6MC15 | Fe | 2 | B17 |
| 6MC16 | Fe | 2 | B30 |
| 6MC17 | Fe | 2 | B33 |
| 6MC18 | Fe | 2 | B83 |
| 6MC19 | Fe | 2 | B84 |
| 6MC20 | Fe | 2 | B103 |
| 6MC21 | Fe | 3 | B9 |
| 6MC22 | Fe | 3 | B14 |
| 6MC23 | Fe | 3 | B71 |
| 6MC24 | Fe | 3 | B17 |
| 6MC25 | Fe | 3 | B30 |
| 6MC26 | Fe | 3 | B33 |
| 6MC27 | Fe | 3 | B83 |
| 6MC28 | Fe | 3 | B84 |
| 6MC29 | Fe | 3 | B103 |
| 6MC30 | Fe | 3 | B109 |
| 6MC31 | Fe | 3 | B110 |
| 6MC32 | Fe | 3 | B111 |
| 6MC33 | Fe | 3 | B112 |
| 6MC34 | Fe | 3 | B113 |
| 6MC35 | Fe | 2 | B5 |
| 6MC36 | Fe | 3 | B5 |
| 6MC37 | Cu | 2 | B5 |
| 6MC38 | In | 3 | B1 |
| 6MC39 | Cu | 2 | B114 |
| 6MC40 | Cu | 2 | B115 |
| 6MC41 | Cu | 2 | B116 |
| 6MC42 | Cu | 2 | B117 |
| 6MC43 | Mn | 2 | B115 |
| 6MC44 | Mn | 2 | B117 |
| 6MC45 | Zn | 2 | B114 |
| 6MC46 | Zn | 2 | B115 |
| 6MC47 | Fe | 3 | B114 |
| 6MC48 | Fe | 3 | B115 |
| MC-25 | Cu | 2 | E116 |
| MC-26 | Fe | 3 | E116 |
| MC-27 | Mn | 2 | E116 |
| MC-28 | Zn | 2 | E116 |
| MC-72 | Mn | 3 | E116 |
| MC-81 | Ru | 3 | E116 |
| MC-90 | In | 3 | E116 |
| MC-99 | Al | 3 | E116 |
| MC-108 | Ce | 4 | E116 |
| MC-115 | Mn | 2 | E116-D |
| MC-116 | Fe | 3 | E116-D |
| MC-117 | Cu | 2 | E116-D |
| MC-118 | Zn | 2 | E116-D |
| MC-29 | Cu | 2 | E117 |
| MC-30 | Fe | 3 | E117 |
| MC-31 | Mn | 2 | E117 |
| MC-32 | Zn | 2 | E117 |
| MC-33 | Cu | 2 | E118 |
| MC-34 | Fe | 3 | E118 |
| MC-35 | Mn | 2 | E118 |
| MC-36 | Zn | 2 | E118 |
| 7MC1 | Cu | 2 | C1 |
| 7MC2 | Cu | 2 | C2 |
| 7MC3 | Cu | 2 | C3 |
| 7MC4 | Cu | 2 | C4 |
| 7MC5 | Cu | 2 | C5 |
| 7MC6 | Cu | 2 | C6 |
| 7MC7 | Cu | 2 | C7 |
| 7MC8 | Cu | 2 | C8 |
| 7MC9 | Cu | 2 | C9 |
| 7MC10 | Cu | 2 | C10 |
| 7MC11 | Cu | 2 | C11 |
| 7MC12 | Cu | 2 | C12 |
| 7MC13 | Cu | 2 | C13 |
| 7MC14 | Cu | 2 | C14 |
| 7MC15 | Cu | 2 | C15 |
| 7MC16 | Cu | 2 | C16 |
| 7MC17 | Cu | 2 | C17 |
| 7MC18 | Cu | 2 | C18 |
| 7MC19 | Cu | 2 | C19 |
| 7MC20 | Cu | 2 | C20 |
| 7MC21 | Cu | 2 | C21 |
| 7MC22 | Cu | 2 | C22 |
| 7MC23 | Cu | 2 | C23 |
| 7MC24 | Cu | 2 | C24 |
| 7MC25 | Cu | 2 | C25 |
| 7MC26 | Cu | 2 | C26 |
| 7MC27 | Cu | 2 | C27 |
| 7MC28 | Fe | 3 | C1 |
| 7MC29 | Fe | 3 | C2 |
| 7MC30 | Fe | 3 | C3 |
| 7MC31 | Fe | 3 | C4 |
| 7MC32 | Fe | 3 | C5 |
| 7MC33 | Fe | 3 | C6 |
| 7MC34 | Fe | 3 | C7 |
| 7MC35 | Fe | 3 | C8 |
| 7MC36 | Fe | 3 | C9 |
| 7MC37 | Fe | 3 | C10 |
| 7MC38 | Fe | 3 | C11 |
| 7MC39 | Fe | 3 | C12 |
| 7MC40 | Fe | 3 | C13 |
| 7MC41 | Fe | 3 | C14 |
| 7MC42 | Fe | 3 | C15 |
| 7MC43 | Fe | 3 | C16 |
| 7MC44 | Fe | 3 | C17 |
| 7MC45 | Fe | 3 | C18 |
| 7MC46 | Fe | 3 | C19 |
| 7MC47 | Fe | 3 | C20 |
| 7MC48 | Fe | 3 | C21 |
| 7MC49 | Fe | 3 | C22 |
| 7MC50 | Fe | 3 | C23 |
| 7MC51 | Fe | 3 | C24 |
| 7MC52 | Fe | 3 | C25 |
| 7MC53 | Fe | 3 | C26 |
| 7MC54 | Fe | 3 | C27 |
| 7MC55 | Ce | 4 | C1 |
| 7MC56 | Ce | 4 | C13 |
| 7MC57 | In | 3 | C1 |
| 7MC58 | In | 3 | C13 |
| 7MC59 | Al | 3 | C1 |
| 7MC60 | Al | 3 | C13 |
| 7MC61 | Co | 3 | C1 |
| 7MC62 | Co | 3 | C13 |
| 7MC63 | Mn | 3 | C1 |
| 7MC64 | Mn | 3 | C13 |
| 7MC65 | Mn | 2 | C13 |
| 7MC66 | Ru | 3 | C1 |
| 7MC67 | Ru | 3 | C13 |
| 7MC68 | Cr | 3 | C1 |
| 7MC69 | Cr | 3 | C13 |
| 7MC70 | Cu | 2 | C28 |
| 7MC71 | Cu | 2 | C29 |
| 7MC72 | Cu | 2 | C30 |
| 7MC73 | Cu | 2 | C31 |
| 7MC74 | Mn | 2 | C29 |
| 7MC75 | Mn | 2 | C31 |
| 7MC76 | Zn | 2 | C28 |
| 7MC77 | Zn | 2 | C29 |
| 7MC78 | Fe | 3 | C30 |
| 7MC79 | Fe | 3 | C31 |
| MC-37 | Cu | 2 | E141 |
| MC-38 | Fe | 3 | E141 |
| MC-39 | Mn | 2 | E141 |
| MC-40 | Zn | 2 | E141 |
| MC-73 | Mn | 3 | E141 |
| MC-82 | Ru | 3 | E141 |
| MC-91 | In | 3 | E141 |
| MC-100 | Al | 3 | E141 |
| MC-109 | Ce | 4 | E141 |
| MC-111 | Mn | 2 | E141-D |
| MC-112 | Fe | 3 | E141-D |
| MC-113 | Cu | 2 | E141-D |
| MC-114 | Zn | 2 | E141-D |
| MC-41 | Cu | 2 | E142 |
| MC-42 | Fe | 3 | E142 |
| MC-43 | Mn | 2 | E142 |
| MC-44 | Zn | 2 | E142 |
| MC-45 | Cu | 2 | E143 |
| MC-46 | Fe | 3 | E143 |
| MC-47 | Mn | 2 | E143 |
| MC-48 | Zn | 2 | E143 |
| 8MC-1 | Cu | 2 | D1 |
| 8MC-2 | Cu | 2 | D2 |
| 8MC-3 | Cu | 2 | D3 |
| 8MC-4 | Cu | 2 | D4 |
| 8MC-5 | Cu | 2 | D5 |
| 8MC-6 | Cu | 2 | D6 |
| 8MC-7 | Cu | 2 | D7 |
| 8MC-8 | Cu | 2 | D8 |
| 8MC-9 | Cu | 2 | D9 |
| 8MC-10 | Cu | 2 | D10 |
| 8MC-11 | Cu | 2 | D11 |
| 8MC-12 | Cu | 2 | D12 |
| 8MC-13 | Cu | 2 | D13 |
| 8MC-14 | Cu | 2 | D14 |
| 8MC-15 | Cu | 2 | D16 |
| 8MC-16 | Cu | 2 | D17 |
| 8MC-17 | Cu | 2 | D18 |
| 8MC-18 | Cu | 2 | D19 |
| 8MC-19 | Cu | 2 | D20 |
| 8MC-20 | Cu | 2 | D21 |
| 8MC-21 | Cu | 2 | D22 |
| 8MC-22 | Cu | 2 | D23 |
| 8MC-23 | Cu | 2 | D24 |
| 8MC-24 | Cu | 2 | D25 |
| 8MC-25 | Cu | 2 | D26 |
| 8MC-26 | Cu | 2 | D27 |
| 8MC-27 | Cu | 2 | D28 |
| 8MC-28 | Cu | 2 | D29 |
| 8MC-29 | Cu | 2 | D30 |
| 8MC-30 | Cu | 2 | D31 |
| 8MC-31 | Cu | 2 | D32 |
| 8MC-32 | Cu | 2 | D33 |
| 8MC-33 | Cu | 2 | D34 |
| 8MC-34 | Cu | 2 | D35 |
| 8MC-35 | Cu | 2 | D36 |
| 8MC-36 | Cu | 2 | D37 |
| 8MC-37 | Cu | 2 | D38 |
| 8MC-38 | Cu | 2 | D39 |
| 8MC-39 | Cu | 2 | D40 |
| 8MC-40 | Cu | 2 | D41 |
| 8MC-41 | Cu | 2 | D42 |
| 8MC-42 | Cu | 2 | D43 |
| 8MC-43 | Cu | 2 | D44 |
| 8MC-44 | Cu | 2 | D45 |
| 8MC-45 | Cu | 2 | D46 |
| 8MC-46 | Cu | 2 | D47 |
| 8MC-47 | Cu | 2 | D48 |
| 8MC-48 | Cu | 2 | D49 |
| 8MC-49 | Cu | 2 | D50 |
| 8MC-50 | Cu | 2 | D51 |
| 8MC-51 | Cu | 2 | D52 |
| 8MC-52 | Cu | 2 | D53 |
| 8MC-53 | Cu | 2 | D54 |
| 8MC-54 | Cu | 2 | D55 |
| 8MC-55 | Cu | 2 | D56 |
| 8MC-56 | Cu | 2 | D57 |
| 8MC-57 | Cu | 2 | D58 |
| 8MC-58 | Cu | 2 | D59 |
| 8MC-59 | Cu | 2 | D60 |
| 8MC-60 | Cu | 2 | D61 |
| 8MC-61 | Cu | 2 | D62 |
| 8MC-62 | Cu | 2 | D63 |
| 8MC-63 | Cu | 2 | D64 |
| 8MC-64 | Cu | 2 | D65 |
| 8MC-65 | Cu | 2 | D66 |
| 8MC-66 | Cu | 2 | D67 |
| 8MC-67 | Cu | 2 | D68 |
| 8MC-68 | Cu | 2 | D69 |
| 8MC-69 | Cu | 2 | D70 |
| 8MC-70 | Cu | 2 | D71 |
| 8MC-71 | Cu | 2 | D72 |
| 8MC-72 | Cu | 2 | D73 |
| 8MC-73 | Cu | 2 | D74 |
| 8MC-74 | Cu | 2 | D75 |
| 8MC-75 | Cu | 2 | D76 |
| 8MC-76 | Cu | 2 | D77 |
| 8MC-77 | Zn | 2 | D1 |
| 8MC-78 | Zn | 2 | D2 |
| 8MC-79 | Zn | 2 | D3 |
| 8MC-80 | Zn | 2 | D4 |
| 8MC-81 | Zn | 2 | D5 |
| 8MC-82 | Zn | 2 | D6 |
| 8MC-83 | Zn | 2 | D7 |
| 8MC-84 | Zn | 2 | D8 |
| 8MC-85 | Zn | 2 | D9 |
| 8MC-86 | Zn | 2 | D10 |
| 8MC-87 | Zn | 2 | D11 |
| 8MC-88 | Zn | 2 | D12 |
| 8MC-89 | Zn | 2 | D13 |
| 8MC-90 | Zn | 2 | D14 |
| 8MC-91 | Zn | 2 | D16 |
| 8MC-92 | Zn | 2 | D17 |
| 8MC-93 | Zn | 2 | D18 |
| 8MC-94 | Zn | 2 | D19 |
| 8MC-95 | Zn | 2 | D20 |
| 8MC-96 | Zn | 2 | D21 |
| 8MC-97 | Zn | 2 | D22 |
| 8MC-98 | Zn | 2 | D23 |
| 8MC-99 | Zn | 2 | D24 |
| 8MC-100 | Zn | 2 | D25 |
| 8MC-101 | Zn | 2 | D26 |
| 8MC-102 | Zn | 2 | D27 |
| 8MC-103 | Zn | 2 | D28 |
| 8MC-104 | Zn | 2 | D29 |
| 8MC-105 | Zn | 2 | D30 |
| 8MC-106 | Zn | 2 | D31 |
| 8MC-107 | Zn | 2 | D32 |
| 8MC-108 | Zn | 2 | D33 |
| 8MC-109 | Zn | 2 | D34 |
| 8MC-110 | Zn | 2 | D35 |
| 8MC-111 | Zn | 2 | D36 |
| 8MC-112 | Zn | 2 | D37 |
| 8MC-113 | Zn | 2 | D38 |
| 8MC-114 | Zn | 2 | D39 |
| 8MC-115 | Zn | 2 | D40 |
| 8MC-116 | Zn | 2 | D41 |
| 8MC-117 | Zn | 2 | D42 |
| 8MC-118 | Zn | 2 | D43 |
| 8MC-119 | Zn | 2 | D44 |
| 8MC-120 | Zn | 2 | D45 |
| 8MC-121 | Zn | 2 | D46 |
| 8MC-122 | Zn | 2 | D47 |
| 8MC-123 | Zn | 2 | D48 |
| 8MC-124 | Zn | 2 | D49 |
| 8MC-125 | Zn | 2 | D50 |
| 8MC-126 | Zn | 2 | D51 |
| 8MC-127 | Zn | 2 | D52 |
| 8MC-128 | Zn | 2 | D53 |
| 8MC-129 | Zn | 2 | D54 |
| 8MC-130 | Zn | 2 | D55 |
| 8MC-131 | Zn | 2 | D56 |
| 8MC-132 | Zn | 2 | D57 |
| 8MC-133 | Zn | 2 | D58 |
| 8MC-134 | Zn | 2 | D59 |
| 8MC-135 | Zn | 2 | D60 |
| 8MC-136 | Zn | 2 | D61 |
| 8MC-137 | Zn | 2 | D62 |
| 8MC-138 | Zn | 2 | D63 |
| 8MC-139 | Zn | 2 | D64 |
| 8MC-140 | Zn | 2 | D65 |
| 8MC-141 | Zn | 2 | D66 |
| 8MC-142 | Zn | 2 | D67 |
| 8MC-143 | Zn | 2 | D68 |
| 8MC-144 | Zn | 2 | D69 |
| 8MC-145 | Zn | 2 | D70 |
| 8MC-146 | Zn | 2 | D71 |
| 8MC-147 | Zn | 2 | D72 |
| 8MC-148 | Zn | 2 | D73 |
| 8MC-149 | Zn | 2 | D74 |
| 8MC-150 | Zn | 2 | D75 |
| 8MC-151 | Zn | 2 | D76 |
| 8MC-152 | Zn | 2 | D77 |
| 8MC-153 | Mn | 2 | D1 |
| 8MC-154 | Mn | 2 | D2 |
| 8MC-155 | Mn | 2 | D3 |
| 8MC-156 | Mn | 2 | D4 |
| 8MC-157 | Mn | 2 | D5 |
| 8MC-158 | Mn | 2 | D6 |
| 8MC-159 | Mn | 2 | D7 |
| 8MC-160 | Mn | 2 | D8 |
| 8MC-161 | Mn | 2 | D9 |
| 8MC-162 | Mn | 2 | D10 |
| 8MC-163 | Mn | 2 | D11 |
| 8MC-164 | Mn | 2 | D12 |
| 8MC-165 | Mn | 2 | D13 |
| 8MC-166 | Mn | 2 | D14 |
| 8MC-167 | Mn | 2 | D16 |
| 8MC-168 | Mn | 2 | D17 |
| 8MC-169 | Mn | 2 | D18 |
| 8MC-170 | Mn | 2 | D19 |
| 8MC-171 | Mn | 2 | D20 |
| 8MC-172 | Mn | 2 | D21 |
| 8MC-173 | Mn | 2 | D22 |
| 8MC-174 | Mn | 2 | D23 |
| 8MC-175 | Mn | 2 | D24 |
| 8MC-176 | Mn | 2 | D25 |
| 8MC-177 | Mn | 2 | D26 |
| 8MC-178 | Mn | 2 | D27 |
| 8MC-179 | Mn | 2 | D28 |
| 8MC-180 | Mn | 2 | D29 |
| 8MC-181 | Mn | 2 | D30 |
| 8MC-182 | Mn | 2 | D31 |
| 8MC-183 | Mn | 2 | D32 |
| 8MC-184 | Mn | 2 | D33 |
| 8MC-185 | Mn | 2 | D34 |
| 8MC-186 | Mn | 2 | D35 |
| 8MC-187 | Mn | 2 | D36 |
| 8MC-188 | Mn | 2 | D37 |
| 8MC-189 | Mn | 2 | D38 |
| 8MC-190 | Mn | 2 | D39 |
| 8MC-191 | Mn | 2 | D40 |
| 8MC-192 | Mn | 2 | D41 |
| 8MC-193 | Mn | 2 | D42 |
| 8MC-194 | Mn | 2 | D43 |
| 8MC-195 | Mn | 2 | D44 |
| 8MC-196 | Mn | 2 | D45 |
| 8MC-197 | Mn | 2 | D46 |
| 8MC-198 | Mn | 2 | D47 |
| 8MC-199 | Mn | 2 | D48 |
| 8MC-200 | Mn | 2 | D49 |
| 8MC-201 | Mn | 2 | D50 |
| 8MC-202 | Mn | 2 | D51 |
| 8MC-203 | Mn | 2 | D52 |
| 8MC-204 | Mn | 2 | D53 |
| 8MC-205 | Mn | 2 | D54 |
| 8MC-206 | Mn | 2 | D55 |
| 8MC-207 | Mn | 2 | D56 |
| 8MC-208 | Mn | 2 | D57 |
| 8MC-209 | Mn | 2 | D58 |
| 8MC-210 | Mn | 2 | D59 |
| 8MC-211 | Mn | 2 | D60 |
| 8MC-212 | Mn | 2 | D61 |
| 8MC-213 | Mn | 2 | D62 |
| 8MC-214 | Mn | 2 | D63 |
| 8MC-215 | Mn | 2 | D64 |
| 8MC-216 | Mn | 2 | D65 |
| 8MC-217 | Mn | 2 | D66 |
| 8MC-218 | Mn | 2 | D67 |
| 8MC-219 | Mn | 2 | D68 |
| 8MC-220 | Mn | 2 | D69 |
| 8MC-221 | Mn | 2 | D70 |
| 8MC-222 | Mn | 2 | D71 |
| 8MC-223 | Mn | 2 | D72 |
| 8MC-224 | Mn | 2 | D73 |
| 8MC-225 | Mn | 2 | D74 |
| 8MC-226 | Mn | 2 | D75 |
| 8MC-227 | Mn | 2 | D76 |
| 8MC-228 | Mn | 2 | D77 |
| 8MC-229 | Fe | 3 | D1 |
| 8MC-230 | Fe | 3 | D2 |
| 8MC-231 | Fe | 3 | D3 |
| 8MC-232 | Fe | 3 | D4 |
| 8MC-233 | Fe | 3 | D5 |
| 8MC-234 | Fe | 3 | D6 |
| 8MC-235 | Fe | 3 | D7 |
| 8MC-236 | Fe | 3 | D8 |
| 8MC-237 | Fe | 3 | D9 |
| 8MC-238 | Fe | 3 | D10 |
| 8MC-239 | Fe | 3 | D11 |
| 8MC-240 | Fe | 3 | D12 |
| 8MC-241 | Fe | 3 | D13 |
| 8MC-242 | Fe | 3 | D14 |
| 8MC-243 | Fe | 3 | D16 |
| 8MC-244 | Fe | 3 | D17 |
| 8MC-245 | Fe | 3 | D18 |
| 8MC-246 | Fe | 3 | D19 |
| 8MC-247 | Fe | 3 | D20 |
| 8MC-248 | Fe | 3 | D21 |
| 8MC-249 | Fe | 3 | D22 |
| 8MC-250 | Fe | 3 | D23 |
| 8MC-251 | Fe | 3 | D24 |
| 8MC-252 | Fe | 3 | D25 |
| 8MC-253 | Fe | 3 | D26 |
| 8MC-254 | Fe | 3 | D27 |
| 8MC-255 | Fe | 3 | D28 |
| 8MC-256 | Fe | 3 | D29 |
| 8MC-257 | Fe | 3 | D30 |
| 8MC-258 | Fe | 3 | D31 |
| 8MC-259 | Fe | 3 | D32 |
| 8MC-260 | Fe | 3 | D34 |
| 8MC-261 | Fe | 3 | D35 |
| 8MC-262 | Fe | 3 | D36 |
| 8MC-263 | Fe | 3 | D37 |
| 8MC-264 | Fe | 3 | D38 |
| 8MC-265 | Fe | 3 | D39 |
| 8MC-266 | Fe | 3 | D40 |
| 8MC-267 | Fe | 3 | D41 |
| 8MC-268 | Fe | 3 | D42 |
| 8MC-269 | Fe | 3 | D43 |
| 8MC-270 | Fe | 3 | D44 |
| 8MC-271 | Fe | 3 | D45 |
| 8MC-272 | Fe | 3 | D46 |
| 8MC-273 | Fe | 3 | D47 |
| 8MC-274 | Fe | 3 | D48 |
| 8MC-275 | Fe | 3 | D49 |
| 8MC-276 | Fe | 3 | D50 |
| 8MC-277 | Fe | 3 | D51 |
| 8MC-278 | Fe | 3 | D52 |
| 8MC-279 | Fe | 3 | D53 |
| 8MC-280 | Fe | 3 | D54 |
| 8MC-281 | Fe | 3 | D55 |
| 8MC-282 | Fe | 3 | D56 |
| 8MC-283 | Fe | 3 | D57 |
| 8MC-284 | Fe | 3 | D58 |
| 8MC-285 | Fe | 3 | D59 |
| 8MC-286 | Fe | 3 | D60 |
| 8MC-287 | Fe | 3 | D61 |
| 8MC-288 | Fe | 3 | D62 |
| 8MC-289 | Fe | 3 | D63 |
| 8MC-290 | Fe | 3 | D64 |
| 8MC-291 | Fe | 3 | D65 |
| 8MC-292 | Fe | 3 | D66 |
| 8MC-293 | Fe | 3 | D67 |
| 8MC-294 | Fe | 3 | D68 |
| 8MC-295 | Fe | 3 | D69 |
| 8MC-296 | Fe | 3 | D70 |
| 8MC-297 | Fe | 3 | D71 |
| 8MC-298 | Fe | 3 | D72 |
| 8MC-299 | Fe | 3 | D73 |
| 8MC-300 | Fe | 3 | D74 |
| 8MC-301 | Fe | 3 | D75 |
| 8MC-302 | Fe | 3 | D76 |
| 8MC-303 | Fe | 3 | D77 |
| 8MC-304 | Al | 3 | D2 |
| 8MC-305 | Al | 3 | D10 |
| 8MC-306 | Al | 3 | D11 |
| 8MC-307 | Al | 3 | D14 |
| 8MC-308 | Al | 3 | D15 |
| 8MC-309 | Al | 3 | D22 |
| 8MC-310 | Al | 3 | D23 |
| 8MC-311 | Al | 3 | D26 |
| 8MC-312 | Al | 3 | D27 |
| 8MC-313 | Al | 3 | D30 |
| 8MC-314 | Al | 3 | D31 |
| 8MC-315 | Al | 3 | D46 |
| 8MC-316 | Al | 3 | D47 |
| 8MC-317 | Al | 3 | D50 |
| 8MC-318 | Al | 3 | D51 |
| 8MC-319 | Al | 3 | D54 |
| 8MC-320 | Al | 3 | D55 |
| 8MC-321 | Al | 3 | D65 |
| 8MC-322 | Al | 3 | D69 |
| 8MC-323 | Al | 3 | D73 |
| 8MC-324 | Al | 3 | D77 |
| 8MC-325 | Al | 3 | D3 |
| 8MC-326 | In | 3 | D2 |
| 8MC-327 | In | 3 | D3 |
| 8MC-328 | In | 3 | D10 |
| 8MC-329 | In | 3 | D11 |
| 8MC-330 | In | 3 | D14 |
| 8MC-331 | In | 3 | D15 |
| 8MC-332 | In | 3 | D22 |
| 8MC-333 | In | 3 | D23 |
| 8MC-334 | In | 3 | D26 |
| 8MC-335 | In | 3 | D27 |
| 8MC-336 | In | 3 | D30 |
| 8MC-337 | In | 3 | D31 |
| 8MC-338 | In | 3 | D46 |
| 8MC-339 | In | 3 | D47 |
| 8MC-340 | In | 3 | D50 |
| 8MC-341 | In | 3 | D51 |
| 8MC-342 | In | 3 | D54 |
| 8MC-343 | In | 3 | D55 |
| 8MC-344 | In | 3 | D65 |
| 8MC-345 | In | 3 | D69 |
| 8MC-346 | In | 3 | D73 |
| 8MC-347 | In | 3 | D77 |
| 8MC-348 | Mn | 3 | D2 |
| 8MC-349 | Mn | 3 | D3 |
| 8MC-350 | Mn | 3 | D10 |
| 8MC-351 | Mn | 3 | D11 |
| 8MC-352 | Mn | 3 | D14 |
| 8MC-353 | Mn | 3 | D15 |
| 8MC-354 | Mn | 3 | D22 |
| 8MC-355 | Mn | 3 | D23 |
| 8MC-356 | Mn | 3 | D26 |
| 8MC-357 | Mn | 3 | D27 |
| 8MC-358 | Mn | 3 | D30 |
| 8MC-359 | Mn | 3 | D31 |
| 8MC-360 | Mn | 3 | D46 |
| 8MC-361 | Mn | 3 | D47 |
| 8MC-362 | Mn | 3 | D50 |
| 8MC-363 | Mn | 3 | D51 |
| 8MC-364 | Mn | 3 | D54 |
| 8MC-365 | Mn | 3 | D55 |
| 8MC-366 | Mn | 3 | D65 |
| 8MC-367 | Mn | 3 | D69 |
| 8MC-368 | Mn | 3 | D73 |
| 8MC-369 | Mn | 3 | D77 |
| 8MC-370 | Ru | 3 | D2 |
| 8MC-371 | Ru | 3 | D3 |
| 8MC-372 | Ru | 3 | D10 |
| 8MC-373 | Ru | 3 | D11 |
| 8MC-374 | Ru | 3 | D14 |
| 8MC-375 | Ru | 3 | D15 |
| 8MC-376 | Ru | 3 | D22 |
| 8MC-377 | Ru | 3 | D23 |
| 8MC-378 | Ru | 3 | D26 |
| 8MC-379 | Ru | 3 | D27 |
| 8MC-380 | Ru | 3 | D30 |
| 8MC-381 | Ru | 3 | D31 |
| 8MC-382 | Ru | 3 | D46 |
| 8MC-383 | Ru | 3 | D47 |
| 8MC-384 | Ru | 3 | D50 |
| 8MC-385 | Ru | 3 | D51 |
| 8MC-386 | Ru | 3 | D54 |
| 8MC-387 | Ru | 3 | D55 |
| 8MC-388 | Ru | 3 | D65 |
| 8MC-389 | Ru | 3 | D69 |
| 8MC-390 | Ru | 3 | D73 |
| 8MC-391 | Ru | 3 | D77 |
| 8MC-392 | Ce | 4 | D10 |
| 8MC-393 | Ce | 4 | D11 |
| 8MC-394 | Ce | 4 | D22 |
| 8MC-395 | Ce | 4 | D23 |
| 8MC-396 | Ce | 4 | D58 |
| 8MC-397 | Ce | 4 | D65 |
| 8MC-398 | Ce | 4 | D77 |
| 8MC-398 | Ce | 4 | D77 |
| 8MC-399 | Cu | 2 | D78 |
| 8MC-400 | Cu | 2 | D79 |
| 8MC-401 | Cu | 2 | D80 |
| 8MC-402 | Cu | 2 | D81 |
| 8MC-403 | Zn | 2 | D78 |
| 8MC-404 | Zn | 2 | D79 |
| 8MC-405 | Zn | 2 | D80 |
| 8MC-406 | Zn | 2 | D81 |
| 8MC-407 | Mn | 2 | D78 |
| 8MC-408 | Mn | 2 | D79 |
| 8MC-409 | Mn | 2 | D80 |
| 8MC-410 | Mn | 2 | D81 |
| 8MC-411 | Fe | 3 | D78 |
| 8MC-412 | Fe | 3 | D79 |
| 8MC-413 | Fe | 3 | D80 |
| 8MC-414 | Fe | 3 | D81 |
| 8MC-415 | Cu | 2 | D82 |
| 8MC-416 | Cu | 2 | D83 |
| 8MC-416 | Zn | 2 | D82 |
| 8MC-417 | Zn | 2 | D83 |
| MC-49 | Cu | 2 | D2 |
| MC-50 | Fe | 3 | D2 |
| MC-51 | Mn | 2 | D2 |
| MC-52 | Zn | 2 | D2 |
| MC-74 | Mn | 3 | D2 |
| MC-83 | Ru | 3 | D2 |
| MC-92 | In | 3 | D2 |
| MC-101 | Al | 3 | D2 |
| MC-110 | Ce | 4 | D2 |
| MC-119 | Mn | 2 | E166 |
| MC-120 | Fe | 3 | E166 |
| MC-121 | Cu | 2 | E166 |
| MC-122 | Zn | 2 | E166 |
| MC-53 | Cu | 2 | D6 |
| MC-54 | Fe | 3 | D6 |
| MC-55 | Mn | 2 | D6 |
| MC-56 | Zn | 2 | D6 |
| MC-57 | Cu | 2 | E167 |
| MC-58 | Fe | 3 | E167 |
| MC-59 | Mn | 2 | E167 |
| MC-60 | Zn | 2 | E167 |
| MC-61 | Cu | 2 | D14 |
| MC-62 | Fe | 3 | D14 |
| MC-63 | Mn | 2 | D14 |
| MC-64 | Zn | 2 | D14 |

According to one embodiment the metal complex of formula (I) is selected from the following compounds:

| **Name:** | **Metal M** | **Valency n** | **Ligand L** |
|---|---|---|---|
| 5MC6 | Cu(II) | 2 | A1 |
| 5MC57 | Cu(II) | 2 | A53 |
| MC-9 | Cu | 2 | E5 |
| 6MC38 | In | 3 | B1 |
| 7MC13 | Cu | 2 | C13 |
| 5MC61 | Cu | 2 | A213 |
| 5MC62 | Cu | 2 | A214 |
| 5MC63 | Cu | 2 | A215 |
| 5MC64 | Cu | 2 | A216 |
| 5MC65 | Cu | 2 | A217 |
| 5MC66 | Mn(II) | 2 | A213 |
| 5MC67 | Mn(II) | 2 | A216 |
| 5MC68 | Zn | 2 | A213 |
| 5MC69 | Zn | 2 | A216 |
| 6MC39 | Cu | 2 | B114 |
| 6MC40 | Cu | 2 | B115 |
| 6MC41 | Cu | 2 | B116 |
| 6MC42 | Cu | 2 | B117 |
| 6MC43 | Mn | 2 | B115 |
| 6MC44 | Mn | 2 | B117 |
| 6MC45 | Zn | 2 | B114 |
| 6MC46 | Zn | 2 | B115 |
| 6MC47 | Fe | 3 | B114 |
| 6MC48 | Fe | 3 | B115 |
| 7MC70 | Cu | 2 | C28 |
| 7MC71 | Cu | 2 | C29 |
| 7MC72 | Cu | 2 | C30 |
| 7MC73 | Cu | 2 | C31 |
| 7MC74 | Mn | 2 | C29 |
| 7MC75 | Mn | 2 | C31 |
| 7MC76 | Zn | 2 | C28 |
| 7MC77 | Zn | 2 | C29 |
| 7MC78 | Fe | 3 | C30 |
| 7MC79 | Fe | 3 | C31 |
| 8MC-415 | Cu | 2 | D82 |
| 8MC-416 | Cu | 2 | D83 |
| 8MC-416 | Zn | 2 | D82 |
| 8MC-417 | Zn | 2 | D83 |

According to one embodiment the metal complex of formula (I) is selected from the following compounds:

| **Name:** | **Metal M** | **Valency n** | **Ligand L** |
|---|---|---|---|
| 5MC6 | Cu(II) | 2 | A1 |
| 5MC57 | Cu(II) | 2 | A53 |
| MC-9 | Cu | 2 | E5 |
| 6MC38 | In | 3 | B1 |
| 7MC13 | Cu | 2 | C13 |
| 5MC61 | Cu | 2 | A213 |
| 5MC62 | Cu | 2 | A214 |
| 5MC63 | Cu | 2 | A215 |
| 5MC64 | Cu | 2 | A216 |
| 5MC65 | Cu | 2 | A217 |
| 6MC39 | Cu | 2 | B114 |
| 6MC40 | Cu | 2 | B115 |
| 6MC41 | Cu | 2 | B116 |
| 6MC42 | Cu | 2 | B117 |
| 6MC43 | Mn | 2 | B115 |
| 6MC44 | Mn | 2 | B117 |
| 6MC45 | Zn | 2 | B114 |
| 6MC46 | Zn | 2 | B115 |
| 6MC47 | Fe | 3 | B114 |
| 6MC48 | Fe | 3 | B115 |
| 7MC70 | Cu | 2 | C28 |
| 7MC71 | Cu | 2 | C29 |
| 7MC72 | Cu | 2 | C30 |
| 7MC73 | Cu | 2 | C31 |
| 7MC74 | Mn | 2 | C29 |
| 7MC75 | Mn | 2 | C31 |
| 7MC76 | Zn | 2 | C28 |
| 7MC77 | Zn | 2 | C29 |
| 7MC78 | Fe | 3 | C30 |
| 7MC79 | Fe | 3 | C31 |

### Organic semiconductor layer

According to an embodiment of the present invention the metal complex of formula (I) can be present in an organic semiconductor layer.

The organic semiconductor layer may be formed on the anode layer or cathode layer by vacuum deposition, spin coating, printing, casting, slot-die coating, Langmuir-Blodgett (LB) deposition, or the like. When the organic semiconductor layer is formed using vacuum deposition, the deposition conditions may vary according to the compound(s) that are used to form the layer, and the desired structure and thermal properties of the layer. In general, however, conditions for vacuum deposition may include a deposition temperature of 100° C to 350° C, a pressure of 10⁻⁸ to 10⁻³ Torr (1 Torr equals 133.322 Pa), and a deposition rate of 0.1 to 10 nm/sec.

When the organic semiconductor layer is formed using spin coating or printing, coating conditions may vary according to the compound(s) that are used to form the layer, and the desired structure and thermal properties of the organic semiconductor layer. For example, the coating conditions may include a coating speed of about 2000 rpm to about 5000 rpm, and a thermal treatment temperature of about 80° C to about 200° C. Thermal treatment removes a solvent after the coating is performed.

The thickness of the organic semiconductor layer may be in the range from about 1 nm to about 20 nm, and for example, from about 2 nm to about 15 nm, alternatively about 2 nm to about 12 nm.

According to one embodiment of the present invention, the organic semiconductor layer may comprise:
- at least about ≥ 0.5 wt.-% to about ≤ 30 wt.-%, preferably about ≥ 0.5 wt.-% to about ≤ 20 wt.-%, and more preferred about ≥ 1 wt.-% to about ≤ 15 wt.-% of a metal complex of formula (I), and
- at least about ≥ 70 wt.-% to about ≤ 99.5 wt.-%, preferably about ≥ 80 wt.-% to about ≤ 99.5 wt.-%, and more preferred about ≥ 85 wt.-% to about ≤ 99 wt.-% of an electron donor compound; preferably the wt.-% of the metal complex of formula (I) is lower than the wt.-% of the electron donor compound; wherein the weight-% of the components are based on the total weight of the organic semiconductor layer.

According to one embodiment of the present invention, the organic semiconductor layer may comprise:
- at least about ≥ 0.5 wt.-% to about ≤ 30 wt.-%, preferably about ≥ 0.5 wt.-% to about ≤ 20 wt.-%, and more preferred about ≥ 1 wt.-% to about ≤ 15 wt.-% of a metal complex of formula (I), and
- at least about ≥ 70 wt.-% to about ≤ 99.5 wt.-%, preferably about ≥ 80 wt.-% to about ≤ 99.5 wt.-%, and more preferred about ≥ 85 wt.-% to about ≤ 99 wt.-% of an electron blocking compound; preferably the wt.-% of the metal complex of formula (I) is lower than the wt.-% of the electron blocking compound; wherein the weight-% of the components are based on the total weight of the organic semiconductor layer.

The electron blocking compound in the electron blocking layer may be selected from N,N-di([1,1'-biphenyl]-4-yl)-7,7-dimethyl-7H-fluoreno[4,3-b]benzofuran-10-amine ([1616706-52-5]), N,N-di([1,1'-biphenyl]-4-yl)-4'-(9H-carbazol-9-yl)-[1,1'-biphenyl]-4-amine ([1069137-74-1]), N,N-di([1,1'-biphenyl]-4-yl)-9-phenyl-9H-carbazol-2-amine ([1259388-72-1]), N¹-([1,1'-biphenyl]-4-yl)-N³-(dibenzo[b,d]thiophen-2-yl)-N¹,N³-diphenyl-5-(9-phenyl-9H-carbazol-2-yl)benzene-1,3-diamine ([1869085-48-2]), N¹-([1,1'-biphenyl]-4-yl)-N³,N³-diphenyl-N¹-(8-(9-phenyl-9H-carbazol-2-yl)dibenzo[b,d]thiophen-2-yl)benzene-1,3-diamine ([2055861-88-4]), N⁷-(dibenzo[b,d]thiophen-3-yl)-N²,N²,N⁷-triphenyldibenzo[b,d]thiophene-2,7-diamine ([2033134-06-2]), 7-(4-(dibenzo[b,d]thiophen-3-yl(phenyl)amino)phenyl)-N,N-diphenyldibenzo[b,d]thiophen-2-amine ([2641671-54-5]), N-([1,1'-biphenyl]-4-yl)-9,9-dimethyl-N-(3-(9-phenyl-9H-fluoren-9-yl)phenyl)-9H-fluoren-2-amine ([2226747-62-0]), N-([1,1'-biphenyl]-2-yl)-N-(9,9-dimethyl-9H-fluoren-2-yl)-9,9'-spirobi[fluoren]-4-amine ([1450933-44-4]), or N-([1,1'-biphenyl]-4-yl)-N-(2-(9,9-diphenyl-9H-fluoren-4-yl)phenyl)-9,9-dimethyl-9H-fluoren-2-amine ([1792219-00-1]), 2',7'-di-tert-butyl-N,N-bis(9,9-dimethyl-9H-fluoren-2-yl)-9,9'-spirobi[fluoren]-4-amine([2379778-94-4]);
preferably N-([1,1'-biphenyl]-4-yl)-9,9-dimethyl-N-(3-(9-phenyl-9H-fluoren-9-yl)phenyl)-9H-fluoren-2-amine ([2226747-62-0]), N-([1,1'-biphenyl]-2-yl)-N-(9,9-dimethyl-9H-fluoren-2-yl)-9,9'-spirobi[fluoren]-4-amine ([1450933-44-4]), or N-([1,1'-biphenyl]-4-yl)-N-(2-(9,9-diphenyl-9H-fluoren-4-yl)phenyl)-9,9-dimethyl-9H-fluoren-2-amine ([1792219-00-1]), 2',7'-di-tert-butyl-N,N-bis(9,9-dimethyl-9H-fluoren-2-yl)-9,9'-spirobi[fluoren]-4-amine([2379778-94-4]).

According to one embodiment of the present invention the organic semiconductor layer and/or the metal complex of formula (I) are non-emissive.

In the context of the present specification the term "essentially non-emissive" or "non-emissive" means that the contribution of the compound or layer to the visible emission spectrum from the device is less than 10%, preferably less than 5 % relative to the visible emission spectrum. The visible emission spectrum is an emission spectrum with a wavelength of about ≥ 380 nm to about ≤ 780 nm.

According to an embodiment, the semiconductor layer is a hole extraction layer. The term "hole extraction layer" especially means a layer which facilitates the extraction of holes from the photoconversion unit.

In other words, a hole extraction layer means a layer which facilitates the entry of an electron into the photoconversion unit, while a positive charge will be formed in the hole extraction layer.

According to one embodiment of the present invention the organic semiconductor layer is in direct contact to the photoconversion unit.

According to one embodiment of the present invention the organic semiconductor layer is an interlayer and in direct contact to the photoconversion unit.

According to one embodiment of the present invention the organic semiconductor layer is a hole extraction layer and in direct contact to the photoconversion unit.

According to one embodiment of the present invention the organic semiconductor layer is an interlayer and comprises a metal complex of formula (I).

According to one embodiment of the present invention the organic semiconductor layer is a hole extraction layer and comprises a metal complex of formula (I).

According to one embodiment of the present invention the organic semiconductor layer is an interlayer in direct contact to the photoconversion unit and comprises a metal complex of formula (I).

According to one embodiment of the present invention the organic semiconductor layer is a hole extraction layer in direct contact to the photoconversion unit and comprises a metal complex of formula (I).

### Photoconversion unit

In the following the further components of the photoconversion unit will be described in more detail whereby all features can be combined *ad libitum:*

### Electron donor compound

According to an embodiment, the electron donor compound absorbs light in the range of ≥ 380 nm to ≤ 2.5 µm, preferably ≥ 490 to ≤ 2.5 µm.

According to an embodiment, the electron donor compound has an energy gap Egap between its HOMO energy level and its LUMO energy level of ≥ 0.4 eV to ≤ 3.3 eV preferably ≥ 0.4 eV to ≤2.8 eV, wherein the energy gap, the HOMO energy level, and the LUMO energy level calculated with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany) wherein the optimized geometries and the HOMO and LUMO energy levels of the molecular structures are determined by applying the hybrid functional B3LYP with a 6-31G* basis set in the gas phase.

According to an embodiment, wherein the electron donor compound absorbs light in the wave length range of ≥ 380 nm to ≤ 2.5 µm, preferably ≥ 490 to ≤ 2.5 µm.

According to an embodiment, wherein the electron donor compound has an energy gap E_{gap} between its LUMO energy level and its HOMO energy level ≥ 0.4 eV to ≤ 3.3 eV preferably ≥ 0.4 eV to ≤2.8 eV,
wherein the energy gap, the HOMO energy level, and the LUMO energy level are calculated with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany) wherein the optimized geometries and the HOMO and LUMO energy levels of the molecular structures are determined by applying the hybrid functional B3LYP with a 6-31G* basis set in the gas phase.

According to an embodiment, wherein the electron donor compound has a LUMO energy level in the range of ≥ -4.0 eV to ≤ -1.0 eV, preferably ≥ -4.0 eV to ≤ -2.0 eV, more preferably ≥ - 4.0 eV to ≤-2.5 eV, wherein the LUMO energy level is calculated with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany) wherein the optimized geometries and the HOMO and LUMO energy levels of the molecular structures are determined by applying the hybrid functional B3LYP with a 6-31G* basis set in the gas phase.

According to an embodiment, the electron donor compound comprises a phthalocyanine compound, a perylene-based compound, a squaraine dye compound, subphthalocyanine (SubPc), zinc phthalocyanine (ZnPc), ditolyaminothienyl-benzothiadiazole-dicyanovinylene (DTDCTB), lead phthalocyanine (PbPc), 5,10,15,20-Tetraphenyl bisbenz[5,6]indeno[1,2,3-cd: 1',2',3'-lm]perylene (DBP), copper phthalocyanine (CuPc), tin phthalocyanine (SnPc), quinacridone, quinacridone-derivatives

### Electron acceptor compound

According to an embodiment, the electron acceptor compound has a LUMO energy level in the range of ≥ -4.5 eV to ≤ -2.5 eV, preferably ≥ -4.5 eV to ≤ -3.0 eV, wherein the LUMO energy level is calculated with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany) wherein the optimized geometries and the HOMO and LUMO energy levels of the molecular structures are determined by applying the hybrid functional B3LYP with a 6-31G* basis set in the gas phase.

According to an embodiment, the electron acceptor compound has a LUMO energy level in the range of ≥ -4.5 eV to ≤ -2.5 eV, preferably ≥ -4.5 eV to ≤ -3.0 eV, wherein the LUMO energy level is calculated with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany) wherein the optimized geometries and the HOMO and LUMO energy levels of the molecular structures are determined by applying the hybrid functional B3LYP with a 6-31G* basis set in the gas phase.

According to an embodiment, wherein the electron acceptor compound has an energy gap Egap between its LUMO energy level and its HOMO energy level in the range of ≥0.01 eV to ≤5.0 eV; preferably ≥0.4 eV to ≤3.3 eV, and more preferably ≥0.4 eV to ≤2.8 eV, wherein the energy gap, the HOMO energy level, and the LUMO energy level are calculated with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany) wherein the optimized geometries and the HOMO and LUMO energy levels of the molecular structures are determined by applying the hybrid functional B3LYP with a 6-31G* basis set in the gas phase.

According to an embodiment, the electron acceptor compound comprises Fullerene-C₇₀ [115383-22-7], Fullerence-C₆₀ [99685-96-8], [6,6]-phenyl-C₇₁-butyric acid methyl ester (abbreviation: PC₇₁BM), [6,6]-phenyl-C₆₁-butyric acid methyl ester (abbreviation: PC₆₁BM), and 1',1",4',4"-tetrahydro-di[1,4]methanonaphthaleno[1,2:2',3',56,60:2",3"][5,6]fullerene-C₆₀ (abbreviation: ICBA), a fullerene derivative, a perylene tetracarboxylic diimide (PTCDI) derivative, a perylene tetracarboxylic dianhydride (PTCDA) derivative, or the like

### Electron donor compound & Electron acceptor compound

According to an embodiment, the electron donor compound has a LUMO energy level and wherein the electron acceptor compound has a LUMO energy level LUMO (electron acceptor compound), wherein the LUMO energy level of the electron donor compound is larger than the LUMO energy level of the electron acceptor compound, wherein the LUMO energy level
is calculated with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany) wherein the optimized geometries and the HOMO and LUMO energy levels of the molecular structures are determined by applying the hybrid functional B3LYP with a 6-31G* basis set in the gas phase.

According to an embodiment, the electron donor compound has a LUMO energy level and wherein the electron acceptor compound has a LUMO energy level LUMO (electron acceptor compound), wherein the LUMO energy level of the electron acceptor compound is further away from vacuum level than the LUMO energy level of the electron donor compound, wherein the LUMO energy level is calculated with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany) wherein the optimized geometries and the HOMO and LUMO energy levels of the molecular structures are determined by applying the hybrid functional B3LYP with a 6-31G* basis set in the gas phase.

### Organic photodetector

According to an embodiment, the organic photodetector has a dark current density of -1×10⁻⁴ mA/cm or less when a reverse Bias of -3V±0.05 is applied.

The dark current density of an organic photodetector may be measured in dark conditions by applying a voltage at -3V±0.05.

The dark current density of an organic photodetector may be measured in dark conditions by applying a voltage at -3V±0.05, wherein the positive pol of the current-voltage measurement device is connected to the anode layer of the organic photodetector, and wherein the negative pol of the current-voltage measurement device is connected to the cathode layer of the organic photodetector.

The voltage may be applied for a period of at least one second before the current is measured.

The minimum measurement time of the current is selected so that the effects of current fluctuations in the external grid on the measured value disappear.

The "dark current density" is the current density which is determined when the organic photodetector is not exposed to light.

The organic photodetector messured may have an area size from 0.01 mm² - 30 mm², preferably 2.0 mm² to 10.0 mm², more preferably 4.0 to 7.0 mm², and most preferably 6.0 mm² to 7.0 mm².

The area size of the organic photodetector is determined by the area wherein the anode layer, cathode layer, and the layers between the anode layer and the cathode layer overlaps.

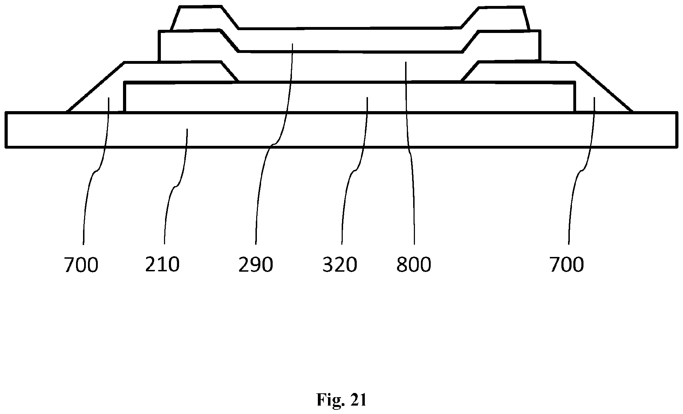

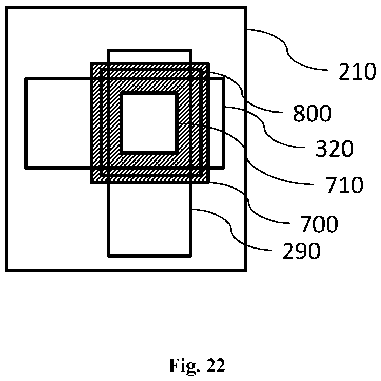

In the overlap of the area anode layer, cathode layer, and the layer between the anode layer and cathode layer are stacked on top of each other. The anode layer area may be defined by a pixel definition layer aperture like shown in figures 21/22.

For the measurement of the dark current density, the organic photodetector may be encapsulated. The encapsulation of the organic photodetector may be a thin film encapsulation or an encapsulation lid, wherein the encapsulation lid provides a cavity, which includes a getter material. In particular, the encapsulation lid is a glass lid.

The applying of the voltage and the measurement of the current may be conducted by means of a Keithley SM2635B.

When using a Keithley SM2635B, the voltage may be applied for a period of at least one second before the current is measured with a minimum measurement time of the current, wherein the minimum measurement time of the current is selected so that the effects of the current fluctuations in the external grid on the measured value disappear.

When using a Keithley SM2635B, the minimum measurement time of the current is at least 5 NPLC (Number of Power Line-Cycles).

According to an embodiment, wherein the hole transport region of the organic photodetector comprises a hole injection layer.

According to an embodiment, wherein the hole transport region of the organic photodetector comprises a hole injection layer, wherein the hole injection layer is adjacent or in direct to the anode layer.

According to an embodiment, wherein the hole transport region of the organic photodetector comprises a hole injection layer, wherein the hole injection layer comprises a p-dopant; preferably an organic p-dopant or a metal compound; more preferably an organic p-dopant or a metal complex of formula (I), wherein the metal complex of formula (I) can be the same or different from the metal complex of formula (I) in the semiconductor layer; and most preferably a metal complex of formula (I) wherein the metal complex of formula (I) can be the same or different from the metal complex of formula (I) in the semiconductor layer.

According to an embodiment, wherein the hole transport region of organic photodetector comprises a hole injection layer, and/or a hole transport layer.

According to an embodiment, wherein the hole transport region of organic photodetector comprises a hole injection layer, and a hole transport layer.

According to an embodiment, wherein the hole transport region of organic photodetector comprises a hole injection layer, and/or a hole transport layer, and wherein the organic photodetector further comprises an electron blocking layer.

According to an embodiment, wherein the hole transport region of organic photodetector comprises a hole injection layer, and a hole transport layer, and wherein the organic photodetector further comprises an electron blocking layer.

According to an embodiment, wherein the organic photodetector further comprises an electron blocking layer.

According to an embodiment, wherein the organic photodetector further comprises an electron transport region, wherein the electron transport region is arranged between the photoconversion unit and the cathode layer. In particular the electron transport region comprises a layer selected from a hole blocking layer, an electron transport layer, an electron injection layer.

According to an embodiment, wherein the organic photodetector further comprises a hole blocking layer, an electron transport layer, an electron injection layer; a hole injection layer, hole transport layer, an electron blocking layer, an electron transport layer, and/or an electron injection layer.

According to an embodiment, wherein the organic photodetector further comprises a hole blocking layer, an electron transport layer and/or an electron injection layer; preferably an electron transport layer and an electron injection layer; more preferably an electron transport layer and an electron injection layer, wherein the organic semiconductor layer is a hole blocking layer.

According to an embodiment, wherein the hole transport region of organic photodetector comprises a hole injection layer, and a hole transport layer, and wherein the organic photodetector further comprises an electron blocking layer, an electron transport layer, and an electron injection layer.

According to an embodiment, wherein the hole transport region of organic photodetector comprises a hole injection layer, and a hole transport layer, and wherein the organic photodetector further comprises an electron blocking layer, a hole blocking layer, an electron transport layer, and an electron injection layer,.

### Organic electronic device

According to another aspect of the present invention, an organic electronic device is provided, comprising a substrate, an organic photodetector, an organic light-emitting device;
wherein the organic photodetector and the organic light-emitting device is disposed on the substrate;
wherein the organic photodetector is an organic photodetector as described in this invention,
wherein the organic light-emitting device comprises
an anode layer, a cathode layer, a hole transport region, a light-emitting layer a cathode layer;
wherein the hole transport region and the light-emitting layer are arranged between the anode layer and the cathode layer;
wherein the hole transport region is arranged between the anode layer and the organic light-emitting layer;

According to one embodiment of the present invention, the organic light-emitting device is an electroluminescent device, an organic light emitting diode (OLED), a light emitting device, thin film transistor, a battery, a display device or an organic photovoltaic cell (OPV).

According to one embodiment, the cathode layer of the organic light-emitting device and the cathode layer of the organic photodetector is a common cathode layer shared by the organic light-emitting device and the organic photodetector,

According to one embodiment, the organic electronic device is a display device.

According to an embodiment, in the organic electronic device the hole transport region of the organic light-emitting device and the hole transport region of the organic photodetector is a common hole transport region shared by at least one organic light-emitting device and at least one organic photodetector.

According to an embodiment, in the organic electronic device the hole transport region of the organic light-emitting device and the hole transport region of the organic photodetector is a common hole transport region shared by the organic light-emitting device and the organic photodetector.

According to an embodiment, in the organic electronic device the common hole transport region is selected from a common hole injection layer, wherein the common hole injection layer comprises a p-dopant; preferably an organic p-dopant or a metal compound; more preferably an organic p-dopant or a metal complex of formula (I), wherein the metal complex of formula (I) can be the same or different from the metal complex of formula (I) in the semiconductor layer; and most preferably a metal complex of formula (I) wherein the metal complex of formula (I) can be the same or different from the metal complex of formula (I) in the semiconductor layer.

According to an embodiment, in the organic electronic device the common transport region is selected from a common hole injection layer, a common hole transport layer.

According to an embodiment, the organic electronic device further comprises a common electron transport region, wherein the common electron transport region is shared by at least one organic light-emitting device and at least one organic photodetector.

According to an embodiment, the organic electronic device further comprises a common electron transport region, wherein the common electron transport region is shared by the organic light-emitting device and the organic photodetector.

According to an embodiment, in the organic electronic device the hole transport region of the organic light-emitting device and the hole transport region of the organic photodetector is a common hole transport region shared by at least one organic light-emitting device and at least one organic photodetector; and wherein the organic electronic device further comprises a common electron transport region, wherein the common electron transport is shared by at least one organic light-emitting device and at least one organic photodetector.

According to an embodiment, in the organic electronic device the hole transport region of the organic light-emitting device and the hole transport region of the organic photodetector is a common hole transport region shared by the organic light-emitting device and the organic photodetector; wherein the organic electronic device further comprises a common electron transport region, wherein the common electron transport is shared by the organic light-emitting device and the organic photodetector.

According to an embodiment, wherein the common electron transport region comprises a common hole blocking layer, a common electron transport layer, and/or a common electron injection layer.

According to an embodiment, the organic photodetector of organic electronic device further comprises an electron blocking layer, wherein the electron blocking layer of the organic photodetector is arranged between semiconductor layer and the hole transport region; and wherein the organic light-emitting device comprises an electron blocking layer, wherein the electron blocking layer is arranged between the hole transport region and the light-emitting layer, wherein the electron blocking layer of the organic photodetector and the electron blocking layer of the organic light-emitting device is a common electron blocking layer shared by at least one of the organic photodetectors and at least one of the organic light-emitting devices.

### Use of a metal complex of Formula (I)

The present invention furthermore relates to the use of a metal complex of formula (I) for photo detection and/or in a photo detector, especially an organic photodetector.

Additionally the present invention furthermore relates to the use of a metal complex of formula (I) for photo detection and/or in a photo detector, especially an organic photodetector as a hole extractio material, especially in a photo detector, especially an organic photodetector.

All specification and embodiments shown for the metal complex of formula (I) and its individual moieties apply *mutatis mutandis.*

### Further layers

In accordance with the invention, the organic photodetector, the organic electronic device, the organic light-emitting device of the organic electronic device, or the organic photodetector of the organic electronic device may comprise, besides the layers already mentioned above, further layers. It is noted that layers may be, depending on the actual embodiment, present in more than one or even all sub-devices of the organic electronic device, especially both in the organic photodetector and the organic light-emitting device. Exemplary embodiments of respective layers are described in the following:

### Substrate

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate.

### Anode layer

The anode layer, also named anode electrode, may be formed by depositing or sputtering a material that is used to form the anode layer. The material used to form the anode layer may be a high work-function material, so as to facilitate hole injection. The anode layer may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode layer. The anode layer may also be formed using metals, typically silver (Ag), gold (Au), or metal alloys.

The anode layer may comprise two or more anode sub-layers.

According to one embodiment, the anode layer comprises a first anode sub-layer and a second anode sub-layer, wherein the first anode sub-layer is arranged closer to the substrate and the second anode sub-layer is arranged closer to the cathode layer.

According to one embodiment, the anode layer may comprise a first anode sub-layer comprising or consisting of Ag or Au and a second anode-sub-layer comprising or consisting of transparent conductive oxide.

According to one embodiment, the anode layer comprises a first anode sub-layer, a second anode sub-layer and a third anode sub-layer, wherein the first anode sub-layer is arranged closer to the substrate and the second anode sub-layer is arranged closer to the cathode layer, and the third anode sub-layer is arranged between the substrate and the first anode sub-layer.

According to one embodiment, the anode layer may comprise a first anode sub-layer comprising or consisting of Ag or Au, a second anode-sub-layer comprising or consisting of transparent conductive oxide and optionally a third anode sub-layer comprising or consisting of transparent conductive oxide. Preferably the first anode sub-layer may comprise or consists of Ag, the second anode-sublayer may comprise or consists of ITO or IZO and the third anode sublayer may comprises or consists of ITO or IZO.

Preferably the first anode sub-layer may comprise or consists of Ag, the second anode-sublayer may comprise or consists of ITO and the third anode sub-layer may comprise or consist of ITO.

Preferably, the transparent conductive oxide in the second and third anode sub-layer may be selected the same.

According to one embodiment, the anode layer may comprise a first anode sub-layer comprising Ag or Au having a thickness of 100 to 150 nm, a second anode sub-layer comprising or consisting of a transparent conductive oxide having a thickness of 3 to 20 nm and a third anode sub-layer comprising or consisting of a transparent conductive oxide having a thickness of 3 to 20 nm.

It is to be understood that the third anode layer is not part of the substrate.

### Hole injection layer

A hole injection layer (HIL) may be formed on the anode electrode by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of 10⁻⁸ to 10⁻³ Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluoro-tetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto. The HIL may be selected from a holetransporting matrix compound doped with a p-type dopant. Typical examples of known doped hole transport materials are: copper phthalocyanine (CuPc), which HOMO level is approximately -5.2 eV, doped with tetrafluoro-tetracyanoquinonedimethane (F4TCNQ), which LUMO level is about -5.2 eV; zinc phthalocyanine (ZnPc) (HOMO = -5.2 eV) doped with F4TCNQ; α-NPD (N,N'-Bis(naphthalen-1-yl)-N,N'-bis(phenyl)-benzidine) doped with F4TCNQ. α-NPD doped with 2,2'-(perfluoronaphthalen-2,6-diylidene) dimalononitrile. The p-type dopant concentrations can be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

### Hole transport layer

According to one embodiment of the present invention, the organic electronic device may further comprise a hole transport layer, wherein the hole transport layer is arranged between the anode layer and the cathode layer.

The hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010 and incorporated by reference. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

According to a preferred embodiment of the present invention, the hole transport layer may comprise a substantially covalent matrix compound.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in driving voltage.

### Substantially covalent matrix compound/ covalent matrix compound

According to one embodiment of the present invention, at least one of the layers of the organic electronic device may comprise a substantially covelant matrix compound or a covalent matrix compound.

The substantially covalent matrix compound, also named matrix compound, may be an organic aromatic matrix compounds, which comprises organic aromatic covalent bonded carbon atoms. The substantially covalent matrix compound may be an organic compound, consisting substantially from covalently bound C, H, O, N, S, which may optionally comprise also covalently bound B, P or Si. The substantially covalent matrix compound may be an organic aromatic covalent bonded compound, which is free of metal atoms, and the majority of its skeletal atoms may be selected from C, O, S, N and preferably from C, O and N, wherein the majority of atoms are C-atoms. Alternatively, the covalent matrix compound is free of metal atoms and majority of its skeletal atoms may be selected from C and N, preferably the covalent matrix compound is free of metal atoms and majority of its skeletal atoms may be selected from C and the minority of its skeletal atoms may be N.

According to one embodiment, the substantially covalent matrix compound may have a molecular weight Mw of ≥ 400 and ≤ 2000 g/mol, preferably a molecular weight Mw of ≥ 450 and ≤ 1500 g/mol, further preferred a molecular weight Mw of ≥ 500 and ≤ 1000 g/mol, in addition preferred a molecular weight Mw of ≥ 550 and ≤ 900 g/mol, also preferred a molecular weight Mw of ≥ 600 and ≤ 800 g/mol.

In one embodiment, the HOMO level of the substantially covalent matrix compound may be more negative than the HOMO level of N2,N2,N2',N2',N7,N7,N7',N7'-octakis(4-methoxyphenyl)-9,9'-spirobi[fluorene]-2,2',7,7'-tetraamine (CAS 207739-72-8) when determined under the same conditions.

In one embodiment of the present invention, the substantially covalent matrix compound may be free of alkoxy groups.

Preferably, the substantially covalent matrix compound comprises at least one arylamine moiety, alternatively a diarylamine moiety, alternatively a triarylamine moiety.

Preferably, the substantially covalent matrix compound is free of TPD or NPB.

### Compound of formula (IIIa) or a compound of formula (IIIb)

According to another aspect of the present invention, the substantially covalent matrix compound or covalent matrix compound, also referred to as matrix compound herein, may comprises at least one arylamine compound, diarylamine compound, triarylamine compound, a compound of formula (IIIa) or a compound of formula (IIIb): 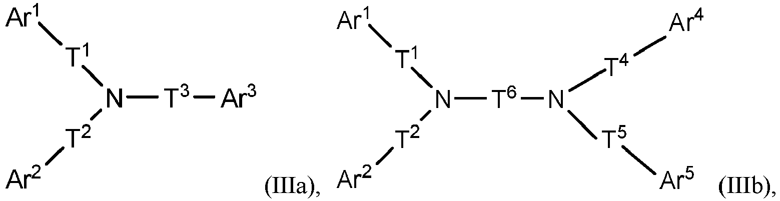 wherein:
T¹, T², T³, T⁴ and T⁵ are independently selected from a single bond, phenylene, biphenylene, terphenylene or naphthenylene, preferably a single bond or phenylene;
T⁶ is phenylene, biphenylene, terphenylene or naphthenylene;
Ar¹, Ar², Ar³, Ar⁴ and Ar⁵ are independently selected from substituted or unsubstituted C₆ to C₂₀ aryl, or substituted or unsubstituted C₃ to C₂₀ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted nonhetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;
wherein
the substituents of Ar¹, Ar², Ar³, Ar⁴ and Ar⁵ are selected the same or different from the group comprising H, D, F, C(-O)R², CN, Si(R²)₃, P(-O)(R²)₂, OR², S(-O)R², S(-O)₂R², substituted or unsubstituted straight-chain alkyl having 1 to 20 carbon atoms, substituted or unsubstituted branched alkyl having 1 to 20 carbon atoms, substituted or unsubstituted cyclic alkyl having 3 to 20 carbon atoms, substituted or unsubstituted alkenyl or alkynyl groups having 2 to 20 carbon atoms, substituted or unsubstituted substituted or unsubstituted aromatic ring systems having 6 to 40 aromatic Atoms, and substituted or unsubstituted heteroaromatic ring systems having 5 to 40 aromatic Atoms, unsubstituted C₆ to C₁₈ aryl, unsubstituted C₃ to C₁₈ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle,
   wherein R² may be selected from H, D, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, C₆ to Cis aryl or C₃ to Cis heteroaryl.

Preferably, the substituents of Ar¹, Ar², Ar³, Ar⁴ and Ar⁵ are selected the same or different from the group comprising H, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, C₆ to Cis aryl, C₃ to Cis heteroaryl, a fused ring system comprising 2 to 4 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle; more preferred the substituents are selected the same or different from the group consisting of H, straight-chain alkyl having 1 to 4 carbon atoms, branched alkyl having 1 to 4 carbon atoms, cyclic alkyl having 3 to 4 carbon atoms and/or phenyl.
Thereby, the compound of formula (IIIa) or (IIIb) may have a rate onset temperature suitable for mass production.

According to an embodiment the substantially covalent matrix compound comprises a compound of formula (IIIa) or formula (IIIb): 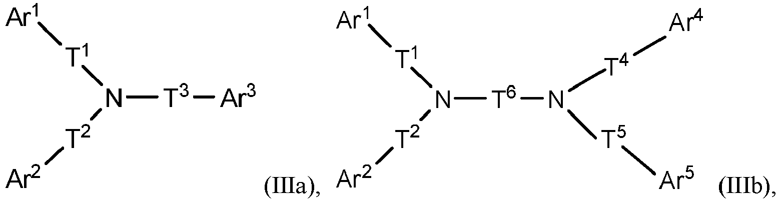 wherein
T¹, T², T³, T⁴ and T⁵ may be independently selected from a single bond, phenylene, biphenylene, terphenylene or naphthenylene, preferably a single bond or phenylene;
T⁶ is phenylene, biphenylene, terphenylene or naphthenylene;
Ar¹, Ar², Ar³, Ar⁴ and Ar⁵ may be independently selected from unsubstituted C₆ to C₂₀ aryl, or unsubstituted C₃ to C₂₀ heteroarylene, unsubstituted biphenylene, unsubstituted fluorene, substituted 9-fluorene, substituted 9,9-fluorene, unsubstituted naphthalene, unsubstituted anthracene, unsubstituted phenanthrene, unsubstituted pyrene, unsubstituted perylene, unsubstituted triphenylene, unsubstituted tetracene, unsubstituted tetraphene, unsubstituted dibenzofurane, unsubstituted dibenzothiophene, unsubstituted xanthene, unsubstituted carbazole, substituted 9-phenylcarbazole, unsubstituted azepine, unsubstituted dibenzo[b,f]azepine, unsubstituted 9,9'-spirobi[fluorene], unsubstituted spiro[fluorene-9,9'-xanthene], or a unsubstituted aromatic fused ring system comprising at least three unsubstituted aromatic rings selected from the group comprising unsubstituted non-hetero, unsubstituted hetero 5-member rings, unsubstituted 6-member rings and/or unsubstituted 7-member rings, unsubstituted fluorene, or a fused ring system comprising 2 to 6 unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle.

According to an embodiment, the substantially covalent matrix compound comprises a compound of formula (IIIa) or formula (IIIb): 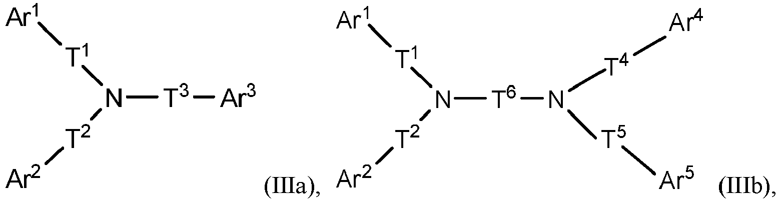 wherein
T¹, T², T³, T⁴ and T⁵ may be independently selected from a single bond, phenylene, biphenylene, terphenylene or naphthenylene, preferably a single bond or phenylene;
T⁶ is phenylene, biphenylene, terphenylene or naphthenylene;
Ar¹, Ar², Ar³, Ar⁴ and Ar⁵ may be independently selected from unsubstituted C₆ to C₂₀ aryl, or unsubstituted C₃ to C₂₀ heteroarylene, unsubstituted biphenylene, unsubstituted fluorene, substituted 9-fluorene, substituted 9,9-fluorene, unsubstituted naphthalene, unsubstituted anthracene, unsubstituted phenanthrene, unsubstituted pyrene, unsubstituted perylene, unsubstituted triphenylene, unsubstituted tetracene, unsubstituted tetraphene, unsubstituted dibenzofurane, unsubstituted dibenzothiophene, unsubstituted xanthene, unsubstituted carbazole, substituted 9-phenylcarbazole, unsubstituted azepine, unsubstituted dibenzo[b,f]azepine, unsubstituted 9,9'-spirobi[fluorene], unsubstituted spiro[fluorene-9,9'-xanthene].

Thereby, the compound of formula (IIIa) or (IIIb) may have a rate onset temperature suitable for mass production.

According to an embodiment wherein T¹, T², T³, T⁴ and T⁵ may be independently selected from a single bond, phenylene, biphenylene or terphenylene.
According to an embodiment wherein T¹, T², T³, T⁴ and T⁵ may be independently selected from phenylene, biphenylene or terphenylene and one of T¹, T², T³, T⁴ and T⁵ are a single bond.
According to an embodiment wherein T¹, T², T³, T⁴ and T⁵ may be independently selected from phenylene or biphenylene and one of T¹, T², T³, T⁴ and T⁵ are a single bond.
According to an embodiment wherein T¹, T², T³, T⁴ and T⁵ may be independently selected from phenylene or biphenylene and two of T¹, T², T³, T⁴ and T⁵ are a single bond.

According to an embodiment wherein T¹, T² and T³ may be independently selected from phenylene and one of T¹, T² and T³ are a single bond.

According to an embodiment wherein T¹, T² and T³ may be independently selected from phenylene and two of T¹, T² and T³ are a single bond.

According to an embodiment wherein T⁶ may be phenylene, biphenylene, terphenylene. According to an embodiment wherein T⁶ may be phenylene.

According to an embodiment wherein T⁶ may be biphenylene. According to an embodiment wherein T⁶ may be terphenylene.

According to an embodiment wherein Ar¹, Ar², Ar³, Ar⁴ and Ar⁵ may be independently selected from formulae (G1) to (G16): 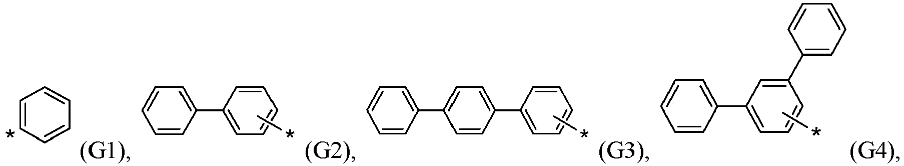 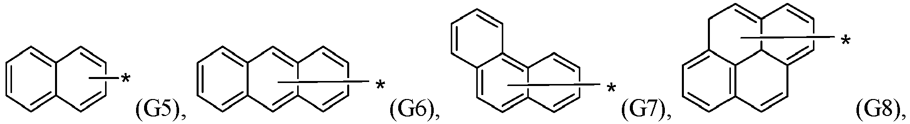 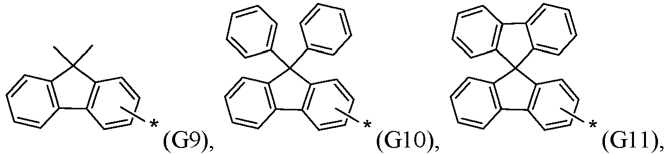 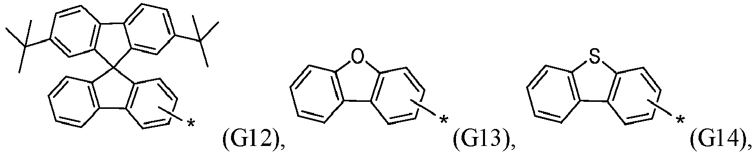 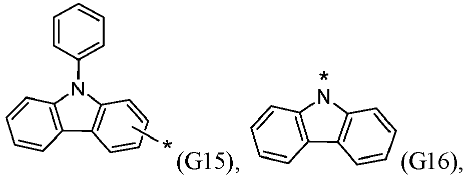 wherein the asterix "*" denotes the binding position.

According to an embodiment, wherein Ar¹, Ar², Ar³, Ar⁴ and Ar⁵ may be independently selected from (G1) to (G15); alternatively selected from (G1) to (G10) and (G13) to (G15).

According to an embodiment, wherein Ar¹, Ar², Ar³, Ar⁴ and Ar⁵ may be independently selected from the group consisting of (G1), (G2), (G5), (G7), (G9), (G10), (G13) to (G16).

The rate onset temperature may be in a range particularly suited to mass production, when Ar¹, Ar², Ar³, Ar⁴ and Ar⁵ are selected in this range.

The "matrix compound of formula (IIIa) or formula (IIIb) " may be also referred to as "hole transport compound".

According to one embodiment the compound of formula (IIIa) or formula (IIIb) may comprises at least ≥ 1 to ≤ 6 substituted or unsubstituted aromatic fused ring systems comprising heteroaromatic rings.

According to one embodiment the compound of formula (IIIa) or formula (IIIb) may comprises at least ≥ 1 to ≤ 6 substituted or unsubstituted aromatic fused ring systems comprising heteroaromatic rings and at least ≥ 1 to ≤ 3 substituted or unsubstituted unsaturated 5- to 7-member ring of a heterocycle, preferably ≥ 2 to ≤ 5 substituted or unsubstituted aromatic fused ring systems comprising heteroaromatic rings.

According to one embodiment the compound of formula (IIIa) or formula (IIIb) may comprises at least ≥ 1 to ≤ 6 substituted or unsubstituted aromatic fused ring systems comprising heteroaromatic rings and at least ≥ 1 to ≤ 3 substituted or unsubstituted unsaturated 5- to 7-member ring of a heterocycle, preferably ≥ 2 to ≤ 5 substituted or unsubstituted aromatic fused ring systems comprising heteroaromatic rings, and at least ≥ 1 to ≤ 3 substituted or unsubstituted unsaturated 5- to 7-member ring of a heterocycle, further preferred 3 or 4 substituted or unsubstituted aromatic fused ring systems comprising heteroaromatic rings and optional at least ≥ 1 to ≤ 3 substituted or unsubstituted unsaturated 5- to 7-member ring of a heterocycle, and additional preferred wherein the aromatic fused ring systems comprising heteroaromatic rings are unsubstituted and optional at least ≥ 1 to ≤ 3 unsubstituted unsaturated 5- to 7-member ring of a heterocycle.

According to one embodiment the compound of formula (IIIa) or formula (IIIb) may comprise:
- a substituted or unsubstituted aromatic fused ring systems with at least ≥ 2 to ≤ 6, preferably ≥ 3 to ≤ 5, or 4 fused aromatic rings selected from the group comprising substituted or unsubstituted non-hetero aromatic rings, substituted or unsubstituted hetero 5-member rings, substituted or unsubstituted 6-member rings and/or substituted or unsubstituted unsaturated 5-to 7- member ring of a heterocycle; or
- an unsubstituted aromatic fused ring systems with at least ≥ 2 to ≤ 6, preferably ≥ 3 to ≤ 5, or 4 fused aromatic rings selected from the group comprising unsubstituted non-hetero aromatic rings, unsubstituted hetero 5-member rings, unsubstituted 6-member rings and/or unsubstituted unsaturated 5- to 7-member ring of a heterocycle.

It should be noted here that the wording "aromatic fused ring system" may include at least one aromatic and at least one substituted or unsubstituted unsaturated 5- to 7- member ring. It should be noted here that the substituted or unsubstituted unsaturated 5- to 7- member ring may not be an aromatic ring.

According to one embodiment, the substantially covalent matrix compound comprises at least one naphthyl group, carbazole group, dibenzofurane group, dibenzothiophene group and/or substituted fluorenyl group, wherein the substituents are independently selected from methyl, phenyl or fluorenyl.

According to an embodiment of the present invention, the compound of formula (IIIa) or formula (IIIb) are selected from formulae (F1) to (F23): 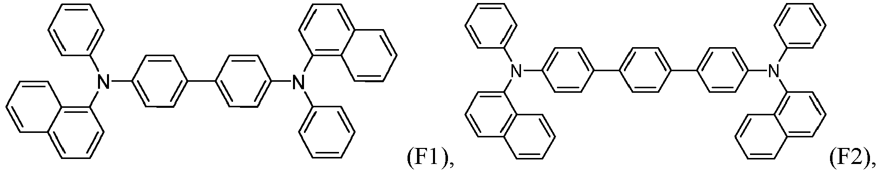 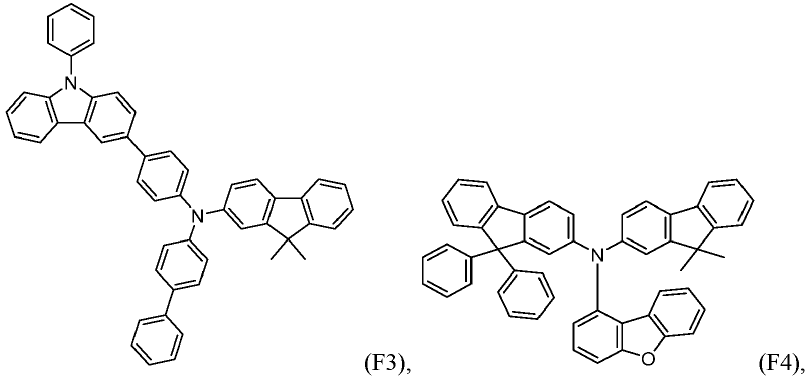 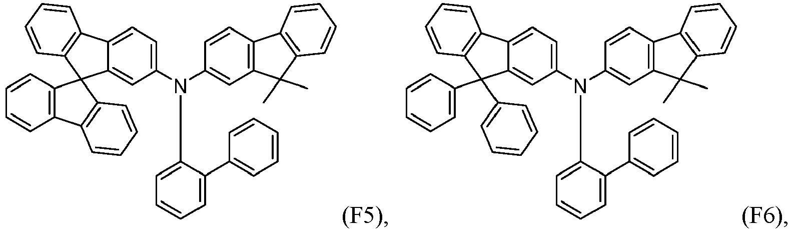 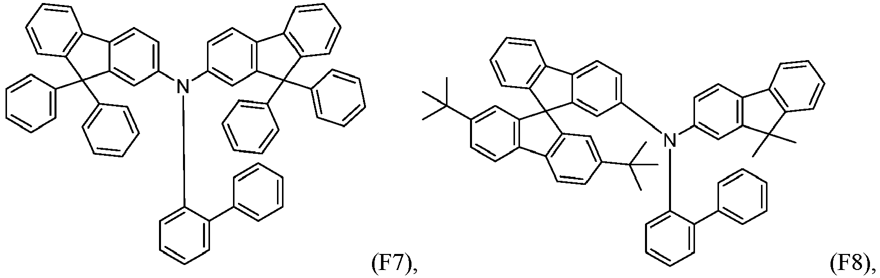 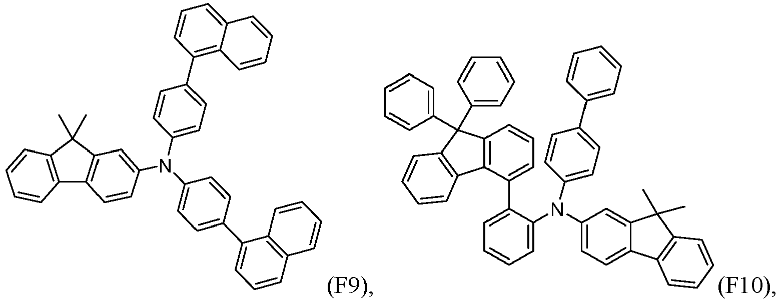 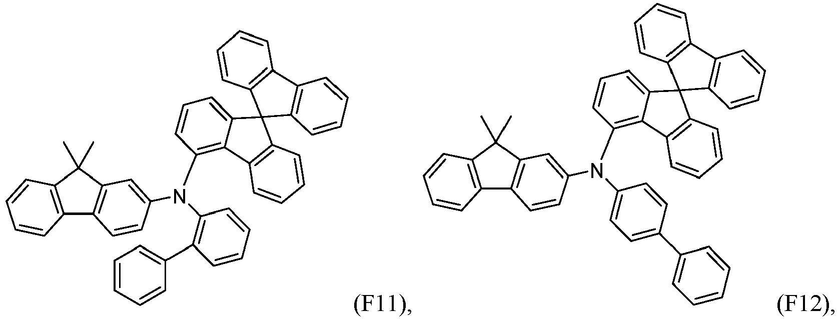 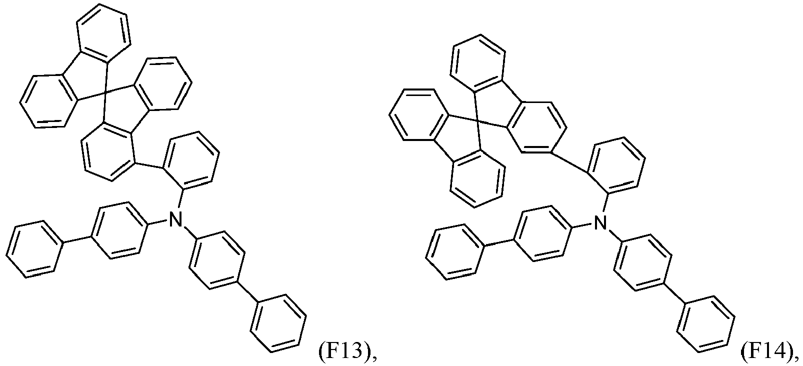 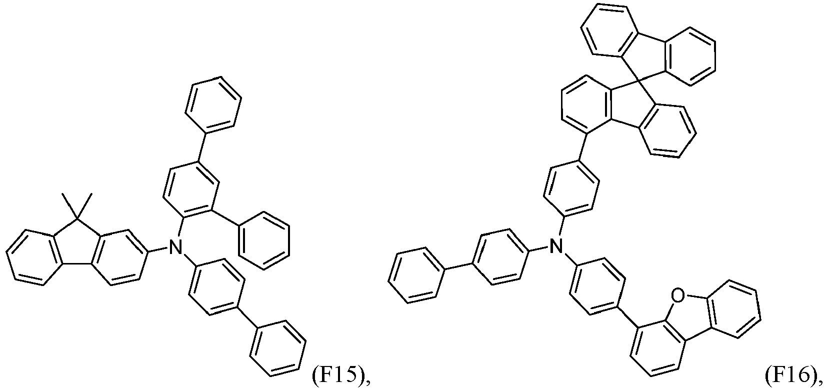 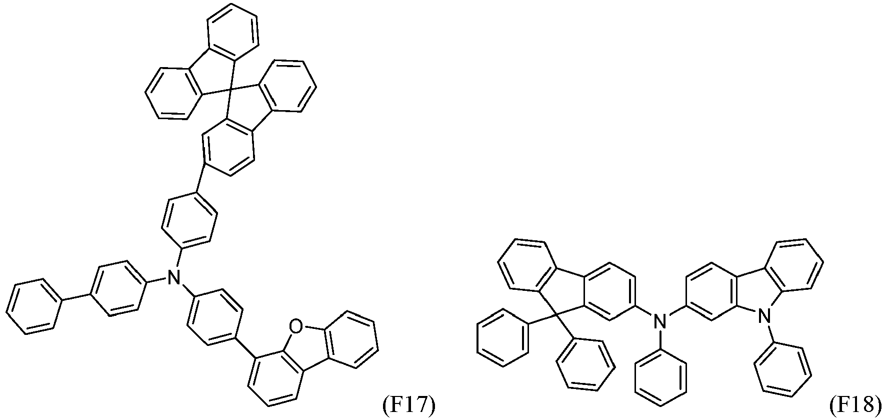 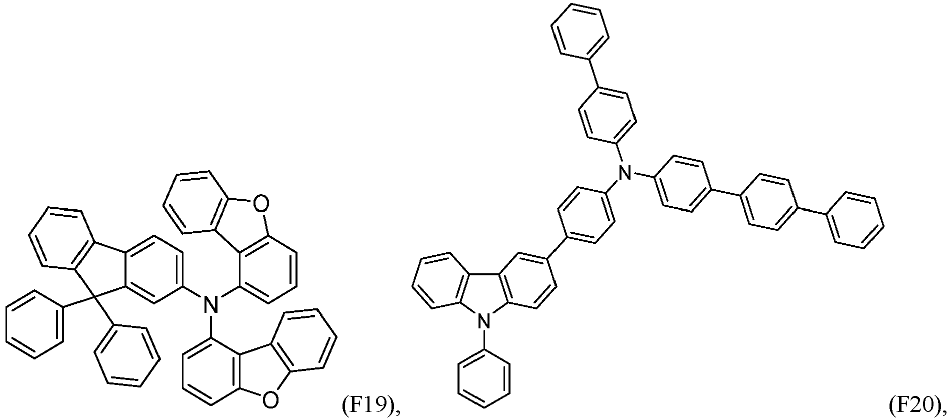 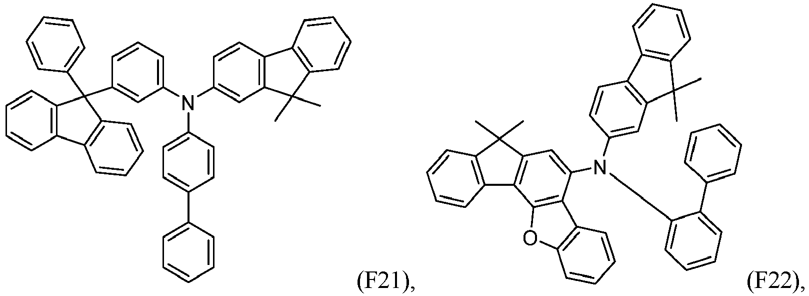 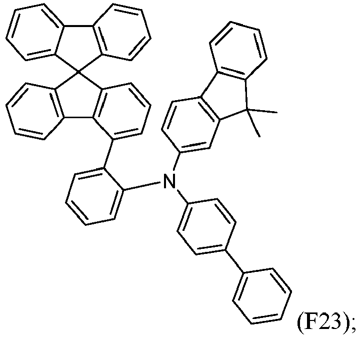 preferably the compound of formula (IIIa) or formula (IIIb) is selected from formulae (F3) to (F23), more preferred from (F4) to (F23), most preferred from (F18).

### Electron blocking layer

The function of an electron blocking layer (EBL) is to prevent electrons from being transferred from an emission layer to the hole transport layer and thereby confine electrons to the emission layer. Thereby, efficiency, operating voltage and/or lifetime may be improved.

The electron blocking layer comprises an electron blocking compound.

Typically, the electron blocking layer comprises a triarylamine compound such as N,N-di([1,1'-biphenyl]-4-yl)-7,7-dimethyl-7H-fluoreno[4,3-b]benzofuran-10-amine ([1616706-52-5]), ]), N,N-di([1,1'-biphenyl]-4-yl)-4'-(9H-carbazol-9-yl)-[1,1'-biphenyl]-4-amine ([1069137-74-1]), N,N-di([1,1'-biphenyl]-4-yl)-9-phenyl-9H-carbazol-2-amine ([1259388-72-1]), N¹-([1,1'-biphenyl]-4-yl)-N³-(dibenzo[b,d]thiophen-2-yl)-N¹,N³-diphenyl-5-(9-phenyl-9H-carbazol-2-yl)benzene-1,3-diamine ([1869085-48-2]), N¹-([1,1'-biphenyl]-4-yl)-N³,N³-diphenyl-N¹-(8-(9-phenyl-9H-carbazol-2-yl)dibenzo[b,d]thiophen-2-yl)benzene-1,3-diamine ([2055861-88-4]), N⁷-(dibenzo[b,d]thiophen-3-yl)-N²,N²,N⁷-triphenyldibenzo[b,d]thiophene-2,7-diamine ([2033134-06-2]), 7-(4-(dibenzo[b,d]thiophen-3-yl(phenyl)amino)phenyl)-N,N-diphenyldibenzo[b,d]thiophen-2-amine ([2641671-54-5]), N-([1,1'-biphenyl]-4-yl)-9,9-dimethyl-N-(3-(9-phenyl-9H-fluoren-9-yl)phenyl)-9H-fluoren-2-amine (2226747-62-0), N-([1,1'-biphenyl]-2-yl)-N-(9,9-dimethyl-9H-fluoren-2-yl)-9,9'-spirobi[fluoren]-4-amine ([1450933-44-4]), N-([1,1'-biphenyl]-4-yl)-N-(2-(9,9-diphenyl-9H-fluoren-4-yl)phenyl)-9,9-dimethyl-9H-fluoren-2-amine ([1792219-00-1]), 2',7'-di-tert-butyl-N,N-bis(9,9-dimethyl-9H-fluoren-2-yl)-9,9'-spirobi[fluoren]-4-amine ([2379778-94-4]),
N,N-di([1,1'-biphenyl]-4-yl)-3'-(9H-carbazol-9-yl)-[1,1'-biphenyl]-4-amine ([1464822-27-2]),
N,N-bis(4-(dibenzo[b,d]furan-4-yl)phenyl)-[1,1':4',1"-terphenyl]-4-amine ([N,N-bis(4-(dibenzo[b,d]furan-4-yl)phenyl)-[1,1':4',1"-terphenyl]-4-amine]) ([1198399-61-9]),
N-(4-(naphthalen-1-yl)phenyl)-N-(3-(6-phenyldibenzo[b,d]furan-4-yl)phenyl)naphthalen-1-amine ([1868149-26-1]),
N,9,9-triphenyl-N-(4'-(triphenylsilyl)-[1,1'-biphenyl]-4-yl)-9H-fluoren-2-amine ([2209040-18-4]),
N-([1,1'-biphenyl]-4-yl)-N-(4-(phenanthren-9-yl)phenyl)-[1,1':4',1"-terphenyl]-4-amine ([2233544-05-1]),
N-([1,1'-biphenyl]-4-yl)-9,9-diphenyl-N-(4-(triphenylsilyl)phenyl)-9H-fluoren-2-amine ([1613079-70-1]),
N-([1,1'-biphenyl]-4-yl)-3'-(9H-carbazol-9-yl)-N-(4-(dibenzo[b,d]furan-4-yl)phenyl)-[1,1'-biphenyl]-4-amine ([2245098-00-2]),
N,N-di([1,1'-biphenyl]-4-yl)-9,9'-spirobi[fluoren]-2-amine ([1364602-86-7]), N,N-di([1,1'-biphenyl]-4-yl)-3'-(dibenzo[b,d]furan-4-yl)-[1,1'-biphenyl]-4-amine ([N,N-di([1,1'-biphenyl]-4-yl)-3'-(dibenzo[b,d]furan-4-yl)-[1,1'-biphenyl]-4-amine]),
N-([1,1'-biphenyl]-4-yl)-N-(4-(9-phenyl-9H-carbazol-3-yl)phenyl)-[1,1':4',1"-terphenyl]-4-amine ([1569603-30-0]),
N-(3'-(9H-carbazol-9-yl)-[1,1'-biphenyl]-4-yl)-N-([1,1'-biphenyl]-4-yl)-[1,1':4',1"-terphenyl]-4-amine ([1887177-93-6]),
N3,N3'-di([1,1'-biphenyl]-4-yl)-N3,N3'-dimesityl-[1,1'-biphenyl]-3,3'-diamine ([1887177-93-6]).

The electron blocking compound in particular a triarylamine compound may have a LUMO level closer to vacuum level than the LUMO level of the hole transport layer. The electron blocking compound may have a HOMO level that is further away from vacuum level compared to the HOMO level of the hole transport layer.

The electron blocking compound in particular a triarylamine compound may have a LUMO level closer to vacuum level than the LUMO level of the electron donor compound in the emission layer.

The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

In case the organic electronic device comprises at least one organic photodetector and at least one organic light-emittting device, it is one embodiment of the present invention that at least one electron blocking layer is shared by at least one organic photodetector and by at least one organic light-emitting diode

### Photoconversion unit

The photoconversion unit converts photons into an electrical current.

The photoconversion unit may be formed by vacuum deposition, spin coating, slot-die coat-ing, printing, casting, LB deposition, or the like. When the photoconversion unit is formed using vacuum deposition or spin coating, the conditions for deposition and coating may be similar to those for the formation of the HIL. However, the conditions for deposition and coating may vary, according to the compound that is used to form the PAL.

### Emission layer (EML)

The emission layer (EML) converts an electrical current into photons.

According to an embodiment, the emission layer is arranged between the anode layer and the cathode layer, preferably the emission layer is arranged between the organic semiconductor layer and the cathode layer.

The EML may be formed by vacuum deposition, spin coating, slot-die coat-ing, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

The emission layer (EML) may comprise an organic emitter host and a light-emitting compound dopant. Examples of the organic emitter host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp)3.

Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

It may be provided that the emission layer does not comprise the metal complex of formula (I).

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

According to a preferred embodiment of the present invention, the emission layer comprises a light-emitting compound of formula (IV): 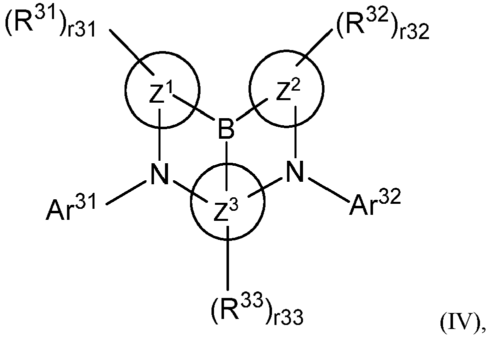 wherein
Z¹, Z² and Z³ are the same as or different from each other, and are each independently selected from the group comprising a monocyclic to polycyclic aromatic hydrocarbon ring or monocyclic to polycyclic aromatic hetero ring;
Ar³¹ and Ar³² are the same as or selected different from each other, and are each independently a substituted or unsubstituted alkyl group, a substituted or unsubstituted aryl group, or a substituted or unsubstituted heteroaryl group, or are bonded to an adjacent substituent to form a substituted or unsubstituted aromatic ring or a substituted or unsubstituted aliphatic ring;
R³¹, R³² and R³³ are the same as or different from each other, and are each independently selected from the group comprising hydrogen, deuterium, a substituted or unsubstituted alkyl group, a substituted or unsubstituted silyl group, a substituted or unsubstituted amine group, a substituted or unsubstituted aryl group, or a substituted or unsubstituted heteroaryl group, or adjacent substituents are bonded to each other to form a substituted or unsubstituted aromatic ring or a substituted or unsubstituted aliphatic ring,
wherein one or more substituents selected from the group consisting of deuterium, an alkyl group having 1 to 6 carbon atoms, an alkyl silyl group having 1 to 30 carbon atoms, an aryl silyl group having 6 to 50 carbon atoms, an alkylamine group having 1 to 30 carbon atoms, an alkylarylamine group having 1 to 50 carbon atoms, an arylamine group having 6 to 50 carbon atoms, an aryl group having 6 to 30 carbon atoms, and a heteroaryl group having 2 to 30 carbon atoms, or a substituent to which two or more substituents selected from the group are linked, or adjacent substituents are bonded to each other to form an aliphatic hydrocarbon ring having 3 to 60 carbon atoms, which is unsubstituted or substituted with the substituent;
r³¹, r³² and r³³ are each an integer from 0, 1, 2, 3 or 4, and when r³¹ to r³³ are 2 or higher, substituents in the parenthesis are the same as or different from each other.

According to one embodiment, for formula (III):
- Z¹, Z² and Z³: are the same as or selected different from each other, and are each independently selected from the group comprising a monocyclic to bicyclic aromatic hydrocarbon ring, or a monocyclic to bicyclic aromatic hetero ring containing O, N or S;
- Ar³¹ and Ar³²: are the same as or selected different from, and are each independently selected from the group comprising an alkyl group having 1 to 10 carbon atoms, which is unsubstituted or substituted with an aryl group, an aryl group having 6 to 30 carbon atoms, which is unsubstituted or substituted with an aryl group, or a heteroaryl group having 2 to 30 carbon atoms;
- R³¹, R³² and R³³: are the same as or selected different from each other, and are each independently selected from the group comprising hydrogen, deuterium, a substituted or unsubstituted alkyl group, a substituted or unsubstituted cycloalkyl group, a substituted or unsubstituted silyl group, a substituted or unsubstituted aryl group, or a substituted or unsubstituted heteroaryl group.

According to one embodiment, wherein for formula (III):
- Z¹, Z² and Z³: are the same as or selected different from each other, and are each independently selected from the group comprising a benzene ring or a thiophene ring;
- Ar³¹ and Ar³²: are the same as or selected different from each other, and are each independently selected from the group comprising phenyl group, a biphenyl group, a naphthyl group, a dimethyl fluorenyl group, a diphenyl fluorenyl group, a dibenzofuran group, or a dibenzothiophene group;
- R³¹, R³² and R³³: are the same as or selected different from each other, and are each independently selected from the group comprising hydrogen, deuterium, a substituted or unsubstituted alkyl group having 1 to 10 carbon atoms, a substituted or unsubstituted cycloalkyl group having 5 to 30 carbon atoms, a substituted or unsubstituted silyl group having 1 to 10 carbon atoms, a substituted or unsubstituted aryl group having 6 to 30 carbon atoms, or a substituted or unsubstituted heteroaryl group having 2 to 30 carbon atoms.

According to a preferred embodiment of the present invention, the emission layer comprises a light-emitting compound of formula (IV) is selected from formulae (BD1) to (BD9):

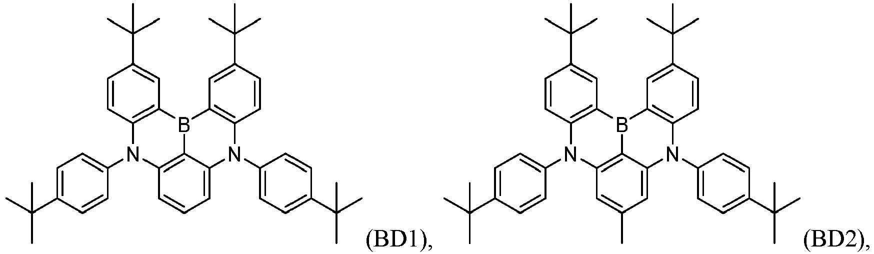

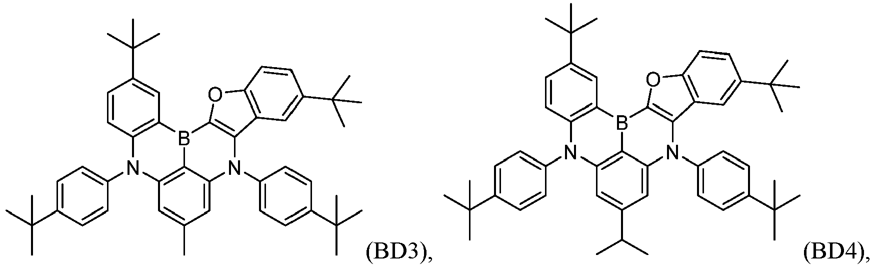

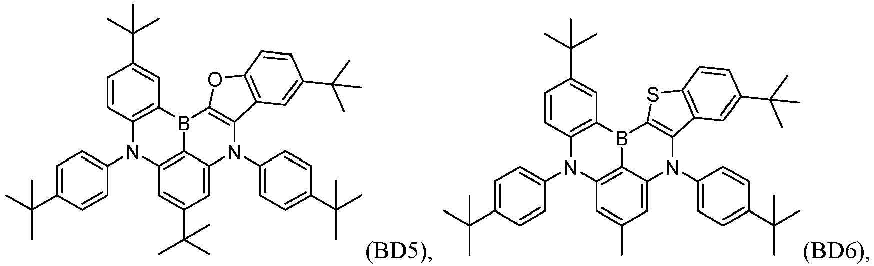

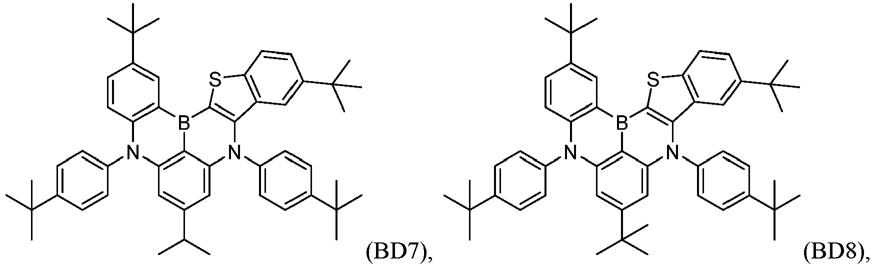

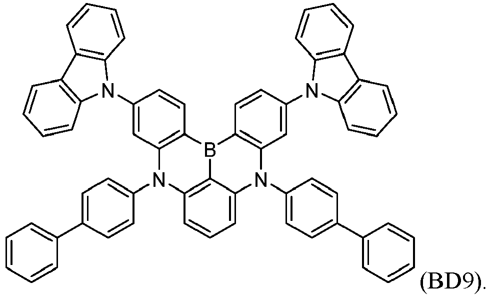

According to a preferred embodiment of the present invention, the emission layer comprises an organic emitter host compound, wherein the organic emitter host compound comprises
- at least one condensed aromatic ring system consisting of 3 to 5 rings, and
- 3 to 7 aromatic or heteroaromatic rings, wherein one or more sub-groups of the aromatic and/or heteroaromatic rings may be condensed to form fused aromatic or heteroaromatic ring systems;
wherein the molecular weight Mw of the organic emitter host compound is in the range of ≥ 400 and ≤ 2000 g/mol.

According to a preferred embodiment of the present invention, the organic emitter host compound has the formula (V) 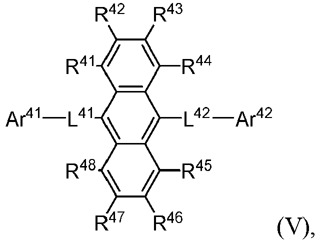 wherein
Ar⁴¹ and Ar⁴² are independently selected from substituted or unsubstituted C₆ to C₂₄ aryl, substituted or unsubstituted C₃ to C₂₄ heteroaryl;
L⁴¹ and L⁴² are independently selected from a direct bond or substituted or unsubstituted C₆ to C₂₄ arylene, substituted or unsubstituted C₃ to C₂₄ heteroarylene;
R⁴¹ to R⁴¹ are independently selected from H, D, substituted or unsubstituted Ci to C₁₂ alkyl, substituted or unsubstituted C₆ to C₁₉ aryl, substituted or unsubstituted C₃ to C₁₂ heteroaryl;
wherein
the substituents on Ar⁴¹, Ar⁴², L⁴¹, L⁴², R⁴¹ to R⁴⁸ are independently selected from D, C₆ to C₁₀ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, halogen, F or CN.

According to a preferred embodiment of the present invention, the organic emitter host and/or compound of formula (V) is selected from formulae (BH1) to (BH13):

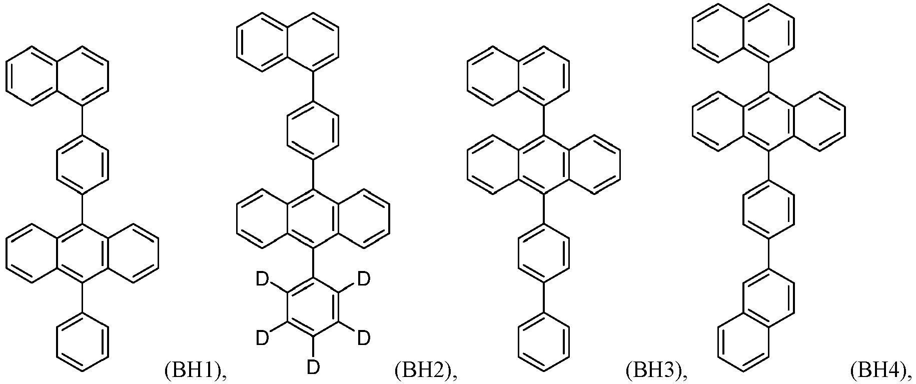

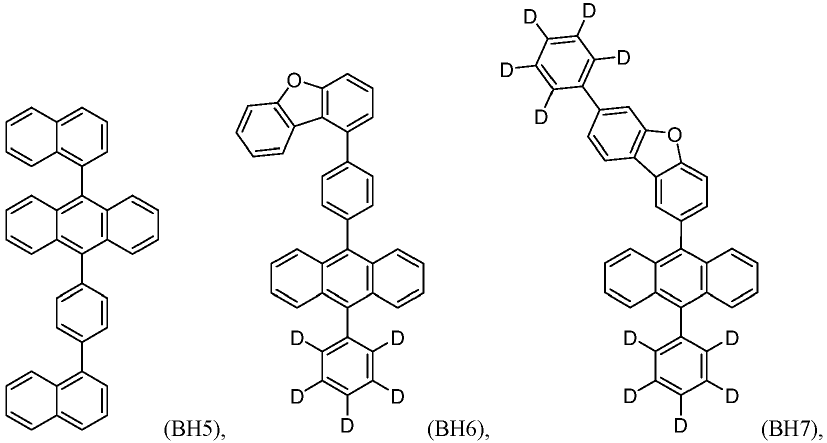

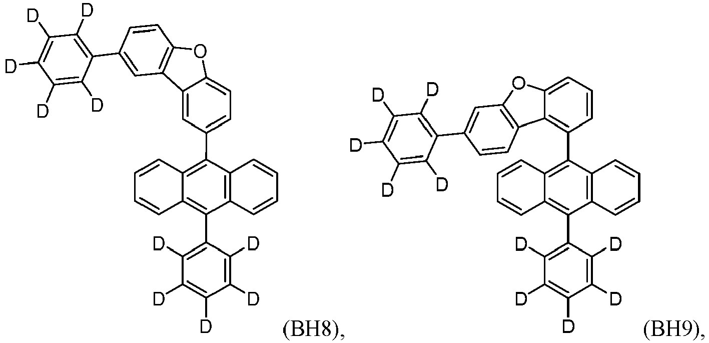

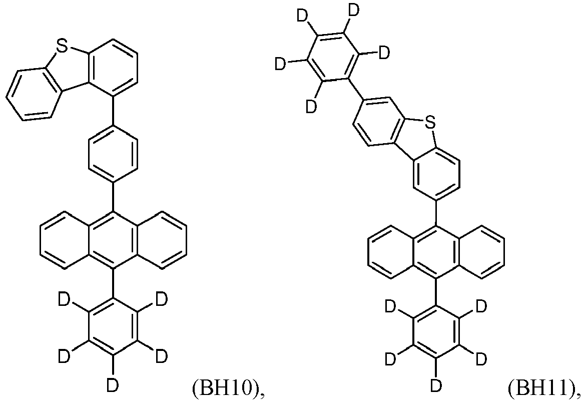

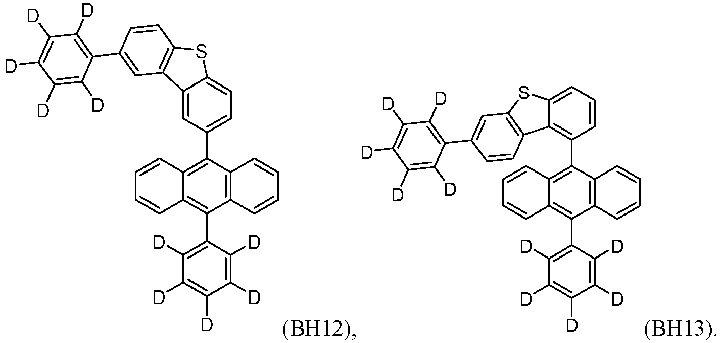

According to a preferred embodiment of the present invention, the light-emitting layer comprises a light-emitting dopant of formula (IV) and an organic emitter host of formula (V).

### Hole blocking layer (HBL)

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function.

The HBL may also be named auxiliary ETL or α-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, phenanthroline derivatives and triazine derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

### Electron transport layer (ETL)

The organic electronic device according to the present invention may further comprise an electron transport layer (ETL), wherein the electron transport layer is arranged between the anode layer and the cathode layer, preferably between the organic semiconductor layer and the cathode layer.

According to another embodiment of the present invention, the electron transport layer may further comprise an azine compound, preferably a triazine compound.

In one embodiment, the electron transport layer may further comprise a dopant selected from an alkali organic complex, preferably LiQ.

The thickness of the ETL may be in the range from about 15 nm to about 50 nm, for example, in the range from about 20 nm to about 40 nm. When the thickness of the EII, is within this range, the ETL may have satisfactory electron-injecting properties, without a substantial penalty in driving voltage.

According to another embodiment of the present invention, the organic electronic device may further comprise a hole blocking layer and an electron transport layer, wherein the hole blocking layer and the electron transport layer comprise an azine compound. Preferably, the azine compound is a triazine compound.

### Electron injection layer (EIL)

An optional EIL, which may facilitates injection of electrons from the cathode, may be formed on the ETL, preferably directly on the electron transport layer. Examples of materials for forming the EII, include lithium 8-hydroxyquinolinolate (LiQ), LiF, NaCl, CsF, Li2O, BaO, Ca, Ba, Yb, Mg which are known in the art. Deposition and coating conditions for forming the EII, are similar to those for formation of the HIL, although the deposition and coating conditions may vary, according to the material that is used to form the EIL.

The thickness of the EIL may be in the range from about 0.1 nm to about 10 nm, for example, in the range from about 0.5 nm to about 9 nm. When the thickness of the EII, is within this range, the EII, may have satisfactory electron-injecting properties, without a substantial penalty in driving voltage.

### Cathode layer

The cathode layer is formed on the ETL or optional EIL. The cathode layer may be formed of a metal, an alloy, an electrically conductive compound, or a mixture thereof. The cathode layer may have a low work function. For example, the cathode layer may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode layer may be formed of a transparent conductive oxide, such as ITO or IZO.

The thickness of the cathode layer may be in the range from about 5 nm to about 1000 nm, for example, in the range from about 10 nm to about 100 nm. When the thickness of the cathode layer is in the range from about 5 nm to about 50 nm, the cathode layer may be transparent or semitransparent even if formed from a metal or metal alloy.

It is to be understood that the cathode layer is not part of an electron injection layer or the electron transport layer.

### Pixel definition layer

According to an embodiment of the invention, the organic electronic device may comprise a pixel definition layer by which the organic photodetector and/or the organic-light emitting device are separated from each other.

The pixel definition layer preferably is in direct contact with the substrate.

According to one embodiment, the pixel definition layer is not positive charged and/or is not an anode or comprises an anode material, preferably the pixel definition layer comprises Si based compound, SiN, a non-positive charged oligomer and/or polymer material. Preferred oligomer material include a polyacrylate-based resin or a polyimide-based resin. Also the pixel definition layer may futher include an inorganic matteri in addition to the polymer material as well as an light absorbing material, such a black pigment and/or a black dye, such as carbon black.

Hereinafter, the embodiments are illustrated in more detail with reference to examples. However, the present disclosure is not limited to the following examples. Reference will now be made in detail to the exemplary aspects.

### Description of the Drawings

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.

### Figures 1 to 20

FIG. 1 is a schematic view of an organic photodetector according an exemplary embodiment of the present invention;
FIG. 2 is a schematic view of an organic photodetector according an exemplary embodiment of the present invention;
FIG. 3 is a schematic view of an organic photodetector according an exemplary embodiment of the present invention;
FIG. 4 is a schematic view of an organic photodetector according an exemplary embodiment of the present invention;
FIG. 5 is a schematic view of an organic photodetector according an exemplary embodiment of the present invention;
FIG. 6 is a schematic view of an organic photodetector according an exemplary embodiment of the present invention.
FIG. 7 is a schematic view of an organic photodetector according an exemplary embodiment of the present invention;
FIG. 8 is a schematic view of an organic photodetector according an exemplary embodiment of the present invention;
FIG. 9 is a schematic view of an organic photodetector according an exemplary embodiment of the present invention;
FIG. 10 is a schematic view of an organic photodetector according an exemplary embodiment of the present invention;
FIG. 11 is a schematic view of an organic electronic device device according to an exemplary embodiment of the present invention;
FIG. 12 is a schematic view of an organic electronic device device according to an exemplary embodiment of the present invention;
FIG. 13 is a schematic view of an organic electronic device device according to an exemplary embodiment of the present invention;
FIG. 14 is a schematic view of an organic electronic device device according to an exemplary embodiment of the present invention;
FIG. 15 is a schematic view of an organic electronic device device according to an exemplary embodiment of the present invention;
FIG. 16 is a schematic view of an organic electronic device device according to an exemplary embodiment of the present invention;
FIG. 17 is a schematic view of an organic electronic device device according to an exemplary embodiment of the present invention;
FIG. 18 is a schematic view of an organic electronic device device according to an exemplary embodiment of the present invention;
FIG. 19 is a schematic view of an organic electronic device device according to an exemplary embodiment of the present invention;
FIG. 20 is a schematic view of an organic electronic device device according to an exemplary embodiment of the present invention;
FIG. 21 is a schematic view of an organic photodetector device according to an exemplary embodiment of the present invention;and
FIG. 22 is a schematic top view of an organic photodetector device according to an exemplary embodiment of the present invention.

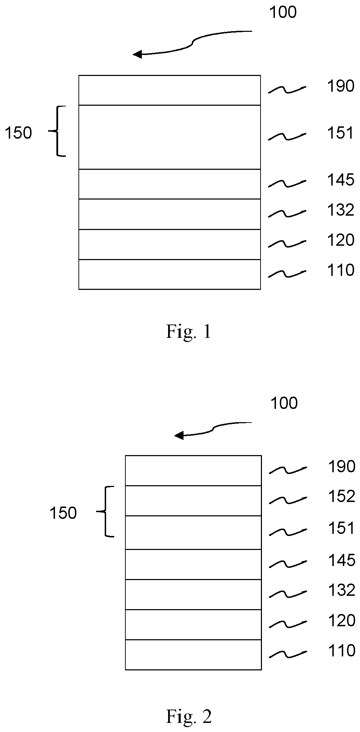

Hereinafter, the figures 1 to 22 are illustrated in more detail with reference to examples. However, the present disclosure is not limited to the following figures, which are of exemplary nature only.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

Referring to Fig. 1 the organic photodetector 100 includes a substrate 110, an anode layer 120, a hole transport region 132, a semiconductor layer 145, a photoconversion unit 150, and a cathode layer 190; wherein the photoconversion unit 150 may comprise one or more layer, wherein the photoconversion unit comprises an electron donor compound and an electron acceptor compound. The photoconversion unit 150 may comprise a first layer 151, wherein the first layer 151 comprises the electron donor compound and the electron acceptor compound. The semiconductor layer 145 comprises a metal complex of formula (I).

Referring to Fig. 2 the organic photodetector 100 includes a substrate 110, an anode layer 120, a hole transport region 132, a semiconductor layer 145, a first layer 151 of a photoconversion unit 150, a second layer 152 of a photoconversion unit 150, and a cathode layer 190. The first layer 151 of the photoconversion unit 150 may comprise the electron donor compound, the second layer 152 of the photoconversion unit 150 may comprise the electron acceptor compound.

The semiconductor layer 145 comprises a metal complex of formula (I).

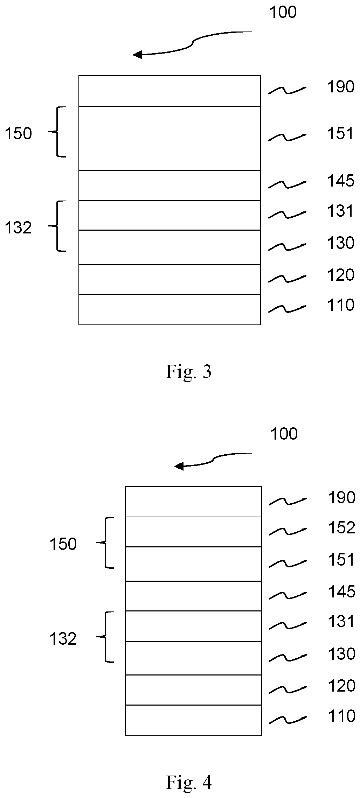

Referring to Fig. 3 the organic photodetector 100 includes a substrate 110, an anode layer 120, a hole injection layer 130 of the hole transport region 132, a hole transport layer 131 of the hole transport region 131, a semiconductor layer 145, a photoconversion unit 150, and a cathode layer 190; wherein the photoconversion unit 150 may comprise one or more layer, wherein the photoconversion unit comprises an electron donor compound and an electron acceptor compound. The photoconversion unit 150 may comprise a first layer 151, wherein the first layer 151 comprises the electron donor compound and the electron acceptor compound. The semiconductor layer 145 comprises a metal complex of formula (I).

Referring to Fig. 4 the organic photodetector 100 includes a substrate 110, an anode layer 120, a hole injection layer 130 of the hole transport region 132, a hole transport layer 131 of the hole transport region 131, a semiconductor layer 145, a first layer 151 of a photoconversion unit 150, a second layer 152 of a photoconversion unit 150, and a cathode layer 190. The first layer 151 of the photoconversion unit 150 may comprise the electron donor compound, the second layer 152 of the photoconversion unit 150 may comprise the electron acceptor compound.

The semiconductor layer 145 comprises a metal complex of formula (I).

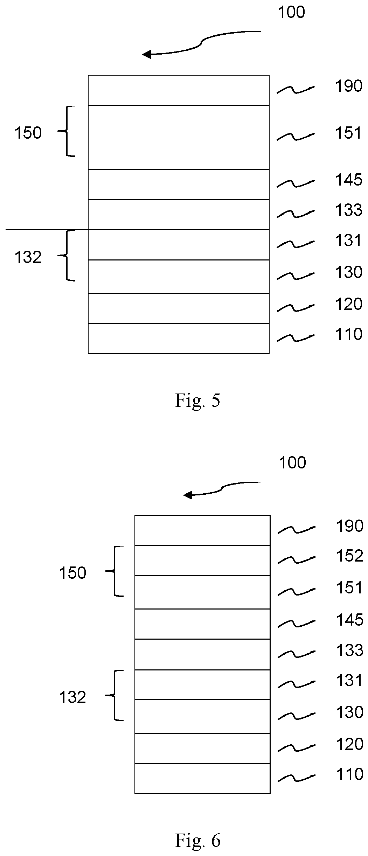

Referring to Fig. 5 the organic photodetector 100 includes a substrate 110, an anode layer 120, a hole injection layer 130 of the hole transport region 132, a hole transport layer 131 of the hole transport region 131, an electron blocking layer 133, a semiconductor layer 145, a photoconversion unit 150, and a cathode layer 190; wherein the photoconversion unit 150 may comprise one or more layer, wherein the photoconversion unit comprises an electron donor compound and an electron acceptor compound. The photoconversion unit 150 may comprise a first layer 151, wherein the first layer 151 comprises the electron donor compound and the electron acceptor compound. The semiconductor layer 145 comprises a metal complex of formula (I).

Referring to Fig. 6 the organic photodetector 100 includes a substrate 110, an anode layer 120, a hole injection layer 130 of the hole transport region 132, a hole transport layer 131 of the hole transport region 131, an electron blocking layer 133, a semiconductor layer 145, a first layer 151 of a photoconversion unit 150, a second layer 152 of a photoconversion unit 150, and a cathode layer 190. The first layer 151 of the photoconversion unit 150 may comprise the electron donor compound, the second layer 152 of the photoconversion unit 150 may comprise the electron acceptor compound.

The semiconductor layer 145 comprises a metal complex of formula (I).

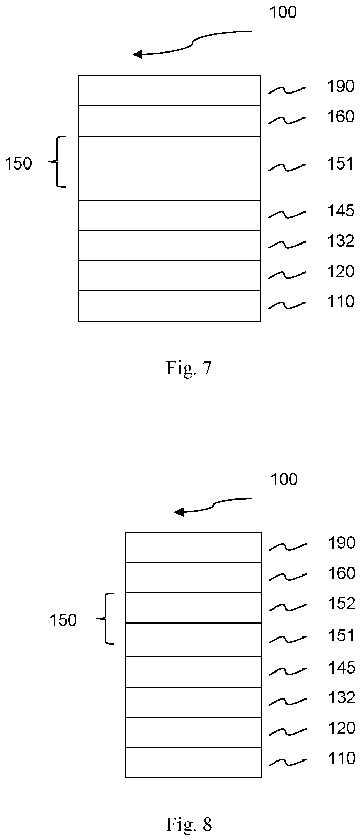

Referring to Fig. 7 the organic photodetector 100 includes a substrate 110, an anode layer 120, a hole transport region 132, a semiconductor layer 145, a photoconversion unit 150, an organic semiconductor layer 160 which is an electron transport layer and/or hole blocking layer, and a cathode layer 190;
wherein the photoconversion unit 150 may comprise one or more layer, wherein the photoconversion unit comprises an electron donor compound and an electron acceptor compound. The photoconversion unit 150 may comprise a first layer 151, wherein the first layer 151 comprises the electron donor compound and the electron acceptor compound. The semiconductor layer 145 comprises a metal complex of formula (I).

Referring to Fig. 8 the organic photodetector 100 includes a substrate 110, an anode layer 120, a hole transport region 132, a semiconductor layer 145, a first layer 151 of a photoconversion unit 150, a second layer 152 of a photoconversion unit 150, an organic semiconductor layer 160 which can be an electron transport layer and/or hole blocking layer, and a cathode layer 190. The first layer 151 of the photoconversion unit 150 may comprise the electron donor compound, the second layer 152 of the photoconversion unit 150 may comprise the electron acceptor compound.

The semiconductor layer 145 comprises a metal complex of formula (I).

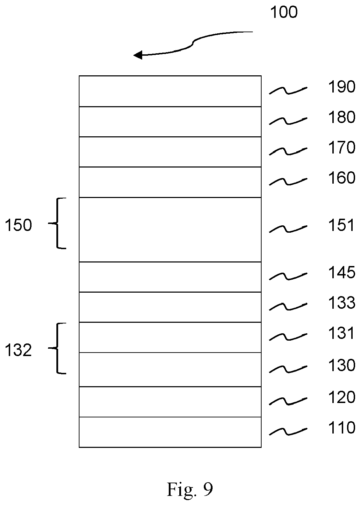

Referring to Fig. 9 the organic photodetector 100 includes a substrate 110, an anode layer 120, a hole injection layer 130 of the hole transport region 132, a hole transport layer 131 of the hole transport region 131, an electron blocking layer 133, a semiconductor layer 145, a photoconversion unit 150, a hole blocking layer 160, an electron transport layer 170, an electron injection layer 180, and a cathode layer 190; wherein the photoconversion unit 150 may comprise one or more layer, wherein the photoconversion unit comprises an electron donor compound and an electron acceptor compound. The photoconversion unit 150 may comprise a first layer 151, wherein the first layer 151 comprises the electron donor compound and the electron acceptor compound. The semiconductor layer 145 comprises a metal complex of formula (I).

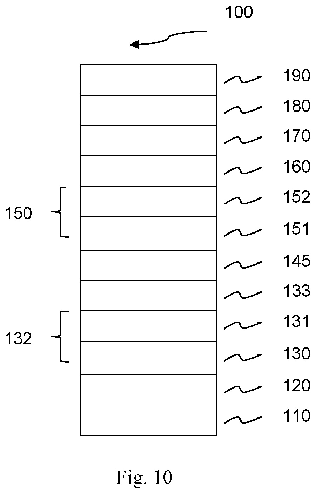

Referring to Fig. 10 the organic photodetector 100 includes a substrate 110, an anode layer 120, a hole injection layer 130 of the hole transport region 132, a hole transport layer 131 of the hole transport region 131, an electron blocking layer 133, a semiconductor layer 145, a first layer 151 of a photoconversion unit 150, a second layer 152 of a photoconversion unit 150, a hole blocking layer 160, an electron transport layer 170, an electron injection layer 180, and a cathode layer 190. The first layer 151 of the photoconversion unit 150 may comprise the electron donor compound, the second layer 152 of the photoconversion unit 150 may comprise the electron acceptor compound.

The semiconductor layer 145 comprises a metal complex of formula (I).

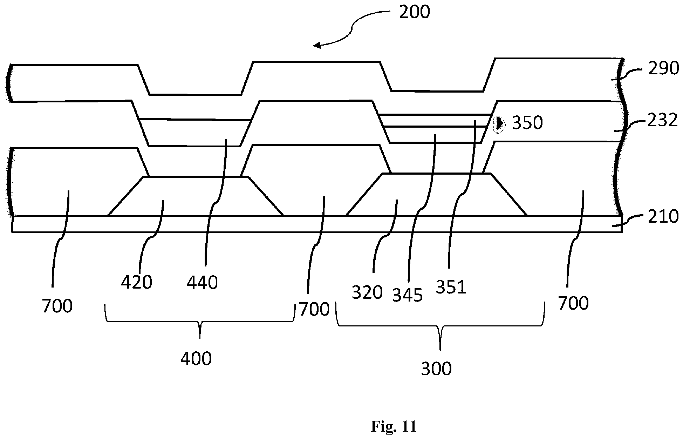

Referring to Fig. 11 the organic electronic device, which in particular is a display, includes a substrate 210, an organic photodetector 300 and an organic light-emitting device 400, which are separated by a pixel definition layer 700.

The organic photodetector includes 300 includes an anode layer 320, a hole transport region 232, a semiconductor layer 345, and a photoconversion unit 350, and a cathode layer 290; wherein the photoconversion unit 350 may comprise one or more layer, wherein the photoconversion unit comprises an electron donor compound and an electron acceptor compound. The photoconversion unit 350 may comprise a first layer 351, wherein the first layer 351 comprises the electron donor compound and the electron acceptor compound. The semiconductor layer 345 comprises a metal complex of formula (I).

The organic light-emitting device 400 includes an anode layer 420, a hole transport layer 232, a light-emitting layer 440, and a cathode layer 290.

The organic electronic device 200 have a hole transport region 232, wherein hole transport can a common hole transport region which is shared by the organic photodetector 300 and the organic light-emitting device 400, or (not shown) the organic photodetector 300 and organic light-emitting device 400 may each have its own hole transport region and each organic light emitting device 400 may have its own hole transport region. Preferably, the hole transport regions a common hole transport region.

Further, the organic electronic device 200 have a common cathode layer 290, wherein the common cathode layer 290 is shared by the organic photodetector 300 and the organic light-emitting device 400.

The organic photodetector 300 may further comprise an electron blocking layer (not shown).

The organic light-emitting device 400 may further comprises an electron blocking layer (not shown).

The electron blocking layer (not shown) of the organic photodetector 300 and the electron blocking layer ((not shown)) of the organic light-emitting device 400 may be shared by at least one organic photodetector and by at least one organic light-emitting diode.

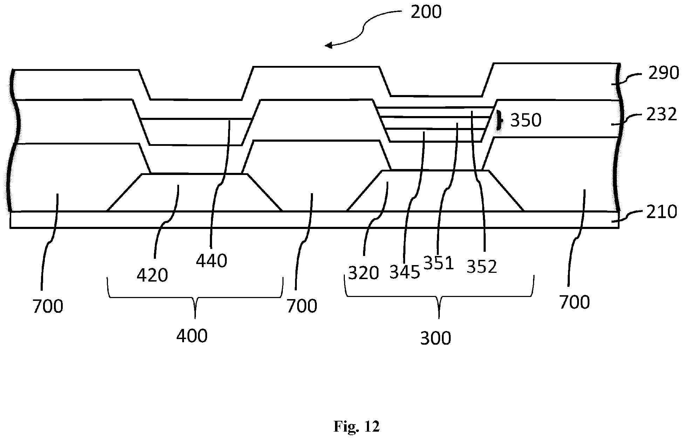

Referring to Fig. 12 the organic electronic device, which in particular is a display, includes a substrate 210, an organic photodetector 300 and an organic light-emitting device 400 which are separated by a pixel definition layer 700.

The organic photodetector includes 300 includes an anode layer 320, a hole transport region 232, a semiconductor layer 345, and a photoconversion unit 350, and a cathode layer 290; wherein the photoconversion unit 350 may comprise one or more layer, wherein the photoconversion unit comprises an electron donor compound and an electron acceptor compound. The first layer 351 of the photoconversion unit 350 may comprise the electron donor compound, the second layer 352 of the photoconversion unit 350 may comprise the electron acceptor compound. The semiconductor layer 345 comprises a metal complex of formula (I).

The organic light-emitting device 400 includes an anode layer 420, a hole transport region 232, a light-emitting layer 440, and a cathode layer 290.

The organic electronic device 200 have a hole transport region 232, wherein hole transport region can be a common hole transport region which is shared by the organic photodetector 300 and the organic light-emitting device 400, or (not shown) the organic photodetector 300 and organic light-emitting device 400 may each have its own hole transport region and each organic light emitting device 400 may have its own hole transport region. Preferably, the hole transport regions a common hole transport region.

Further, the organic electronic device 200 have a common cathode layer 290, wherein the common cathode layer 290 is shared by the organic photodetector 300 and the organic light-emitting device 400.

The organic photodetector 300 may further comprise an electron blocking layer (not shown).

The organic light-emitting device 400 may further comprises an electron blocking layer (not shown).

The electron blocking layer (not shown) of the organic photodetector 300 and the electron blocking layer ((not shown)) of the organic light-emitting device 400 may be shared by at least one organic photodetector and by at least one organic light-emitting diode.

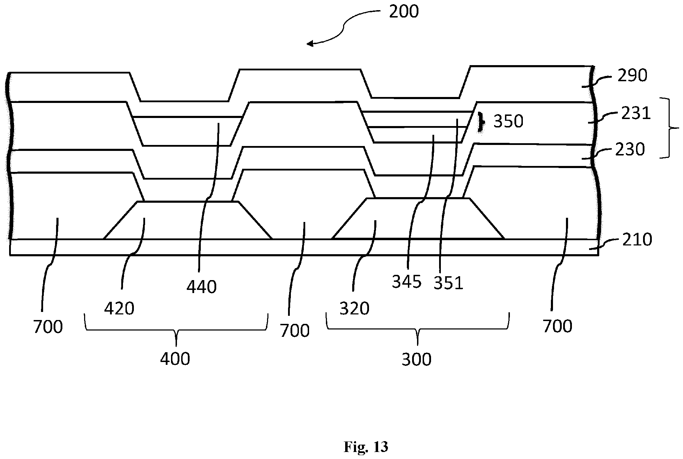

Referring to Fig. 13 the organic electronic device, which in particular is a display, includes a substrate 210, an organic photodetector 300 and an organic light-emitting device 400 which are separated by a pixel definition layer 700.

The organic photodetector includes 300 includes an anode layer 320, a hole injection layer 230 of the hole transport region 232, a hole transport layer 231 of the hole transport region 232, a semiconductor layer 345, and a photoconversion unit 350, and a cathode layer 290; wherein the photoconversion unit 350 may comprise one or more layer, wherein the photoconversion unit comprises an electron donor compound and an electron acceptor compound. The photoconversion unit 350 may comprise a first layer 351, wherein the first layer 351 comprises the electron donor compound and the electron acceptor compound. The semiconductor layer 345 comprises a metal complex of formula (I).

The organic light-emitting device 400 includes an anode layer 420, a hole injection layer 230 of the hole transport region 232, a hole transport layer 231 of the hole transport region 232, a light-emitting layer 440, and a cathode layer 290.

The organic electronic device 200 have a common hole injection layer 230 of the hole transport region 232, wherein the common hole injection layer 230 is shared by the organic photodetector 300 and the organic light-emitting device 400.

The organic electronic device 200 have a common hole transport layer 231 of the hole transport region 232, wherein the common hole transport layer 231 is shared by the organic photodetector 300 and the organic light-emitting device 400.

Further, the organic electronic device 200 have a common cathode layer 290, wherein the common cathode layer 290 is shared by the organic photodetector 300 and the organic light-emitting device 400.

The organic photodetector 300 may further comprise an electron blocking layer (not shown).

The organic light-emitting device 400 may further comprises an electron blocking layer (not shown).

The electron blocking layer (not shown) of the organic photodetector 300 and the electron blocking layer ((not shown)) of the organic light-emitting device 400 may be shared by at least one organic photodetector and by at least one organic light-emitting diode.

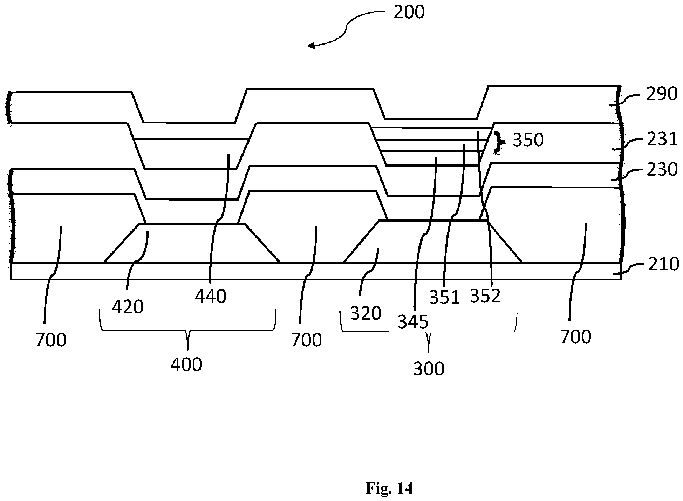

Referring to Fig. 14 the organic electronic device, which in particular is a display, includes a substrate 210, an organic photodetector 300 and an organic light-emitting device 400 which are each separated by a pixel definition layer 700.

The organic photodetector includes 300 includes an anode layer 320, a hole injection layer 230 of the hole transport region 232, a hole transport layer 231 of the hole transport region 232, a semiconductor layer 345, and a photoconversion unit 350, and a cathode layer 290; wherein the photoconversion unit 350 may comprise one or more layer, wherein the photoconversion unit comprises an electron donor compound and an electron acceptor compound. The first layer 351 of the photoconversion unit 350 may comprise the electron donor compound, the second layer 352 of the photoconversion unit 350 may comprise the electron acceptor compound. The semiconductor layer 345 comprises a metal complex of formula (I).

The organic light-emitting device 400 includes an anode layer 420, a hole injection layer 230 of the hole transport region 232, a hole transport layer 231 of the hole transport region 232, a light-emitting layer 440, and a cathode layer 290.

The organic electronic device 200 have a common hole injection layer 230 of the hole transport region 232, wherein the common hole injection layer 230 is shared by the organic photodetector 300 and the organic light-emitting device 400.

The organic electronic device 200 have a common hole transport layer 231 of the hole transport region 232, wherein the common hole transport layer 231 is shared by the organic photodetector 300 and the organic light-emitting device 400.

Further, the organic electronic device 200 have a common cathode layer 290, wherein the common cathode layer 290 is shared by the organic photodetector 300 and the organic light-emitting device 400.

The organic photodetector 300 may further comprise an electron blocking layer (not shown).

The organic light-emitting device 400 may further comprises an electron blocking layer (not shown).

The electron blocking layer (not shown) of the organic photodetector 300 and the electron blocking layer ((not shown)) of the organic light-emitting device 400 may be shared by at least one organic photodetector and by at least one organic light-emitting diode.

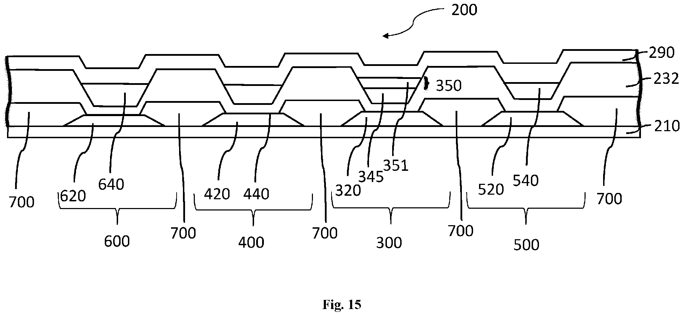

Referring to Fig. 15 the organic electronic device, which in particular is a display, includes a substrate 210, an organic photodetector 300, an organic light-emitting device 400, an organic light-emitting device 500, and an organic light-emitting device 600 which are each separated by a pixel definition layer 700.

The organic photodetector includes 300 includes an anode layer 320, a hole transport region 232, a semiconductor layer 345, and a photoconversion unit 350, and a cathode layer 290; wherein the photoconversion unit 350 may comprise one or more layer, wherein the photoconversion unit comprises an electron donor compound and an electron acceptor compound. The photoconversion unit 350 may comprise a first layer 351, wherein the first layer 351 comprises the electron donor compound and the electron acceptor compound. The semiconductor layer 345 comprises a metal complex of formula (I).

The organic light-emitting device 400 includes an anode layer 420, a hole transport region 232, a light-emitting layer 440, and a cathode layer 290; wherein the organic light-emitting device 400 emits green light.

The organic light-emitting device 500 includes an anode layer 520, a hole transport region 232, a light-emitting layer 540, and a cathode layer 290; wherein the organic light-emitting device 500 emits blue light.

The organic light-emitting device 400 includes an anode layer 620, a hole transport region 232, a light-emitting layer 640, and a cathode layer 290; wherein the organic light-emitting device 600 emits red-light.

The organic electronic device 200 have a hole transport region 232, wherein the hole transport region can be a common hole transport region which is shared by the organic the organic photodetector 300 the organic light-emitting device 400, the organic light-emitting device 500, and the organic light-emitting device 600, or (not shown) the organic photodetector 300, the organic light-emitting device 400, the organic light-emitting device 500, and the organic light-emitting device 600 may each have its own hole transport region. Preferably, the hole transport regions a common hole transport region.

Further, the organic electronic device 200 have a common have a common cathode layer 290, wherein the common cathode layer 290 is shared by the organic photodetector 300 the organic light-emitting device 400, the organic light-emitting device 500, and the organic light-emitting device 600.

The organic photodetector 300 may further comprise an electron blocking layer (not shown).

The organic light-emitting device 400 may further comprises an electron blocking layer (not shown).

The organic light-emitting device 500 may further comprises an electron blocking layer (not shown).

The organic light-emitting device 600 may further comprises an electron blocking layer (not shown).

The electron blocking layer (not shown) of the organic photodetector 300 and the electron blocking layer (not shown) of the organic light-emitting device 400 may be shared by the organic photodetector 300 and by the organic light-emitting diode 400; or the electron blocking layer (not shown) of the organic photodetector 300 and the electron blocking layer (not shown) of the organic light-emitting device 600 may be shared by the organic photodetector 300 and by the organic light-emitting diode 600.

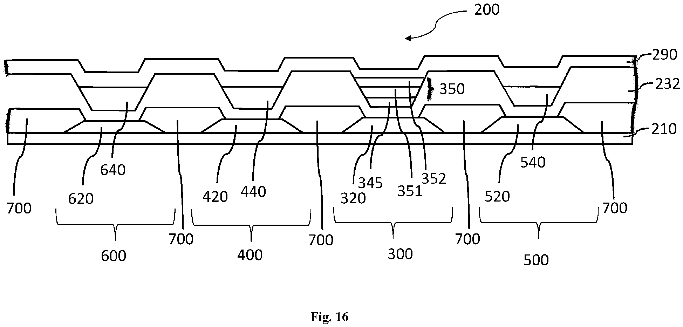

Referring to Fig. 16 the organic electronic device, which in particular is a display, includes a substrate 210, an organic photodetector 300, an organic light-emitting device 400, an organic light-emitting device 500, and an organic light-emitting device 600 which are each separated by a pixel definition layer 700.

The organic photodetector includes 300 includes an anode layer 320, a hole transport region 232, a semiconductor layer 345, and a photoconversion unit 350, and a cathode layer 290; wherein the photoconversion unit 350 may comprise one or more layer, wherein the photoconversion unit comprises an electron donor compound and an electron acceptor compound. The first layer 351 of the photoconversion unit 350 may comprise the electron donor compound, the second layer 352 of the photoconversion unit 150 may comprise the electron acceptor compound.

The semiconductor layer 345 comprises a metal complex of formula (I).

The organic light-emitting device 400 includes an anode layer 420, a hole transport region 232, a light-emitting layer 440, and a cathode layer 290; wherein the organic light-emitting device 400 emits green light.

The organic light-emitting device 500 includes an anode layer 520, a hole transport region 232, a light-emitting layer 540, and a cathode layer 290; wherein the organic light-emitting device 500 emits blue light.

The organic light-emitting device 400 includes an anode layer 620, a hole transport region 232, a light-emitting layer 640, and a cathode layer 290; wherein the organic light-emitting device 600 emits red-light.

The organic electronic device 200 have a hole transport region 232, wherein the hole transport region can be a common hole transport region which is shared by the organic the organic photodetector 300 the organic light-emitting device 400, the organic light-emitting device 500, and the organic light-emitting device 600, or (not shown) the organic photodetector 300, the organic light-emitting device 400, the organic light-emitting device 500, and the organic light-emitting device 600 may each have its own hole transport region. Preferably, the hole transport regions a common hole transport region.

Further, the organic electronic device 200 have a common have a common cathode layer 290, wherein the common cathode layer 290 is shared by the organic photodetector 300 the organic light-emitting device 400, the organic light-emitting device 500, and the organic light-emitting device 600.

The organic photodetector 300 may further comprise an electron blocking layer (not shown).

The organic light-emitting device 400 may further comprises an electron blocking layer (not shown).

The organic light-emitting device 500 may further comprises an electron blocking layer (not shown).

The organic light-emitting device 600 may further comprises an electron blocking layer (not shown).

The electron blocking layer (not shown) of the organic photodetector 300 and the electron blocking layer (not shown) of the organic light-emitting device 400 may be shared by the organic photodetector 300 and by the organic light-emitting diode 400; or the electron blocking layer (not shown) of the organic photodetector 300 and the electron blocking layer (not shown) of the organic light-emitting device 600 may be shared by the organic photodetector 300 and by the organic light-emitting diode 600.

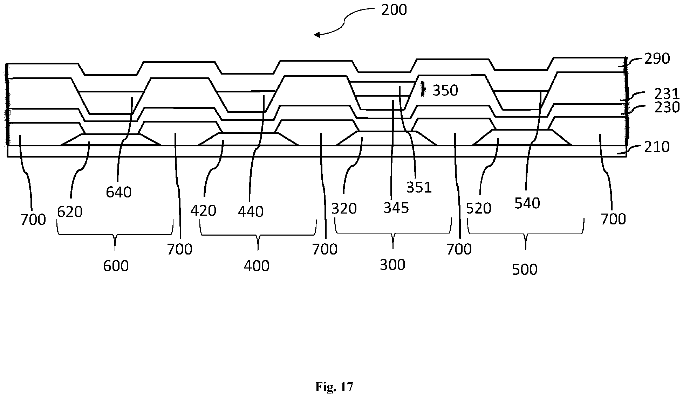

Referring to Fig. 17 the organic electronic device, which in particular is a display, includes a substrate 210, an organic photodetector 300, an organic light-emitting device 400, an organic light-emitting device 500, and an organic light-emitting device 600 which are each separated by a pixel definition layer 700.

The organic photodetector includes 300 includes an anode layer 320, a hole injection layer 230 of the hole transport region 232, a hole transport layer 231 of the hole transport region 232, a semiconductor layer 345, and a photoconversion unit 350, and a cathode layer 290; wherein the photoconversion unit 350 may comprise one or more layer, wherein the photoconversion unit comprises an electron donor compound and an electron acceptor compound. The photoconversion unit 350 may comprise a first layer 351, wherein the first layer 351 comprises the electron donor compound and the electron acceptor compound. The semiconductor layer 345 comprises a metal complex of formula (I).

The organic light-emitting device 400 includes an anode layer 420, a hole injection layer 230 of the hole transport region 232, a hole transport layer 231 of the hole transport region 232, a light-emitting layer 440, and a cathode layer 290; wherein the organic light-emitting device 400 emits green light.

The organic light-emitting device 500 includes an anode layer 520, a hole injection layer 230 of the hole transport region 232, a hole transport layer 231 of the hole transport region 232, a light-emitting layer 540, and a cathode layer 290; wherein the organic light-emitting device 500 emits blue light.

The organic light-emitting device 400 includes an anode layer 620, a hole injection layer 230 of the hole transport region 232, a hole transport layer 231 of the hole transport region 232,a light-emitting layer 640, and a cathode layer 290; wherein the organic light-emitting device 600 emits red-light.

The organic electronic device 200 have a common hole injection layer 230 of the hole transport region 232, wherein the common hole injection layer 230 is shared by the organic photodetector 300, and the organic light-emitting device 400, the organic light-emitting device 500, and the organic light-emitting device 600.

The organic electronic device 200 have a common hole transport layer 231 of the hole transport region 232, wherein the common hole transport layer 231 is shared by the organic photodetector 300 and the organic light-emitting device 400, the organic light-emitting device 500, and the organic light-emitting device 600.

Further, the organic electronic device 200 have a common have a common cathode layer 290, wherein the common cathode layer 290 is shared by the organic photodetector 300 the organic light-emitting device 400, the organic light-emitting device 500, and the organic light-emitting device 600.

The organic photodetector 300 may further comprise an electron blocking layer (not shown).

The organic light-emitting device 400 may further comprises an electron blocking layer (not shown).

The organic light-emitting device 500 may further comprises an electron blocking layer (not shown).

The organic light-emitting device 600 may further comprises an electron blocking layer (not shown).

The electron blocking layer (not shown) of the organic photodetector 300 and the electron blocking layer (not shown) of the organic light-emitting device 400 may be shared by the organic photodetector 300 and by the organic light-emitting diode 400; or the electron blocking layer (not shown) of the organic photodetector 300 and the electron blocking layer (not shown) of the organic light-emitting device 600 may be shared by the organic photodetector 300 and by the organic light-emitting diode 600.

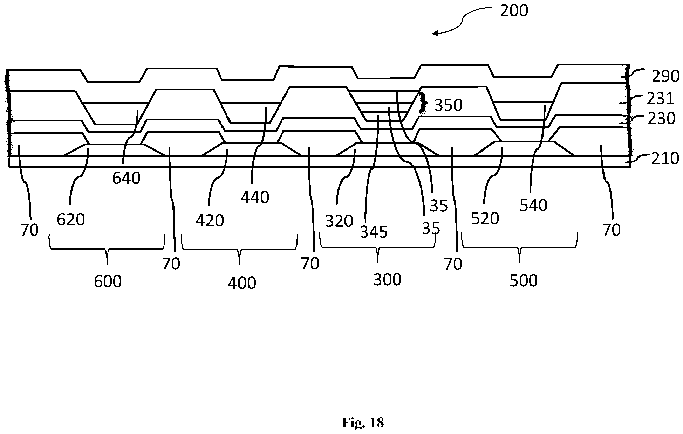

Referring to Fig. 18 the organic electronic device, which in particular is a display, includes a substrate 210, an organic photodetector 300, an organic light-emitting device 400, an organic light-emitting device 500, and an organic light-emitting device 600 which are each separated by a pixel definition layer 700.

The organic photodetector includes 300 includes an anode layer 320, a hole transport region 232, a semiconductor layer 345, and a photoconversion unit 350, and a cathode layer 290; wherein the photoconversion unit 350 may comprise one or more layer, wherein the photoconversion unit comprises an electron donor compound and an electron acceptor compound. The first layer 351 of the photoconversion unit 350 may comprise the electron donor compound, the second layer 352 of the photoconversion unit 150 may comprise the electron acceptor compound.

The semiconductor layer 345 comprises a metal complex of formula (I).

The organic light-emitting device 400 includes an anode layer 420, a hole transport region 232, a light-emitting layer 440, and a cathode layer 290; wherein the organic light-emitting device 400 emits green light.

The organic light-emitting device 500 includes an anode layer 520, a hole transport region 232, a light-emitting layer 540, and a cathode layer 290; wherein the organic light-emitting device 500 emits blue light.

The organic light-emitting device 400 includes an anode layer 620, a hole transport region 232, a light-emitting layer 640, and a cathode layer 290; wherein the organic light-emitting device 600 emits red-light.

The organic electronic device 200 have a hole transport region 232, wherein the hole transport region can be a common hole transport region which is shared by the organic the organic photodetector 300 the organic light-emitting device 400, the organic light-emitting device 500, and the organic light-emitting device 600, or (not shown) the organic photodetector 300, the organic light-emitting device 400, the organic light-emitting device 500, and the organic light-emitting device 600 may each have its own hole transport region. Preferably, the hole transport regions a common hole transport region.

Further, the organic electronic device 200 have a common have a common cathode layer 290, wherein the common cathode layer 290 is shared by the organic photodetector 300 the organic light-emitting device 400, the organic light-emitting device 500, and the organic light-emitting device 600.

The organic photodetector 300 may further comprise an electron blocking layer (not shown).

The organic light-emitting device 400 may further comprises an electron blocking layer (not shown).

The organic light-emitting device 500 may further comprises an electron blocking layer (not shown).

The organic light-emitting device 600 may further comprises an electron blocking layer (not shown).

The electron blocking layer (not shown) of the organic photodetector 300 and the electron blocking layer (not shown) of the organic light-emitting device 400 may be shared by the organic photodetector 300 and by the organic light-emitting diode 400; or the electron blocking layer (not shown) of the organic photodetector 300 and the electron blocking layer (not shown) of the organic light-emitting device 600 may be shared by the organic photodetector 300 and by the organic light-emitting diode 600.

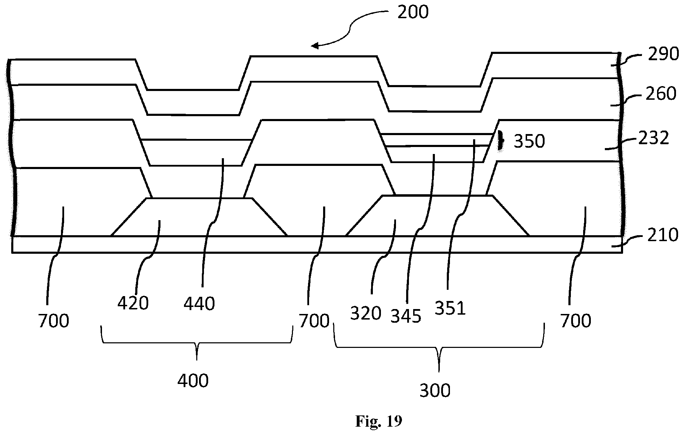

Referring to Fig. 19 the organic electronic device, which in particular is a display, includes a substrate 210, an organic photodetector 300 and an organic light-emitting device 400 which are separated by a pixel definition layer 700.

The organic photodetector includes 300 includes an anode layer 320, a hole transport region 232, a semiconductor layer 345, and a photoconversion unit 350, an electron transport region 260 which may comprises an electron transport layer, hole blocking layer and/or electron injection layer, and a cathode layer 290; wherein the photoconversion unit 350 may comprise one or more layer, wherein the photoconversion unit comprises an electron donor compound and an electron acceptor compound. The photoconversion unit 350 may comprise a first layer 351, wherein the first layer 351 comprises the electron donor compound and the electron acceptor compound. The semiconductor layer 345 comprises a metal complex of formula (I).

The organic light-emitting device 400 includes an anode layer 420, a hole transport region 232, a light-emitting layer 440, an electron transport region 260 which is in particular an electron transport layer, hole blocking layer and/or electron injection layer, and a cathode layer 290.

The organic electronic device 200 have a hole transport region 232, wherein hole transport region can a common hole transport region which is shared by the organic photodetector 300 and the organic light-emitting device 400, or (not shown) the organic photodetector 300 and organic light-emitting device 400 may each have its own hole transport region and each organic light emitting device 400 may have its own hole transport region. Preferably, the hole transport regions a common hole transport region.

The organic electronic device 200 have a common electron transport region 260 which may comprises a common electron transport layer, a common hole blocking layer and/or a common electron injection layer, wherein the common electron transport region 260 is shared by the organic photodetector 300 and the organic light-emitting device 400.

Further, the organic electronic device 200 have a common cathode layer 290, wherein the common cathode layer 290 is shared by the organic photodetector 300 and the organic light-emitting device 400.

The organic photodetector 300 may further comprise an electron blocking layer (not shown).

The organic light-emitting device 400 may further comprises an electron blocking layer (not shown).

The electron blocking layer (not shown) of the organic photodetector 300 and the electron blocking layer (not shown) of the organic light-emitting device 400 may be shared by the organic photodetector 300 and by the organic light-emitting diode 400.

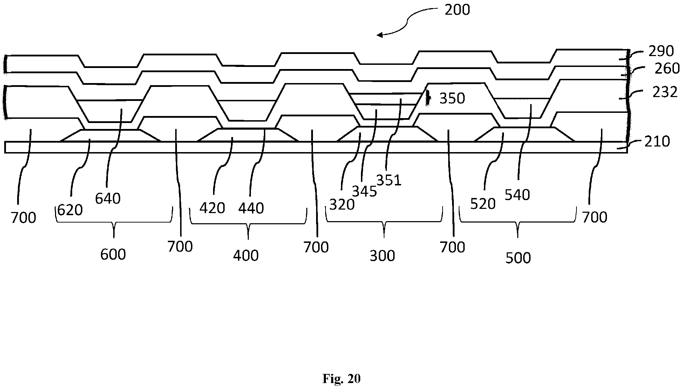

Referring to Fig. 20 the organic electronic device, which in particular is a display, includes a substrate 210, an organic photodetector 300, an organic light-emitting device 400, an organic light-emitting device 500, and an organic light-emitting device 600, which are each separated by a pixel definition layer.

The organic photodetector includes 300 includes an anode layer 320, a hole transport region 232, a semiconductor layer 345, and a photoconversion unit 350, an electron transport region 260 which may comprises an electron transport layer, hole blocking layer and/or electron injection layer, and a cathode layer 290; wherein the photoconversion unit 350 may comprise one or more layer, wherein the photoconversion unit comprises an electron donor compound and an electron acceptor compound. The photoconversion unit 350 may comprise a first layer 351, wherein the first layer 351 comprises the electron donor compound and the electron acceptor compound. The semiconductor layer 345 comprises a metal complex of formula (I).

The organic light-emitting device 400 includes an anode layer 420, a hole transport region 232, a light-emitting layer 440, an electron transport region 260 which may comprises an electron transport layer, hole blocking layer and/or electron injection layer, and a cathode layer 290; wherein the organic light-emitting device 400 emits green light.

The organic light-emitting device 500 includes an anode layer 520, a hole transport region 232, a light-emitting layer 540, an electron transport region 260 which may comprises an electron transport layer, hole blocking layer and/or electron injection layer, and a cathode layer 290; wherein the organic light-emitting device 500 emits blue light.

The organic light-emitting device 400 includes an anode layer 620, a hole transport region 232, a light-emitting layer 640, an electron transport region 260 which may comprises an electron transport layer, hole blocking layer and/or electron injection layer, and a cathode layer 290; wherein the organic light-emitting device 600 emits red-light.

The organic electronic device 200 have a hole transport region 232, wherein the hole transport region can be a common hole transport region which is shared by the organic the organic photodetector 300 the organic light-emitting device 400, the organic light-emitting device 500, and the organic light-emitting device 600, or (not shown) the organic photodetector 300, the organic light-emitting device 400, the organic light-emitting device 500, and the organic light-emitting device 600 may each have its own hole transport region. Preferably, the hole transport regions a common hole transport region.

The organic electronic device 200 have a common electron transport region 260 which may comprises a common electron transport layer, a common hole blocking layer and/or a common electron injection layer, wherein the common electron transport region 260 is shared by the organic photodetector, the organic light-emitting device 400, the organic light-emitting device 500, and the organic light-emitting device 600.

Further, the organic electronic device 200 have a common have a common cathode layer 290, wherein the common cathode layer 290 is shared by the organic photodetector 300 the organic light-emitting device 400, the organic light-emitting device 500, and the organic light-emitting device 600.

The organic photodetector 300 may further comprise an electron blocking layer (not shown).

The organic light-emitting device 400 may further comprises an electron blocking layer (not shown).

The organic light-emitting device 500 may further comprises an electron blocking layer (not shown).

The organic light-emitting device 600 may further comprises an electron blocking layer (not shown).

The electron blocking layer (not shown) of the organic photodetector 300 and the electron blocking layer (not shown) of the organic light-emitting device 400 may be shared by the organic photodetector 300 and by the organic light-emitting diode 400; or the electron blocking layer (not shown) of the organic photodetector 300 and the electron blocking layer (not shown) of the organic light-emitting device 600 may be shared by the organic photodetector 300 and by the organic light-emitting diode 600.

Fig. 21 is a schematic view of an organic photodetector device, used to determine dark and photo current, according to an exemplary embodiment of the present invention.

Referring to Fig. 21 the organic photodetector device includes a substrate 210, an anode layer 320, a pixel definition layer 700 defining the area size of the organic photodetector device, an organic semiconductor layer stack 800 and a cathode layer.

Fig. 22 is a schematic top view of an organic photodetector device, used to determine dark and photo current, according to an exemplary embodiment of the present invention.

Referring to Fig. 22 the organic photodetector device includes a substrate 210, an anode layer 320, a pixel definition layer 700 defining the area size 710 of the organic photodetector device, an organic semiconductor layer stack 800 and a cathode layer.

Hereinafter, one or more exemplary embodiments of the present invention will be described in detail with, reference to the following examples. However, these examples are not intended to limit the purpose and scope of the one or more exemplary embodiments of the present invention.

### Detailed discussion

The invention is furthermore illustrated by the following examples which are illustrative only and non-binding.

### Calculated LUMP of compounds of Formula (I) and comparative compounds

The energy of the lowest unoccupied molecular orbital (LUMP) of compounds of Formula (I) and comparative compounds were calculated with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany). The LUMP energy levels of the molecular structures are determined by applying the hybrid functional B3LYP with a Def2-TZVP basis set and the Stuttgart/Dresden (SDD) effective core potential (ECP) for the metals from the optimized geometries obtained by applying the functional BP86 with a Def2-SVP basis set the Stuttgart/Dresden (SDD) effective core potential (ECP) for the metals. For materials containing a Ce(IV) cation, the LUMP energy levels of the molecular structures are determined by applying the hybrid functional B3LYP with a SARC-ZORA-TZVP basis set for the metals and a ZORA-Def2-TZVP basis set for all other atoms from the optimized geometries obtained by applying the functional BP86 with a SARC-ZORA-TZVP basis set for the metals and a ZORA-Def2-SVP basis set for all other atoms. All the calculations were performed in the gas phase. All relativistic calculations were performed by applying the zero order regular approximation (ZORA). If more than one conformation is viable, the conformation with the lowest total energy is selected. Depending on the metal cation different multiplicities may be applied. For the following metal cations the multiplicity is shown in brackets: Cu²⁺(doublet), Cr³⁺(quartet), Mn²⁺(sextet), Mn³⁺(quintet), Fe³⁺(sextet), Co³⁺(quintet), Al³⁺(singlet), In³⁺(singlet), Ru³⁺(sextet), Ce⁴⁺(singlet). The LUMO values in Table 2 were calculated by this method unless noted otherwise.

According to an embodiment of compound of Formula (I), the LUMP of compound of Formula (I) is selected in the range of ≤ -2 eV and ≥ -6.5 eV, preferably ≤ -2.05 eV and ≥ -6 eV, more preferred ≤ -2.1 eV and ≥ -5.3 eV; wherein the LUMO is calculated by the method described above.

Thereby, particularly improved performance may be obtained in an organic photodector comprising a semiconductor layer comprising compound of Formula (I).

### Synthesis examples

Compounds of formula (I) in particular with a ligand of Formula (IIb) may be prepared by known methods or as described below.

### Synthesis of Tris((3-methyl-1-phenyl-4-(2,2,2-trifluoroacetyl)-1H-pyrazol-5-yl)oxy)iron (5MC7)

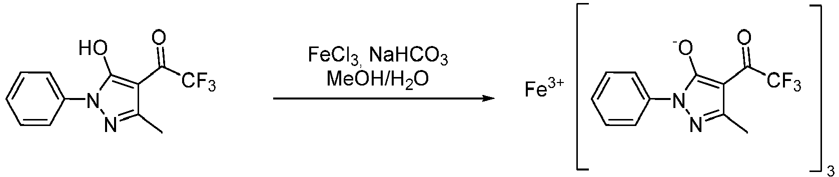

To a mixture of 12.8g (47.4mmol) Ligand and 3.98g (47.4mmol) NaHCO3 in 120ml methanol was added in portions a solution of 2.56g (15.8mmol) of FeCl3 in 15ml of water at ice-bath-temperature. The mixture was allowed to stir overnight, then the suspension was filtered and the product washed with little water-methanol. Drying resulted in 12.4g (91%) of the desired product.

### Synthesis of Bis((3-methyl-1-phenyl-4-(2,2,2-trifluoroacetyl)-1H-pyrazol-5-yl)oxy)copper (5MC6)

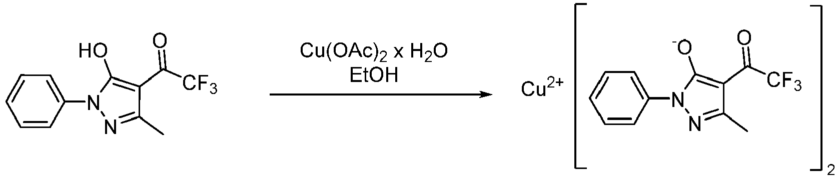

To a solution of 2.5g (9.25mmol) of ligand in 50ml of Ethanol was added 0.92g (4.63mmol) of copper (II) acetate and the mixture was stirred overnight. The resulting green suspension was filtered off and the solid was dried in vacuum to obtain 2.3g (83%) light green-yellowish powder.

### Synthesis of Tetrakis((3-methyl-1-phenyl-4-(2,2,2-trifluoroacetyl)-1H-pyrazol-5-yl)oxy)cerium (5MC9)

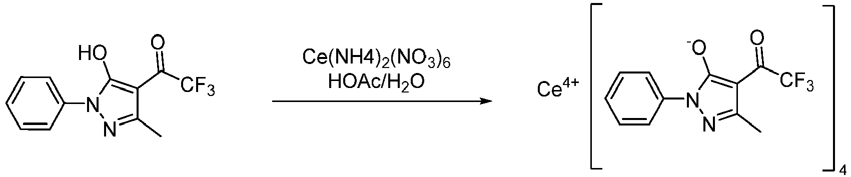

3.76g (13.92mmol) of ligand and 1.07g ammonium acetate (13.92mmol) were dissolved in 50ml acetic acid. 2.86g (5.22mmol) cerium ammonium nitrate were dissolved in 30ml water and added drop wise to the ligand. After 30min the resulting precipitate was filtered off and dried overnight at 100°C in vacuum to obtain 2.63g (62%) black solid.

### Synthesis of 1-(1-(3,5-bis(trifluoromethyl)phenyl)-5-hydroxy-3-methyl-1H-pyrazol-4-yl)-2,2,2-trifluoroethan-1-one

### Step 1. 1-(3,5-bis(trifluoromethyl)phenyl)-3-methyl-1H-pyrazol-5-ol

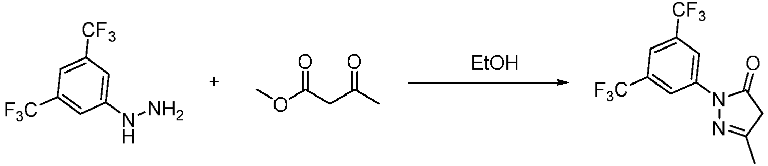

4.0g (16.38mmol) 3,5-bis(trifluoromethyl)phenyl hydrazine were dissolved in 60ml ethanol and a solution of 1.9g (16.38mmol) methyl acetoacetate was added dropwise. The reactions for heated to reflux for 4h. After removing the solvent, the residue was distilled from bulb to bulb in vacuum. The residue was further purified by flash chromatography using ethyl acetate/hexane and sublimation to obtain 2.33g (46%) slightly yellow, crystalline solid.

### Step 2. 1-(1-(3,5-bis(trifluoromethyl)phenyl)-5-hydroxy-3-methyl-1H-pyrazol-4-yl)-2,2,2-trifluoroethan-1-one

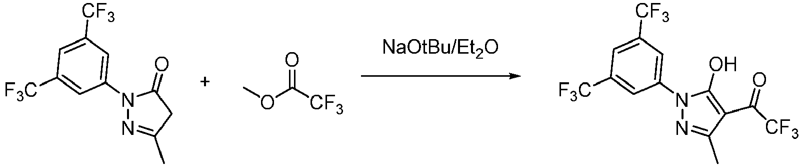

1.38g (14.38mmol) sodium tert-butylate and 1.1g (8.63mmol) trifluoroaceticacid methyl ester were dissolved in 10ml dry diethyl ether and cooled with an ice bath. A solution of 2.23g (7. 19mmol) 1-(3,5-bis(trifluoromethyl)phenyl)-3-methyl-1H-pyrazol-5-ol in 15ml diethyl ether was added dropwise. The mixture was stirred at room temperature overnight. After drying with magnesium sulfate the solvent was removed and the crude product sublimed to obtain 2.1g (72%) slightly reddish solid.

### Synthesis of Bis((1-(3,5-bis(trifluoromethyl)phenyl)-3-methyl-4-(2,2,2-trifluoroacetyl)-1H-pyrazol-5-yl)oxy)copper (5MC56)

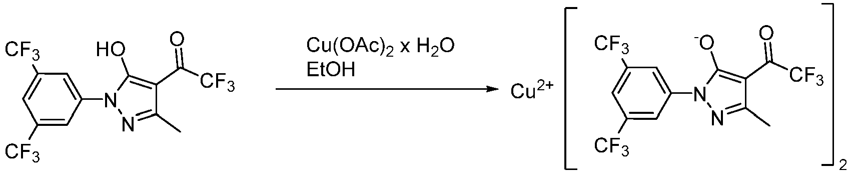

2.0g (4.92mmol) ligand was dissolved in 20ml ethanol and 0.49g (2.46mmol) copper (II) acetate were added. The mixture was stirred overnight at room temperature, cooled with an ice bath and the precipitate was filtered off, washed with ethanol and dried at 70°C in vacuum to obtain 1.95g (91%) bright green powder.

Further compounds according to invention may be prepared by the methods described above or by methods known in the art.

Further compounds according to invention may be prepared by the methods described above or by methods known in the art.

Compounds of formula (I) in particular with a ligand of formula (IIc) and (II£) may be prepared by methods known in the art and as described below.

Compounds of formula (I), wherein M is Cu, Zn, Cd, Pb and n is 2 may be prepared by methods described in Enchev, Venelin et al., J. Mol. Struct., 2001, 595(1-3), 67-76.

Compounds of formula (I), wherein M is Fe and n is 3 may be prepared by methods described in Ahmedova, Anife et al.,_Inorg. Chim. Acta, 2006, 359(10), 3123-3128.

Compounds of formula (I), wherein M is Fe and n is 2 may be prepared by methods described in Rusanov, Ventzislav et al. Eur. J. Chem., 2014, 5(1), 176-180.

Compounds of formula (III), (IV) and (V) may be prepared by methods known in the art
The protonated form of the ligand of formula (II) in particular of Formula (IIb) L-H may be prepared by methods known in the art, for example as described in J. G. Lombardino, J. Org. Chem. 1968, 33, 10, 3938-3941.

Compounds according to formula (I) in particular with a ligand of Formula (IId) or (IIg) may be prepared by methods known in the art and as described below.

### Synthesis of bis(l-(1,3-dioxo-1,3-dihydro-2H-inden-2-ylidene)-2,2,2-trifluoroethoxy)copper (7MC1)

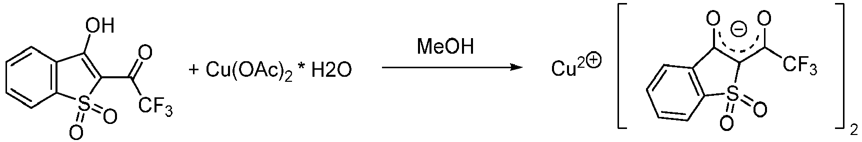

2.42g (10mmol) of 3-hydroxy-2-(2,2,2-trifluoroacetyl)-1H-inden-1-one and 1g (5mmol) of copper acetate monohydrate were added to 50ml methanol and stirred vigorously for 3 days. The suspension was filtered and the solid washed with methanol. After drying in high vacuum at 60°C, 2.39g (95%) product was obtained as a pale green powder, which was further purified by sublimation in vacuo.

### Synthesis of tris((2-(3,5-bis(trifluoromethyl)benzoyl)-1.1-dioxidobenzo[b]thiophen-3-yl)oxy)iron (7MC40)

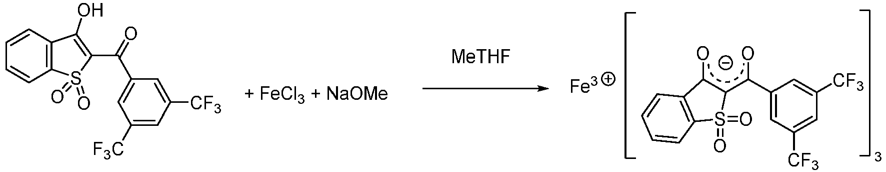

3g (7.1mmol) of (3,5-bis(trifluoromethyl)phenyl)(3-hydroxy-1,1-dioxidobenzo[b]thiophen-2-yl)methanone was dissolved in 30mL 2-methyltetrahydrofurane and 0.38g (7.1mmol) sodium methoxide and 0.41g (2.49mmol) iron trichloride were added. The mixture was stirred at room temperature for 2 hours. The precipitate was filtered off and the solvent was removed under reduced pressure. The residue was dissolved in dichloromethane and the product was precipitated by adding hexane. After filtration the product was dried in high vacuum to obtain 2.82g (90%) dark red solid, which was further purified by sublimation in vacuo.

### Synthesis of bis((2-(3,5-bis(trifluoromethyl)benzovl)-1,1-dioxidobenzo[b]thiophen-3-yl)oxy)manganese (7MC65)

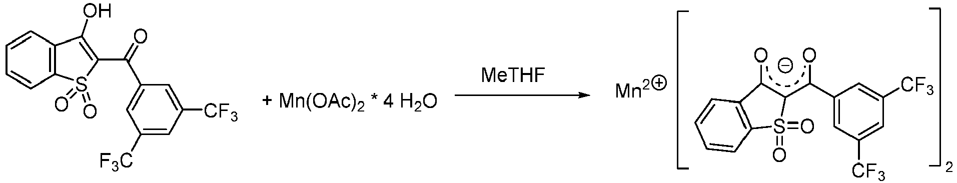

4,0g (9.47mmol) of (3,5-bis(trifluoromethyl)phenyl)(3-hydroxy-1,1-dioxidobenzo[b]thiophen-2-yl)methanone was dissolved in 40mL ethanol and a solution of 1.16g (4,74mmol) of manganese acetate tetrahydrate in 35ml ethanol was added. The mixture was stirred under reflux for 2 hours. After cooling the precipitate was filtered off and washed with ethanol and the solvent was removed under reduced pressure. The residue was dissolved in tetrahydrofurane and precipitated with hexane. The solid was filtered off, washed with hexane and dried in high vacuum to obtain 2,41g (57%) pale orange solid, which was further purified by sublimation in vacuo.

The protonated form of the ligand of the following ligand according to formula (II) L-H may be prepared by methods known in the art, for example in M. Kolb, J. Barth, B. Neises, Tetrahedron Lett., 1986, 27(14), 1579-82 and R. Saijo, K. Kurihara, M. Kawase, Heterocycles, 2013, 87(12), 2533-2553, and as described below.

### Synthesis of 2-phenyl-4-(2.2.2-trifluoroacetyl)oxazol-5(4H)-one (B3-H)

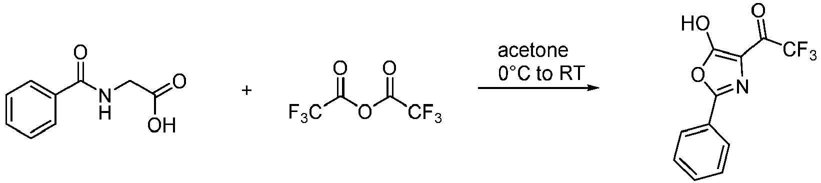

52.9g (252mmol) of 2,2,2-trifluoroacetic anhydride was added dropwise to a mixture of 15.1g (84mmol) of benzoylglycine in 150mL dry acetone at 0°C to 5°C. The resulting mixture was warmed to room temperature over 20h. 300mL water was added to the reaction and the precipitate was filtered, washed with water and dried. The crude product was stirred in 150mL ethylacetate, filtrated, washed with ethylacetate and dried. 15 g (69%) product was obtained as a pink solid.

Compounds of Formula (I) in particular with a ligand of Formula (IIb) may be prepared by methods known in the art and as described below.

### Synthesis of bis((2-phenyl-4-(2.2.2-trifluoroacetyl)oxazol-5-yl)oxy)copper (8MC-3)

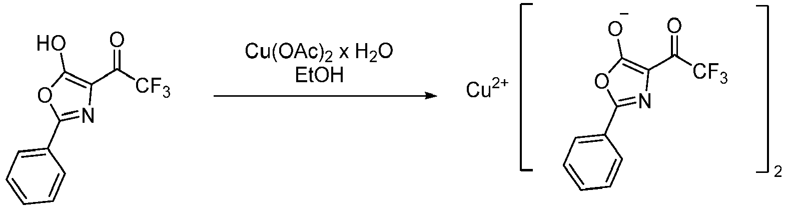

A suspension of 3.0g (11.7mmol) of 2,2,2-trifluoro-1-(5-hydroxy-2-phenyloxazol-4-yl)ethan-1-one in 5mL ethanol was added to a suspension of 1.16g (5.83mmol) copper acetate monohydrate in 10mL ethanol and stirred at room temperature for 2h. The solid was filtered, washed with ethanol and dried in vacuum. 3.1g (93%) product was obtained as an orange-brown solid. The compound may be purified further by sublimation in vacuum.

### Synthesis of bis((2-phenyl-4-(2.2.2-trifluoroacetyl)oxazol-5-yl)oxy)zinc (8MC-79)

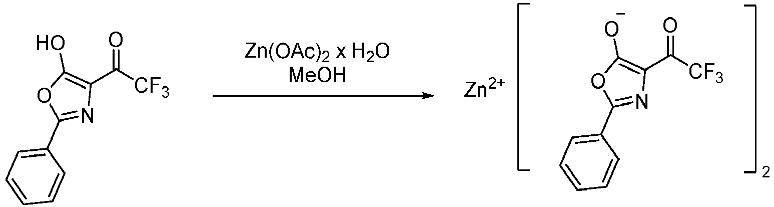

4.0g (15.6mmol) of 2,2,2-trifluoro-1-(5-hydroxy-2-phenyloxazol-4-yl)ethan-1-one was added to a suspension of 1.43g (7.78mmol) zinc acetate in 40mL methanol and stirred at room temperature overnight. The solid was filtered, washed with methanol and dried in vacuum. 4.1g (92%) product was obtained as a white powder. The compound may be purified further by sublimation in vacuum.

The protonated form of the ligand of formula (II) in particular (IIb) L-H may be prepared by methods known in the art.

Compounds of Formula (I) in particular with a ligand of Formula (IIb) may be prepared by methods known in the art and as described below.

### Synthesis of bis((4-(2.2-difluoroacetyl)-3-(difluoromethyl)-1-phenyl-1H-pyrazol-5-vl)oxy)copper (MC-9)

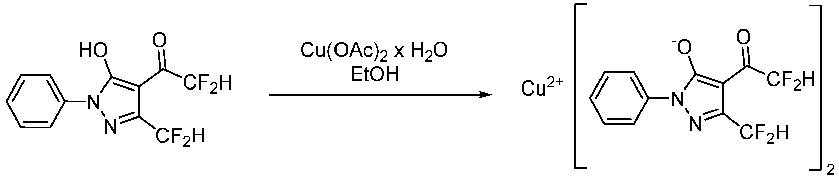

1.64g (8.2mmol) copper acetate monohydrate was suspended in 20mL methanol and a solution of 4.72g (16.4mmol) 1-(3-(difluoromethyl)-5-hydroxy-1-phenyl-1H-pyrazol-4-yl)-2,2-difluoroethan-1-one in 10ml methanol was added dropwise. The reaction was stirred overnight at room temperature. The precipitated was filtered and washed with methanol and dried in high vacuum. 4.57g (88%) product was obtained as a yellow-greenish powder. The compound may be purified further by sublimation in vacuum.

Compounds MC-17 and MC-65 have been prepared by the same method.

### Calculated HOMO and LUMP of other compounds than the compound formula (I) and its comparative example

The HOMO and LUMO are calculated with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany). The LUMO energy levels of the molecular structures are determined by applying the hybrid functional B3LYP with a Def2-TZVP basis set and the Stuttgart/Dresden (SDD) effective core potential (ECP) for the metals from the optimized geometries obtained by applying the functional BP86 with a Def2-SVP basis set the Stuttgart/Dresden (SDD) effective core potential (ECP) for the metals. For materials containing a Ce(IV) cation, the LUMO energy levels of the molecular structures are determined by applying the hybrid functional B3LYP with a SARC-ZORA-TZVP basis set for the metals and a ZORA-Def2-TZVP basis set for all other atoms from the optimized geometries obtained by applying the functional BP86 with a SARC-ZORA-TZVP basis set for the metals and a ZORA-Def2-SVP basis set for all other atoms. All the calculations were performed in the gas phase. All relativistic calculations were performed by applying the zero order regular approximation (ZORA). If more than one conformation is viable, the conformation with the lowest total energy is selected.

Depending on the metal cation different multiplicities may be applied. For the following metal cations the multiplicity is shown in brackets: Cu²⁺(doublet), Cr³⁺(quartet), Mn²⁺(sextet), Mn³⁺(quintet), Fe³⁺(sextet), Co³⁺(quintet), Al³⁺(singlet), In³⁺(singlet), Ru³⁺(sextet), Ce⁴⁺(singlet).

### General procedure for fabrication of OPDs

For inventive examples and comparative examples in Table 2, a glass substrate with an anode layer comprising a first anode sub-layer of 120 nm Ag, a second anode sub-layer of 8 nm ITO and a third anode sub-layer of 10 nm ITO was cut to a size of 25 mm × 25 mm × 0.7 mm, ultrasonically washed with water for 60 minutes and then with isopropanol for 20 minutes. The liquid film was removed in a nitrogen stream, followed by plasma treatment to prepare the anode layer. The plasma treatment was performed in an atmosphere comprising 97.6 vol.-% nitrogen and 2.4 vol.-% oxygen.

Then N,N'-Bis(naphthalen-1-yl)-N,N'-bis(phenyl)-benzidine ([123847-85-8]) was vacuum co-deposited with 3 wt% of 4,4',4"-((1E,1'E,1"E)-cyclopropane-1,2,3-triylidenetris(cyanomethanylylidene))tris(2,3,5,6-tetrafluorobenzonitrile) ([1224447-88-4]) to form a hole injection layer having a thickness of 10 nm.

Then N,N'-Bis(naphthalen-1-yl)-N,N'-bis(phenyl)-benzidine ([123847-85-8]) was vacuum deposited on the hole injection layer, to form a hole transport layer having a thickness of 135 nm.

Then Boron subphthalocyanine chloride ([36530-06-0]) was vacuum co-deposited with 4 vol% of a compound of formula (I) or a comparative example (the exact compound can be seen in table 2) on the hole transport layer, to form hole extraction layer having a thickness of 5 nm.

Then Boron subphthalocyanine chloride ([36530-06-0]) and Fullerene-C60 ([99685-96-8]) in a volume ratio of 25:75 were vacuum co-deposited on the hole extraction layer, to form a photoconversion unit having a thickness of 40 nm.

Then tris(quinolin-8-yloxy)aluminum ([2085-33-8]) was vacuum deposited to form the hole blocking layer on the photoconversion unit having a thickness of 5 nm.

Then, 50 vol% tris(quinolin-8-yloxy)aluminum ([2085-33-8]) and 50 vol% LiQ ([850918-68-2]) were vacuum co-deposited on the hole blocking layer to form an electron transport layer having a thickness of 21 nm.

Then Yb was vacuum deposited at a rate of 0.01 to 1 Å/s at 10⁻⁷ mbar on the electron transport layer to form an electron injection layer having a thickness of 1.3 nm.

Ag/Mg (90 vol% Ag) was vacuum deposited at a rate of 0.01 to 1 Å/s at 10⁻⁷ mbar to form a cathode having a thickness of 13 nm.

Then, N-(f[1,1-'biphenyl]-4-yl)-9,9,dimethyl-N-(4-(9-phenyl-9H-carbazol-3-yl)phenyl)-9H-fluoren-2-amine} (N-1) was vacuum deposited on the cathode layer to form a capping layer with a thickness of 75 nm.

Substrate temperature during deposition process is at room temperature. Waiting time in between individual layers is less than 60 minutes. Deposition is done in the dark.

The device stack is protected from ambient conditions by encapsulation of the device with a glass lid. The glass lid provides a cavity, which includes a getter material for further protection.

### Measurement of the photocurrent by illumination of the organic photodetector

The photocurrent by illumination of the organic photodetector having an area size of 6.35 mm² is measured in a box under illumination by applying a voltage of -3V±0.05 at 22°C.

The voltage is applied by means of Keithley SM2635B and the current densityis determined by means of Keithley SM2635B. The positive pol of current-voltage measuring device (source measure unit) Keithley SM2635B is connected to the anode layer of the organic photodetector, and the negative pol of source measure unit Keithley SM2635B is connected to the cathode layer of the organic photodetector. The voltage is applied for a period (duration) of at least one second before the current is measured with a measurement time of 5 NPLC (Number of Power Line-Cycles).

For device illumination 2 sets of RGB LEDs (Cree CLX6F-FKC-CKNNQDGBB7A363) are used. In total 6 LEDs: 2x red, 2x blue, 2x green, while each is driven with 0.63mA. LEDs are circular arranged and have lateral distance of 7.6mm to each other. Distance each diode to center is 3.8mm, respectively. Vertical distance from center to organic photodetector is 13mm. The area size of the organic photodetector is determined by the area wherein the anode layer, cathode layer, and the layers between the anode layer and the cathode layer overlaps.

In the overlap of the area anode layer, cathode layer, and the layer between the anode layer and cathode layer are stacked on top of each other. The anode layer area may be defined by a pixel definition layer aperture like shown in figures 21/22.

### Measurement of the dark current (current under dark conditions) of the organic photodetector

The dark current of the organic photodetector having an area size of 6.35 mm² is measured in a box in dark conditions by applying a voltage from -3V±0.05 at 22°C.

The voltage is applied by means of Keithley SM2635B and the current density-is determined by means of Keithley SM2635B. The positive pol of source measure unit Keithley SM2635B is connected to the anode layer of the organic photodetector, and the negative pol of source measure unit Keithley SM2635B is connected to the cathode layer of the organic photodetector. The voltage is applied for a period (duration) of at least one second before the current is measured with a measurement time of 5 NPLC (Number of Power Line-Cycles).

### Technical effect of the invention

In Table 1 are shown the calculated LUMO energy levels of metal complexes of Formula (I). If more than one spin-state is viable, the spin-state is shown in the respective column.

**Table 1: Calculated LUMO energy levels of metal complexes of Formula (I)**

| Referred to as: | Metal M | Valency n | Ligand L | LUMO [eV] | Spin state |
|---|---|---|---|---|---|
| 5MC1 | Ag(I) | 1 | A1 | -2.20 | |
| 5MC2 | Ir(III) | 3 | A1 | -2.66 | |
| 5MC3 | In(III) | 3 | A1 | -2.67 | |
| 5MC4 | Al(III) | 3 | A1 | -2.80 | |
| 5MC5 | Mn(III) | 3 | A1 | -3.34 | β |
| 5MC6 | Cu(II) | 2 | A1 | -3.61 | β |
| 5MC7 | Fe(III) | 3 | A1 | -4.25 | β |
| 5MC8 | Ru(III) | 3 | A1 | -4.37 | β |
| 5MC9 | CeβIV) | 4 | A1 | -4.50 | |
| 5MC10 | Cu(II) | 2 | A161 | -3.58 | β |
| 5MC11 | Cu(II) | 2 | A158 | -3.50 | β |
| 5MC12 | Cu(II) | 2 | A164 | -3.71 | β |
| 5MC13 | Cu(II) | 2 | A160 | -3.74 | β |
| 5MC14 | Cu(II) | 2 | A166 | -3.46 | β |
| 5MC15 | Cu(II) | 2 | A171 | -3.68 | β |
| 5MC16 | Cu(II) | 2 | A174 | -3.80 | β |
| 5MC17 | Cu(II) | 2 | A168 | -3.75 | β |
| 5MC18 | Cu(II) | 2 | A177 | -3.93 | β |
| 5MC19 | Cu(II) | 2 | A170 | -3.95 | β |
| 5MC20 | Cu(II) | 2 | A180 | -3.54 | β |
| 5MC21 | Cu(II) | 2 | A185 | -3.77 | β |
| 5MC22 | Cu(II) | 2 | A188 | -3.88 | β |
| 5MC23 | Cu(II) | 2 | A182 | -3.84 | β |
| 5MC24 | Cu(II) | 2 | A191 | -4.02 | β |
| 5MC25 | Cu(II) | 2 | A184 | -4.02 | β |
| 5MC26 | Cu(II) | 2 | A194 | -3.66 | β |
| 5MC27 | Cu(II) | 2 | A202 | -3.84 | β |
| 5MC28 | Cu(II) | 2 | A204 | -3.94 | β |
| 5MC29 | Cu(II) | 2 | A199 | -3.87 | β |
| 5MC30 | Cu(II) | 2 | A207 | -4.05 | β |
| 5MC31 | Cu(II) | 2 | A201 | -4.06 | β |
| 5MC32 | Cu(II) | 2 | A169 | -3.71 | β |
| 5MC33 | Fe(III) | 3 | A161 | -4.20 | β |
| 5MC34 | Fe(III) | 3 | A158 | -4.11 | β |
| 5MC35 | Fe(III) | 3 | A164 | -4.38 | β |
| 5MC36 | Fe(III) | 3 | A160 | -4.39 | β |
| 5MC37 | Fe(III) | 3 | A166 | -4.02 | β |
| 5MC38 | Fe(III) | 3 | A171 | -4.33 | β |
| 5MC39 | Fe(III) | 3 | A174 | -4.50 | β |
| 5MC40 | Fe(III) | 3 | A168 | -4.39 | β |
| 5MC41 | Fe(III) | 3 | A178 | -4.67 | β |
| 5MC42 | Fe(III) | 3 | A170 | -4.66 | β |
| 5MC43 | Fe(III) | 3 | A180 | -4.10 | β |
| 5MC44 | Fe(III) | 3 | A185 | -4.42 | β |
| 5MC45 | Fe(III) | 3 | A188 | -4.60 | β |
| 5MC46 | Fe(III) | 3 | A182 | -4.49 | β |
| 5MC47 | Fe(III) | 3 | A191 | -4.77 | β |
| 5MC48 | Fe(III) | 3 | A184 | -4.76 | β |
| 5MC49 | Fe(III) | 3 | A194 | -4.28 | β |
| 5MC50 | Fe(III) | 3 | A202 | -4.53 | β |
| 5MC51 | Fe(III) | 3 | A204 | -4.67 | β |
| 5MC52 | Fe(III) | 3 | A199 | -4.58 | β |
| 5MC53 | Fe(III) | 3 | A207 | -4.81 | β |
| 5MC54 | Fe(III) | 3 | A201 | -4.81 | β |
| 5MC55 | Fe(III) | 3 | A169 | -4.11 | β |
| 5MC56 | Cu(II) | 2 | A9 | -3.76 | β |
| 5MC57 | Cu(II) | 2 | A53 | -3.81 | β |
| 5MC58 | Cu(II) | 2 | A85 | -3.97 | β |
| 5MC59 | Cu(II) | 2 | A212 | -3.92 | β |
| 5MC60 | Fe(III) | 3 | A212 | -4.68 | β |
| 5MC61 | Cu | 2 | A213 | -4.16 | β |
| 5MC62 | Cu | 2 | A214 | -4.24 | β |
| 5MC63 | Cu | 2 | A215 | -4.21 | β |
| 5MC64 | Cu | 2 | A216 | -4.55 | β |
| 5MC65 | Cu | 2 | A217 | -4.38 | β |
| 5MC66 | Mn(II) | 2 | A213 | -3.00 | β |
| 5MC67 | Mn(II) | 2 | A216 | -3.41 | β |
| 5MC68 | Zn | 2 | A213 | -2.75 | |
| 5MC69 | Zn | 2 | A216 | -3.17 | |
| MC-1 | Cu | 2 | E1 | -3.95 | β |
| MC-2 | Fe | 3 | E1 | -4.02 | β |
| MC-3 | Mn | 2 | E1 | -2.51 | β |
| MC-4 | Zn | 2 | E1 | -2.11 | |
| MC-66 | Mn | 3 | E1 | -4.12 | α |
| MC-75 | Ru | 3 | E1 | -3.96 | β |
| MC-84 | In | 3 | E1 | -2.07 | |
| MC-93 | Al | 3 | E1 | -2.11 | |
| MC-102 | Ce | 4 | E1 | -4.12 | |
| MC-5 | Cu | 2 | E3 | -4.08 | β |
| MC-6 | Fe | 3 | E3 | -4.22 | β |
| MC-7 | Mn | 2 | E3 | -2.79 | β |
| MC-8 | Zn | 2 | E3 | -2.43 | |
| MC-67 | Mn | 3 | E3 | -4.32 | α |
| MC-76 | Ru | 3 | E3 | -4.16 | β |
| MC-85 | In | 3 | E3 | -2.44 | |
| MC-94 | Al | 3 | E3 | -2.51 | |
| MC-103 | Ce | 4 | E3 | -4.26 | |
| MC-9 | Cu | 2 | E5 | -4.45 | β |
| MC-10 | Fe | 3 | E5 | -4.64 | β |
| MC-11 | Mn | 2 | E5 | -3.19 | β |
| MC-12 | Zn | 2 | E5 | -2.85 | --- |
| MC-68 | Mn | 3 | E5 | -4.74 | α |
| MC-77 | Ru | 3 | E5 | -4.49 | β |
| MC-86 | In | 3 | E5 | -2.88 | |
| MC-95 | Al | 3 | E5 | -2.96 | |
| MC-104 | Ce | 4 | E5 | -4.68 | |
| MC-13 | Cu | 2 | E6 | -4.54 | β |
| MC-14 | Fe | 3 | E6 | -4.78 | β |
| MC-15 | Mn | 2 | E6 | -3.29 | β |
| MC-16 | Zn | 2 | E6 | -2.96 | |
| MC-69 | Mn | 3 | E6 | -4.88 | α |
| MC-78 | Ru | 3 | E6 | -4.71 | β |
| MC-87 | In | 3 | E6 | -3.08 | |
| MC-96 | Al | 3 | E6 | -3.17 | |
| MC-105 | Ce | 4 | E6 | -4.78 | |
| MC-17 | Cu | 2 | E7 | -4.60 | β |
| MC-18 | Fe | 3 | E7 | -4.80 | β |
| MC-19 | Mn | 2 | E7 | -3.35 | β |
| MC-20 | Zn | 2 | E7 | -3.01 | |
| MC-71 | Mn | 3 | E7 | -4.89 | α |
| MC-80 | Ru | 3 | E7 | -4.70 | β |
| MC-89 | In | 3 | E7 | -3.07 | |
| MC-98 | Al | 3 | E7 | -3.15 | |
| MC-107 | Ce | 4 | E7 | -4.80 | |
| MC-65 | Cu | 2 | E13 | -4.61 | β |
| MC-21 | Cu | 2 | E48 | -4.79 | β |
| MC-22 | Fe | 3 | E48 | -5.05 | β |
| MC-23 | Mn | 2 | E48 | -3.45 | β |
| MC-24 | Zn | 2 | E48 | -3.27 | |
| MC-70 | Mn | 3 | E48 | -5.13 | α |
| MC-79 | Ru | 3 | E48 | -4.90 | β |
| MC-88 | In | 3 | E48 | -3.32 | |
| MC-97 | Al | 3 | E48 | -3.45 | |
| MC-106 | Ce | 4 | E48 | -5.09 | |
| 6MC1 | Cu | 2 | B1 | -4.40 | |
| 6MC2 | Cu | 2 | B9 | -4.73 | |
| 6MC3 | Cu | 2 | B14 | -4.92 | |
| 6MC4 | Cu | 2 | B71 | -4.25 | |
| 6MC5 | Cu | 2 | B17 | -5.06 | |
| 6MC6 | Cu | 2 | B30 | -4.50 | |
| 6MC7 | Cu | 2 | B33 | -4.82 | |
| 6MC8 | Cu | 2 | B83 | -4.18 | |
| 6MC9 | Cu | 2 | B84 | -4.53 | |
| 6MC10 | Cu | 2 | B103 | -4.47 | |
| 6MC11 | Fe | 2 | B1 | -3.08 | |
| 6MC12 | Fe | 2 | B9 | -3.62 | |
| 6MC13 | Fe | 2 | B14 | -3.96 | |
| 6MC14 | Fe | 2 | B71 | -3.47 | |
| 6MC15 | Fe | 2 | B17 | -4.20 | |
| 6MC16 | Fe | 2 | B30 | -3.44 | |
| 6MC17 | Fe | 2 | B33 | -3.79 | |
| 6MC18 | Fe | 2 | B83 | -3.05 | |
| 6MC19 | Fe | 2 | B84 | -3.38 | |
| 6MC20 | Fe | 2 | B103 | -3.63 | |
| 6MC21 | Fe | 3 | B9 | -5.16 | |
| 6MC22 | Fe | 3 | B14 | -5.35 | |
| 6MC23 | Fe | 3 | B71 | -4.62 | |
| 6MC24 | Fe | 3 | B17 | -5.54 | |
| 6MC25 | Fe | 3 | B30 | -4.86 | |
| 6MC26 | Fe | 3 | B33 | -5.23 | |
| 6MC27 | Fe | 3 | B83 | -4.55 | |
| 6MC28 | Fe | 3 | B84 | -4.96 | |
| 6MC29 | Fe | 3 | B103 | -4.90 | |
| 6MC30 | Fe | 3 | B109 | -4.48 | |
| 6MC31 | Fe | 3 | B110 | -5.54 | |
| 6MC32 | Fe | 3 | B111 | -4.52 | |
| 6MC33 | Fe | 3 | B112 | -4.58 | |
| 6MC34 | Fe | 3 | B113 | -4.65 | |
| 6MC35 | Fe | 2 | B5 | -3.64 | |
| 6MC36 | Fe | 3 | B5 | -5.30 | |
| 6MC37 | Cu | 2 | B5 | -4.85 | |
| 6MC38 | In | 3 | B1 | -3.23 | |
| 6MC39 | Cu | 2 | B114 | -4.04 | β |
| 6MC40 | Cu | 2 | B115 | -4.31 | β |
| 6MC41 | Cu | 2 | B116 | -4.15 | β |
| 6MC42 | Cu | 2 | B117 | -4.41 | β |
| 6MC43 | Mn | 2 | B115 | -3.44 | β |
| 6MC44 | Mn | 2 | B117 | -3.56 | β |
| 6MC45 | Zn | 2 | B114 | -2.99 | |
| 6MC46 | Zn | 2 | B115 | -3.26 | |
| 6MC47 | Fe | 3 | B114 | -4.39 | β |
| 6MC48 | Fe | 3 | B115 | -4.70 | β |
| MC-25 | Cu | 2 | E116 | -4.23 | β |
| MC-26 | Fe | 3 | E116 | -4.56 | β |
| MC-27 | Mn | 2 | E116 | -3.31 | β |
| MC-28 | Zn | 2 | E116 | -3.10 | |
| MC-72 | Mn | 3 | E116 | -4.60 | α |
| MC-81 | Ru | 3 | E116 | -4.39 | β |
| MC-90 | In | 3 | E116 | -3.17 | |
| MC-99 | Al | 3 | E116 | -3.22 | |
| MC-108 | Ce | 4 | E116 | -4.49 | |
| MC-115 | Mn | 2 | E116-D | -3.31 | β |
| MC-116 | Fe | 3 | E116-D | -4.56 | β |
| MC-117 | Cu | 2 | E116-D | -4.23 | β |
| MC-118 | Zn | 2 | E116-D | -3.10 | β |
| MC-29 | Cu | 2 | E117 | -4.40 | β |
| MC-30 | Fe | 3 | E117 | -4.75 | β |
| MC-31 | Mn | 2 | E117 | -3.51 | β |
| MC-32 | Zn | 2 | E117 | -3.30 | |
| MC-33 | Cu | 2 | E118 | -4.51 | β |
| MC-34 | Fe | 3 | E118 | -4.88 | β |
| MC-35 | Mn | 2 | E118 | -3.60 | β |
| MC-36 | Zn | 2 | E118 | -3.40 | |
| 7MC1 | Cu | 2 | C1 | -4.62 | |
| 7MC2 | Cu | 2 | C2 | -4.63 | |
| 7MC3 | Cu | 2 | C3 | -4.17 | |
| 7MC4 | Cu | 2 | C4 | -4.51 | |
| 7MC5 | Cu | 2 | C5 | -4.49 | |
| 7MC6 | Cu | 2 | C6 | -4.45 | |
| 7MC7 | Cu | 2 | C7 | -4.69 | |
| 7MC8 | Cu | 2 | C8 | -4.78 | |
| 7MC9 | Cu | 2 | C9 | -4.67 | |
| 7MC10 | Cu | 2 | C10 | -4.19 | |
| 7MC11 | Cu | 2 | C11 | -4.33 | |
| 7MC12 | Cu | 2 | C12 | -4.51 | |
| 7MC13 | Cu | 2 | C13 | -4.38 | |
| 7MC14 | Cu | 2 | C14 | -4.71 | |
| 7MC15 | Cu | 2 | C15 | -4.79 | |
| 7MC16 | Cu | 2 | C16 | -4.27 | |
| 7MC17 | Cu | 2 | C17 | -4.32 | |
| 7MC18 | Cu | 2 | C18 | -5.08 | |
| 7MC19 | Cu | 2 | C19 | -5.10 | |
| 7MC20 | Cu | 2 | C20 | -4.90 | |
| 7MC21 | Cu | 2 | C21 | -5.02 | |
| 7MC22 | Cu | 2 | C22 | -4.61 | |
| 7MC23 | Cu | 2 | C23 | -4.71 | |
| 7MC24 | Cu | 2 | C24 | -5.08 | |
| 7MC25 | Cu | 2 | C25 | -4.73 | |
| 7MC26 | Cu | 2 | C26 | -4.25 | |
| 7MC27 | Cu | 2 | C27 | -4.48 | |
| 7MC28 | Fe | 3 | C1 | -5.06 | |
| 7MC29 | Fe | 3 | C2 | -5.08 | |
| 7MC30 | Fe | 3 | C3 | -4.53 | |
| 7MC31 | Fe | 3 | C4 | -4.89 | |
| 7MC32 | Fe | 3 | C5 | -4.93 | |
| 7MC33 | Fe | 3 | C6 | -4.90 | |
| 7MC34 | Fe | 3 | C7 | -5.09 | |
| 7MC35 | Fe | 3 | C8 | -5.19 | |
| 7MC36 | Fe | 3 | C9 | -5.15 | |
| 7MC37 | Fe | 3 | C10 | -4.59 | |
| 7MC38 | Fe | 3 | C11 | -4.76 | |
| 7MC39 | Fe | 3 | C12 | -4.95 | |
| 7MC40 | Fe | 3 | C13 | -4.80 | |
| 7MC41 | Fe | 3 | C14 | -5.20 | |
| 7MC42 | Fe | 3 | C15 | -5.22 | |
| 7MC43 | Fe | 3 | C16 | -4.66 | |
| 7MC44 | Fe | 3 | C17 | -4.76 | |
| 7MC45 | Fe | 3 | C18 | -5.58 | |
| 7MC46 | Fe | 3 | C19 | -5.67 | |
| 7MC47 | Fe | 3 | C20 | -5.36 | |
| 7MC48 | Fe | 3 | C21 | -5.54 | |
| 7MC49 | Fe | 3 | C22 | -5.06 | |
| 7MC50 | Fe | 3 | C23 | -5.20 | |
| 7MC51 | Fe | 3 | C24 | -5.57 | |
| 7MC52 | Fe | 3 | C25 | -5.20 | |
| 7MC53 | Fe | 3 | C26 | -4.57 | |
| 7MC54 | Fe | 3 | C27 | -4.92 | |
| 7MC55 | Ce | 4 | C1 | -3.63 | |
| 7MC56 | Ce | 4 | C13 | -3.50 | |
| 7MC57 | In | 3 | C1 | -3.71 | |
| 7MC58 | In | 3 | C13 | -3.57 | |
| 7MC59 | Al | 3 | C1 | -4.94 | |
| 7MC60 | Al | 3 | C13 | -4.68 | |
| 7MC61 | Co | 3 | C1 | -5.70 | |
| 7MC62 | Co | 3 | C13 | -5.45 | |
| 7MC63 | Mn | 3 | C1 | -4.19 | |
| 7MC64 | Mn | 3 | C13 | -3.95 | |
| 7MC65 | Mn | 2 | C13 | -3.76 | |
| 7MC66 | Ru | 3 | C1 | -4.99 | |
| 7MC67 | Ru | 3 | C13 | -4.70 | |
| 7MC68 | Cr | 3 | C1 | -4.01 | |
| 7MC69 | Cr | 3 | C13 | -3.82 | |
| 7MC70 | Cu | 2 | C28 | -4.25 | β |
| 7MC71 | Cu | 2 | C29 | -4.50 | β |
| 7MC72 | Cu | 2 | C30 | -4.36 | β |
| 7MC73 | Cu | 2 | C31 | -4.59 | β |
| 7MC74 | Mn | 2 | C29 | -3.72 | β |
| 7MC75 | Mn | 2 | C31 | -3.85 | β |
| 7MC76 | Zn | 2 | C28 | -3.26 | |
| 7MC77 | Zn | 2 | C29 | -3.50 | |
| 7MC78 | Fe | 3 | C30 | -4.51 | β |
| 7MC79 | Fe | 3 | C31 | -4.82 | β |
| MC-37 | Cu | 2 | E141 | -4.49 | β |
| MC-38 | Fe | 3 | E141 | -4.88 | β |
| MC-39 | Mn | 2 | E141 | -3.64 | β |
| MC-40 | Zn | 2 | E141 | -3.35 | |
| MC-73 | Mn | 3 | E141 | -4.90 | α |
| MC-82 | Ru | 3 | E141 | -4.57 | β |
| MC-91 | In | 3 | E141 | -3.51 | |
| MC-100 | Al | 3 | E141 | -3.55 | |
| MC-109 | Ce | 4 | E141 | -4.95 | |
| MC-111 | Mn | 2 | E141-D | -3.64 | β |
| MC-112 | Fe | 3 | E141-D | -4.88 | β |
| MC-113 | Cu | 2 | E141-D | -4.49 | β |
| MC-114 | Zn | 2 | E141-D | -3.43 | β |
| MC-41 | Cu | 2 | E142 | -4.67 | β |
| MC-42 | Fe | 3 | E142 | -5.97 | β |
| MC-43 | Mn | 2 | E142 | -3.79 | β |
| MC-44 | Zn | 2 | E142 | -3.62 | |
| MC-45 | Cu | 2 | E143 | -4.78 | β |
| MC-46 | Fe | 3 | E143 | -5.21 | β |
| MC-47 | Mn | 2 | E143 | -3.93 | β |
| MC-48 | Zn | 2 | E143 | -3.68 | |
| 8MC-1 | Cu | 2 | D1 | -4.33 | β |
| 8MC-2 | Cu | 2 | D2 | -4.52 | β |
| 8MC-3 | Cu | 2 | D3 | -4.74 | β |
| 8MC-4 | Cu | 2 | D4 | -4.10 | β |
| 8MC-5 | Cu | 2 | D5 | -4.40 | β |
| 8MC-6 | Cu | 2 | D6 | -4.61 | β |
| 8MC-7 | Cu | 2 | D7 | -4.83 | β |
| 8MC-8 | Cu | 2 | D8 | -4.29 | β |
| 8MC-9 | Cu | 2 | D9 | -4.60 | β |
| 8MC-10 | Cu | 2 | D10 | -4.83 | β |
| 8MC-11 | Cu | 2 | D11 | -5.01 | β |
| 8MC-12 | Cu | 2 | D12 | -4.35 | β |
| 8MC-13 | Cu | 2 | D13 | -4.67 | β |
| 8MC-14 | Cu | 2 | D14 | -4.88 | β |
| 8MC-15 | Cu | 2 | D16 | -4.25 | β |
| 8MC-16 | Cu | 2 | D17 | -4.54 | β |
| 8MC-17 | Cu | 2 | D18 | -4.77 | β |
| 8MC-18 | Cu | 2 | D19 | -4.98 | β |
| 8MC-19 | Cu | 2 | D20 | -4.48 | β |
| 8MC-20 | Cu | 2 | D21 | -4.76 | β |
| 8MC-21 | Cu | 2 | D22 | -4.97 | β |
| 8MC-22 | Cu | 2 | D23 | -5.22 | β |
| 8MC-23 | Cu | 2 | D24 | -4.79 | β |
| 8MC-24 | Cu | 2 | D25 | -5.05 | β |
| 8MC-25 | Cu | 2 | D26 | -5.29 | β |
| 8MC-26 | Cu | 2 | D27 | -5.52 | β |
| 8MC-27 | Cu | 2 | D28 | -4.19 | β |
| 8MC-28 | Cu | 2 | D29 | -4.49 | β |
| 8MC-29 | Cu | 2 | D30 | -4.73 | β |
| 8MC-30 | Cu | 2 | D31 | -4.93 | β |
| 8MC-31 | Cu | 2 | D32 | -4.37 | β |
| 8MC-32 | Cu | 2 | D33 | -4.67 | β |
| 8MC-33 | Cu | 2 | D34 | -4.90 | β |
| 8MC-34 | Cu | 2 | D35 | -5.10 | β |
| 8MC-35 | Cu | 2 | D36 | -4.15 | β |
| 8MC-36 | Cu | 2 | D37 | -4.44 | β |
| 8MC-37 | Cu | 2 | D38 | -4.67 | β |
| 8MC-38 | Cu | 2 | D39 | -4.89 | β |
| 8MC-39 | Cu | 2 | D40 | -4.29 | β |
| 8MC-40 | Cu | 2 | D41 | -4.58 | β |
| 8MC-41 | Cu | 2 | D42 | -4.82 | β |
| 8MC-42 | Cu | 2 | D43 | -5.00 | β |
| 8MC-43 | Cu | 2 | D44 | -4.46 | β |
| 8MC-44 | Cu | 2 | D45 | -4.74 | β |
| 8MC-45 | Cu | 2 | D46 | -5.00 | β |
| 8MC-46 | Cu | 2 | D47 | -5.19 | β |
| 8MC-47 | Cu | 2 | D48 | -4.71 | β |
| 8MC-48 | Cu | 2 | D49 | -4.99 | β |
| 8MC-49 | Cu | 2 | D50 | -5.22 | β |
| 8MC-50 | Cu | 2 | D51 | -5.39 | β |
| 8MC-51 | Cu | 2 | D52 | -4.62 | β |
| 8MC-52 | Cu | 2 | D53 | -4.89 | β |
| 8MC-53 | Cu | 2 | D54 | -5.14 | β |
| 8MC-54 | Cu | 2 | D55 | -5.33 | β |
| 8MC-55 | Cu | 2 | D56 | -4.52 | β |
| 8MC-56 | Cu | 2 | D57 | -5.00 | β |
| 8MC-57 | Cu | 2 | D58 | -5.42 | β |
| 8MC-58 | Cu | 2 | D59 | -4.00 | β |
| 8MC-59 | Cu | 2 | D60 | -4.18 | β |
| 8MC-60 | Cu | 2 | D61 | -4.34 | β |
| 8MC-61 | Cu | 2 | D62 | -4.37 | β |
| 8MC-62 | Cu | 2 | D63 | -4.66 | β |
| 8MC-63 | Cu | 2 | D64 | -4.85 | β |
| 8MC-64 | Cu | 2 | D65 | -5.01 | β |
| 8MC-65 | Cu | 2 | D66 | -4.30 | β |
| 8MC-66 | Cu | 2 | D67 | -4.55 | β |
| 8MC-67 | Cu | 2 | D68 | -4.75 | β |
| 8MC-68 | Cu | 2 | D69 | -4.91 | β |
| 8MC-69 | Cu | 2 | D70 | -4.63 | β |
| 8MC-70 | Cu | 2 | D71 | -4.93 | β |
| 8MC-71 | Cu | 2 | D72 | -5.09 | β |
| 8MC-72 | Cu | 2 | D73 | -5.25 | β |
| 8MC-73 | Cu | 2 | D74 | -4.29 | β |
| 8MC-74 | Cu | 2 | D75 | -4.57 | β |
| 8MC-75 | Cu | 2 | D76 | -4.76 | β |
| 8MC-76 | Cu | 2 | D77 | -4.92 | β |
| 8MC-77 | Zn | 2 | D1 | -2.29 | |
| 8MC-78 | Zn | 2 | D2 | -2.59 | |
| 8MC-79 | Zn | 2 | D3 | -2.73 | |
| 8MC-80 | Zn | 2 | D4 | -2.01 | |
| 8MC-81 | Zn | 2 | D5 | -2.36 | |
| 8MC-82 | Zn | 2 | D6 | -2.67 | |
| 8MC-83 | Zn | 2 | D7 | -2.81 | |
| 8MC-84 | Zn | 2 | D8 | -2.41 | |
| 8MC-85 | Zn | 2 | D9 | -2.61 | |
| 8MC-86 | Zn | 2 | D10 | -2.98 | |
| 8MC-87 | Zn | 2 | D11 | -3.11 | |
| 8MC-88 | Zn | 2 | D12 | -2.46 | |
| 8MC-89 | Zn | 2 | D13 | -2.73 | |
| 8MC-90 | Zn | 2 | D14 | -3.03 | |
| 8MC-91 | Zn | 2 | D16 | -2.27 | |
| 8MC-92 | Zn | 2 | D17 | -2.60 | |
| 8MC-93 | Zn | 2 | D18 | -2.89 | |
| 8MC-94 | Zn | 2 | D19 | -3.00 | |
| 8MC-95 | Zn | 2 | D20 | -2.59 | |
| 8MC-96 | Zn | 2 | D21 | -2.87 | |
| 8MC-97 | Zn | 2 | D22 | -3.14 | |
| 8MC-98 | Zn | 2 | D23 | -3.27 | |
| 8MC-99 | Zn | 2 | D24 | -3.09 | |
| 8MC-100 | Zn | 2 | D25 | -3.22 | |
| 8MC-101 | Zn | 2 | D26 | -3.59 | |
| 8MC-102 | Zn | 2 | D27 | -3.67 | |
| 8MC-103 | Zn | 2 | D28 | -2.18 | |
| 8MC-104 | Zn | 2 | D29 | -2.50 | |
| 8MC-105 | Zn | 2 | D30 | -2.81 | |
| 8MC-106 | Zn | 2 | D31 | -2.93 | |
| 8MC-107 | Zn | 2 | D32 | -2.41 | |
| 8MC-108 | Zn | 2 | D33 | -2.63 | |
| 8MC-109 | Zn | 2 | D34 | -3.01 | |
| 8MC-110 | Zn | 2 | D35 | -3.13 | |
| 8MC-111 | Zn | 2 | D36 | -2.14 | |
| 8MC-112 | Zn | 2 | D37 | -2.50 | |
| 8MC-113 | Zn | 2 | D38 | -2.76 | |
| 8MC-114 | Zn | 2 | D39 | -2.89 | |
| 8MC-115 | Zn | 2 | D40 | -2.33 | |
| 8MC-116 | Zn | 2 | D41 | -2.69 | |
| 8MC-117 | Zn | 2 | D42 | -2.94 | |
| 8MC-118 | Zn | 2 | D43 | -3.05 | |
| 8MC-119 | Zn | 2 | D44 | -2.69 | |
| 8MC-120 | Zn | 2 | D45 | -2.90 | |
| 8MC-121 | Zn | 2 | D46 | -3.19 | |
| 8MC-122 | Zn | 2 | D47 | -3.28 | |
| 8MC-123 | Zn | 2 | D48 | -3.21 | |
| 8MC-124 | Zn | 2 | D49 | -3.37 | |
| 8MC-125 | Zn | 2 | D50 | -3.62 | |
| 8MC-126 | Zn | 2 | D51 | -3.69 | |
| 8MC-127 | Zn | 2 | D52 | -2.88 | |
| 8MC-128 | Zn | 2 | D53 | -3.20 | |
| 8MC-129 | Zn | 2 | D54 | -3.41 | |
| 8MC-130 | Zn | 2 | D55 | -3.51 | |
| 8MC-131 | Zn | 2 | D56 | -1.66 | |
| 8MC-132 | Zn | 2 | D57 | -2.38 | |
| 8MC-133 | Zn | 2 | D58 | -3.03 | |
| 8MC-134 | Zn | 2 | D59 | -3.37 | |
| 8MC-135 | Zn | 2 | D60 | -2.21 | |
| 8MC-136 | Zn | 2 | D61 | -2.52 | |
| 8MC-137 | Zn | 2 | D62 | -2.68 | |
| 8MC-138 | Zn | 2 | D63 | -2.80 | |
| 8MC-139 | Zn | 2 | D64 | -2.55 | |
| 8MC-140 | Zn | 2 | D65 | -2.87 | |
| 8MC-141 | Zn | 2 | D66 | -3.06 | |
| 8MC-142 | Zn | 2 | D67 | -3.19 | |
| 8MC-143 | Zn | 2 | D68 | -2.45 | |
| 8MC-144 | Zn | 2 | D69 | -2.77 | |
| 8MC-145 | Zn | 2 | D70 | -2.95 | |
| 8MC-146 | Zn | 2 | D71 | -3.09 | |
| 8MC-147 | Zn | 2 | D72 | -2.85 | |
| 8MC-148 | Zn | 2 | D73 | -3.16 | |
| 8MC-149 | Zn | 2 | D74 | -3.34 | |
| 8MC-150 | Zn | 2 | D75 | -3.48 | |
| 8MC-151 | Zn | 2 | D76 | -2.92 | |
| 8MC-152 | Zn | 2 | D77 | -3.20 | |
| 8MC-153 | Mn | 2 | D1 | -3.35 | β |
| 8MC-154 | Mn | 2 | D2 | -3.48 | β |
| 8MC-155 | Mn | 2 | D3 | -3.12 | β |
| 8MC-156 | Mn | 2 | D4 | -2.40 | β |
| 8MC-157 | Mn | 2 | D5 | -2.73 | β |
| 8MC-158 | Mn | 2 | D6 | -3.03 | β |
| 8MC-159 | Mn | 2 | D7 | -3.21 | β |
| 8MC-160 | Mn | 2 | D8 | -2.66 | β |
| 8MC-161 | Mn | 2 | D9 | -2.88 | β |
| 8MC-162 | Mn | 2 | D10 | -3.28 | β |
| 8MC-163 | Mn | 2 | D11 | -3.44 | β |
| 8MC-164 | Mn | 2 | D12 | -2.72 | β |
| 8MC-165 | Mn | 2 | D13 | -2.93 | β |
| 8MC-166 | Mn | 2 | D14 | -3.34 | β |
| 8MC-167 | Mn | 2 | D16 | -2.58 | β |
| 8MC-168 | Mn | 2 | D17 | -2.94 | β |
| 8MC-169 | Mn | 2 | D18 | -3.21 | β |
| 8MC-170 | Mn | 2 | D19 | -3.37 | β |
| 8MC-171 | Mn | 2 | D20 | -2.84 | β |
| 8MC-172 | Mn | 2 | D21 | -3.12 | β |
| 8MC-173 | Mn | 2 | D22 | -3.45 | β |
| 8MC-174 | Mn | 2 | D23 | -3.62 | β |
| 8MC-175 | Mn | 2 | D24 | -3.22 | β |
| 8MC-176 | Mn | 2 | D25 | -3.44 | β |
| 8MC-177 | Mn | 2 | D26 | -3.80 | β |
| 8MC-178 | Mn | 2 | D27 | -3.95 | β |
| 8MC-179 | Mn | 2 | D28 | -2.52 | β |
| 8MC-180 | Mn | 2 | D29 | -2.76 | β |
| 8MC-181 | Mn | 2 | D30 | -3.15 | β |
| 8MC-182 | Mn | 2 | D31 | -3.31 | β |
| 8MC-183 | Mn | 2 | D32 | -2.71 | β |
| 8MC-184 | Mn | 2 | D33 | -2.95 | β |
| 8MC-185 | Mn | 2 | D34 | -3.33 | β |
| 8MC-186 | Mn | 2 | D35 | -3.49 | β |
| 8MC-187 | Mn | 2 | D36 | -2.47 | β |
| 8MC-188 | Mn | 2 | D37 | -2.71 | β |
| 8MC-189 | Mn | 2 | D38 | -3.10 | β |
| 8MC-190 | Mn | 2 | D39 | -3.27 | β |
| 8MC-191 | Mn | 2 | D40 | -2.63 | β |
| 8MC-192 | Mn | 2 | D41 | -2.95 | β |
| 8MC-193 | Mn | 2 | D42 | -3.26 | β |
| 8MC-194 | Mn | 2 | D43 | -3.41 | β |
| 8MC-195 | Mn | 2 | D44 | -2.86 | β |
| 8MC-196 | Mn | 2 | D45 | -3.12 | β |
| 8MC-197 | Mn | 2 | D46 | -3.44 | β |
| 8MC-198 | Mn | 2 | D47 | -3.59 | β |
| 8MC-199 | Mn | 2 | D48 | -3.27 | β |
| 8MC-200 | Mn | 2 | D49 | -3.45 | β |
| 8MC-201 | Mn | 2 | D50 | -3.77 | β |
| 8MC-202 | Mn | 2 | D51 | -3.90 | β |
| 8MC-203 | Mn | 2 | D52 | -3.05 | β |
| 8MC-204 | Mn | 2 | D53 | -3.28 | β |
| 8MC-205 | Mn | 2 | D54 | -3.64 | β |
| 8MC-206 | Mn | 2 | D55 | -3.80 | β |
| 8MC-207 | Mn | 2 | D56 | -2.83 | β |
| 8MC-208 | Mn | 2 | D57 | -3.42 | β |
| 8MC-209 | Mn | 2 | D58 | -3.79 | β |
| 8MC-210 | Mn | 2 | D59 | -2.78 | β |
| 8MC-211 | Mn | 2 | D60 | -2.94 | β |
| 8MC-212 | Mn | 2 | D61 | -3.08 | β |
| 8MC-213 | Mn | 2 | D62 | -2.88 | β |
| 8MC-214 | Mn | 2 | D63 | -3.19 | β |
| 8MC-215 | Mn | 2 | D64 | -3.38 | β |
| 8MC-216 | Mn | 2 | D65 | -3.52 | β |
| 8MC-217 | Mn | 2 | D66 | -2.78 | β |
| 8MC-218 | Mn | 2 | D67 | -3.09 | β |
| 8MC-219 | Mn | 2 | D68 | -3.27 | β |
| 8MC-220 | Mn | 2 | D69 | -3.42 | β |
| 8MC-221 | Mn | 2 | D70 | -3.15 | β |
| 8MC-222 | Mn | 2 | D71 | -3.45 | β |
| 8MC-223 | Mn | 2 | D72 | -3.64 | β |
| 8MC-224 | Mn | 2 | D73 | -3.78 | β |
| 8MC-225 | Mn | 2 | D74 | -3.15 | β |
| 8MC-226 | Mn | 2 | D75 | -3.42 | β |
| 8MC-227 | Mn | 2 | D76 | -3.58 | β |
| 8MC-228 | Mn | 2 | D77 | -3.71 | β |
| 8MC-229 | Fe | 3 | D1 | -4.19 | β |
| 8MC-230 | Fe | 3 | D2 | -4.47 | β |
| 8MC-231 | Fe | 3 | D3 | -4.73 | β |
| 8MC-232 | Fe | 3 | D4 | -4.07 | β |
| 8MC-233 | Fe | 3 | D5 | -4.35 | β |
| 8MC-234 | Fe | 3 | D6 | -4.60 | β |
| 8MC-235 | Fe | 3 | D7 | -4.85 | β |
| 8MC-236 | Fe | 3 | D8 | -4.33 | β |
| 8MC-237 | Fe | 3 | D9 | -4.58 | β |
| 8MC-238 | Fe | 3 | D10 | -4.85 | β |
| 8MC-239 | Fe | 3 | D11 | -5.10 | β |
| 8MC-240 | Fe | 3 | D12 | -4.42 | β |
| 8MC-241 | Fe | 3 | D13 | -4.69 | β |
| 8MC-242 | Fe | 3 | D14 | -4.98 | β |
| 8MC-243 | Fe | 3 | D16 | -4.27 | β |
| 8MC-244 | Fe | 3 | D17 | -4.50 | β |
| 8MC-245 | Fe | 3 | D18 | -4.87 | β |
| 8MC-246 | Fe | 3 | D19 | -4.97 | β |
| 8MC-247 | Fe | 3 | D20 | -4.57 | β |
| 8MC-248 | Fe | 3 | D21 | -4.83 | β |
| 8MC-249 | Fe | 3 | D22 | -5.17 | β |
| 8MC-250 | Fe | 3 | D23 | -5.33 | β |
| 8MC-251 | Fe | 3 | D24 | -4.98 | β |
| 8MC-252 | Fe | 3 | D25 | -5.21 | β |
| 8MC-253 | Fe | 3 | D26 | -5.51 | β |
| 8MC-254 | Fe | 3 | D27 | -5.76 | β |
| 8MC-255 | Fe | 3 | D28 | -4.19 | β |
| 8MC-256 | Fe | 3 | D29 | -4.50 | β |
| 8MC-257 | Fe | 3 | D30 | -4.75 | β |
| 8MC-258 | Fe | 3 | D31 | -4.99 | β |
| 8MC-259 | Fe | 3 | D32 | -4.44 | β |
| 8MC-260 | Fe | 3 | D34 | -4.99 | β |
| 8MC-261 | Fe | 3 | D35 | -5.22 | β |
| 8MC-262 | Fe | 3 | D36 | -4.15 | β |
| 8MC-263 | Fe | 3 | D37 | -4.43 | β |
| 8MC-264 | Fe | 3 | D38 | -4.72 | β |
| 8MC-265 | Fe | 3 | D39 | -4.95 | β |
| 8MC-266 | Fe | 3 | D40 | -4.34 | β |
| 8MC-267 | Fe | 3 | D41 | -4.65 | β |
| 8MC-268 | Fe | 3 | D42 | -4.91 | β |
| 8MC-269 | Fe | 3 | D43 | -5.18 | β |
| 8MC-270 | Fe | 3 | D44 | -4.54 | β |
| 8MC-271 | Fe | 3 | D45 | -4.85 | β |
| 8MC-272 | Fe | 3 | D46 | -5.09 | β |
| 8MC-273 | Fe | 3 | D47 | -5.32 | β |
| 8MC-274 | Fe | 3 | D48 | -4.87 | β |
| 8MC-275 | Fe | 3 | D49 | -5.15 | β |
| 8MC-276 | Fe | 3 | D50 | -5.39 | β |
| 8MC-277 | Fe | 3 | D51 | -5.62 | β |
| 8MC-278 | Fe | 3 | D52 | -4.77 | β |
| 8MC-279 | Fe | 3 | D53 | -5.05 | β |
| 8MC-280 | Fe | 3 | D54 | -5.32 | β |
| 8MC-281 | Fe | 3 | D55 | -5.45 | β |
| 8MC-282 | Fe | 3 | D56 | -4.61 | β |
| 8MC-283 | Fe | 3 | D57 | -5.20 | β |
| 8MC-284 | Fe | 3 | D58 | -5.67 | β |
| 8MC-285 | Fe | 3 | D59 | -4.07 | β |
| 8MC-286 | Fe | 3 | D60 | -4.30 | β |
| 8MC-287 | Fe | 3 | D61 | -4.49 | β |
| 8MC-288 | Fe | 3 | D62 | -4.38 | β |
| 8MC-289 | Fe | 3 | D63 | -4.81 | β |
| 8MC-290 | Fe | 3 | D64 | -5.01 | β |
| 8MC-291 | Fe | 3 | D65 | -5.20 | β |
| 8MC-292 | Fe | 3 | D66 | -4.25 | β |
| 8MC-293 | Fe | 3 | D67 | -4.61 | β |
| 8MC-294 | Fe | 3 | D68 | -4.86 | β |
| 8MC-295 | Fe | 3 | D69 | -5.04 | β |
| 8MC-296 | Fe | 3 | D70 | -4.66 | β |
| 8MC-297 | Fe | 3 | D71 | -5.02 | β |
| 8MC-298 | Fe | 3 | D72 | -5.27 | β |
| 8MC-299 | Fe | 3 | D73 | -5.47 | β |
| 8MC-300 | Fe | 3 | D74 | -4.40 | β |
| 8MC-301 | Fe | 3 | D75 | -4.76 | β |
| 8MC-302 | Fe | 3 | D76 | -4.99 | β |
| 8MC-303 | Fe | 3 | D77 | -5.17 | β |
| 8MC-304 | Al | 3 | D2 | -2.65 | |
| 8MC-305 | Al | 3 | D10 | -3.07 | |
| 8MC-306 | Al | 3 | D11 | -3.25 | |
| 8MC-307 | Al | 3 | D14 | -3.15 | |
| 8MC-308 | Al | 3 | D15 | -3.34 | |
| 8MC-309 | Al | 3 | D22 | -3.32 | |
| 8MC-310 | Al | 3 | D23 | -3.48 | |
| 8MC-311 | Al | 3 | D26 | -3.76 | |
| 8MC-312 | Al | 3 | D27 | -3.93 | |
| 8MC-313 | Al | 3 | D30 | -2.90 | |
| 8MC-314 | Al | 3 | D31 | -3.10 | |
| 8MC-315 | Al | 3 | D46 | -3.31 | |
| 8MC-316 | Al | 3 | D47 | -3.49 | |
| 8MC-317 | Al | 3 | D50 | -3.75 | |
| 8MC-318 | Al | 3 | D51 | -3.88 | |
| 8MC-319 | Al | 3 | D54 | -3.62 | |
| 8MC-320 | Al | 3 | D55 | -3.67 | |
| 8MC-321 | Al | 3 | D65 | -3.40 | |
| 8MC-322 | Al | 3 | D69 | -3.28 | |
| 8MC-323 | Al | 3 | D73 | -3.76 | |
| 8MC-324 | Al | 3 | D77 | -3.69 | |
| 8MC-325 | Al | 3 | D3 | -2.85 | |
| 8MC-326 | In | 3 | D2 | -2.59 | |
| 8MC-327 | In | 3 | D3 | -2.78 | |
| 8MC-328 | In | 3 | D10 | -3.01 | |
| 8MC-329 | In | 3 | D11 | -3.18 | |
| 8MC-330 | In | 3 | D14 | -3.09 | |
| 8MC-331 | In | 3 | D15 | -3.27 | |
| 8MC-332 | In | 3 | D22 | -3.26 | |
| 8MC-333 | In | 3 | D23 | -3.40 | |
| 8MC-334 | In | 3 | D26 | -3.70 | |
| 8MC-335 | In | 3 | D27 | -3.85 | |
| 8MC-336 | In | 3 | D30 | -2.85 | |
| 8MC-337 | In | 3 | D31 | -3.03 | |
| 8MC-338 | In | 3 | D46 | -3.27 | |
| 8MC-339 | In | 3 | D47 | -3.42 | |
| 8MC-340 | In | 3 | D50 | -3.71 | |
| 8MC-341 | In | 3 | D51 | -3.82 | |
| 8MC-342 | In | 3 | D54 | -3.57 | |
| 8MC-343 | In | 3 | D55 | -3.60 | |
| 8MC-344 | In | 3 | D65 | -3.32 | |
| 8MC-345 | In | 3 | D69 | -3.20 | |
| 8MC-346 | In | 3 | D73 | -3.66 | |
| 8MC-347 | In | 3 | D77 | -3.62 | |
| 8MC-348 | Mn | 3 | D2 | -4.62 | α |
| 8MC-349 | Mn | 3 | D3 | -4.87 | α |
| 8MC-350 | Mn | 3 | D10 | -5.02 | α |
| 8MC-351 | Mn | 3 | D11 | -5.28 | α |
| 8MC-352 | Mn | 3 | D14 | -5.11 | α |
| 8MC-353 | Mn | 3 | D15 | -5.37 | α |
| 8MC-354 | Mn | 3 | D22 | -5.29 | α |
| 8MC-355 | Mn | 3 | D23 | -5.50 | α |
| 8MC-356 | Mn | 3 | D26 | -5.65 | α |
| 8MC-357 | Mn | 3 | D27 | -5.92 | α |
| 8MC-358 | Mn | 3 | D30 | -4.87 | α |
| 8MC-359 | Mn | 3 | D31 | -5.13 | α |
| 8MC-360 | Mn | 3 | D46 | -5.20 | α |
| 8MC-361 | Mn | 3 | D47 | -5.46 | α |
| 8MC-362 | Mn | 3 | D50 | -5.53 | α |
| 8MC-363 | Mn | 3 | D51 | -5.79 | α |
| 8MC-364 | Mn | 3 | D54 | -5.52 | α |
| 8MC-365 | Mn | 3 | D55 | -5.63 | α |
| 8MC-366 | Mn | 3 | D65 | -5.37 | α |
| 8MC-367 | Mn | 3 | D69 | -5.23 | α |
| 8MC-368 | Mn | 3 | D73 | -5.66 | α |
| 8MC-369 | Mn | 3 | D77 | -5.33 | α |
| 8MC-370 | Ru | 3 | D2 | -4.33 | β |
| 8MC-371 | Ru | 3 | D3 | -4.54 | β |
| 8MC-372 | Ru | 3 | D10 | -4.70 | β |
| 8MC-373 | Ru | 3 | D11 | -4.92 | β |
| 8MC-374 | Ru | 3 | D14 | -4.80 | β |
| 8MC-375 | Ru | 3 | D15 | -5.02 | β |
| 8MC-376 | Ru | 3 | D22 | -4.95 | β |
| 8MC-377 | Ru | 3 | D23 | -5.14 | β |
| 8MC-378 | Ru | 3 | D26 | -5.34 | β |
| 8MC-379 | Ru | 3 | D27 | -5.56 | β |
| 8MC-380 | Ru | 3 | D30 | -4.57 | β |
| 8MC-381 | Ru | 3 | D31 | -4.76 | β |
| 8MC-382 | Ru | 3 | D46 | -4.90 | β |
| 8MC-383 | Ru | 3 | D47 | -5.10 | β |
| 8MC-384 | Ru | 3 | D50 | -5.17 | β |
| 8MC-385 | Ru | 3 | D51 | -5.42 | β |
| 8MC-386 | Ru | 3 | D54 | -5.18 | β |
| 8MC-387 | Ru | 3 | D55 | -5.26 | β |
| 8MC-388 | Ru | 3 | D65 | -5.11 | β |
| 8MC-389 | Ru | 3 | D69 | -4.99 | β |
| 8MC-390 | Ru | 3 | D73 | -5.40 | β |
| 8MC-391 | Ru | 3 | D77 | -5.08 | β |
| 8MC-392 | Ce | 4 | D10 | -4.66 | |
| 8MC-393 | Ce | 4 | D11 | -4.89 | |
| 8MC-394 | Ce | 4 | D22 | -4.92 | |
| 8MC-395 | Ce | 4 | D23 | -5.16 | |
| 8MC-396 | Ce | 4 | D58 | -5.57 | |
| 8MC-397 | Ce | 4 | D65 | -5.08 | |
| 8MC-398 | Ce | 4 | D77 | -5.14 | |
| 8MC-398 | Ce | 4 | D77 | -4.78 | β |
| 8MC-399 | Cu | 2 | D78 | -4.99 | β |
| 8MC-400 | Cu | 2 | D79 | -5.01 | β |
| 8MC-401 | Cu | 2 | D80 | -5.22 | β |
| 8MC-402 | Cu | 2 | D81 | -3.04 | |
| 8MC-403 | Zn | 2 | D78 | -3.29 | |
| 8MC-404 | Zn | 2 | D79 | -3.36 | |
| 8MC-405 | Zn | 2 | D80 | -3.59 | |
| 8MC-406 | Zn | 2 | D81 | -3.31 | β |
| 8MC-407 | Mn | 2 | D78 | -3.55 | β |
| 8MC-408 | Mn | 2 | D79 | -3.58 | β |
| 8MC-409 | Mn | 2 | D80 | -3.82 | β |
| 8MC-410 | Mn | 2 | D81 | -4.83 | β |
| 8MC-411 | Fe | 3 | D78 | -5.06 | β |
| 8MC-412 | Fe | 3 | D79 | -5.19 | β |
| 8MC-413 | Fe | 3 | D80 | -5.97 | β |
| 8MC-414 | Fe | 3 | D81 | -5.97 | β |
| 8MC-415 | Cu | 2 | D82 | -4.52 | β |
| 8MC-416 | Cu | 2 | D83 | -4.64 | β |
| 8MC-416 | Zn | 2 | D82 | -3.13 | |
| 8MC-417 | Zn | 2 | D83 | -3.32 | |
| MC-49 | Cu | 2 | D2 | -4.52 | β |
| MC-50 | Fe | 3 | D2 | -4.47 | β |
| MC-51 | Mn | 2 | D2 | -2.95 | β |
| MC-52 | Zn | 2 | D2 | -2.59 | |
| MC-74 | Mn | 3 | D2 | -4.62 | α |
| MC-83 | Ru | 3 | D2 | -4.33 | β |
| MC-92 | In | 3 | D2 | -2.59 | |
| MC-101 | Al | 3 | D2 | -2.65 | |
| MC-110 | Ce | 4 | D2 | -4.22 | |
| MC-119 | Mn | 2 | E166 | -2.95 | β |
| MC-120 | Fe | 3 | E166 | -4.49 | β |
| MC-121 | Cu | 2 | E166 | -4.53 | β |
| MC-122 | Zn | 2 | E166 | -2.59 | β |
| MC-53 | Cu | 2 | D6 | -4.61 | β |
| MC-54 | Fe | 3 | D6 | -4.6 | β |
| MC-55 | Mn | 2 | D6 | -3.03 | β |
| MC-56 | Zn | 2 | D6 | -2.67 | |
| MC-57 | Cu | 2 | E167 | -4.83 | β |
| MC-58 | Fe | 3 | E167 | -4.85 | β |
| MC-59 | Mn | 2 | E167 | -3.28 | β |
| MC-60 | Zn | 2 | E167 | -2.98 | |
| MC-61 | Cu | 2 | D14 | -4.88 | β |
| MC-62 | Fe | 3 | D14( | -4.98 | β |
| MC-63 | Mn | 2 | D14 | -3.34 | β |
| MC-64 | Zn | 2 | D14 | -3.03 | |

Table 2 (shown below) shows the setup and experimental results for several comparative and inventive devices. The dark current of the organic photodetector (OPD), i.e. the current density of the OPD refers to a state when no light (dark conditions) is applied on the OPD, and the illumination of the OPD, i.e. the current of the OPD refers to a state when light is applied on the OPD:
Comparative device 1 (comparative example 1) comprises RbI in the hole extraction layer.
Comparative device 2 (comparative example 2) comprises a metal sulfonamide compound instead of RbI in the hole extraction layer.
Inventive device 1 (inventive example 1) comprises a compound of formula (I), namely 5MC6 instead of RbI in the hole extraction layer.
Inventive device 2 (inventive example 2) comprises a compound of formula (I), namely 5MC57 instead of RbI in the hole extraction layer.
Inventive device 3 (inventive example 3) comprises a compound of formula (I), namely MC-9 instead of RbI in the hole extraction layer.
Inventive device 4 (inventive example 4) comprises a compound of formula (I), namely 6MC-3 8 instead of RbI in the hole extraction layer.
Inventive device 5 (inventive example 5) comprises a compound of formula (I), namely 7MC40 instead of RbI in the hole extraction layer.

All inventive devices (inventive examples 1 to 5) exhibit a lower dark current and a higher signal (photocurrent) to noise (dark current) ratio (SNR) than the comparative devices (Comparative examples 1 and 2),

**Table 2: Setup and experimental results for several comparative and inventive devices**

| **Type** | **Structure** | **Current density @ -3V-Voltage [mA/cm²]** | | | | | |
|---|---|---|---|---|---|---|---|
| | | Dark current of the OPD | Rel. to ref. (Dark current) | Illumination of the OPD | Rel. to ref. (illumination) | SNR | SNR(%) |
| Comp. Ex. | RbI | -1.7E-04 | 100% | -8.6E-03 | 100% | 52 | 100% |
| Comp. Ex. | 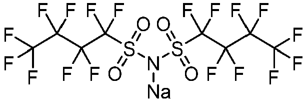 | -1.5E-04 | 91% | -1.9E-02 | 222% | 127 | 244% |
| Inv. Ex. 1 | 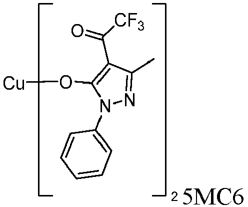 | -3.8E-06 | 2% | -2.4E-02 | 282% | 6423 | 12294% |
| Inv. Ex. 2 | 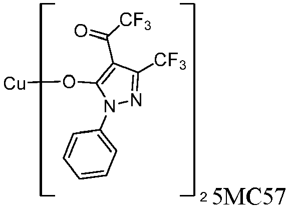 | -4.4E-06 | 3% | -2.3E-02 | 268% | 5269 | 10087% |
| Inv. Ex. 3 | 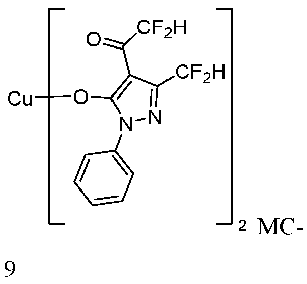 | -3.2E-06 | 2% | -1.9E-02 | 224% | 6013 | 11509% |
| Inv. Ex. 4 | 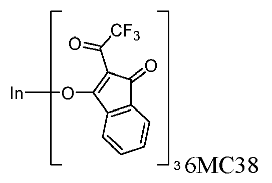 | -1.5E-06 | 1% | -8.3E-03 | 96% | 5663 | 10839% |
| Inv. Ex. 5 | 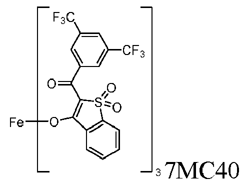 | -3.5E-06 | 2% | -7.0E-03 | 82% | 2016 | 3859% |

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. The invention's scope is defined in the following claims and the equivalents thereto. Furthermore, reference signs used in the description and claims do not limit the scope of the invention as claimed.

## Claims

1. A organic photodetector comprising
an anode layer, a cathode layer, a hole transport region, a photoconversion unit, a semiconductor layer,
wherein the hole transport region, the photoconversion unit, and the semiconductor layer are arranged between the anode layer and the cathode layer;
wherein the hole transport region comprises one or more layer;
wherein the hole transport region is arranged between the anode layer and the photoconversion unit;
wherein the semiconductor layer is arranged between the hole transport region and photoconversion unit;
wherein the semiconductor layer is arranged adjacent or in direct contact to the photoconversion unit;
wherein the photoconversion unit comprises one or more layers;
wherein the photoconversion unit comprises an electron donor compound and electron acceptor compound;
wherein the semiconductor layer or the photoconversion unit comprises a metal complex; compound of Formula (I):
M^{n⊕}(L^{⊝})ₙ(AL)ₘ (I),
wherein:
M is a metal ion,
n is the valency of M and selected from 1 to 4;
L is a ligand independently selected from formula (II) 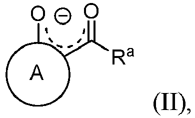 wherein
A is selected from a substituted or unsubstituted C₃ to C₄₀ carbocyclic ring or ring system, substituted or unsubstituted C₂ to C₄₀ heterocyclic ring or ring system, substituted or unsubstituted C₆ to C₄₀ aryl ring or ring system, or substituted or unsubstituted C₂ to C₄₀ heteroaryl ring or ring system, wherein the carbocyclic ring or the heterocyclic ring may contain one or more double bonds, and wherein the ring system may comprise two or three rings, preferably two rings;
R^{a} is selected from CN, substituted or unsubstituted C₁ to C₂₀ alkyl, CH₃, CH₂D, CHD₂, CD₃, a partially fluorinated or perfluorinated C₁ to C₂₀ alkyl, CFH₂, CFDH, CFD₂, CF₂H, CF₂D, CF₃, substituted or unsubstituted C₁ to C₂₀ alkenyl, substituted or unsubstituted C₁ to C₂₀ alkinyl, substituted or unsubstituted C₃ to C₄₀ cycloalkyl, substituted or unsubstituted C₂ to C₄₀ heterocycloalkyl, substituted or unsubstituted C₃ to C₄₀ carbocyclyl ring, substituted or unsubstituted C₂ to C₄₀ heterocyclyl, substituted or unsubstituted C₂ to C₄₀ heteroaryl, or substituted or unsubstituted C₆ to C₄₀ aryl;
AL is an ancillary ligand which coordinates to the metal ion M;
m is an integer selected from 0 to 2.

2. The organic photodetector of the claim 1, wherein L is a ligand of formula (IIa): 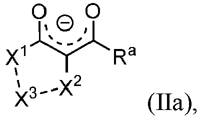 wherein the dashed lines in formula (IIa) denote a single bond or a double bond;
X¹ is selected from a direct bond, N, O, NR¹, CR¹;
X² is selected from N, O, CR², CO, SO₂;
X³ is selected from N, O, CR³, NR³ or substituted or unsubstituted C₁ to C₃ alkylenediyl group;
R¹ is selected from substituted or unsubstituted C₁ to C₁₂ alkyl, substituted or unsubstituted C₆ to C₁₉ aryl or C₂ to C₂₀ heteroaryl group;
R² is selected from H, D, CN, substituted or unsubstituted C₁ to C₁₂ alkyl, substituted or unsubstituted C₃ to C₅ alkenyl, substituted or unsubstituted C₆ to C₁₉ aryl or C₂ to C₂₀ heteroaryl group;
R³ is selected from H, D, substituted or unsubstituted C₁ to C₁₂ alkyl, substituted or unsubstituted C₃ to C₅ alkenyl, substituted or unsubstituted C₆ to C₁₉ aryl or C₂ to C₂₀ heteroaryl group;
wherein two of X¹, X³, R¹ and R³ may form a 5 to 7 membered ring;
R^{a} is selected from CN, substituted or unsubstituted C₁ to C₂₀ alkyl, CH₃, CH₂D, CHD₂, CD₃, a partially fluorinated or perfluorinated C₁ to C₂₀ alkyl, CFH₂, CFDH, CFD₂, CF₂H, CF₂D, CF₃, substituted or unsubstituted C₁ to C₂₀ alkenyl, substituted or unsubstituted C₁ to C₂₀ alkinyl, substituted or unsubstituted C₃ to C₄₀ cycloalkyl, substituted or unsubstituted C₂ to C₄₀ heterocycloalkyl, substituted or unsubstituted C₃ to C₄₀ carbocyclyl ring, substituted or unsubstituted C₂ to C₄₀ heterocyclyl, substituted or unsubstituted C₂ to C₄₀ heteroaryl, or substituted or unsubstituted C₆ to C₄₀ aryl.

3. The organic photodetector of claim 1 or 2, wherein L is a ligand of formulae (IIb) to (IIe): 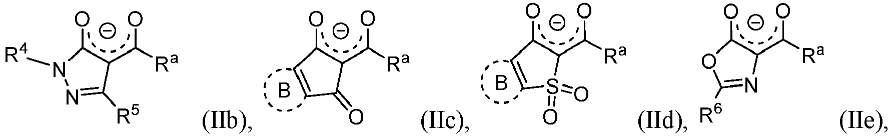 wherein
R⁴ is selected from unsubstituted and substituted C₁ to C₁₂ alkyl, substituted or unsubstituted C₆ to C₁₉ aryl, substituted or unsubstituted C₂ to C₂₀ heteroaryl, substituted or unsubstituted 6-membered heteroaryl, preferably R² is selected from substituted or unsubstituted C₆ to C₁₉ aryl, substituted or unsubstituted C₂ to C₂₀ heteroaryl, substituted or unsubstituted 6-membered heteroaryl;
R⁵ is selected from substituted or unsubstituted C₁ to C₁₂ alkyl, substituted or unsubstituted C₆ to C₁₉ aryl, substituted or unsubstituted C₂ to C₂₀ heteroaryl, substituted or unsubstituted 6-membered heteroaryl, or CN;
R⁶ is selected from unsubstituted and substituted C₁ to C₁₂ alkyl, substituted or unsubstituted C₆ to C₁₉ aryl, substituted or unsubstituted C₂ to C₂₀ heteroaryl, substituted or unsubstituted 6-membered heteroaryl;
B is selected from substituted or unsubstituted C₆ to C₁₉ aryl, substituted or unsubstituted C₂ to C₂₀ heteroaryl ring or a ring system, wherein the ring system may comprise one or two rings, preferably one ring;
R^{a} is selected from CN, substituted or unsubstituted C₁ to C₂₀ alkyl, CH₃, CH₂D, CHD₂, CD₃, a partially fluorinated or perfluorinated C₁ to C₂₀ alkyl, CFH₂, CFDH, CFD₂, CF₂H, CF₂D, CF₃, substituted or unsubstituted C₁ to C₂₀ alkenyl, substituted or unsubstituted C₁ to C₂₀ alkinyl, substituted or unsubstituted C₃ to C₄₀ cycloalkyl, substituted or unsubstituted C₂ to C₄₀ heterocycloalkyl, substituted or unsubstituted C₃ to C₄₀ carbocyclyl ring, substituted or unsubstituted C₂ to C₄₀ heterocyclyl, substituted or unsubstituted C₂ to C₄₀ heteroaryl, or substituted or unsubstituted C₆ to C₄₀ aryl.

4. The organic photodetector of any of the claims 1 to 3 wherein L is selected from formulae (IIf) or (IIg): 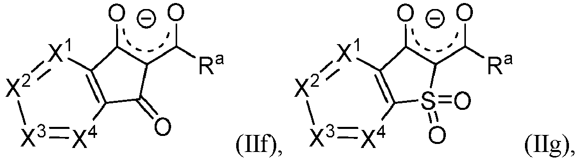 wherein
X¹ is selected from CR²¹ or N;
X² is selected from CR²² or N;
X³ is selected from CR²³ or N;
X⁴ is selected from CR²⁴ or N;
wherein 0, 1 or 2 of the group consisting of X¹, X², X³, X⁴ are selected from N;
R²¹ to R²⁴ are independently selected from H, D, halogen, Cl, F, CN, NO₂, C₁ to C₁₂ alkyl, C₁ to C₁₂ alkoxy, partially or perfluorinated C₁ to C₁₂ alkyl, CF₃, CF₂R, CFR₂, CF₂H, CFH₂, partially or perfluorinated C₁ to C₁₂ alkoxy, or a combination thereof, wherein R is H or D, preferably H; and whereby any R^{k} to R^{k+1} may form a ring, wherein k is an integer from 1 to 3;
R^{a} is selected from CN, substituted or unsubstituted C₁ to C₂₀ alkyl, CH₃, CH₂D, CHD₂, CD₃, a partially fluorinated or perfluorinated C₁ to C₂₀ alkyl, CFH₂, CFDH, CFD₂, CF₂H, CF₂D, CF₃, substituted or unsubstituted C₁ to C₂₀ alkenyl, substituted or unsubstituted C₁ to C₂₀ alkinyl, substituted or unsubstituted C₃ to C₄₀ cycloalkyl, substituted or unsubstituted C₂ to C₄₀ heterocycloalkyl, substituted or unsubstituted C₃ to C₄₀ carbocyclyl ring, substituted or unsubstituted C₂ to C₄₀ heterocyclyl, substituted or unsubstituted C₂ to C₄₀ heteroaryl, or substituted or unsubstituted C₆ to C₄₀ aryl.

5. The organic photodetector of any of the claims 1 to 4, whereby one or more substituents of A are independently selected from D, electron-withdrawing group, =O, =S, CN, halogen, Cl, F, substituted or unsubstituted C₁ to C₂₀ alkyl, CH₃, CH₂D, CHD₂, CD₃, a partially fluorinated or perfluorinated C₁ to C₂₀ alkyl, CFH₂, CFDH, CFD₂, CF₂H, CF₂D, CF₃, substituted or unsubstituted C₁ to C₂₀ alkenyl, substituted or unsubstituted C₁ to C₂₀ alkinyl, substituted or unsubstituted C₃ to C₄₀ cycloalkyl, substituted or unsubstituted C₂ to C₄₀ heterocycloalkyl, substituted or unsubstituted C₃ to C₄₀ carbocyclyl ring, or substituted or unsubstituted C₂ to C₄₀ heterocyclyl, substituted or unsubstituted C₂ to C₄₀ heteroaryl, or substituted or unsubstituted C₆ to C₄₀ aryl.

6. The organic photodetector of any of the claims 1 to 5, whereby one or more substituents of A are independently selected from D, electron-withdrawing group, =O, =S, CN, halogen, Cl, F, substituted or unsubstituted C₁ to C₁₂ alkyl, CH₃, CH₂D, CHD₂, CD₃, a partially fluorinated or perfluorinated C₁ to C₁₂ alkyl, CFH₂, CFDH, CFD₂, CF₂H, CF₂D, CF₃, substituted or unsubstituted C₁ to C₁₂ alkenyl, substituted or unsubstituted C₁ to C₁₂ alkinyl, substituted or unsubstituted C₅ to C₁₉ cycloalkyl, substituted or unsubstituted C₂ to C₂₀ heterocycloalkyl, substituted or unsubstituted C₅ to C₁₉ carbocyclyl ring, or substituted or unsubstituted C₂ to C₂₀ heterocyclyl, substituted or unsubstituted C₂ to C₂₀ heteroaryl, or substituted or unsubstituted C₆ to C₁₉ aryl.

7. The organic photodetector of any of the claims 1 to 6, wherein one or more substituents of A are independently selected from D, electron-withdrawing group, =O, =S, CN, halogen, Cl, F, substituted or unsubstituted C₁ to C₂₀ alkyl, CH₃, CH₂D, CHD₂, CD₃, a partially fluorinated or perfluorinated C₁ to C₂₀ alkyl, CFH₂, CFDH, CFD₂, CF₂H, CF₂D, CF₃, substituted or unsubstituted C₁ to C₂₀ alkenyl, substituted or unsubstituted C₁ to C₂₀ alkinyl, substituted or unsubstituted C₃ to C₄₀ cycloalkyl, substituted or unsubstituted C₂ to C₄₀ heterocycloalkyl, substituted or unsubstituted C₃ to C₄₀ carbocyclyl ring, substituted or unsubstituted C₂ to C₄₀ heterocyclyl, substituted or unsubstituted C₂ to C₄₀ heteroaryl, or substituted or unsubstituted C₆ to C₄₀ aryl;
and wherein the one or more substituents on R^{a} are independently selected from an D, electron-withdrawing group, halogen, Cl, F, CN, partially or perfluorinated C₁ to C₈ alkyl, CH₃, CH₂D, CHD₂, CD₃, CFH₂, CFDH, CFD₂, CF₂H, CF₂D, CF₃, partially or perfluorinated C₁ to C₈ alkoxy, OCH₃, OCH₂D, OCHD₂, OCD₃, OCFH₂, OCFDH, OCFD₂, OCF₂H, OCF₂D, OCF₃.

8. The organic photodetector of any of the claims 1 to 7, whereby if n>1 then each L is selected the same.

9. The organic photodetector of any of the claims 1 to 8, wherein the semiconductor layer is in direct contact to the photoconversion unit.

10. The organic photodetector of any of the claims 1 to 9, wherein the semiconductor layer comprises the metal complex of formula (I).

11. An organic electronic device comprising
a substrate, an organic photodetector, an organic light-emitting device;
wherein the organic photodetector and the organic light-emitting device is disposed on the substrate;
wherein the organic photodetector is an organic photodetector according to any of the claims 1 to 9,
wherein the organic light-emitting device comprises
an anode layer, a cathode layer, a hole transport region, a light-emitting layer a cathode layer;
wherein the hole transport region and the light-emitting layer are arranged between the anode layer and the cathode layer;
wherein the hole transport region is arranged between the anode layer and the organic light-emitting layer;

12. The organic electronic device of Claim 11, wherein the cathode layer of the organic light-emitting device and the cathode layer of the organic photodetector is a common cathode layer shared by at least one of the organic light-emitting device and at least one of the organic photodetector.

13. The organic electronic device according to Claim 11 or 12 whereby the organic electronic device is a display device.

14. Use of a metal complex of Formula (I) according to any one of the claims 1 to 8 for photo detection.

15. Use of a metal complex of Formula (I) according to any one of the claims 1 to 8 as hole extraction material.
